(19) **Europäisches Patentamt / European Patent Office / Office européen des brevets**

(11) **EP 2 177 908 A2**

(12) **EUROPEAN PATENT APPLICATION**

(43) Date of publication:
21.04.2010 Bulletin 2010/16

(51) Int Cl.:
*G01N 33/53* (2006.01)  *G01N 33/567* (2006.01)

(21) Application number: 09010506.5

(22) Date of filing: 11.10.2006

(84) Designated Contracting States:
AT BE BG CH CY CZ DE DK EE ES FI FR GB GR HU IE IS IT LI LT LU LV MC NL PL PT RO SE SI SK TR
Designated Extension States:
AL BA HR MK RS

(30) Priority: 11.10.2005 US 725462 P

(62) Document number(s) of the earlier application(s) in accordance with Art. 76 EPC:
06816821.0 / 1 949 105

(71) Applicant: **Tethys Bioscience, Inc.**
**Emeryville, CA 94608 (US)**

(72) Inventors:
• **Urdea, Mickey**
**Emeryville, CA 94608 (US)**
• **McKenna, Michael**
**Emeryville, CA 94608 (US)**
• **Arensdorf, Patrick**
**Palo Alto, CA 94303 (US)**

(74) Representative: **Marshall, Cameron John et al**
**Carpmaels & Ransford**
**43-45 Bloomsbury Square**
**London WC1A 2RA (GB)**

Remarks:
This application was filed on 14-08-2009 as a divisional application to the application mentioned under INID code 62.

(54) **Diabetes-associated markers and methods of use thereof**

(57) Disclosed are methods of identifying subjects with Diabetes or a pre-diabetic condition, methods of identifying subjects at risk for developing Diabetes or a pre-diabetic condition, methods of differentially diagnosing diseases associated with Diabetes or a pre- diabetic condition from other diseases or within sub-classifications of Diabetes, methods of evaluating the risk of progression to Diabetes or a pre-diabetic condition in patients, methods of evaluating the effectiveness of treatments in subjects with Diabetes or a pre-diabetic condition, and methods of selecting therapies for treating Diabetes or a pre-diabetic condition, using biomarkers.

FIG. 1

EP 2 177 908 A2

(Cont. next page)

FIG. 1(contd.)

FIG. 1(contd.)

FIG. 1(contd.)

**Description**

**INCORPORATION BY REFERENCE**

This application claims priority from U.S. Provisional Application Serial No. 60/725,462, filed on October 11, 2005.

Each of the applications and patents cited in this text, as well as each document or reference cited in each of the applications and patents (including during the prosecution of each issued patent; "application cited documents"), and each of the U.S. and foreign applications or patents corresponding to and/or claiming priority from any of these applications and patents, and each of the documents cited or referenced in each of the application cited documents, are hereby expressly incorporated herein by reference. More generally, documents or references are cited in this text, either in a Reference List before the claims, or in the text itself; and, each of these documents or references ("herein-cited references"), as well as each document or reference cited in each of the herein-cited references (including any manufacturer's specifications, instructions, etc.), is hereby expressly incorporated herein by reference. Documents incorporated by reference into this text may be employed in the practice of the invention.

**FIELD OF THE INVENTION**

The present invention relates generally to the identification of biological markers associated with an increased risk of developing Diabetes, as well as methods of using such biological markers in diagnosis and prognosis of Diabetes.

**BACKGROUND OF THE INVENTION**

Diabetes Mellitus describes a metabolic disorder characterized by chronic hyperglycemia with disturbances of carbohydrate, fat and protein metabolism that result from defects in insulin secretion, insulin action, or both. The effects of Diabetes Mellitus include long-term damage, dysfunction and failure of various organs. Diabetes may be present with characteristic symptoms such as thirst, polyuria, blurring of vision, chronic infections, slow wound healing, and weight loss. In its most severe forms, ketoacidosis or a non-ketotic hyperosmolar state may develop and lead to stupor, coma and, in the absence of effective treatment, death. Often symptoms are not severe, not recognized, or may be absent. Consequently, hyperglycemia sufficient to cause pathological and functional changes may be present for a long time, occasionally up to ten years, before a diagnosis is made, usually by the detection of high levels of glucose in urine after overnight fasting during a routine medical work-up. The long-term effects of Diabetes Mellitus include progressive development of complications such as retinopathy with potential blindness, nephropathy that may lead to renal failure, neuropathy, microvascular changes, and autonomic dysfunction. People with Diabetes are also at increased risk of cardiovascular, peripheral vascular, and cerebrovascular disease (together, "arteriovascular" disease). There is also an increased risk of cancer. Several pathogenetic processes are involved in the development of Diabetes. These include processes which destroy the insulin-secreting beta cells of the pancreas with consequent insulin deficiency, and changes in liver and smooth muscle cells that result in the resistance to insulin uptake. The abnormalities of carbohydrate, fat and protein metabolism are due to deficient action of insulin on target tissues resulting from insensitivity to insulin or lack of insulin.

Regardless of the underlying cause, Diabetes Mellitus is subdivided into Type 1 Diabetes and Type 2 Diabetes. Type 1 Diabetes results from autoimmune mediated destruction of the beta cells of the pancreas. The rate of destruction is variable, and the rapidly progressive form is commonly observed in children, but may also occur in adults. The slowly progressive form of Type 1 Diabetes generally occurs in adults and is sometimes referred to as latent autoimmune Diabetes in adults (LADA). Some patients, particularly children and adolescents, may exhibit ketoacidosis as the first manifestation of the disease. Others have modest fasting hyperglycemia that can rapidly change to severe hyperglycemia and/or ketoacidosis in the presence of infection or other stress. Still others, particularly adults, may retain residual beta cell function sufficient to prevent ketoacidosis for many years. Individuals with this form of Type 1 Diabetes often become dependent on insulin for survival and are at risk for ketoacidosis. Patients with Type 1 Diabetes exhibit little or no insulin secretion as manifested by low or undetectable levels of plasma C-peptide. However, there are some forms of Type 1 Diabetes which have no known etiology, and some of these patients have permanent insulinopenia and are prone to ketoacidosis, but have no evidence of autoimmunity. These patients are referred to as "Type 1 idiopathic."

Type 2 Diabetes is the most common form of Diabetes and is characterized by disorders of insulin action and insulin secretion, either of which may be the predominant feature. Both are usually present at the time that this form of Diabetes is clinically manifested. Type 2 Diabetes patients are characterized with a relative, rather than absolute, insulin deficiency and are resistant to the action of insulin. At least initially, and often throughout their lifetime, these individuals do not need insulin treatment to survive. Type 2 Diabetes accounts for 90-95% of all cases of Diabetes. This form of Diabetes can go undiagnosed for many years because the hyperglycemia is often not severe enough to provoke noticeable symptoms of Diabetes or symptoms are simply not recognized. The majority of patients with Type 2 Diabetes are obese, and obesity itself may cause or aggravate insulin resistance. Many of those who are not obese by traditional weight criteria may have an increased percentage of body fat distributed predominantly in the abdominal region (visceral fat). Ketoacidosis is infrequent in this type of Diabetes and usually arises in association with the stress of another illness. Whereas patients with this form of Diabetes may have insulin levels that appear normal or elevated, the high blood glucose levels in these diabetic patients would be expected to result in even higher insulin values had their beta cell function been normal. Thus, insulin secretion is often defective and insufficient to compensate for the insulin resistance. On the other hand, some hyperglycemic individuals have essentially normal insulin action, but markedly impaired insulin secretion.

Diabetic hyperglycemia may be decreased by weight reduction, increased physical activity, and/or pharmacological treatment. There are several biological mechanisms that are associated with hyperglycemia such as insulin resistance, insulin secretion, and gluconeogenesis, and there are orally active drugs available that act on one or more of these mechanisms. With lifestyle and/or drug intervention, glucose levels can return to near-normal levels, but this is usually temporary. With time, additional second-tier drugs are often required additions to the treatment approach. Often with time, even these multi-drug approaches fail, at which point insulin injections are instituted.

Over 18 million people in the United States have Type 2 Diabetes, and of these, about 5 million do not know they have the disease. These persons who do not know they have the disease and who do not exhibit the classic symptoms of Diabetes present a major diagnostic and therapeutic challenge.

There is a large group in the United States, nearly 41 million persons, who are at significant risk of developing Type 2 Diabetes. They are broadly referred to in the literature as "pre-diabetics." A "pre-diabetic" or a subject with pre-Diabetes represents any person or population with a greater risk than the broad population for conversion to Type 2 Diabetes in a given period of time. The risk of developing Type 2 Diabetes increases with age, obesity, and lack of physical activity. It occurs more frequently in women with prior gestational Diabetes, and in individuals with hypertension and/or dyslipidemia. Its frequency varies in different ethnic subgroups. Type 2 Diabetes is often associated with strong familial, likely genetic, predisposition, however the genetics of this form of Diabetes are complex and not clearly defined.

Pre-diabetics often have fasting glucose levels between normal and frank diabetic levels. Occasionally in research, these persons are tested for their tolerance to glucose. Abnormal glucose tolerance, or "impaired glucose tolerance" can be an indication that an individual is on the path toward Diabetes; it requires the use of a 2-hour oral glucose tolerance test for its detection. However, it has been shown that impaired glucose tolerance is by itself entirely asymptomatic and unassociated with any functional disability. Indeed, insulin secretion is typically greater in response to a mixed meal than in response to a pure glucose load; as a result, most persons with impaired glucose tolerance are rarely, if ever, hyperglycemic in their daily lives, except when they undergo diagnostic glucose tolerance tests. Thus, the importance of impaired glucose tolerance resides exclusively in its ability to identify persons at increased risk of future disease (Stem et al, 2002). In studies conducted by Stem and others, the sensitivity and false-positive rates of impaired glucose tolerance as a predictor of future conversion to Type 2 Diabetes was 50.9% and 10.2%, respectively, representing an area under the Receiver-Operating Characteristic Curve of 77.5% and a $p$-value of 0.20. Because of its cost, reliability, and inconvenience, the oral glucose tolerance test is seldom used in routine clinical practice. Moreover, patients whose Diabetes is diagnosed solely on the basis of an oral glucose tolerance test have a high rate of reversion to normal on follow-up and may in fact represent false-positive diagnoses. Stem and others reported that such cases were almost 5 times more likely to revert to non-diabetic status after 7 to 8 years of follow-up compared with persons meeting conventional fasting or clinical diagnostic criteria. Clearly, there is a need for improved methods of assessing the risk of future Diabetes.

Often a person with impaired glucose tolerance will be found to have at least one or more of the common arteriovascular disease risk factors. This clustering has been termed "Syndrome X," or "Metabolic Syndrome" by some researchers and can be indicative of a prediabetic state. Alone, each component of the cluster conveys increased arteriovascular and diabetic disease risk, but together as a combination they become much more significant. This means that the management of persons with hyperglycemia and other features of Metabolic Syndrome should focus not only on blood glucose control but also include strategies for reduction of other arteriovascular disease risk factors. Furthermore, such risk factors are non-specific for Diabetes or pre-Diabetes and are not in themselves a basis for a diagnosis of Diabetes, or of diabetic status.

It should furthermore be noted that an increased risk of conversion to Diabetes implies an increased risk of converting to arteriovascular disease and events. Diabetes itself is one of the most significant single risk factors for arteriovascular disease, and is in fact often termed a "coronary heart disease equivalent" by itself, indicating a greater than 20 percent ten-year risk of an arteriovascular event, in a similar range with stable angina and just below the most significant independent risk factors, such as survivorship of a previous arteriovascular event. The same is true of other arteriovascular disease, such as peripheral artery disease or cerebrovascular disease.

It is well documented that pre-Diabetes can be present for ten or more years before the detection of glycemic disorders like Diabetes. Treatment of pre-diabetics with drugs such as acarbose, metformin, troglitazone and rosiglitazone can postpone or prevent Diabetes; yet few pre-diabetics are treated. A major reason, as indicated above, is that no simple laboratory test exists to determine the actual risk of an individual to develop Diabetes. Thus, there remains a need in the art for methods of identifying and diagnosing these individuals who are not yet diabetics, but who are at significant risk of developing Diabetes.

## SUMMARY OF THE INVENTION

The present invention relates in part to the discovery that certain biological markers, such as proteins, nucleic acids, polymorphisms, metabolites, and other analytes, as well as certain physiological conditions and states, are present in subjects with an increased risk of developing Diabetes Mellitus or a pre-diabetic condition such as, but not limited to, Metabolic Syndrome (Syndrome X), conditions characterized by impaired glucose regulation and/or insulin resistance, such as Impaired Glucose Tolerance (IGT) and Impaired Fasting Glycemia (IFG), but where such subjects do not exhibit some or all of the conventional risk factors of these conditions, or subjects who are asymptomatic for these conditions.

Accordingly, the invention provides biological markers of Diabetes or pre-diabetic conditions that can be used to monitor or assess the risk of subjects experiencing such diabetic or pre-diabetic conditions, to diagnose or identify subjects with a diabetic or prediabetic condition, to monitor the risk for development of a diabetic or pre-diabetic condition, to monitor subjects that are undergoing therapies for Diabetes or a pre-diabetic condition, to differentially diagnose disease states associated with Diabetes or a pre-diabetic condition from other diseases, or within sub-classifications of Diabetes or pre-diabetic conditions, to evaluate changes in the risk of Diabetes or pre-diabetic conditions, and to select therapies for use in treating subjects with Diabetes or a pre-diabetic condition, or for use in treating subjects who are at risk for developing Diabetes or a pre-diabetic condition. Preferably, the present invention provides use of biological markers, some of which are unrelated to Diabetes or have not heretofore been identified as related to Diabetes, but are related to early biological changes that can lead to the development of Diabetes or a pre-diabetic condition, to detect and identify subjects who exhibit none of the symptoms for Diabetes, i.e., who are asymptomatic for Diabetes or pre-diabetic conditions or have only non-specific indivators of potential pre-diabetic conditions, such as arteriovascular risk factors, or who exhibit none or few of the conventional risk factor of Diabetes. Significantly, many of the biomarkers disclosed herein have shown little individual significance in the diagnosis of Diabetes, but when used in combination (in "panels") with other disclosed markers and combined with the herein disclosed mathematical classification algorithms, becomes significant discriminates of the pre-Diabetes patient or population from one who is not pre-diabetic.

Accordingly, in one aspect, the present invention provides a method with a predetermined level of predictability for assessing a risk of development of Diabetes Mellitus or a pre-diabetic condition in a subject comprising: measuring the level of an effective amount of one or more, preferably two or more DBRISKMARKERS selected from the group consisting of DBRISKMARKERS 1-260 in a sample from the subject, and measuring a clinically significant alteration in the level of the one or more, preferably two or more DBRISKMARKERS in the sample, wherein the alteration indicates an increased risk of developing Diabetes Mellitus or a pre-diabetic condition in the subject.

In one embodiment, the Diabetes Mellitus comprises Type 1 Diabetes, Type 2 Diabetes, or gestational Diabetes. In other embodiments, the pre-diabetic condition comprises IFG, IGT, Metabolic Syndrome, or Syndrome X.

The level of DBRISKMARKERS can be measured electrophoretically or immunochemically. Where the detection is immunochemical, the detection can be by radioimmunoassay, immunofluorescence assay or by an enzyme-linked immunosorbent assay. The detection can also be achieved by specific oligonucleotide hybridization.

In some embodiments, the subject has not been previously diagnosed or identified as having the Diabetes Mellitus or the pre-diabetic condition. In other embodiments, the subject is asymptomatic for the Diabetes Mellitus or the pre-diabetic condition.

The sample as defined by the present invention can be serum, blood plasma, blood cells, endothelial cells, tissue biopsies, ascites fluid, bone marrow, interstitial fluid, sputum, or urine.

In one embodiment of the present invention, the level of expression of five or more DBRISKMARKERS is measured, but can also encompass measurement of ten or more, twenty-five or more, or fifty or more DBRISKMARKERS.

In another aspect, a method with a predetermined level of predictability for diagnosing or identifying a subject having Diabetes Mellitus or a pre-diabetic condition is provided, comprising measuring the level of an effective amount of one or more, preferably two or more DBRISKMARKERS selected from the group consisting of DBRISKMARKERS 1-260 in a sample from the subject, and comparing the level of the effective amount of the one or more (or two or more) DBRISKMARKERS to a reference value.

In one embodiment, the reference value is an index value. The reference value can also be derived from one or more risk prediction algorithms or computed indices for the Diabetes or pre-diabetic condition.

Another aspect of the present invention provides a method with a predetermined level of predictability for assessing a risk of impaired glucose tolerance in a subject comprising measuring the level of an effective amount of one or more, preferably two or more DBRISKMARKERS selected from the group consisting of DBRISKMARKERS 1-260 in a sample from the subject, and measuring a clinically significant alteration in the level of the one or more (or two or more) DBRISKMARKERS in the sample, wherein the alteration indicates an increased risk of impaired glucose tolerance in the subject.

In one embodiment, the subject has not been previously diagnosed as having impaired glucose tolerance. In another embodiment, the subject is asymptomatic for the impaired glucose tolerance.

In another aspect, a method with a predetermined level of predictability for diagnosing or identifying a subject having impaired glucose tolerance is provided, comprising measuring the level of an effective amount of one or more, preferably two or more DBRISKMARKERS selected from the group consisting of DBRISKMARKERS 1-260 in a sample from the subject, and comparing the level of the effective amount of the one or more (preferably two or more) DBRISKMARKERS to a reference value. The reference value can be an index value.

Alternatively, the reference value can be derived from one or more risk prediction algorithms or computed indices for impaired glucose tolerance.

Another aspect of the invention provides a method with a predetermined level of predictability for assessing the progression of Diabetes Mellitus or a pre-diabetic condition in a subject, comprising detecting the level of an effective amount of one or more, preferably two or more DBRISKMARKERS selected from the group consisting of DBRISKMARKERS 1-260 in a first sample from the subject at a first period of time; detecting the level of an effective amount of one or more, preferably two or more DBRISKMARKERS in a second sample from the subject at a second period of time; and comparing the level of the effective amount of the one or more (or two or more) DBRISKMARKERS detected in step (a) to the amount detected in step (b), or to a reference value.

In one embodiment, the subject has previously been diagnosed or identified as suffering from the Diabetes Mellitus or the pre-diabetic condition. In another embodiment, the subject has previously been treated for the Diabetes Mellitus or the pre-diabetic condition. In yet another embodiment, the subject has not been previously diagnosed or identified as suffering from the Diabetes Mellitus or the pre-diabetic condition. In other embodiments, the subject is asymptomatic for the Diabetes Mellitus or the pre-diabetic condition.

In the context of the invention, the first sample can be taken from the subject prior to being treated for the Diabetes Mellitus or the pre-diabetic condition. The second sample can taken from the subject after being treated for the Diabetes Mellitus or the pre-diabetic condition. The reference value can be derived from one or more subjects who have suffered from Diabetes Mellitus or a pre-diabetic condition.

In another aspect of the present invention, a method with a predetermined level of predictability for assessing the progression of impaired glucose tolerance associated with Diabetes Mellitus or a pre-diabetic condition in a subject is provided, comprising detecting the level of an effective amount of one or more, preferably two or more DBRISKMARKERS selected from the group consisting of DBRISKMARKERS 1-260 in a first sample from the subject at a first period of time; detecting the level of an effective amount of one or more, preferably two or more DBRISKMARKERS in a second sample from the subject at a second period of time; and comparing the level of the effective amount of the one or more (or two or more) DBRISKMARKERS detected in step (a) to the amount detected in step (b), or to a reference value.

The subject can be one who has previously been treated for the Diabetes Mellitus or the pre-diabetic condition. The subject can also be one who has not been previously diagnosed or identified as having impaired glucose tolerance or suffering from the Diabetes Mellitus or the pre-diabetic condition. Alternatively, the subject can be asymptomatic for the impaired glucose tolerance, or is asymptomatic for the Diabetes Mellitus or the pre-diabetic condition.

In yet another aspect, a method with a predetermined level of predictability for monitoring the effectiveness of treatment for Diabetes Mellitus or a pre-diabetic condition is provided, comprising detecting the level of an effective amount of one or more, preferably two or more DBRISKMARKERS selected from the group consisting of DBRISKMARKERS 1-260 in a first sample from the subject at a first period of time; detecting the level of an effective amount of one or more, preferably two or more DBRISKMARKERS in a second sample from the subject at a second period of time; and comparing the level of the effective amount of the one or more (or two or more) DBRISKMARKERS detected in step (a) to the amount detected in step (b), or to a reference value, wherein the effectiveness of treatment is monitored by a change in the level of the effective amount of one or more, preferably two or more DBRISKMARKERS from the subject.

In one embodiment, the treatment for the Diabetes Mellitus or the pre-diabetic condition comprises exercise regimens, dietary supplements, therapeutic agents, surgical intervention, and prophylactic agents. In another embodiment, the reference value is derived from one or more subjects who show an improvement in Diabetes risk factors as a result of one or more treatments for the Diabetes Mellitus or the pre-diabetic condition. The effectiveness of treatment can be additionally monitored by detecting changes in body mass index (BMI), insulin levels, blood glucose levels, HDL levels, systolic and/or diastolic blood pressure, or combinations thereof. Changes in blood glucose levels can be detected by an oral glucose tolerance test.

Another aspect of the present invention provides a method with a predetermined level of predictability for selecting a treatment regimen for a subject diagnosed with or at risk for Diabetes Mellitus or a pre-diabetic condition comprising detecting the level of an effective amount of one or more, preferably two or more DBRISKMARKERS selected from the group consisting of DBRISKMARKERS 1-260 in a first sample from the subject at a first period of time; optionally detecting the level of an effective amount of one or more, preferably two or more DBRISKMARKERS in a second sample from the subject at a second period of time; and comparing the level of the effective amount of the one or more (or two or more) DBRISKMARKERS detected in step (a) to a reference value, or optionally, to the amount detected in step (b).

The present invention also provides a Diabetes Mellitus reference expression profile, comprising a pattern of marker levels of an effective amount of one or more, preferably two or more markers selected from the group consisting of DBRISKMARKERS 1-260, taken from one or more subjects who do not have the Diabetes Mellitus.

An impaired glucose tolerance reference expression profile is also provided by the invention, comprising a pattern of marker levels of an effective amount of one or more, preferably two or more markers selected from the group consisting of DBRISKMARKERS 1-260, taken from one or more subjects who do not have impaired glucose tolerance.

In another aspect, a Diabetes Mellitus subject expression profile is provided, comprising a pattern of marker levels of an effective amount of one or more, preferably two or more markers selected from the group consisting of DBRISKMARKERS 1-260 taken from one or more subjects who have the Diabetes Mellitus, are at risk for developing the Diabetes Mellitus, or are being treated for the Diabetes Mellitus.

In another aspect, an impaired glucose tolerance subject expression profile is provided, comprising a pattern of marker levels of an effective amount of one or more, preferably two or more markers selected from the group consisting of DBRISKMARKERS 1-260 taken from one or more subjects who have impaired glucose tolerance, are at risk for developing impaired glucose tolerance, or are being treated for impaired glucose tolerance.

The present invention also provides a kit comprising a plurality of DBRISKMARKER detection reagents that detect the corresponding DBRISKMARKERS selected from the group consisting of DBRISKMARKERS 1-260, sufficient to generate the profiles of the invention. The detection reagent can comprise one or more antibodies or fragments thereof. Alternatively, or additionally, the detection reagent can comprise one or more oligonucleotides or one or more aptamers.

The present invention also provides, in another aspect, a machine readable media containing one or more Diabetes Mellitus reference expression profiles according to the invention, or one or more Diabetes Mellitus subject expression profiles according to the invention, and optionally, additional test results and subject information.

A machine readable media containing one or more impaired glucose tolerance reference expression profiles according to invention is also contemplated, or one or more impaired glucose tolerance subject expression profiles according to the invention, and optionally, additional test results and subject information.

In another aspect, a DBRISKMARKER panel comprising one or more DBRISKMARKERS that are indicative of a physiological and/or biochemical pathway associated with Diabetes Mellitus or a pre-diabetic condition is provided. In one embodiment, the physiological and biochemical pathways comprise autoimmune regulation, inflammation and endothelial function (including cytokine-cytokine receptor interactions, cell adhesion molecules (CAMs), focal adhesions, leukocyte transendothelial migration, natural killer cell mediated cytotoxicity, regulation of the actin cytoskeleton, adherens/tight/gap junctions, and extracellular matrix (ECM)-receptor interaction), adipocyte development and maintenance (including adipocytokines, cell cycle, apoptosis, and neuroactive ligand-receptor interaction) as well as hematopoietic cell lineage, complement and coagulation cascades, intra- and extracellular cell signaling pathways (including the mTOR, TGF-β, MAPK, insulin, GnRH, Toll-like receptor, Jak-STAT, PPAR, T-cell receptor, B-cell receptor, FcεRI, calcium, Wnt, and VEGF signaling pathways and other cell communication mechanisms), in addition to those pathways that are commonly associated with Type 1 and Type 2 Diabetes Mellitus.

A DBRISKMARKER panel comprising one or more DBRISKMARKERS that are indicative of a site associated with Diabetes Mellitus or a pre-diabetic condition is also provided, wherein the site can comprise beta cells, endothelial cells, skeletal and smooth muscle, or peripheral, cardiovascular, or cerebrovascular arteries.

In other aspects, a DBRISKMARKER panel comprising one or more DBRISKMARKERS that are indicative of the progression of Diabetes Mellitus or a prediabetic condition is provided.

The present invention further provides a DBRISKMARKER panel comprising one or more DBRISKMARKERS that are indicative of the speed of progression of Diabetes Mellitus or a pre-diabetic condition. The invention also concerns a DBRISKMARKER panel comprising one or more DBRISKMARKERS that are specific to one or more types of Diabetes Mellitus and a DBRISKMARKER panel comprising one or more DBRISKMARKERS that are specific to a pre-diabetic condition.

A DBRISKMARKER panel comprising one or more DBRISKMARKERS selected from mathematical classification algorithms and factor analysis approach is provided, utilizing a relevant past cohort of subjects, or calculated indices which were developed in such past cohorts. In particular, a DBRISKMARKER panel of one or more, preferably two or more DBRISKMARKERS selected from a subset of the disclosed DBRISKMARKERS comprising Leptin (LEP), Haptoglobin (HP), Insulin-like growth factor binding protein 3 (ILGFBP3), Resistin (RETN), Matrix Metallopeptidase 2 (MMP-2), Angiotensin I converting enzyme (peptidyl dipeptidase A)-1 (ACE), complement component 4A (Rogers blood group)(C4A), CD14 molecule (CD14), selectin E (endothelial adhesion molecule)(SELE), colony stimulating factor 1 (macrophage) (CSF1), and vascular endothelial growth factor (VEGF), c-reactive protein, pentraxin-related (CRP), Tumor Necrosis Factor Receptor Superfamily Member 1A (TNFRSF1A), RAGE (Advanced Glycosylation End Product-specific Receptor [AGER]), CD26 (dipeptidyl peptidase 4; DPP4), and their statistical and/or functional equivalents within mathematical classification algorithms using one or more of these DBRISKMARKERS.

A method for treating one or more subjects at risk for developing Diabetes Mellitus or a pre-diabetic condition is also contemplated by the present invention, comprising detecting the presence of increased levels of at least one, preferably two different DBRISKMARKERS present in a sample from the one or more subjects; and treating the one or more subjects with one or more Diabetes-modulating drugs until altered levels of the at least one, preferably two different DBRISKMARKERS return to a baseline value measured in one or more subjects at low risk for developing the Diabetes Mellitus or the pre-diabetic condition, or a baseline value measured in one or more subjects who show improvements in Diabetes risk markers as a result of treatment with one or more Diabetes-modulating drugs.

The Diabetes-modulating drugs can comprise sulfonylureas; biguanides; insulin, insulin analogs; peroximsome proliferator-activated receptor-γ (PPAR-γ) agonists; dual-acting PPAR agonists; insulin secretagogues; analogs of glucagon-like peptide-1 (GLP-1); inhibitors of dipeptidyl peptidase IV (DPP4); pancreatic lipase inhibitors; α-glucosidase inhibitors; and combinations thereof. In one embodiment, the improvements in Diabetes risk markers as a result of treatment with one or more Diabetes-modulating drugs comprise a reduction in body mass index (BMI), a reduction in blood glucose levels, an increase in insulin levels, an increase in HDL levels, a reduction in systolic and/or diastolic blood pressure, or combinations thereof.

In another aspect, a method of evaluating changes in the risk of impaired glucose tolerance in a subject diagnosed with or at risk for developing a pre-diabetic condition is provided, comprising detecting the level of an effective amount of one or more, preferably two or more DBRISKMARKERS selected from the group consisting of DBRISKMARICERS 1-260 in a first sample from the subject at a first period of time; optionally detecting the level of an effective amount of one or more, preferably two or more DBRISKMARKERS in a second sample from the subject at a second period of time; and comparing the level of the effective amount of the one or more (or two or more) DBRISKMARKERS detected in step (a) to a reference value, or optionally, the amount in step (b).

The present invention further provides a method of differentially diagnosing disease states associated with Diabetes Mellitus or a pre-diabetic condition in a subject comprising detecting the level of an effective amount of one or more, preferably two or more DBRISKMARKERS selected from the group consisting of DBRISKMARKERS 1-260 in a sample from the subject; and comparing the level of the effective amount of the one or more (or two or more) DBRISKMARKERS detected in step (a) to the Diabetes Mellitus disease subject expression profile of the invention, to the impaired glucose tolerance subject expression profile of the invention, or to a reference value.

Further, in a method of diagnosing or identifying a subject at risk for developing Diabetes or a pre-diabetic condition by analyzing Diabetes risk factors, the present invention provides an improvement comprising measuring the level of an effective amount of one or more, preferably two or more DBRISKMARKERS selected from the group consisting of DBRISKMARKERS 1-260 in a sample from the subject, and measuring a clinically significant alteration in the level of the one or more (or two or more) DBRISKMARKERS in the sample, wherein the alteration indicates an increased risk of developing Diabetes Mellitus or a pre-diabetic condition in the subject.

In yet another aspect of the present invention, in a method of diagnosing or identifying a subject at risk for developing Diabetes or a pre-diabetic condition by analyzing Diabetes risk factors, the present invention provides an improvement comprising: measuring the level of an effective amount of one or more DBRISKMARKERS selected from the group consisting of: Leptin (LEP), Haptoglobin (HP), Insulin-like growth factor binding protein 3 (ILGFBP3), Resistin (RETN), Matrix Metallopeptidase 2 (MMP-2), Angiotensin I converting enzyme (peptidyl dipeptidase A)-1 (ACE), complement component 4A (C4A), CD14 molecule (CD14), selectin E (SELE), colony stimulating factor 1 (macrophage; CSF1), and vascular endothelial growth factor (VEGF), c-reactive protein, pentraxin-related (CRP), Tumor Necrosis Factor Receptor Superfamily Member 1A (TNFRSF1A), RAGE (Advanced Glycosylation End Product-specific Receptor [AGER]), and CD26 (dipeptidyl peptidase 4; DPP4), and measuring a clinically significant alteration in the level of the one or more DBRISKMARKERS in the sample, wherein the alteration indicates an increased risk of developing Diabetes Mellitus or a pre-diabetic condition in the subject.

In a method of diagnosing.or identifying a subject at risk for developing Diabetes or a pre-diabetic condition by analyzing Diabetes risk factors, the present invention provides an improvement comprising: measuring the level of an effective amount of two or more DBRISKMARKERS selected from the group consisting of: Leptin (LEP), Haptoglobin (HP), Insulin-like growth factor binding protein 3 (ILGFBP3), Resistin (RETN), Matrix Metallopeptidase 2 (MMP-2), Angiotensin I converting enzyme (peptidyl dipeptidase A)-1 (ACE), complement component 4A (C4A), CD14 molecule (CD14), selectin E (SELE), colony stimulating factor 1 (macrophage; CSF1), and vascular endothelial growth factor (VEGF), c-reactive protein, pentraxin-related (CRP), Tumor Necrosis Factor Receptor Superfamily Member 1A (TNFRSF1A), RAGE (Advanced Glycosylation End Product-specific Receptor [AGER]), and CD26 (dipeptidyl peptidase 4; DPP4), and measuring a clinically significant alteration in the level of the two or more DBRISKMARKERS in the sample, wherein the alteration indicates an increased risk of developing Diabetes Mellitus or a pre-diabetic condition in the subject.

Unless otherwise defined, all technical and scientific terms used herein have the same meaning as commonly understood by one of ordinary skill in the art to which this invention pertains. Although methods and materials similar or equivalent to those described herein can be used in the practice of the present invention, suitable methods and materials are described below. All publications, patent applications, patents, and other references mentioned herein are expressly incorporated by reference in their entirety. In cases of conflict, the present specification, including definitions, will control. In addition, the materials, methods, and examples described herein are illustrative only and are not intended to be limiting.

Other features and advantages of the invention will be apparent from and are encompassed by the following detailed description and claims.

**BRIEF DESCRIPTION OF THE DRAWINGS**

The following Detailed Description, given by way of example, but not intended to limit the invention to specific embodiments described, may be understood in conjunction with the accompanying Figures, incorporated herein by reference, in which:

Figure 1 is a flow chart depicting DBRISKMARKER physiological and biological pathways and categories in the context of the disease progression from Normal to Pre-Diabetes to Diabetes.

Figure 2 is an illustration depicting classes and desirable characteristics of DBRISKMARKERS, and illustrating several differing illustrative patterns of markers that are useful in the diagnosis of subjects having Pre-Diabetes, and Diabetes as compared to normal.

Figures 3A - 3RR are graphic illustrations of the KEGG pathways highlighting three or more DBRISKMARKERS in each disclosed pathway.

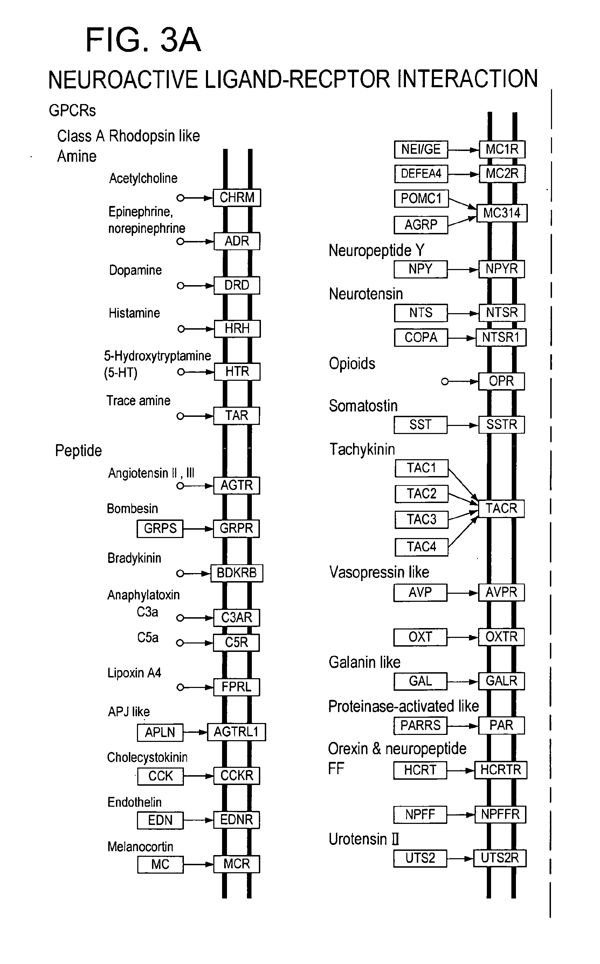

Figure 3A depicts neuroactive ligand-receptor interactions.

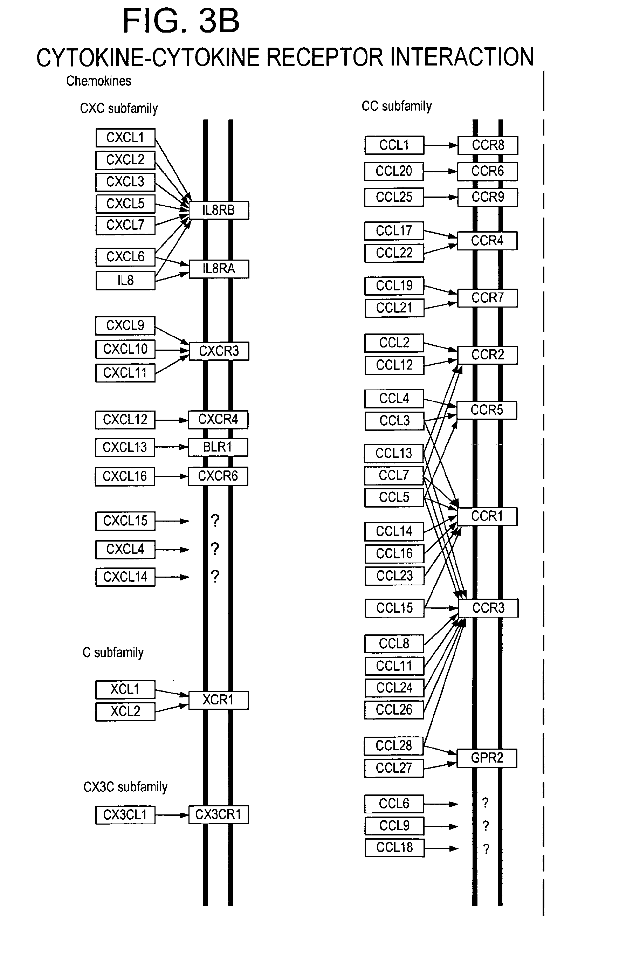

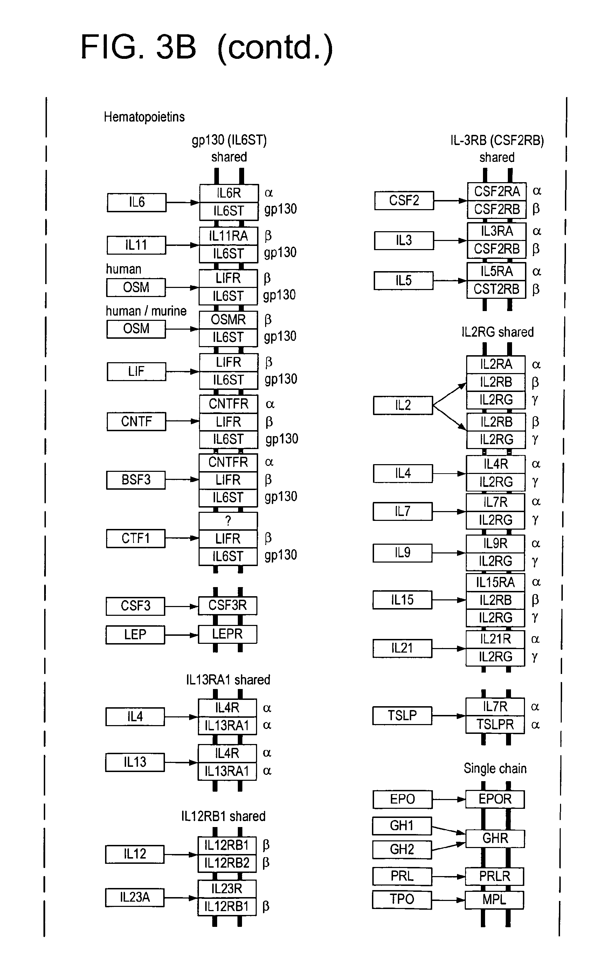

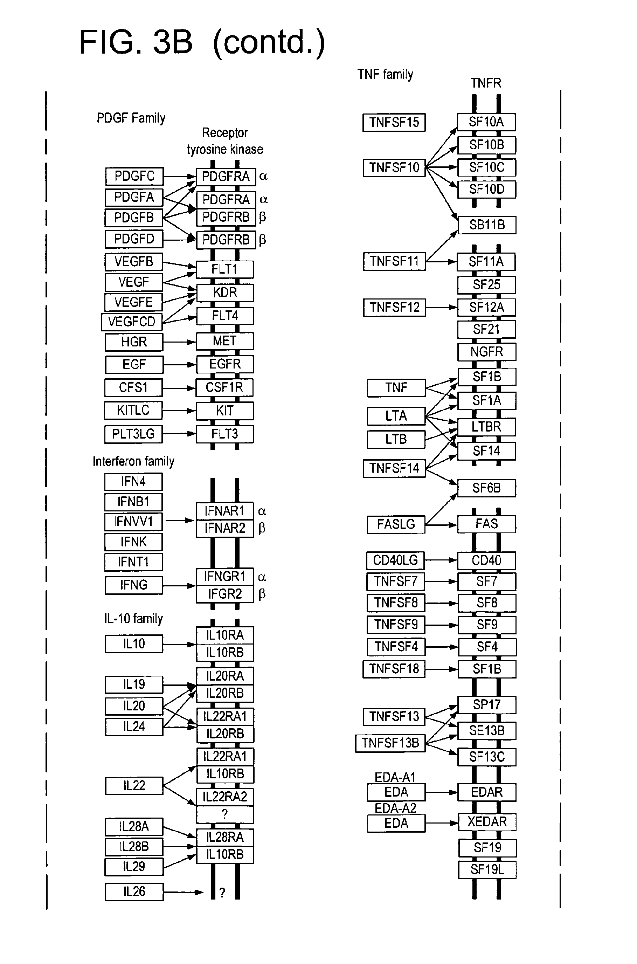

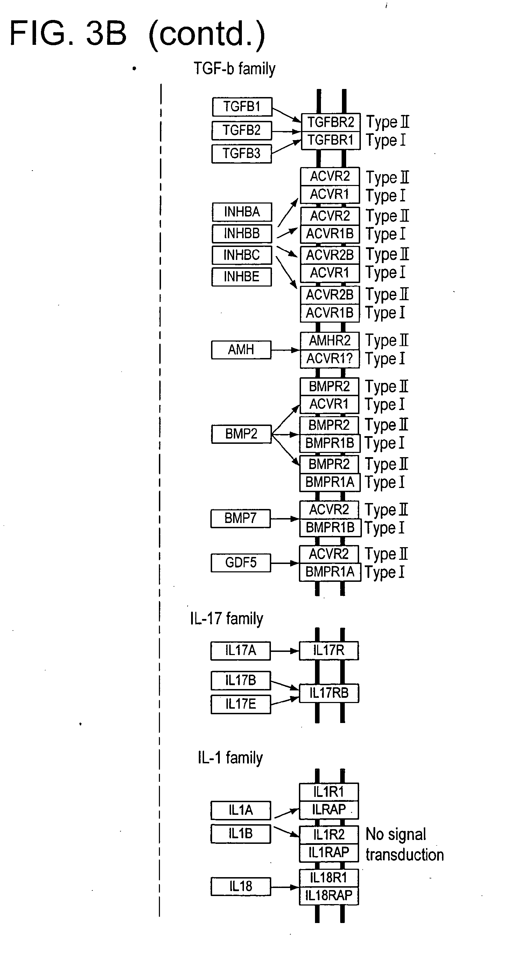

Figure 3B depicts cytokine-cytokine receptor interactions.

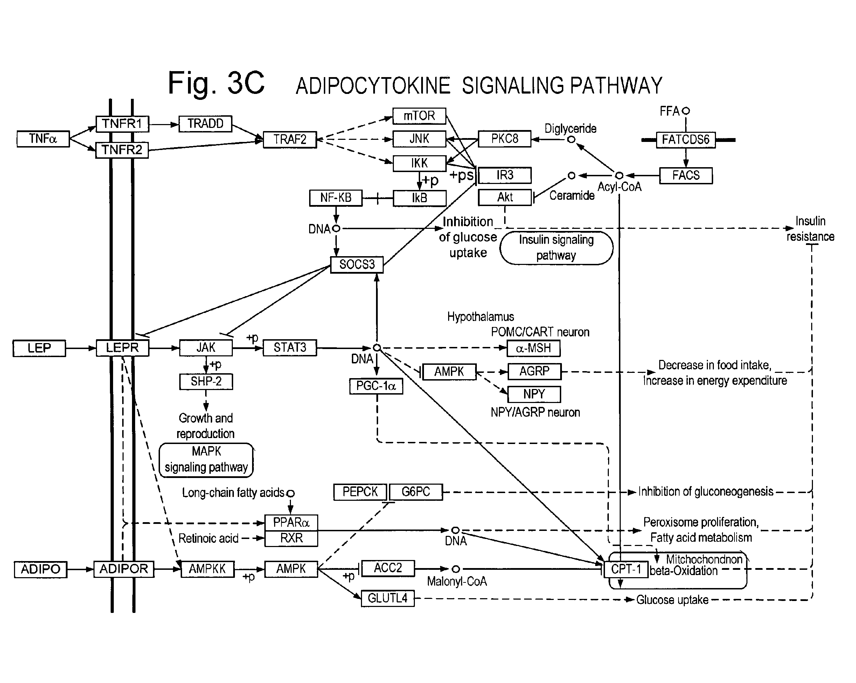

Figure 3C depicts the adipocytokine signaling pathway.

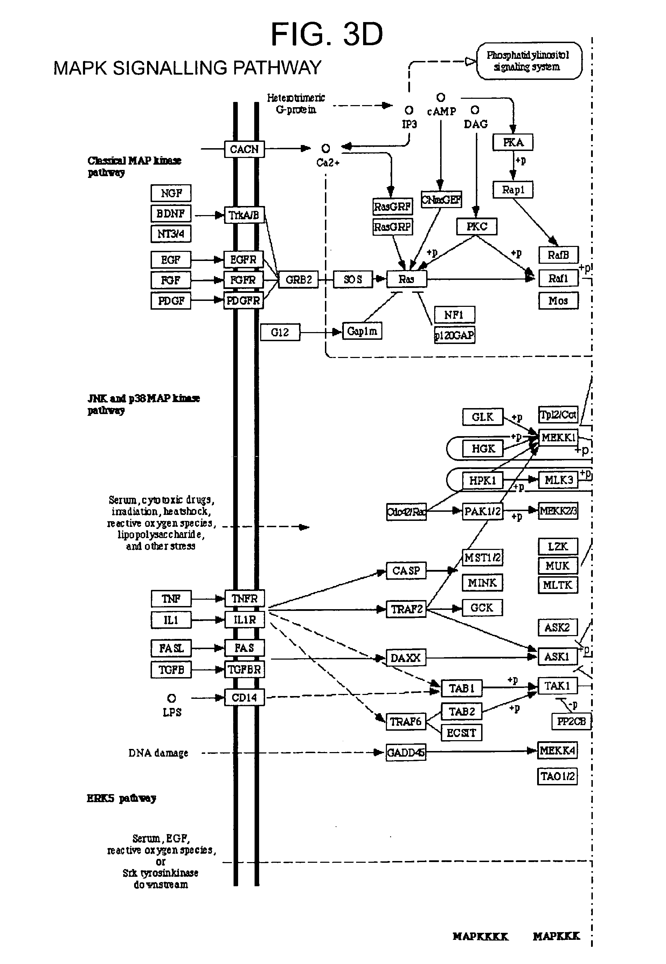

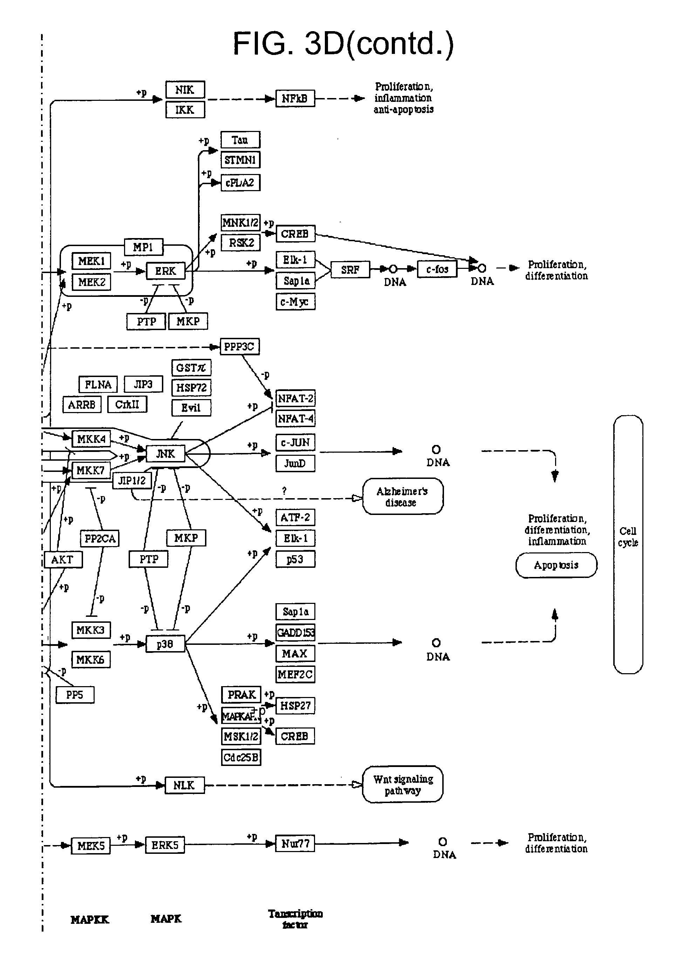

Figure 3D shows the mitogen-activated protein kinase (MAPK) signaling pathway.

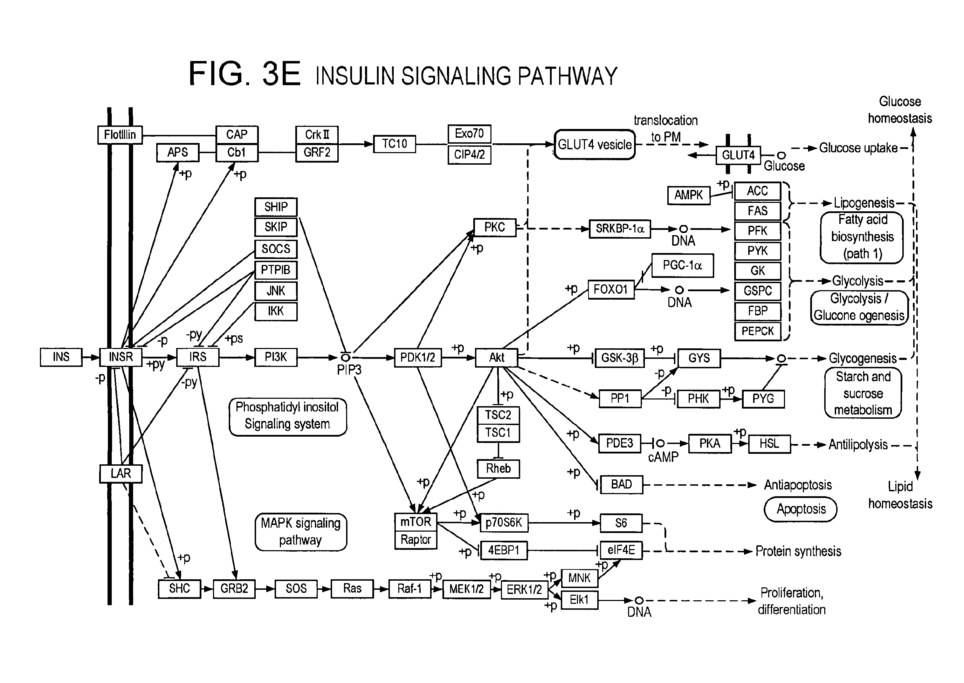

Figure 3E shows the insulin signaling pathway.

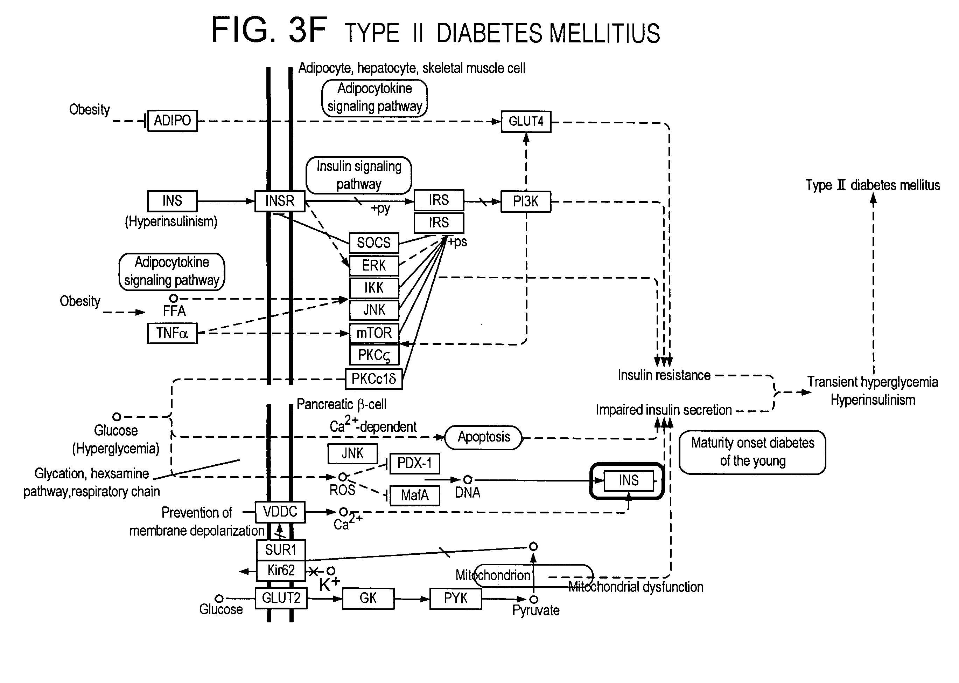

Figure 3F shows the Type II Diabetes Mellitus pathway.

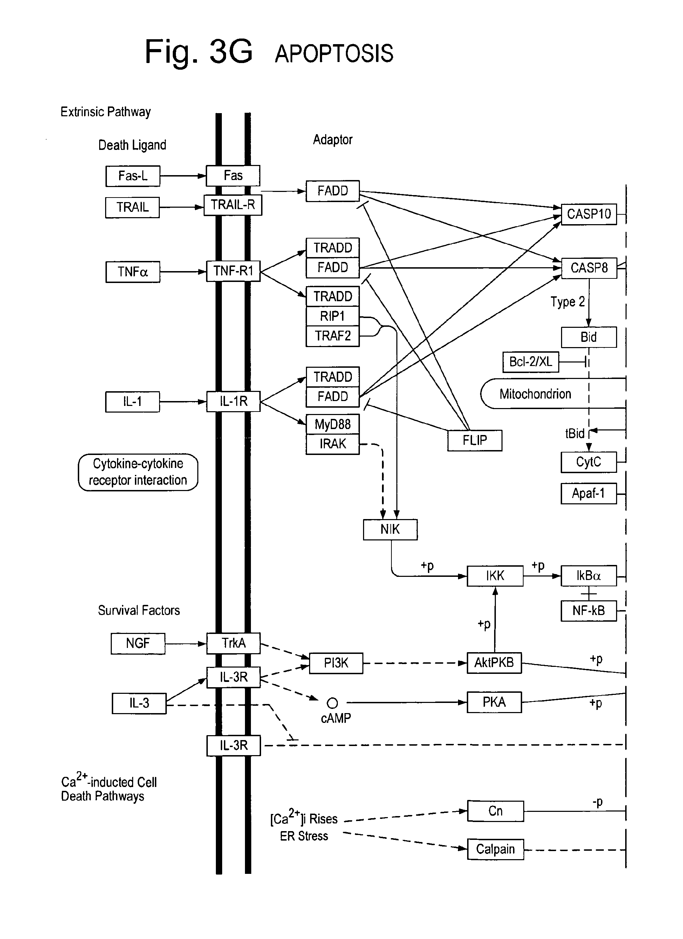

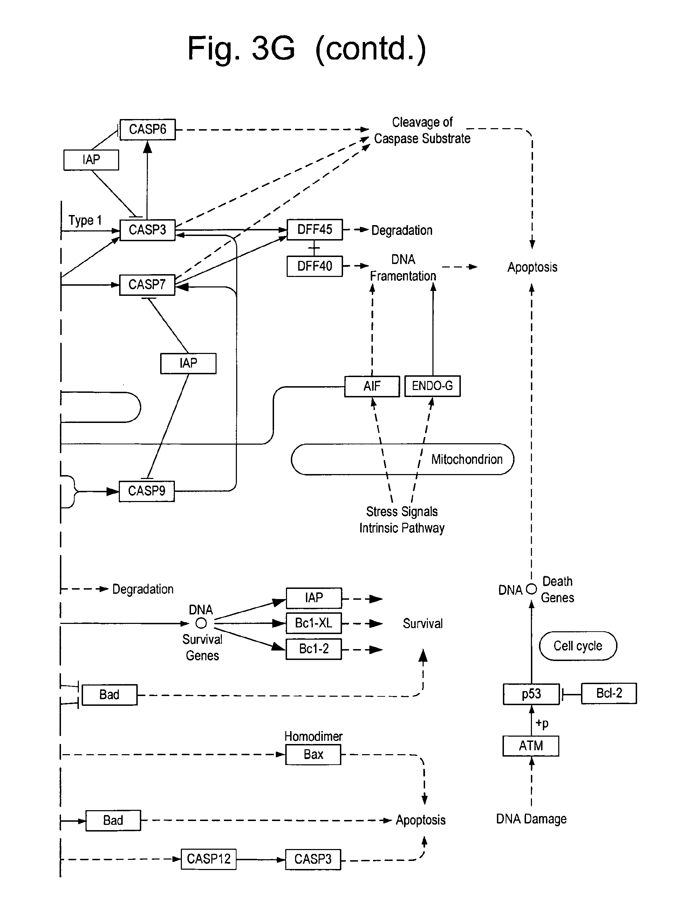

Figure 3G depicts the apoptosis signaling pathway.

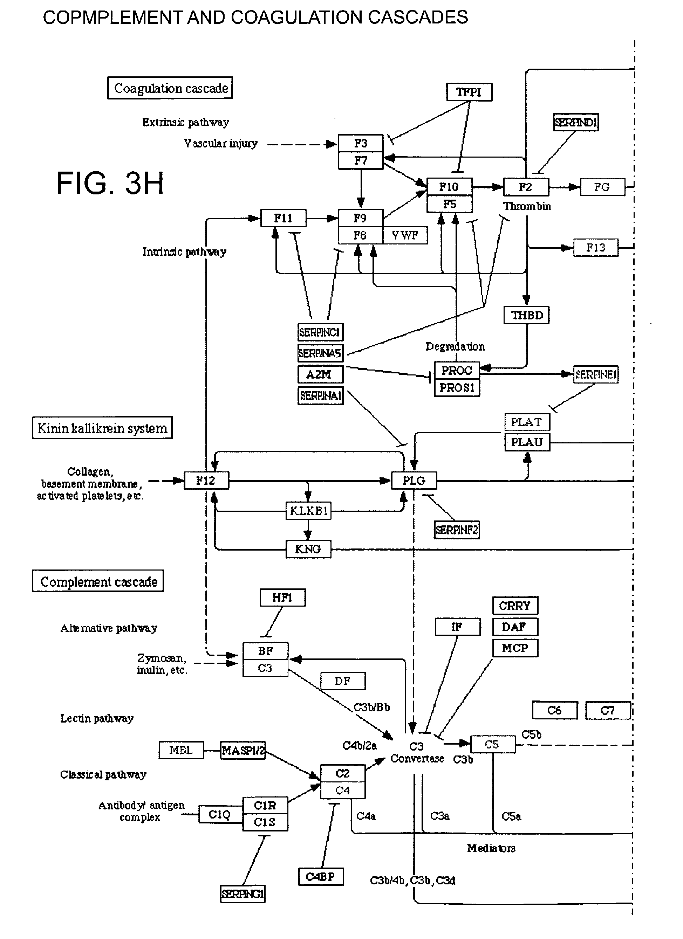

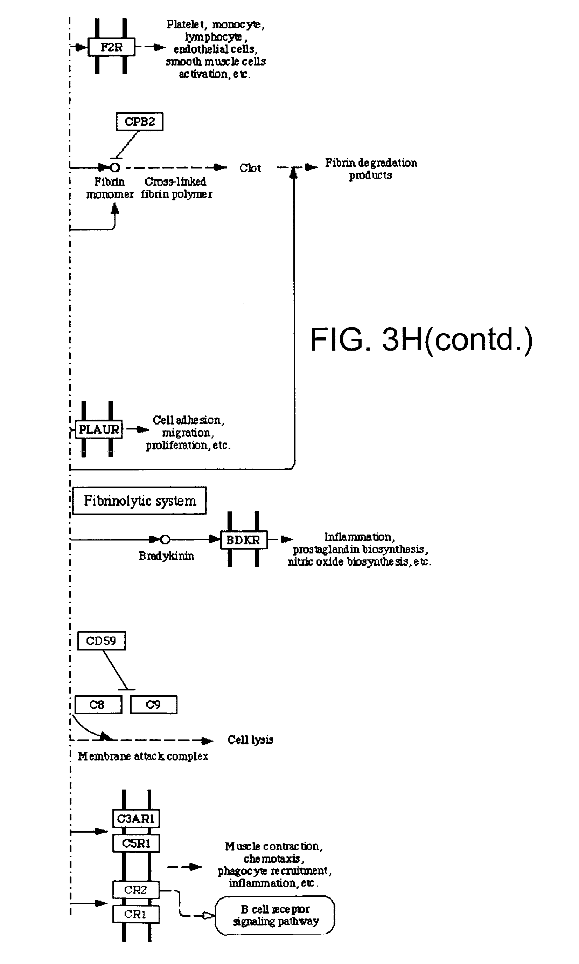

Figure 3H depicts the complement and coagulation cascades.

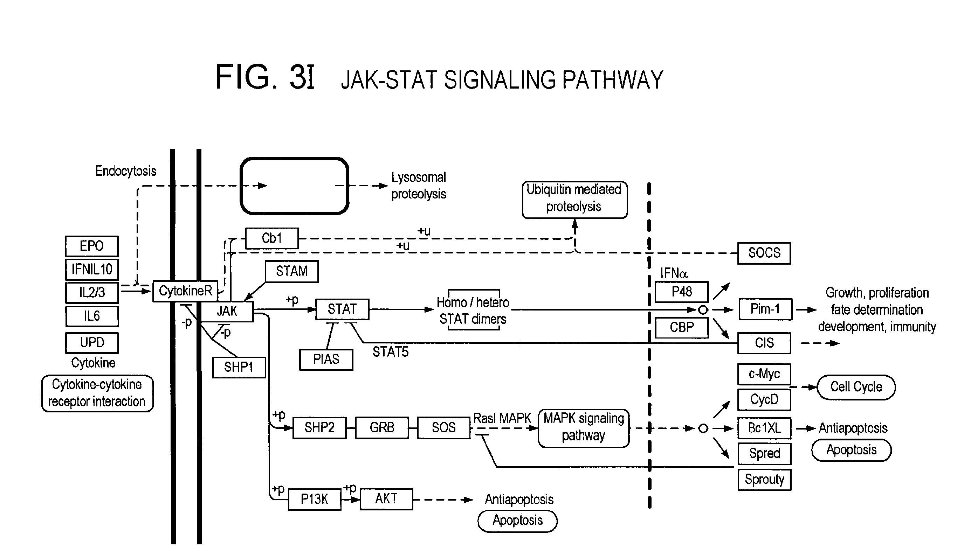

Figure 3I depicts the Jak-STAT signaling pathway.

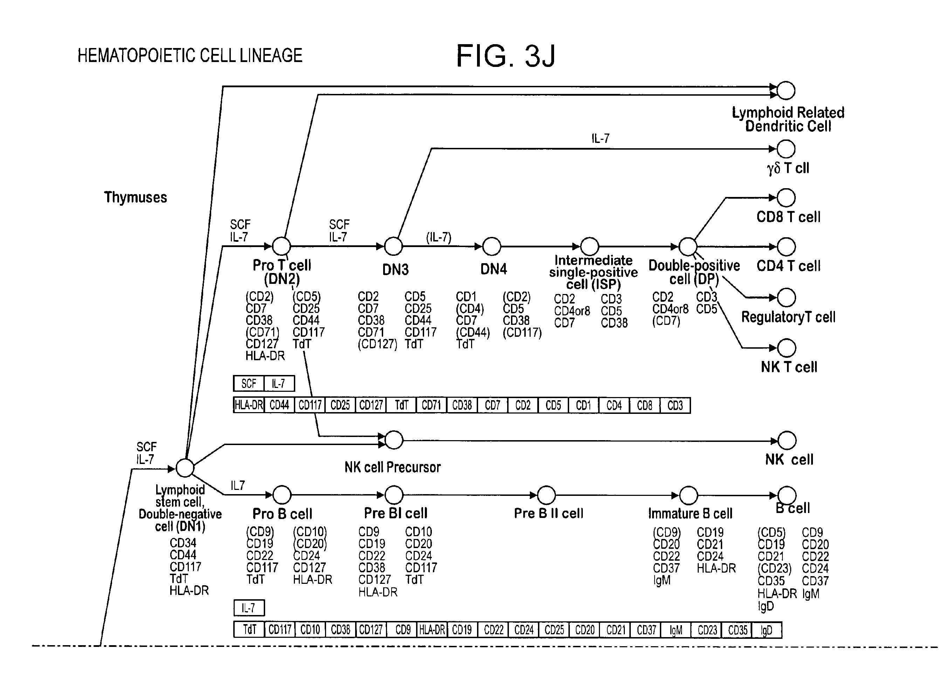

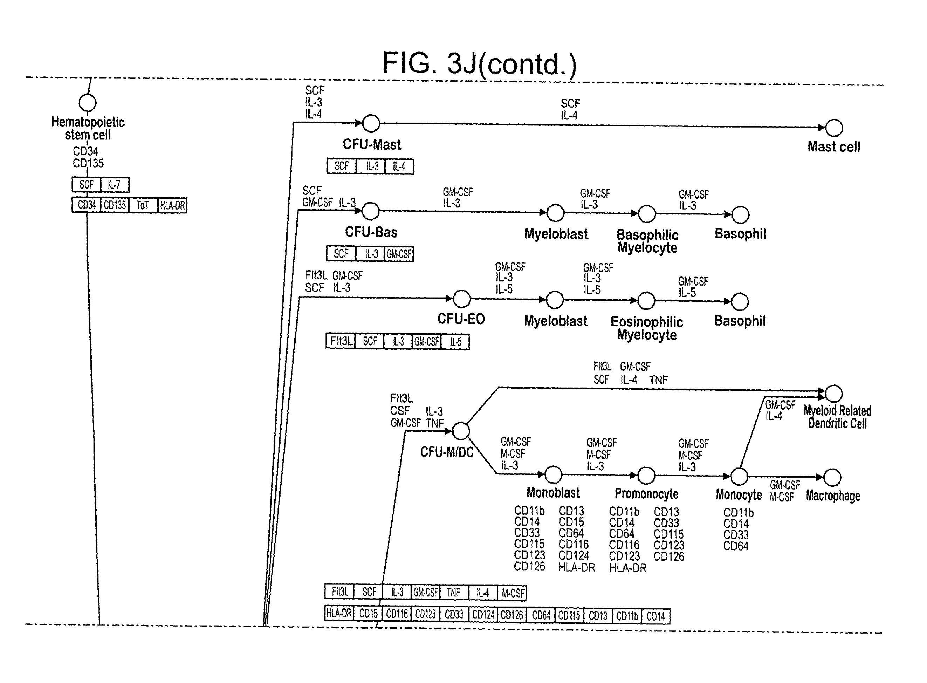

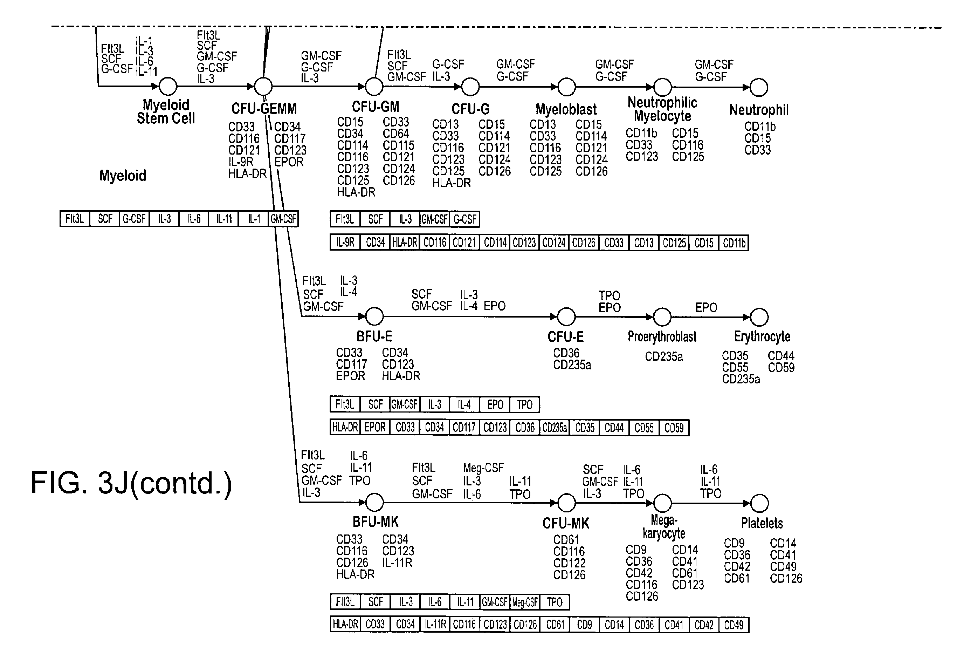

Figure 3J is a representation of the hematopoietic cell lineage.

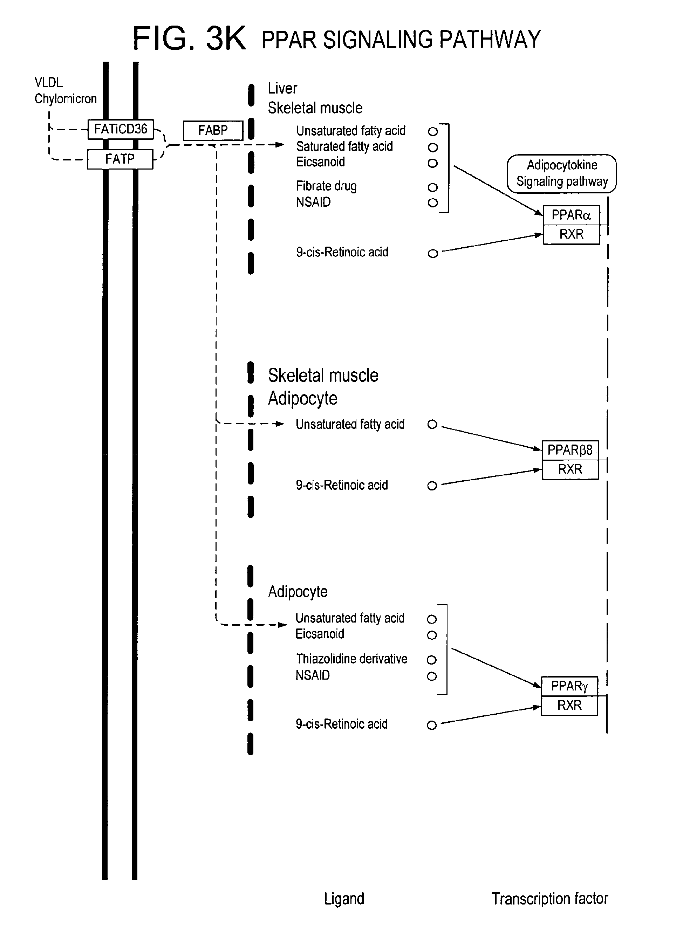

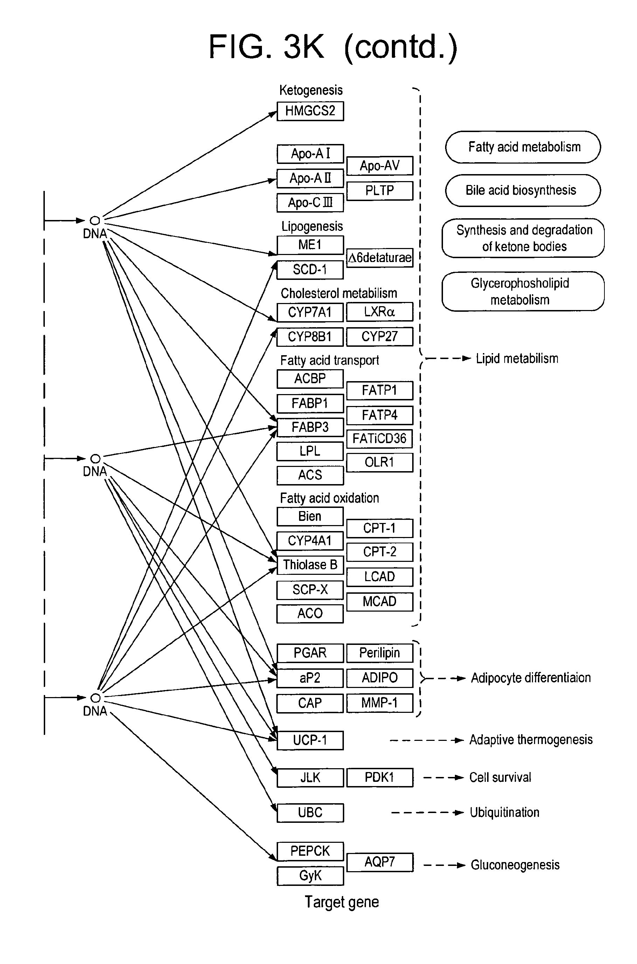

Figure 3K shows the PPAR signaling pathway.

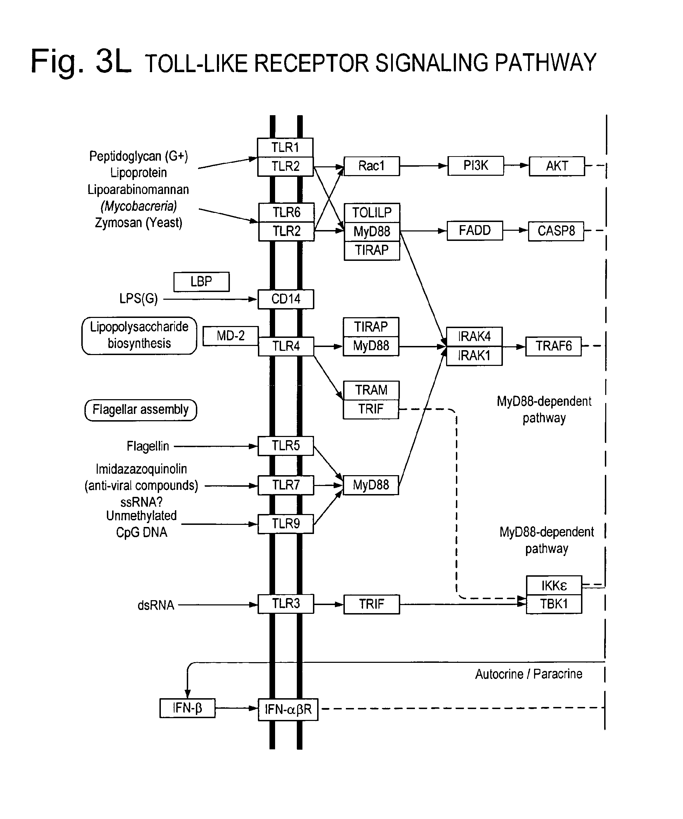

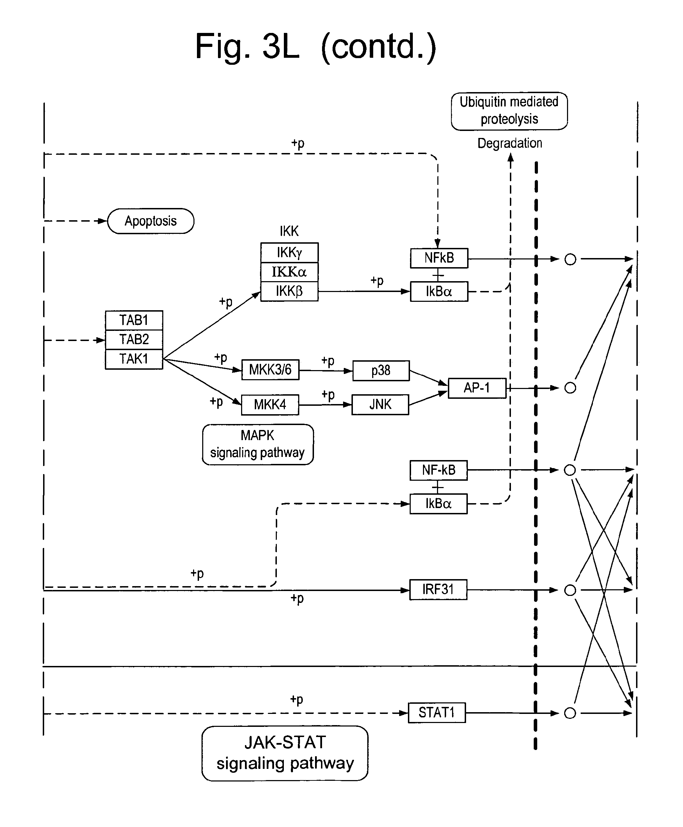

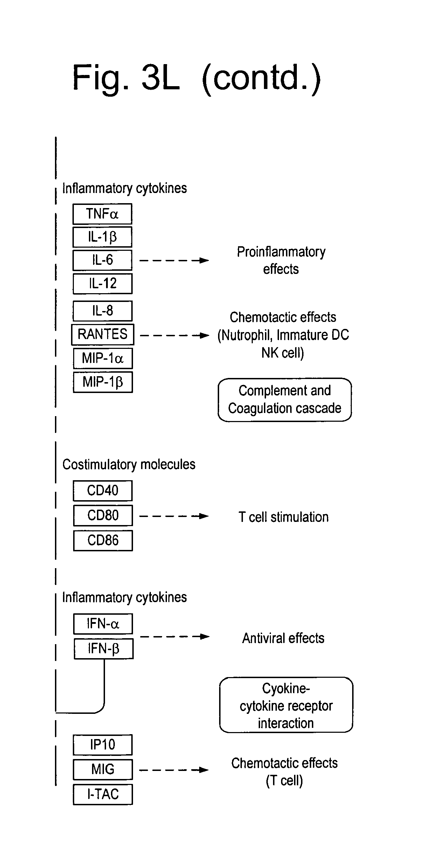

Figure 3L is the Toll-like receptor signaling pathway.

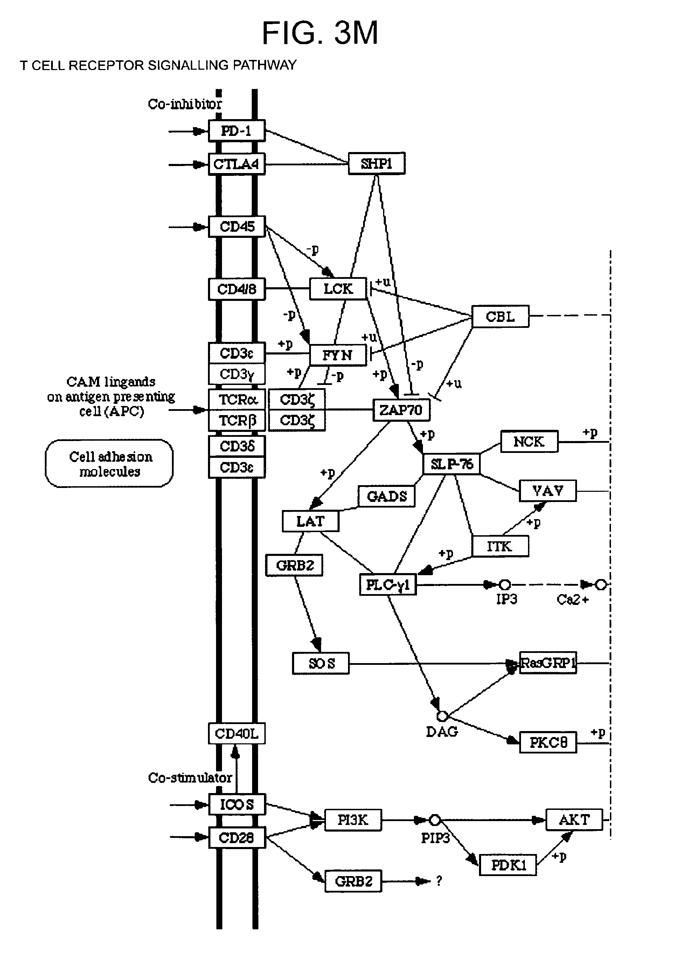

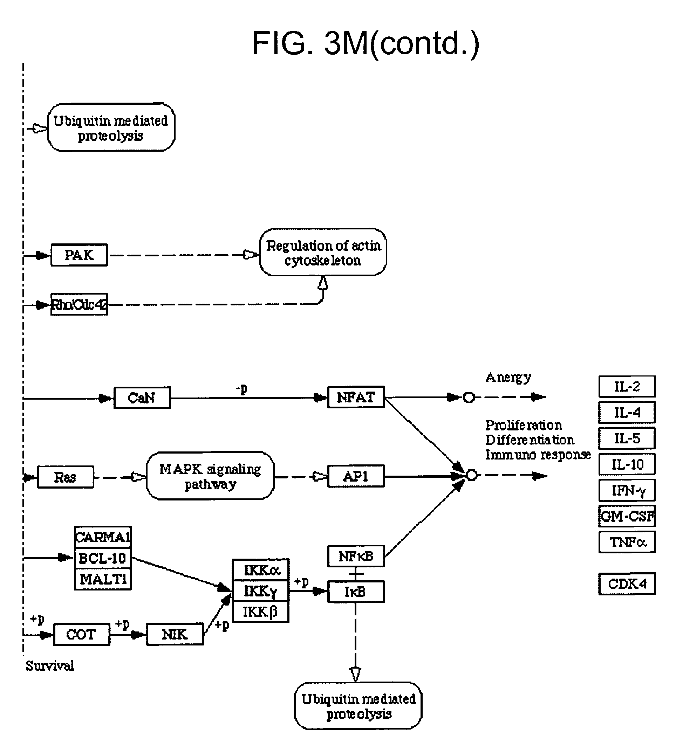

Figure 3M shows the T-cell receptor signaling pathway.

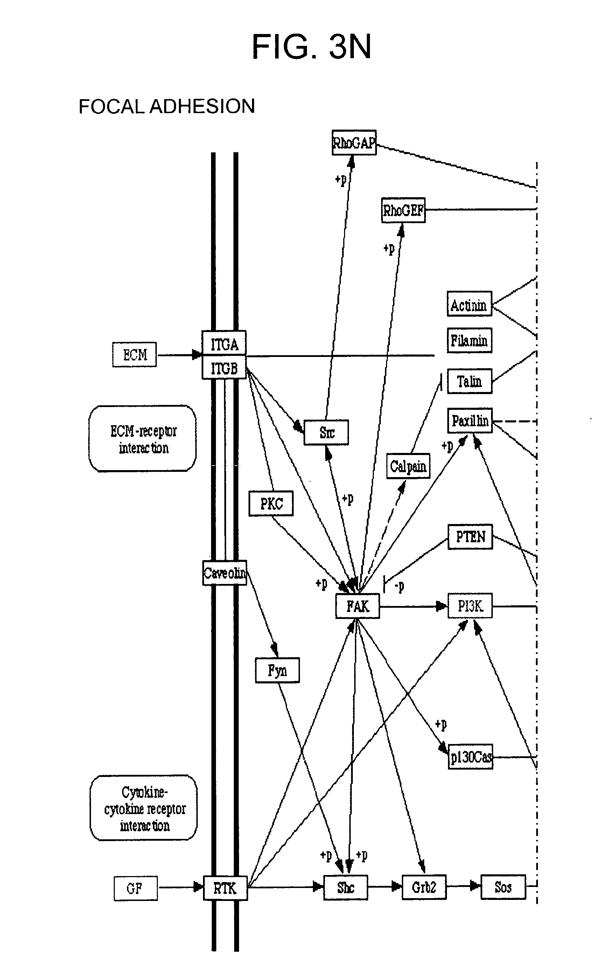

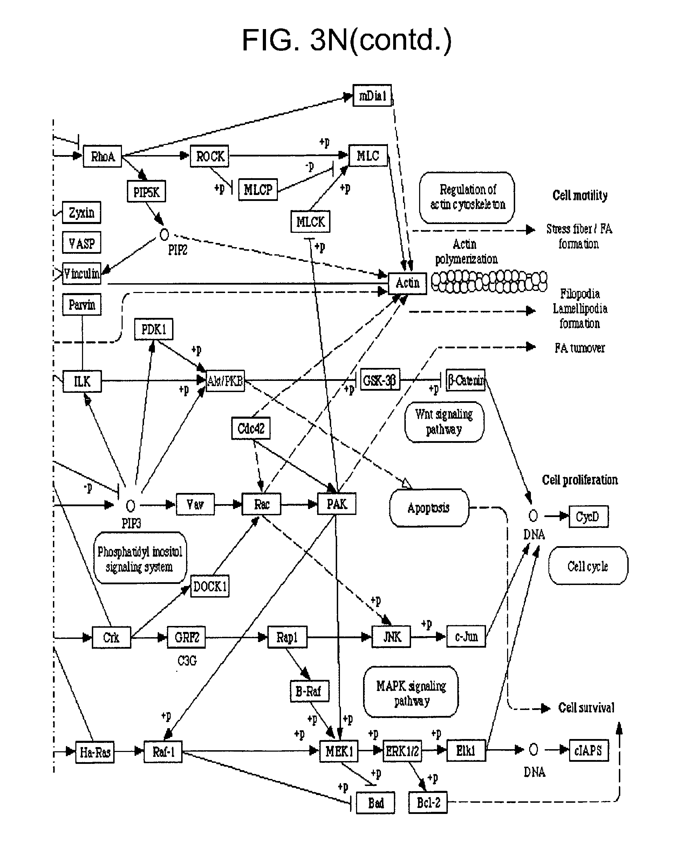

Figure 3N depicts the focal adhesion signaling pathway.

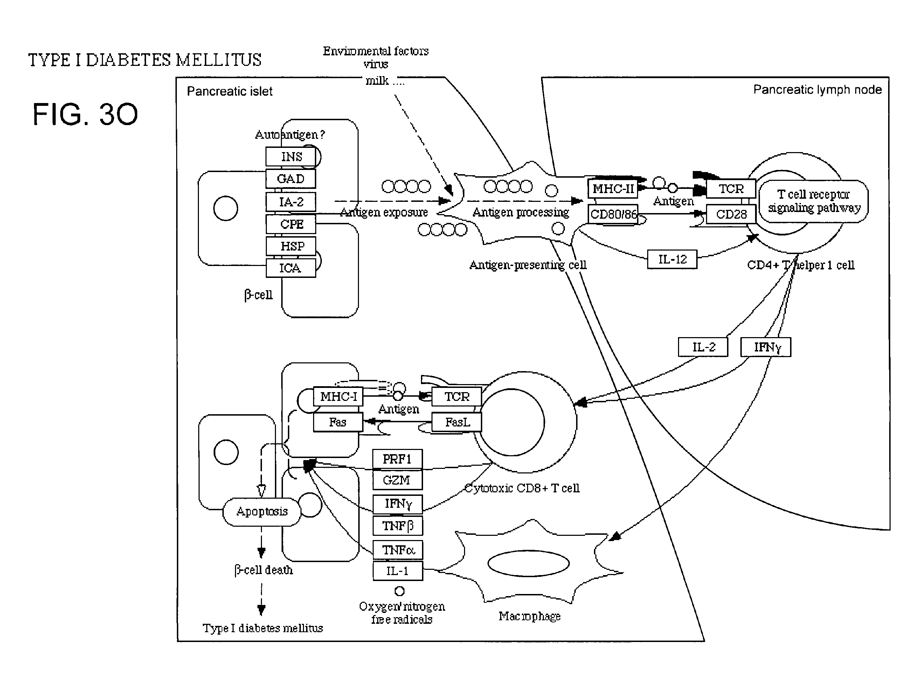

Figure 3O shows the Type I Diabetes Mellitus pathway.

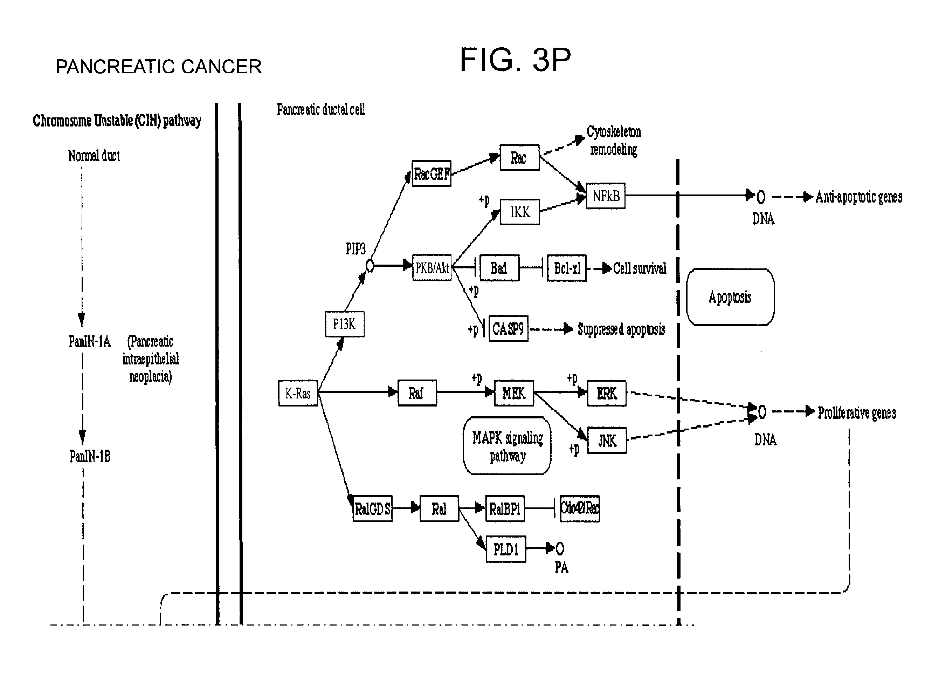

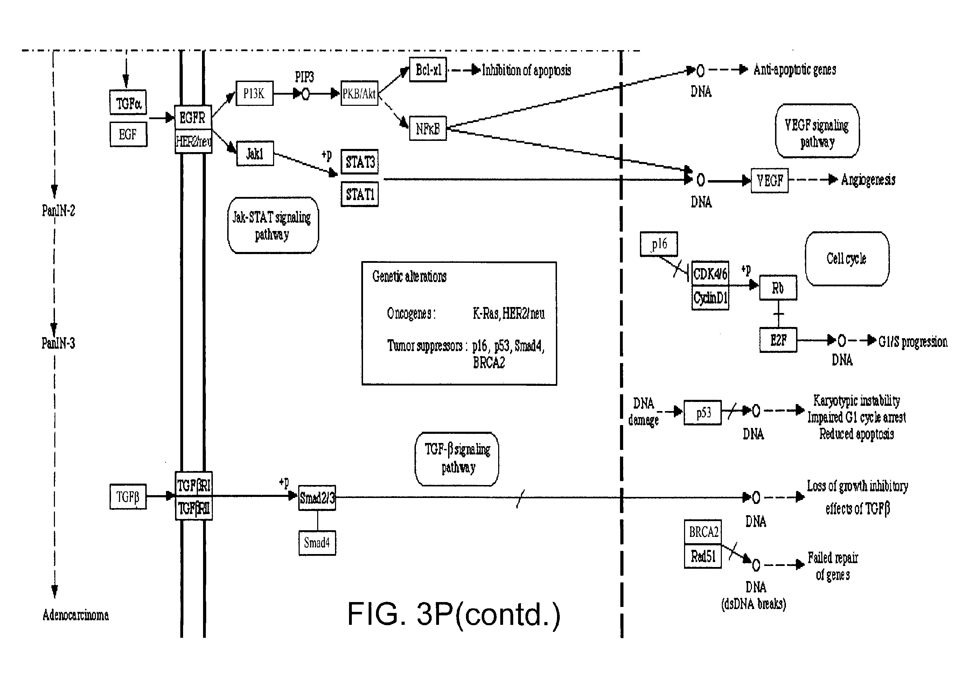

Figure 3P is the pancreatic cancer signaling pathway.

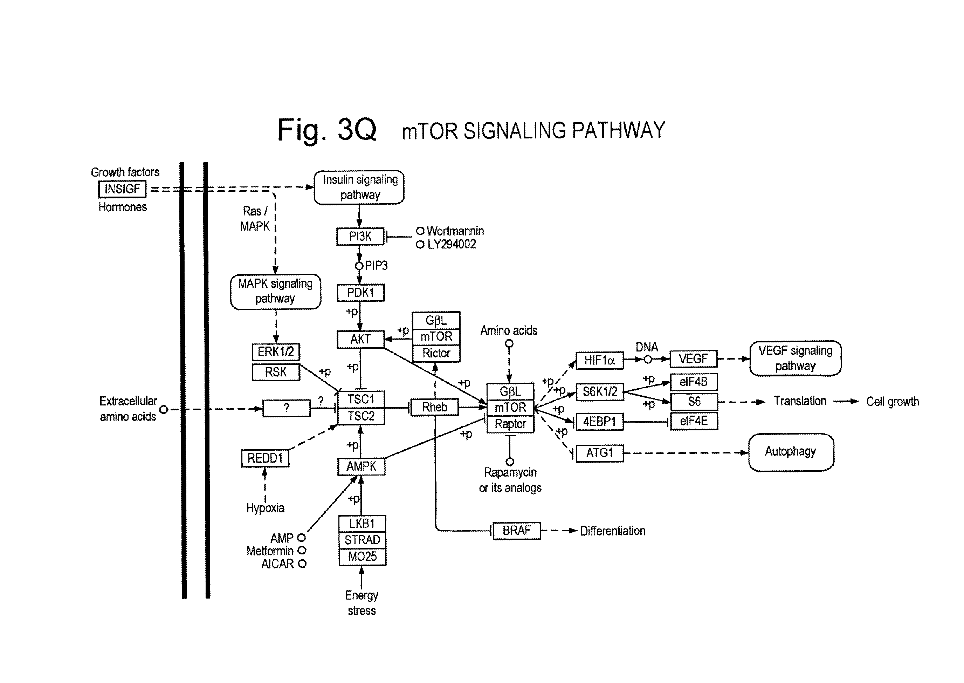

Figure 3Q depicts the mTOR signaling pathway.

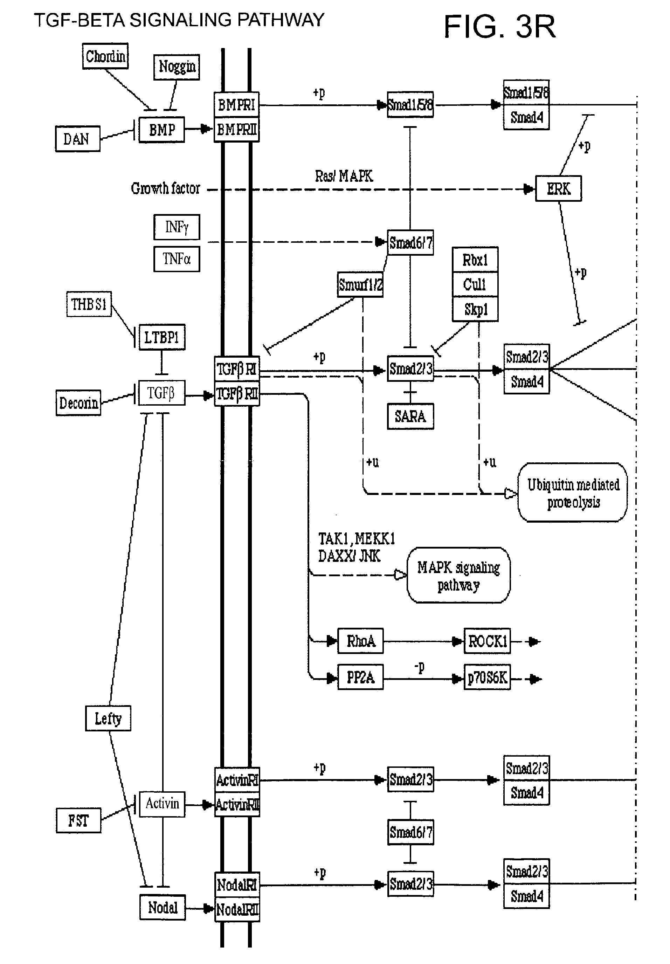

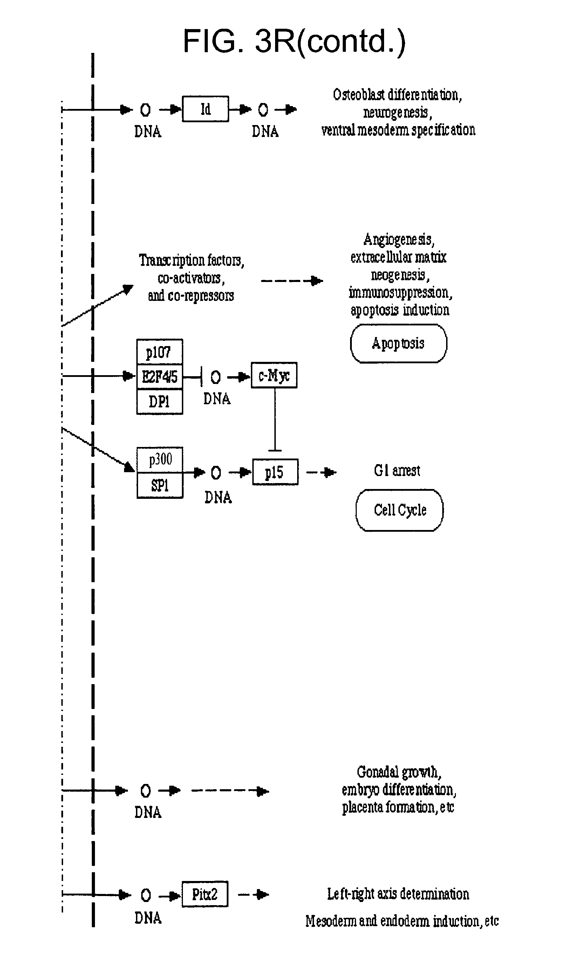

Figure 3R shows the TGF-β signaling pathway.

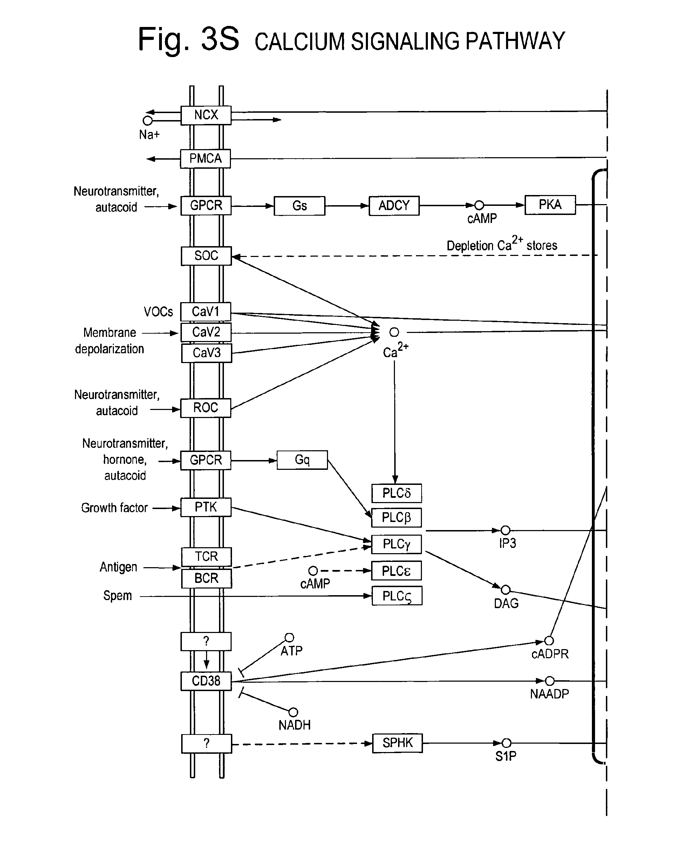

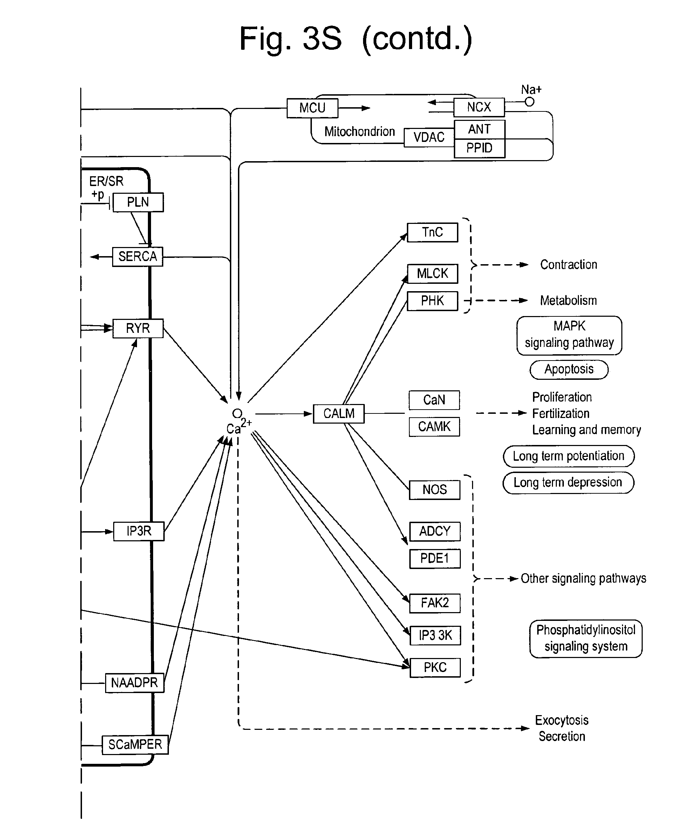

Figure 3S is the calcium signaling pathway.

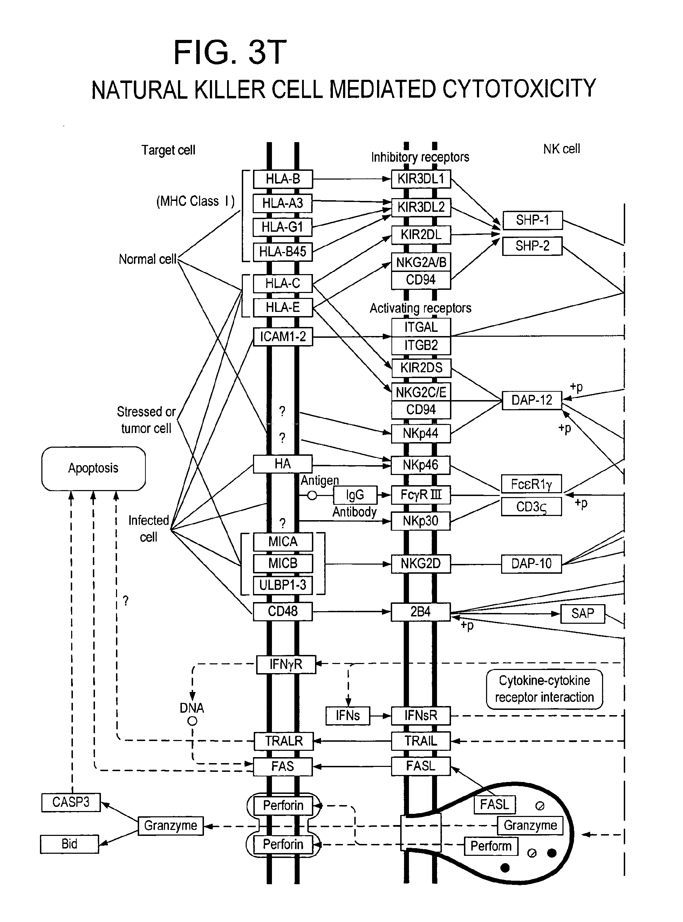

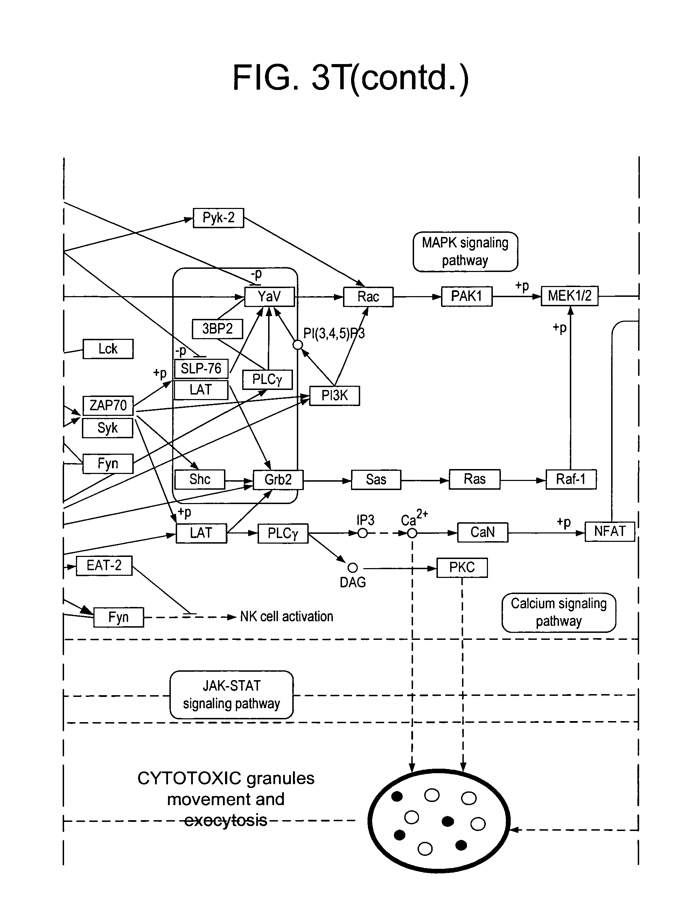

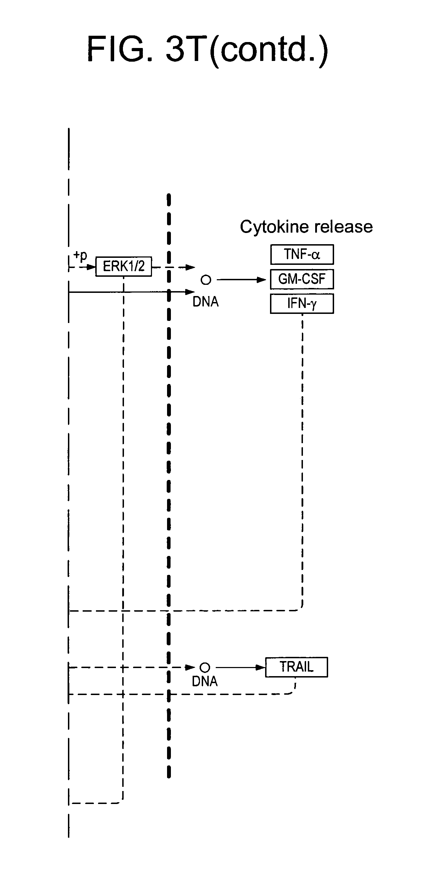

Figure 3T shows the natural killer cell-mediated cytotoxicity pathway.

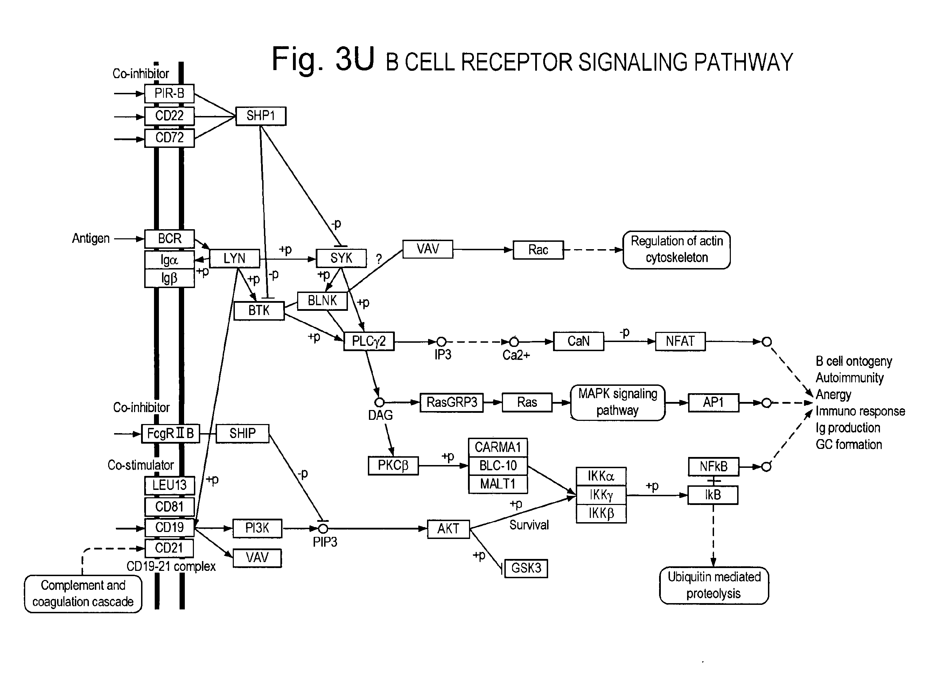

Figure 3U shows the B-cell receptor signaling pathway.

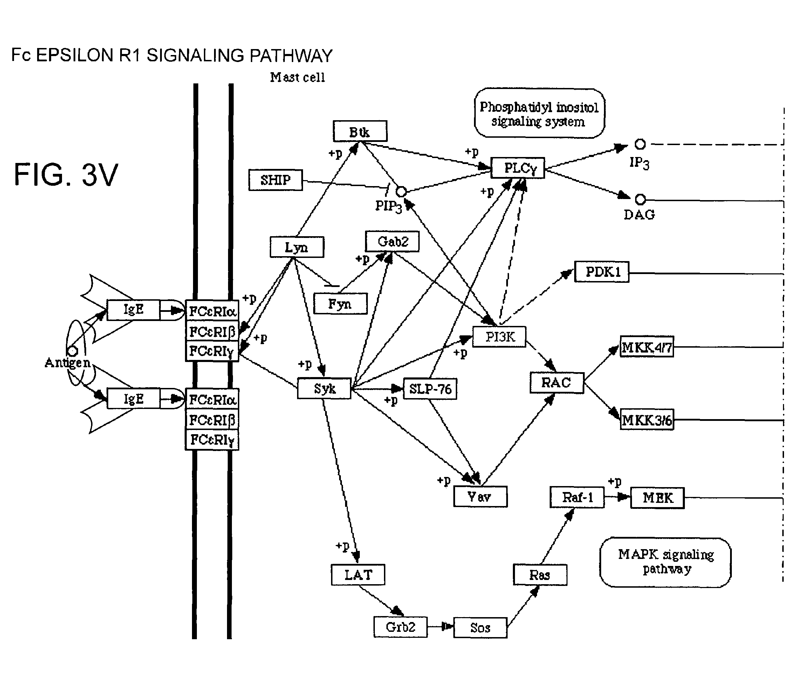

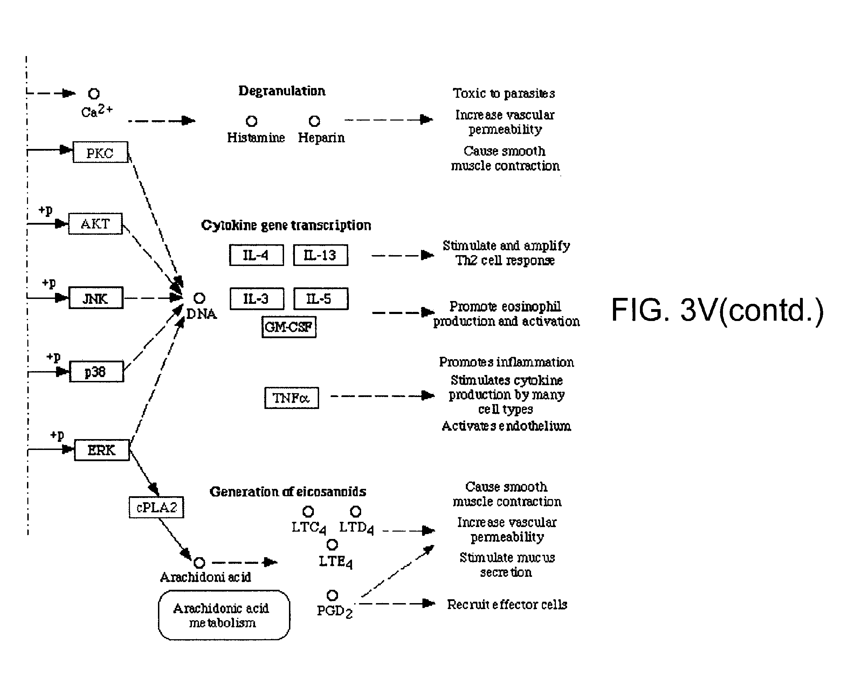

Figure 3V shows the FcεRI signaling pathway.

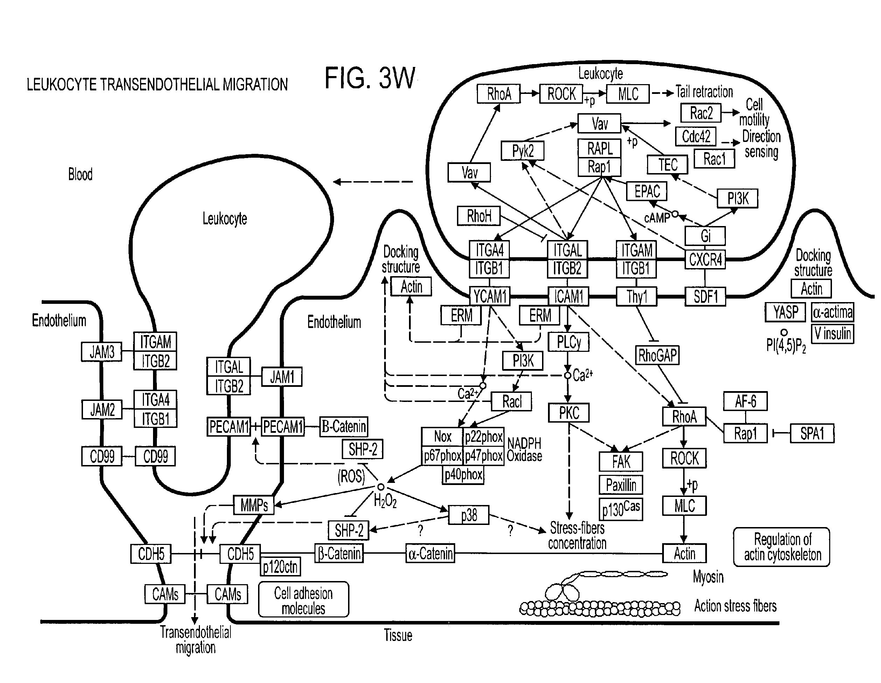

Figure 3W depicts the pathway of leukocyte transendothelial migration.

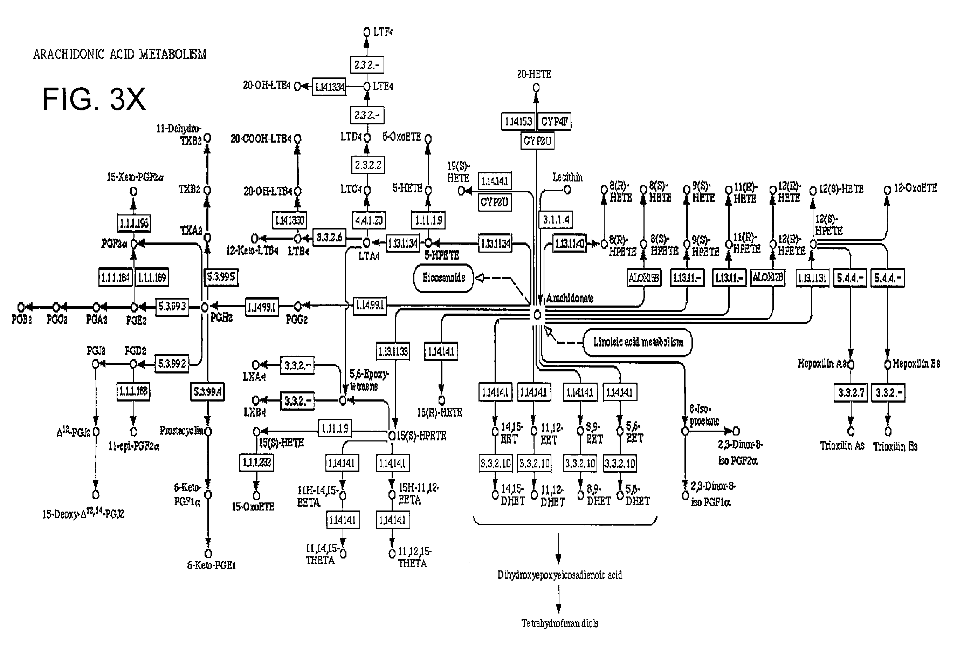

Figure 3X depicts the arachidonic acid metabolic pathway.

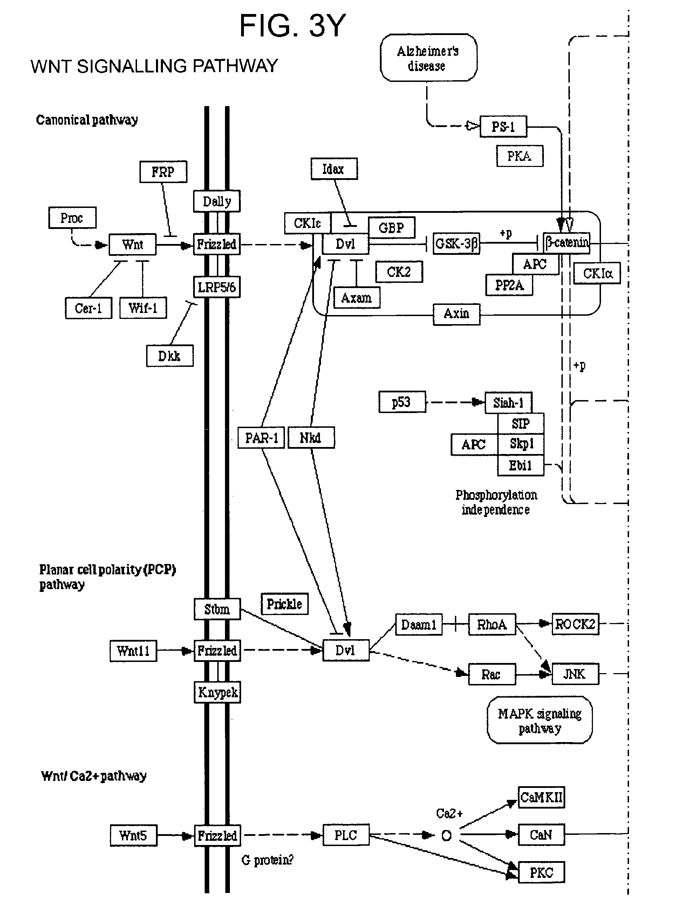

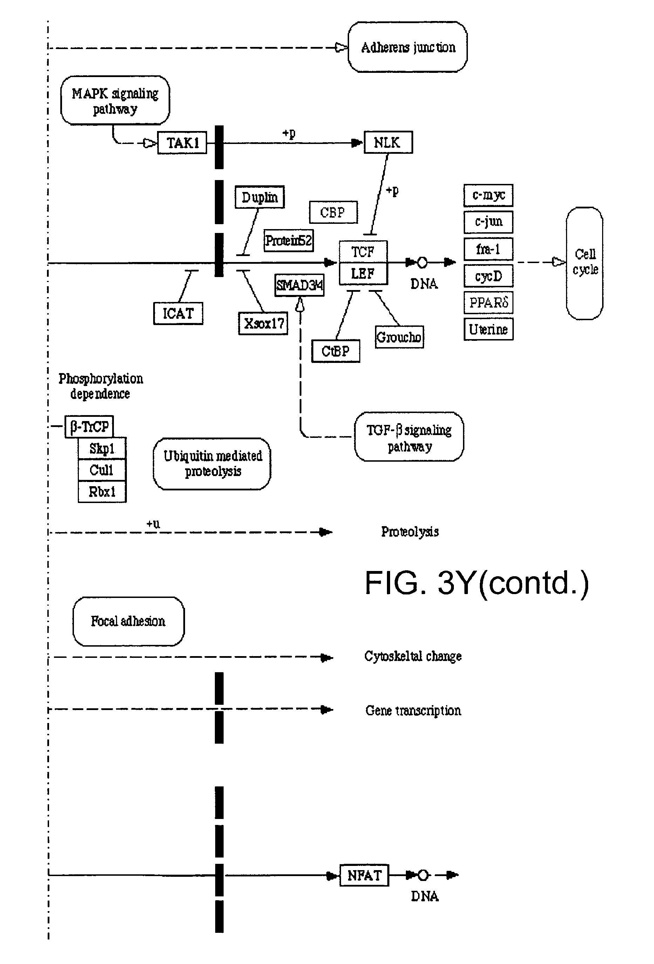

Figure 3Y depicts the Wnt signaling pathway.

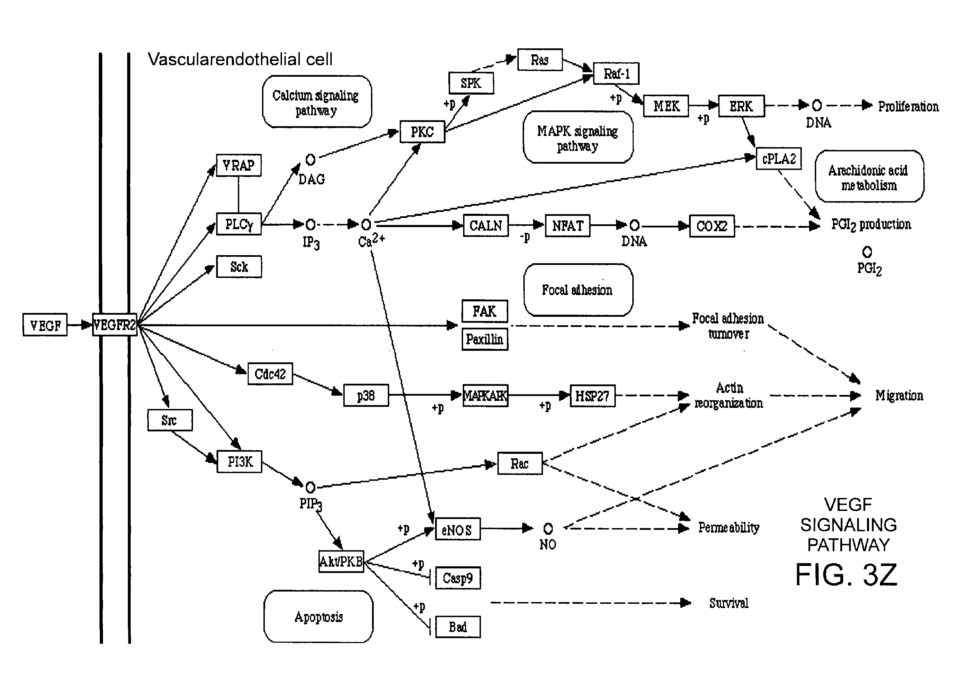

Figure 3Z shows the VEGF signaling pathway.

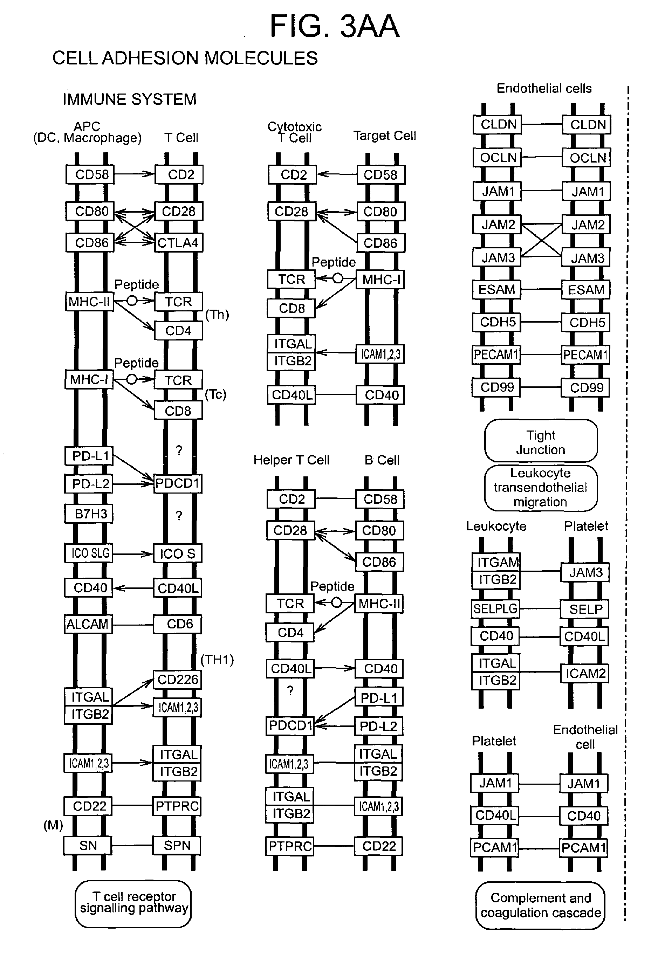

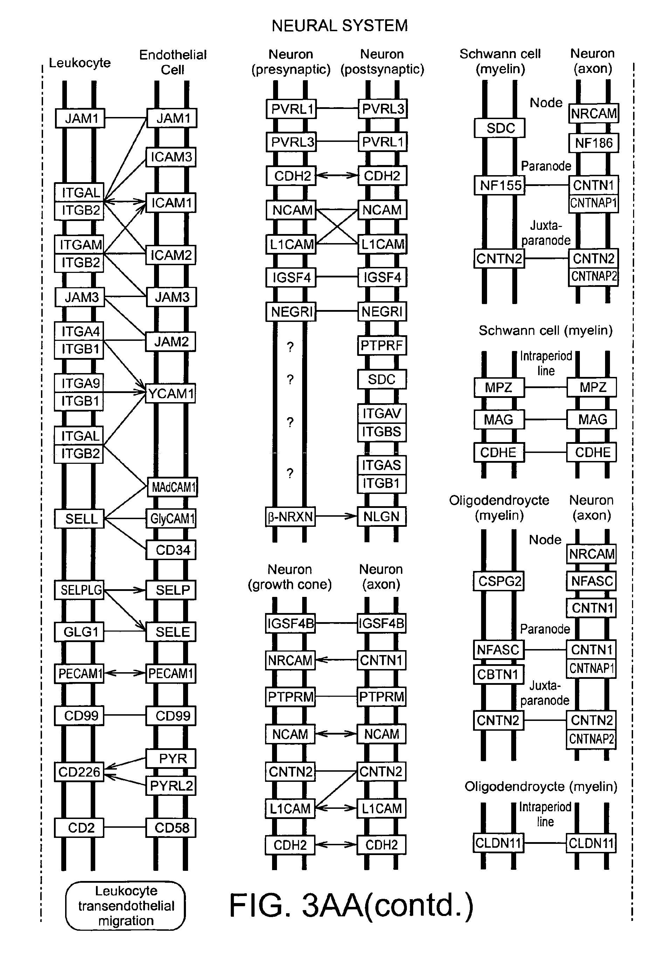

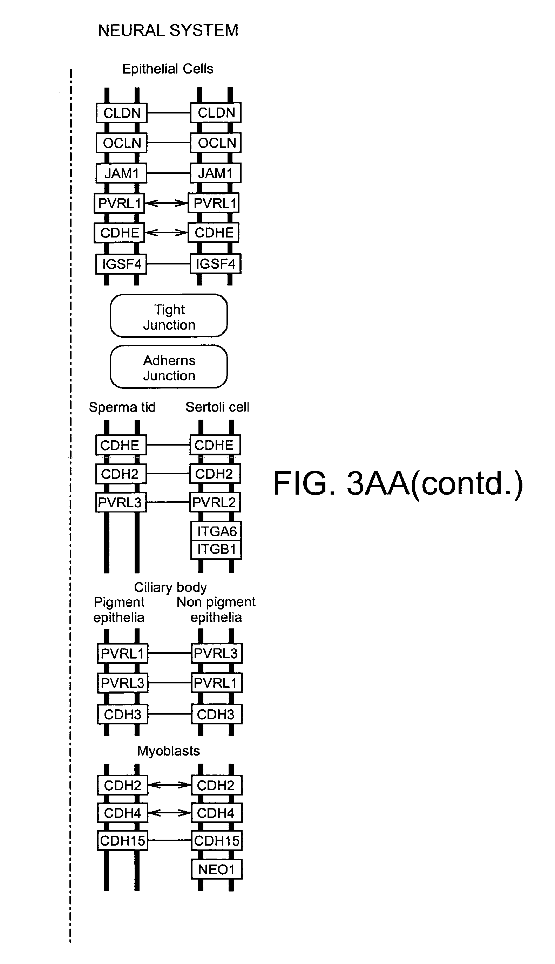

Figure 3AA depicts cell adhesion molecule interactions.

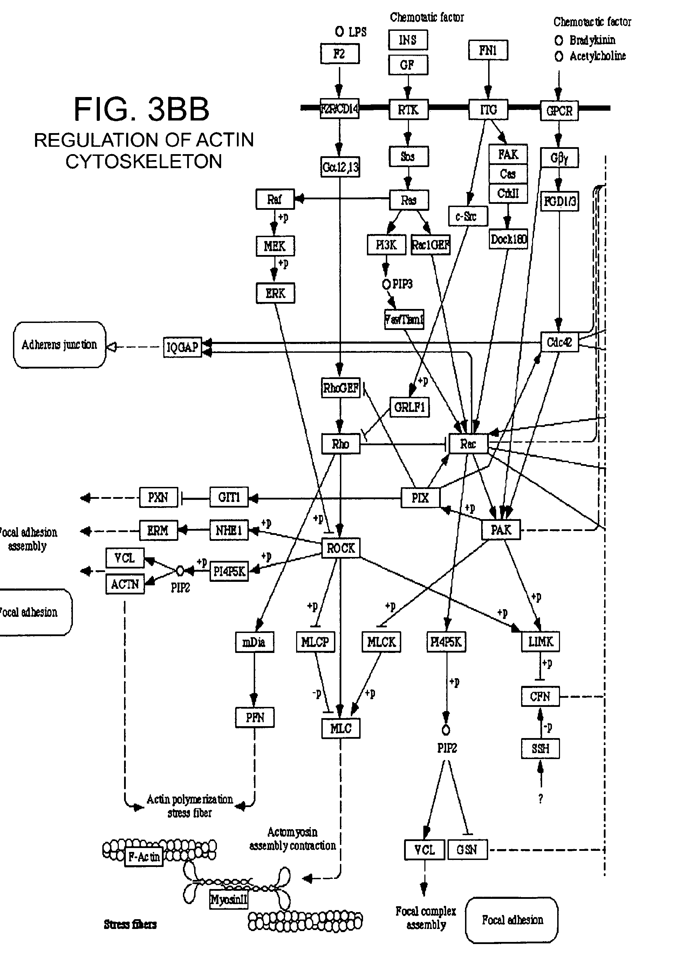

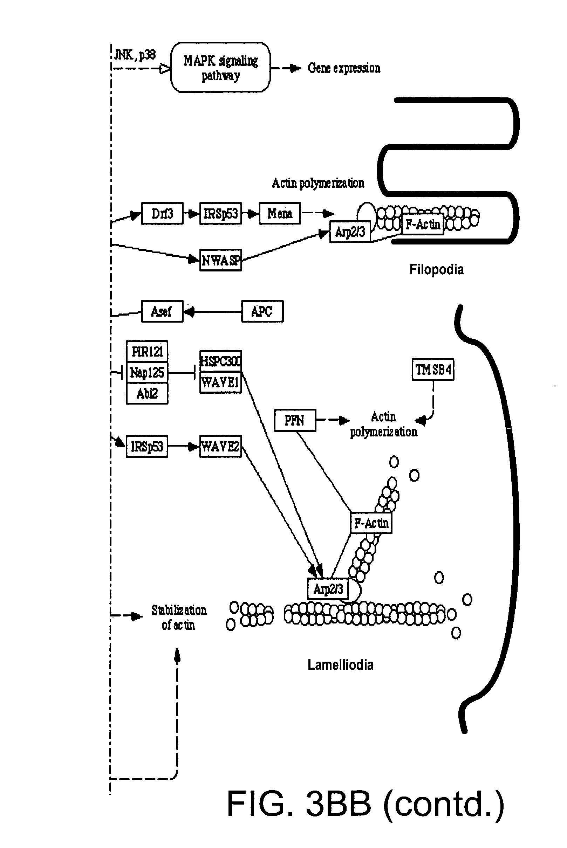

Figure 3BB is a schematic showing regulation of the actin cytoskeleton.

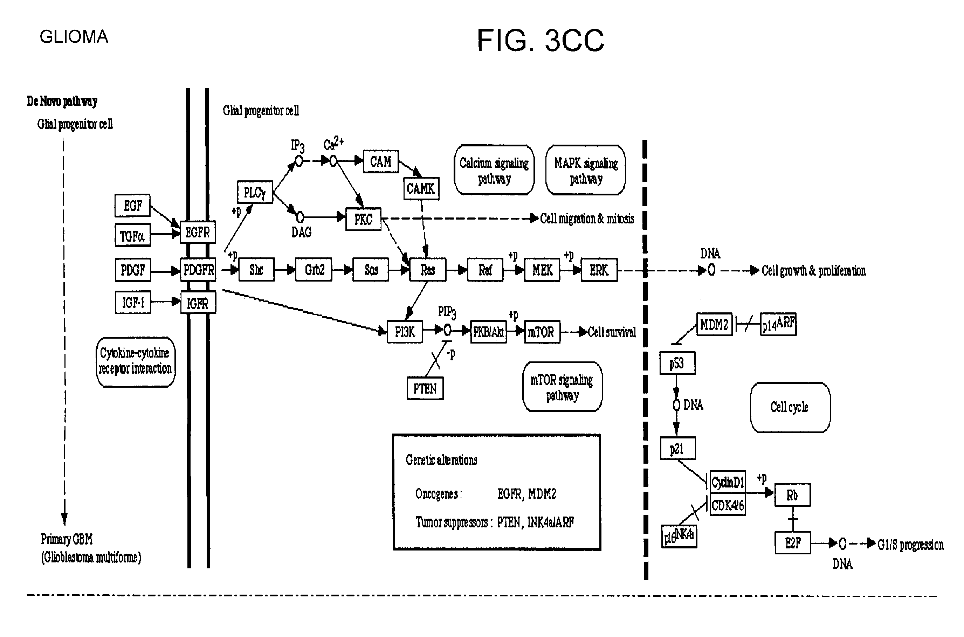

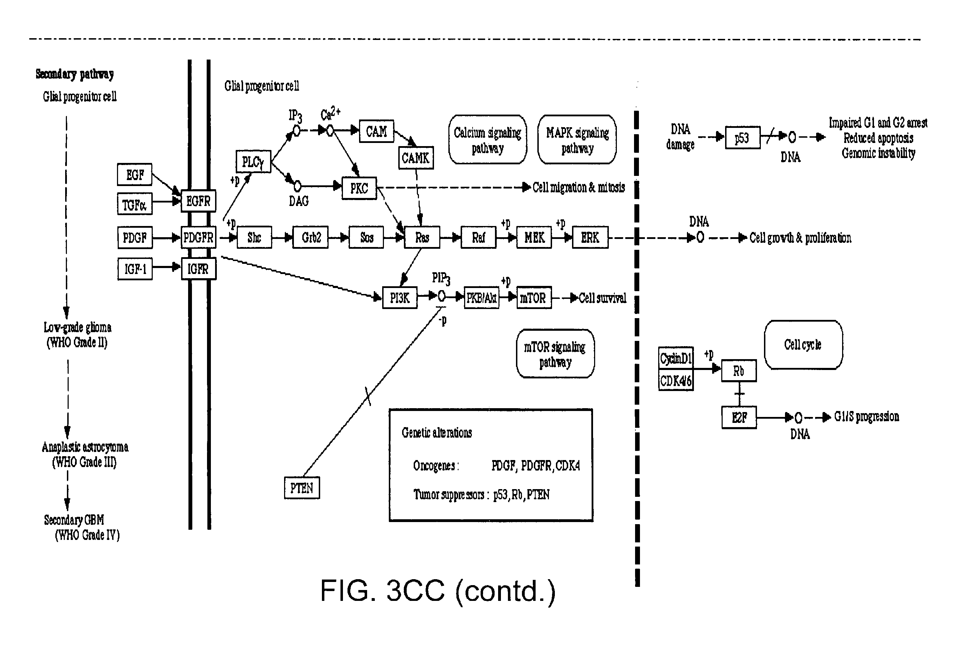

Figure 3CC depicts interactions relating to glioma.

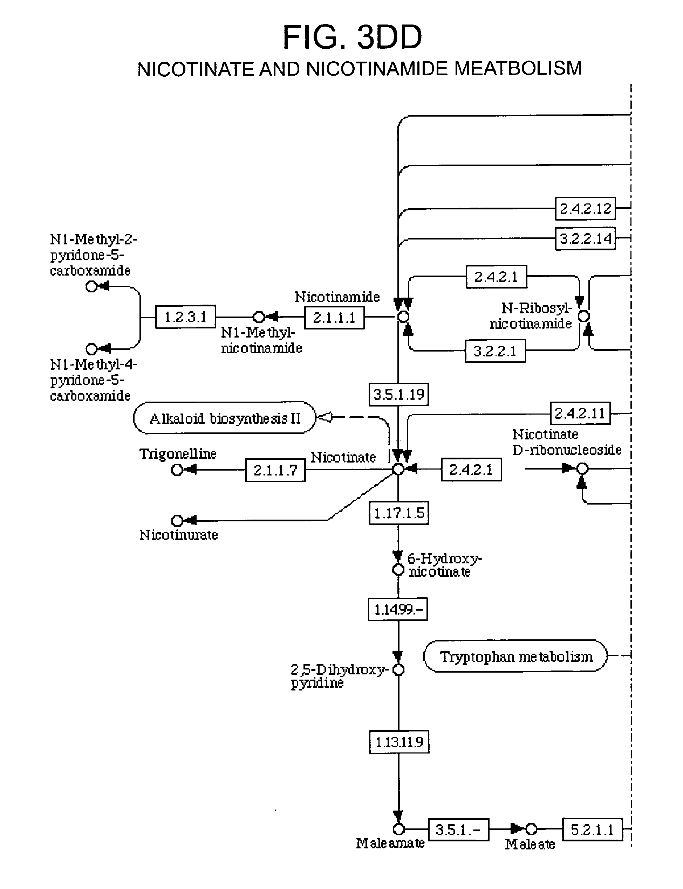

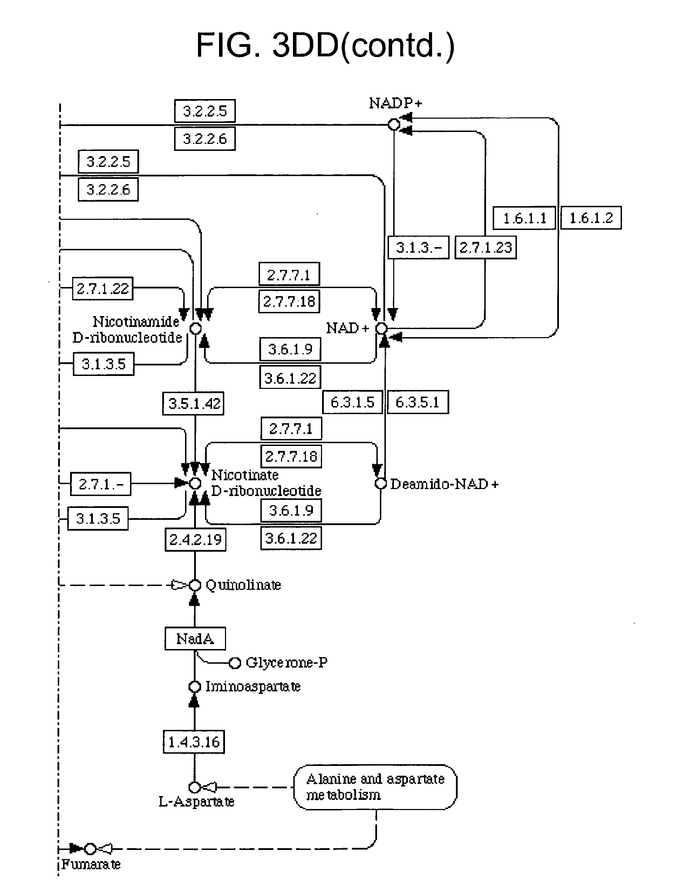

Figure 3DD depicts nicotinate and nicotinamide metabolism.

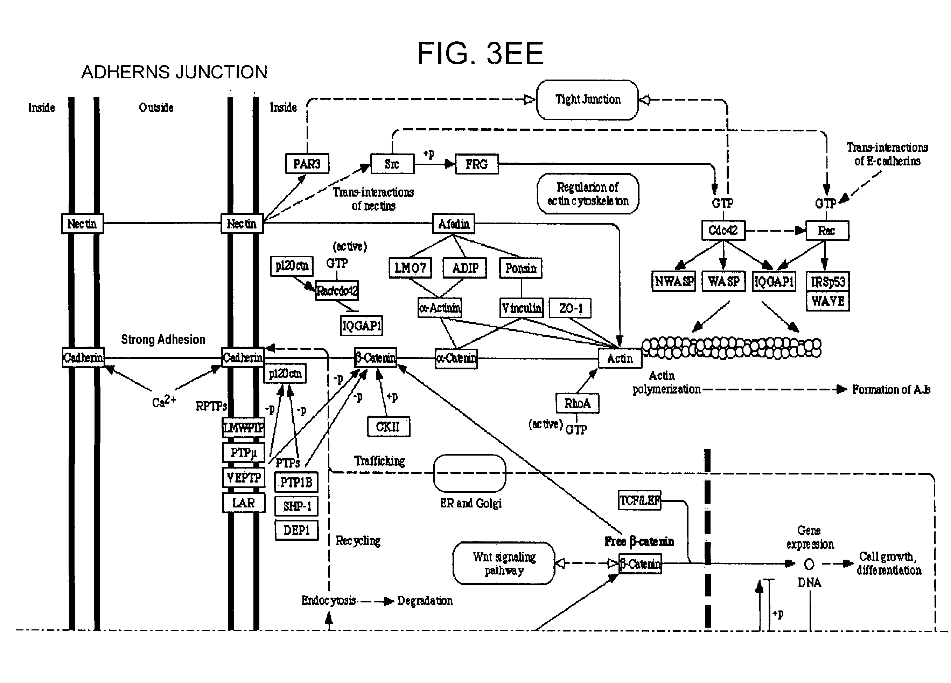

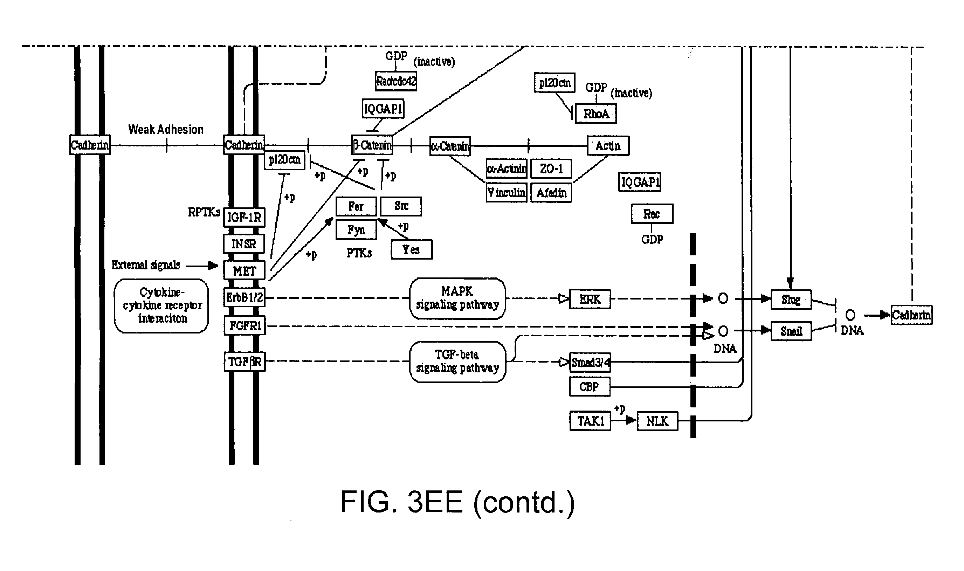

Figure 3EE shows the signaling pathway of adherens junctions.

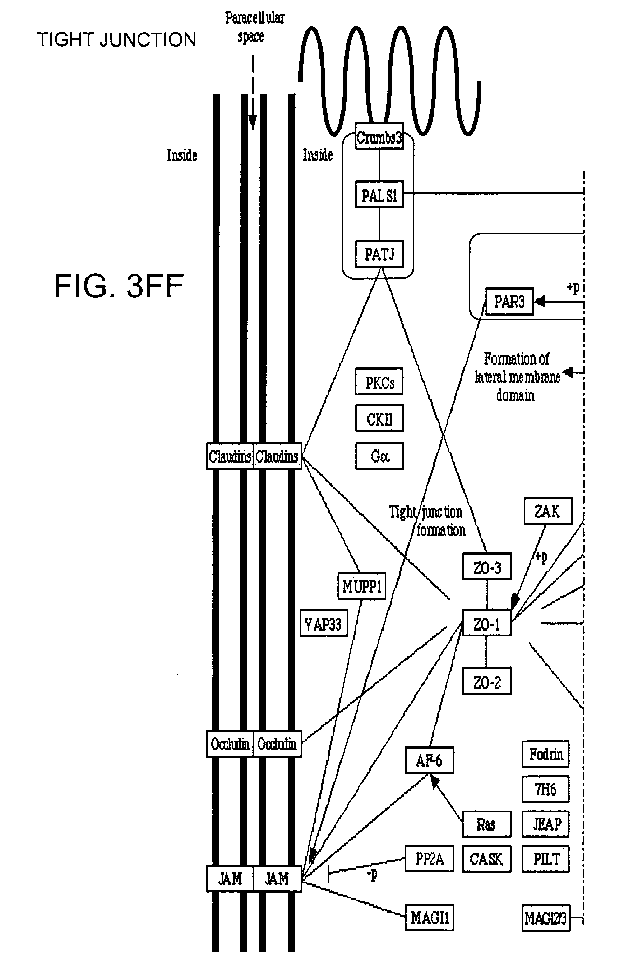

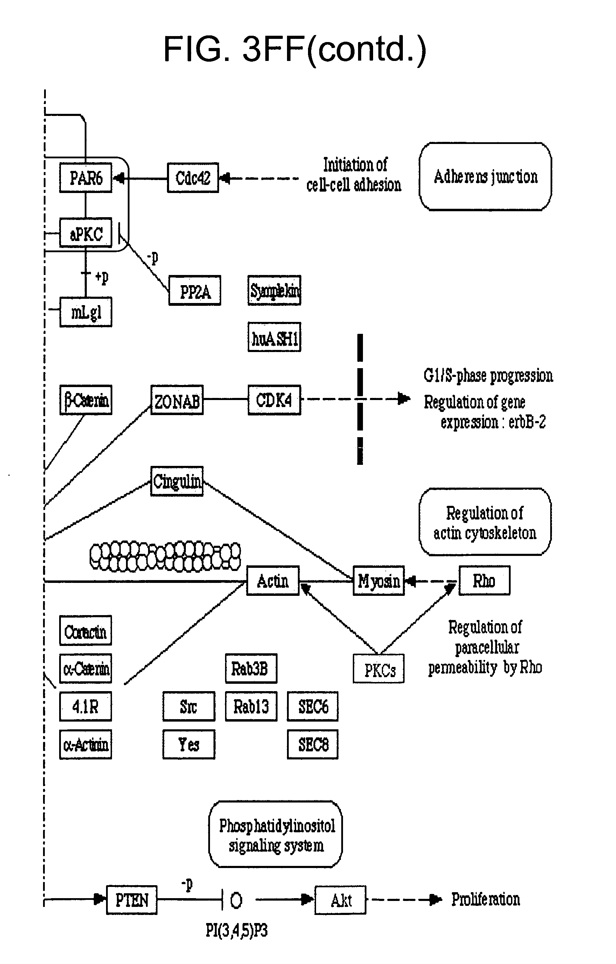

Figure 3FF is a schematic showing the signaling pathway of tight junctions.

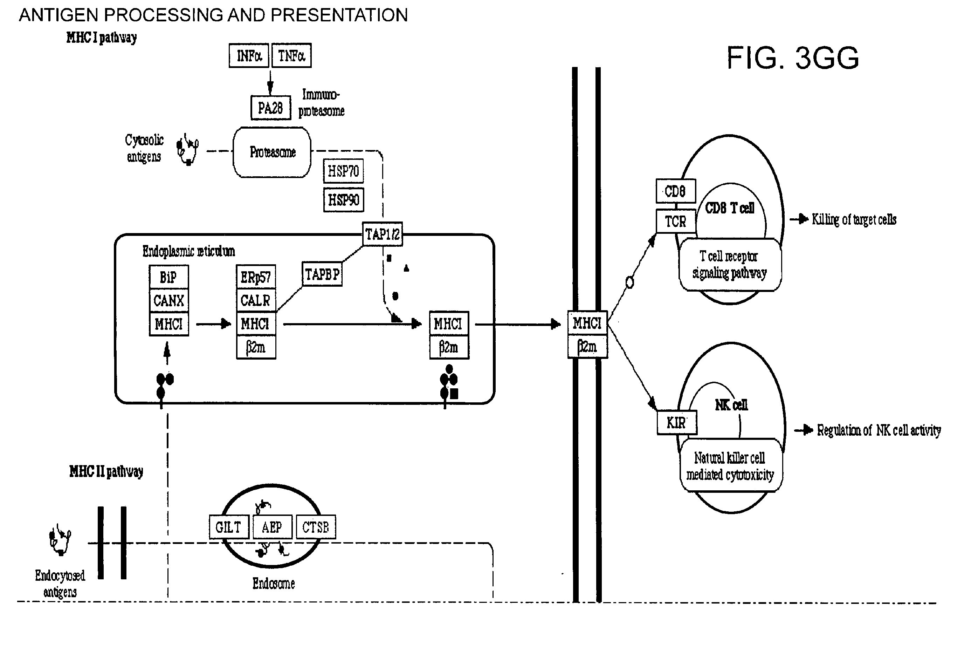

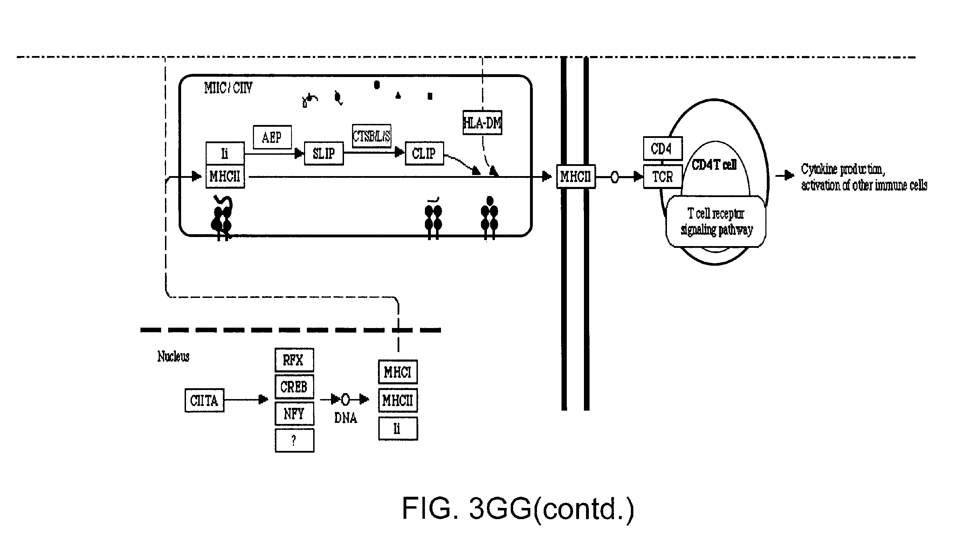

Figure 3GG depicts interactions relating to antigen processing and presentation.

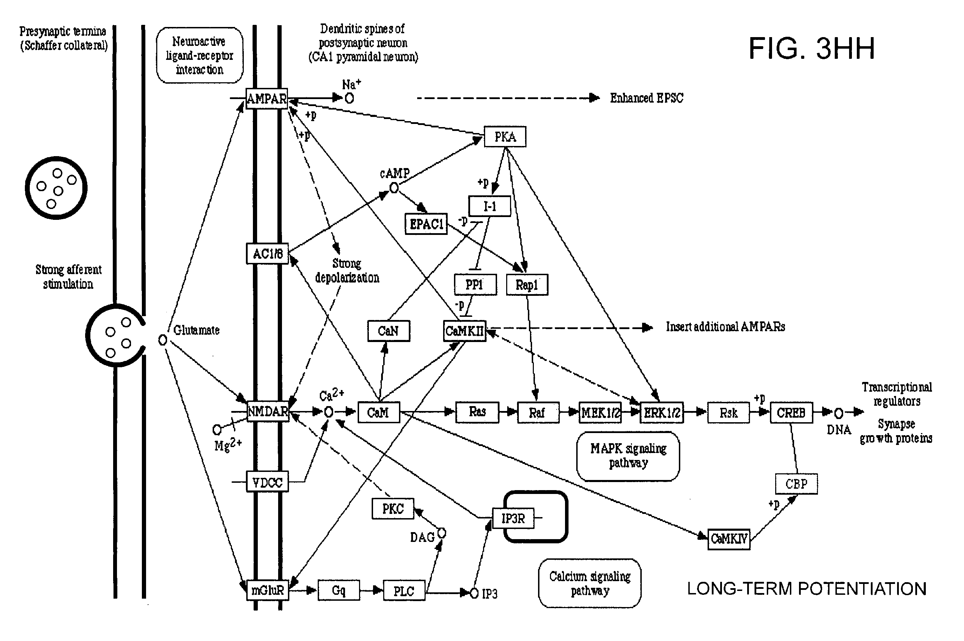

Figure 3HH shows interactions relating to long-term potentiation.

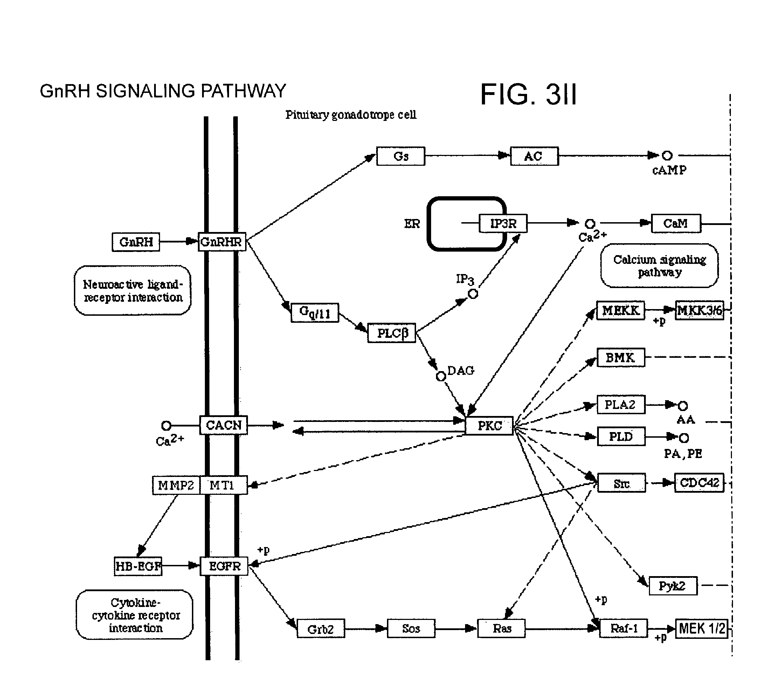

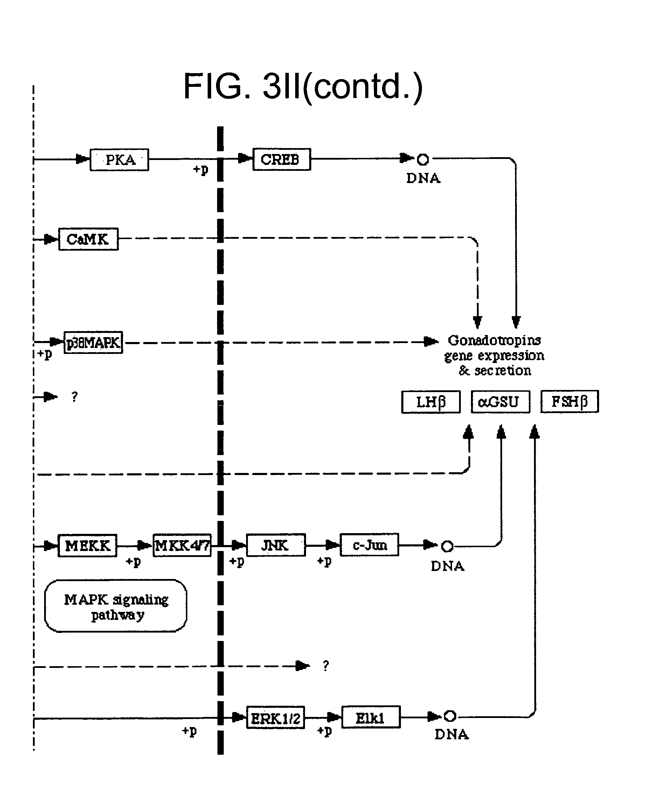

Figure 3II shows the GnRH signaling pathway.

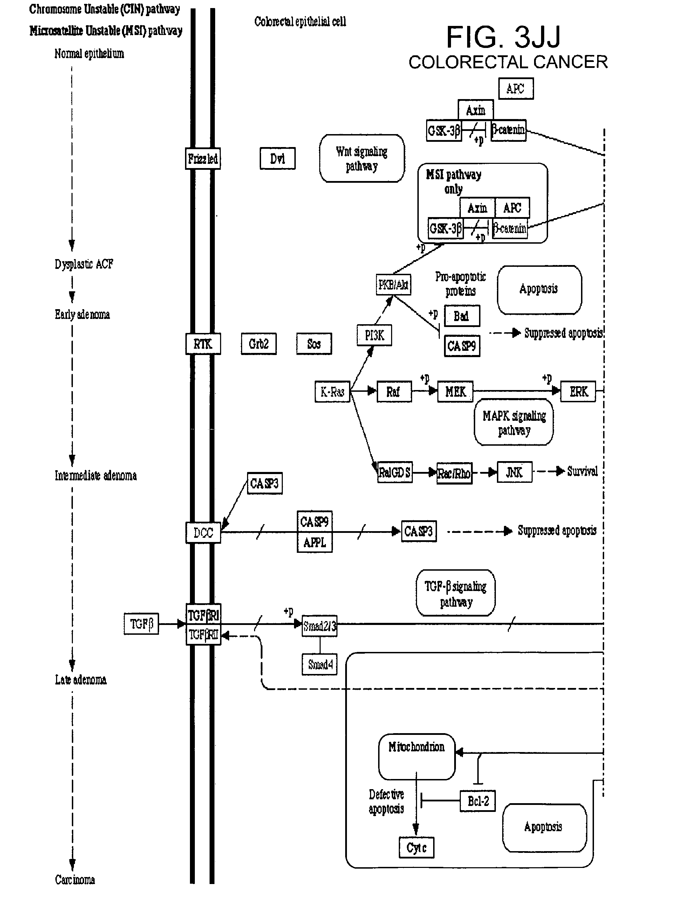

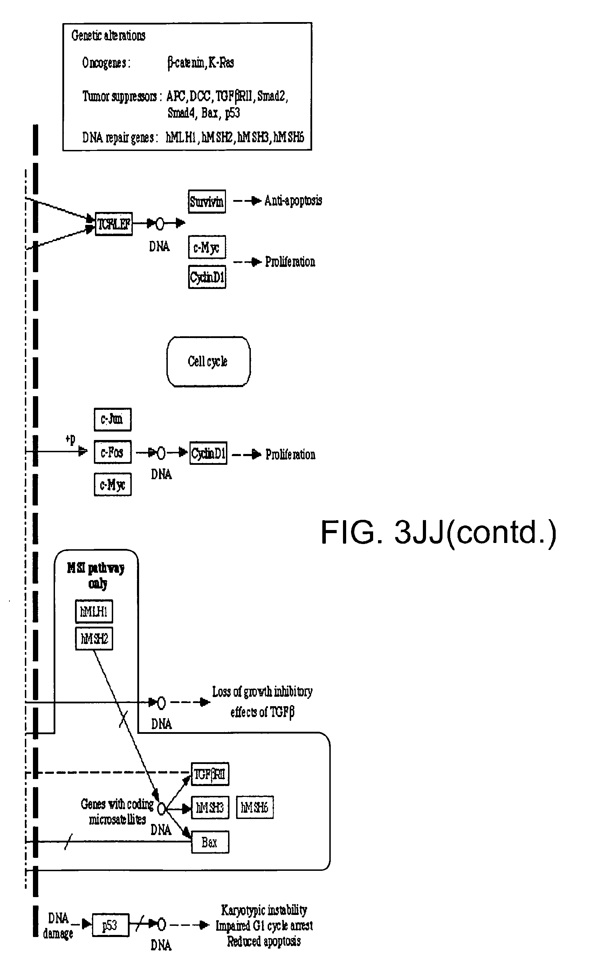

Figure 3JJ shows the interactions relating to colorectal cancer.

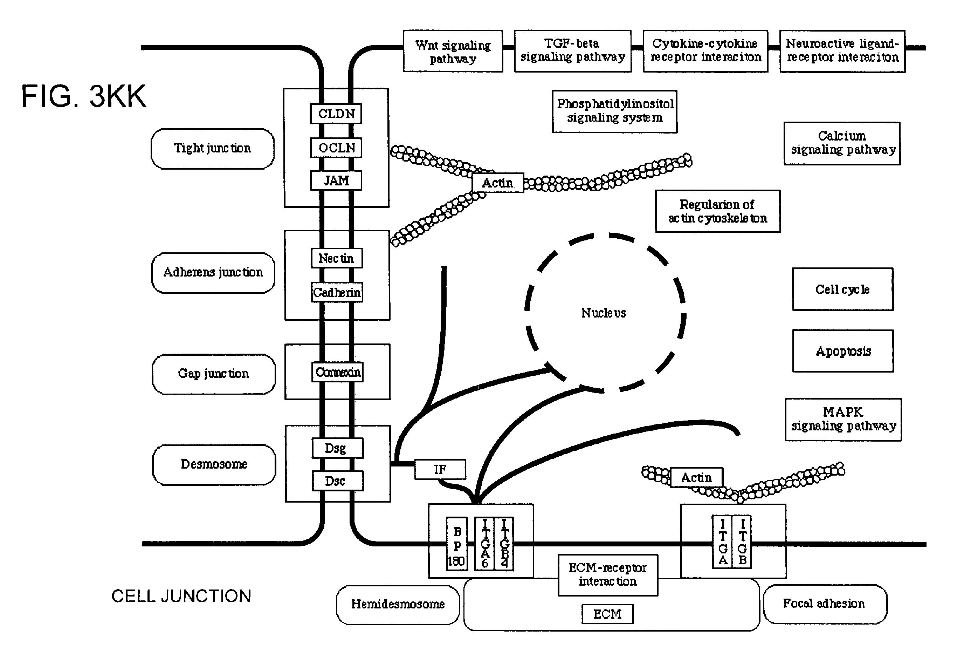

Figure 3KK shows the interactions at cell junctions.

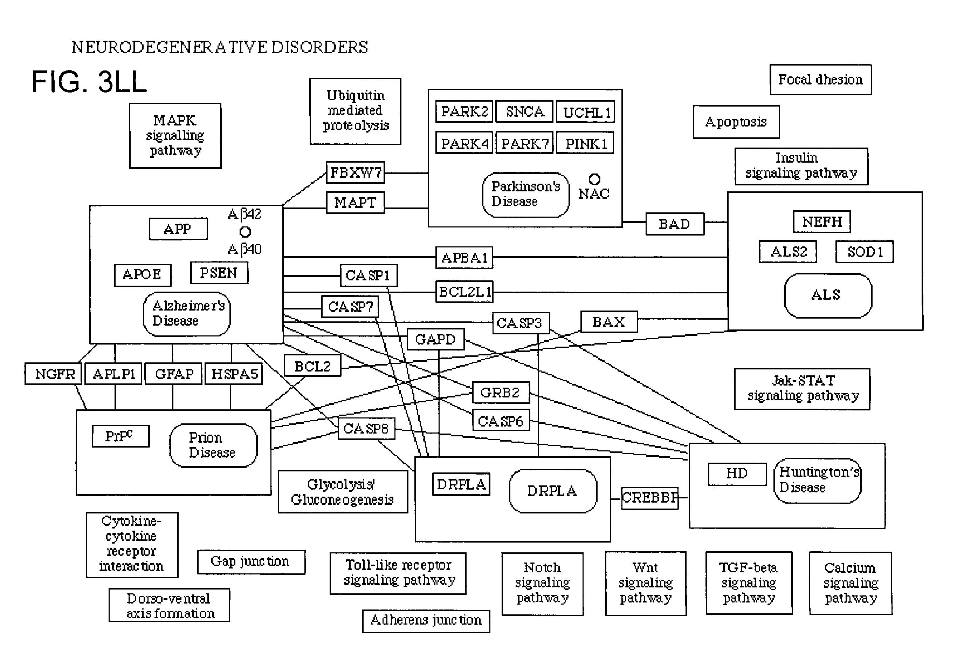

Figure 3LL is a schematic showing the pathways involved in neurodegenerative disorders.

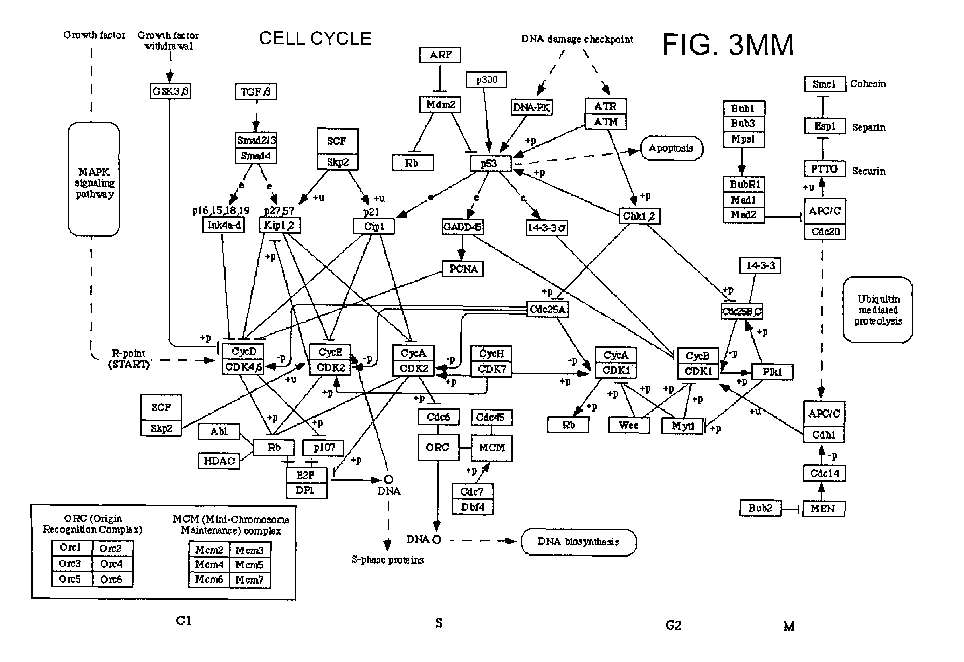

Figure 3MM depicts the cell cycle signaling pathway.

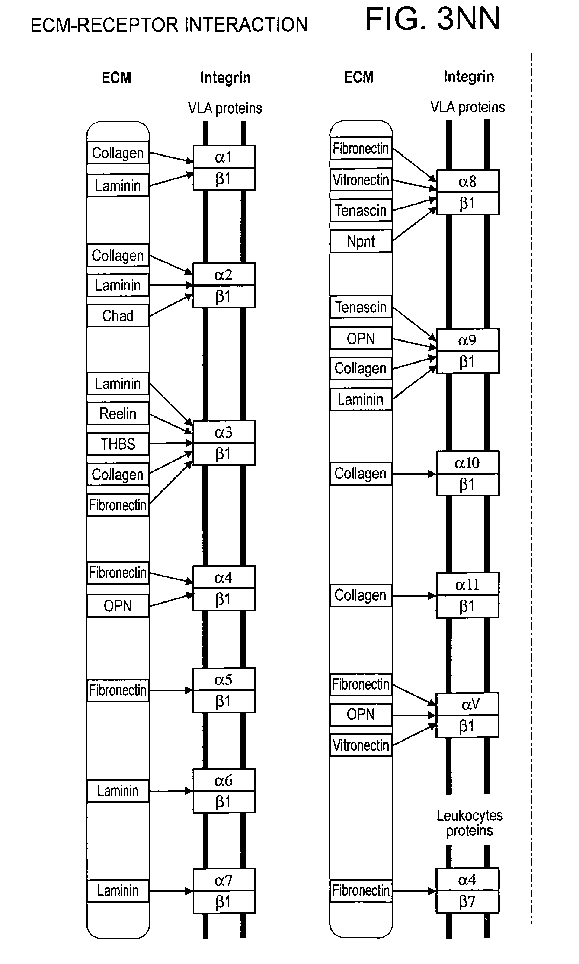

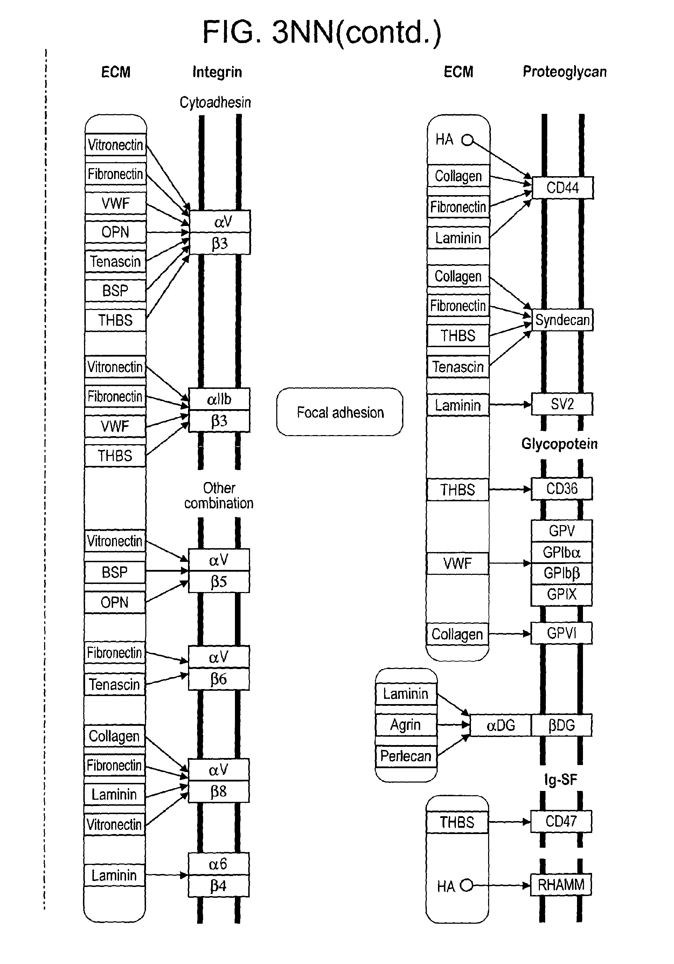

Figure 3NN shows ECM-receptor interactions.

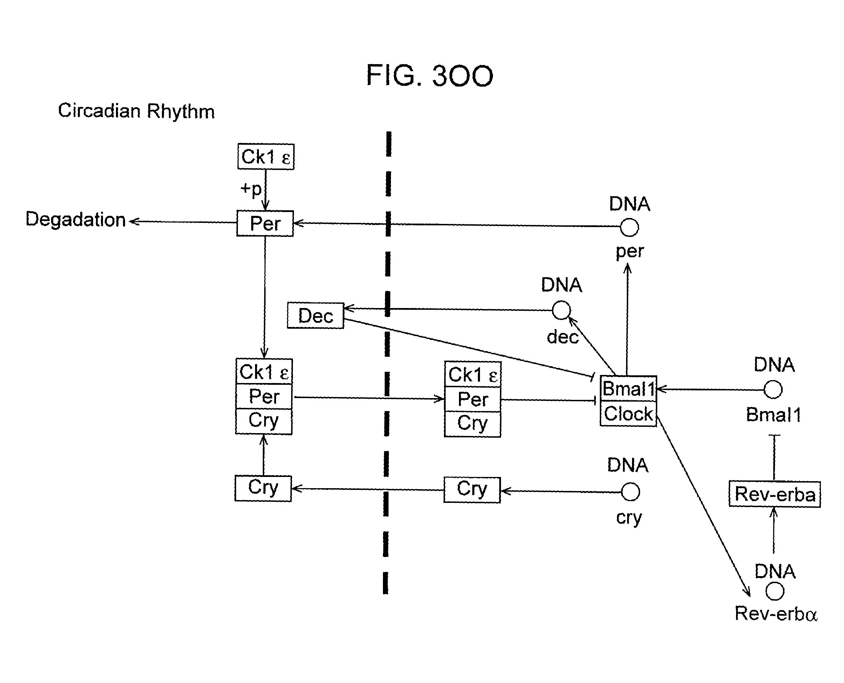

Figure 3OO shows the interactions involved in circadian rhythms.

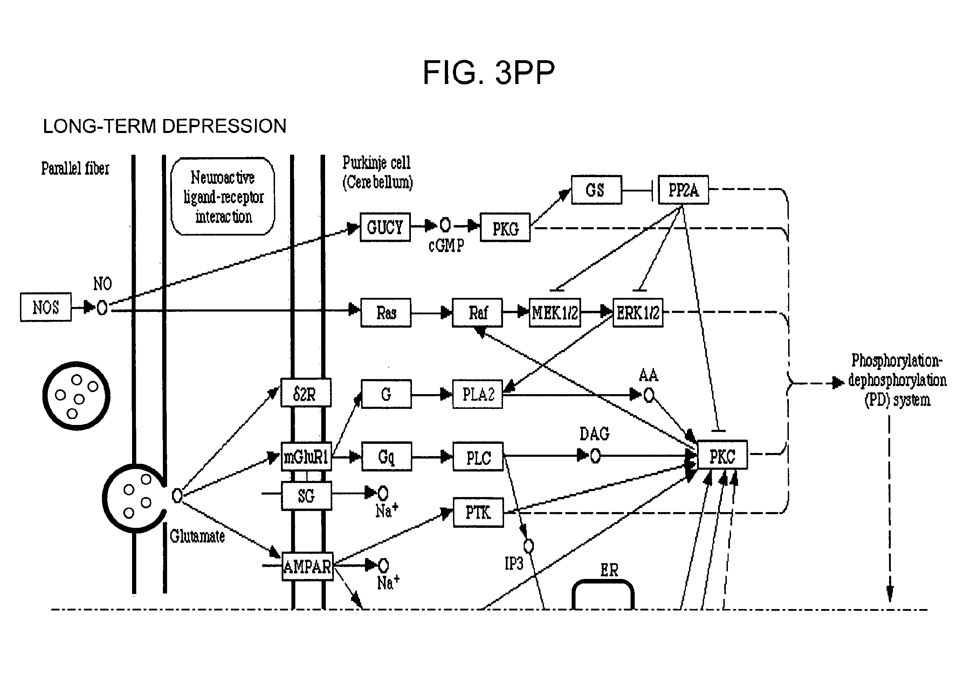

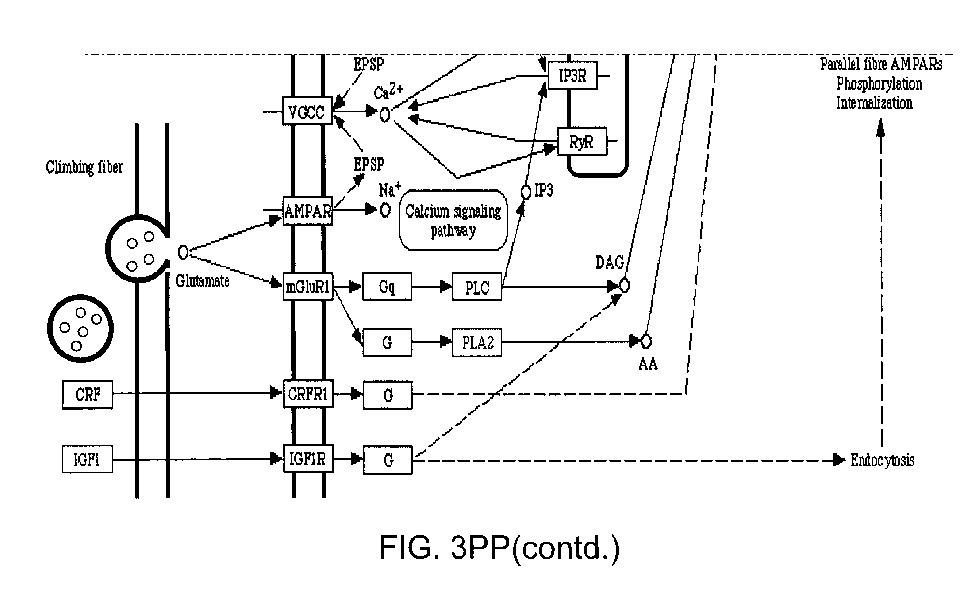

Figure 3PP is a schematic showing the interactions involved in long-term depression.

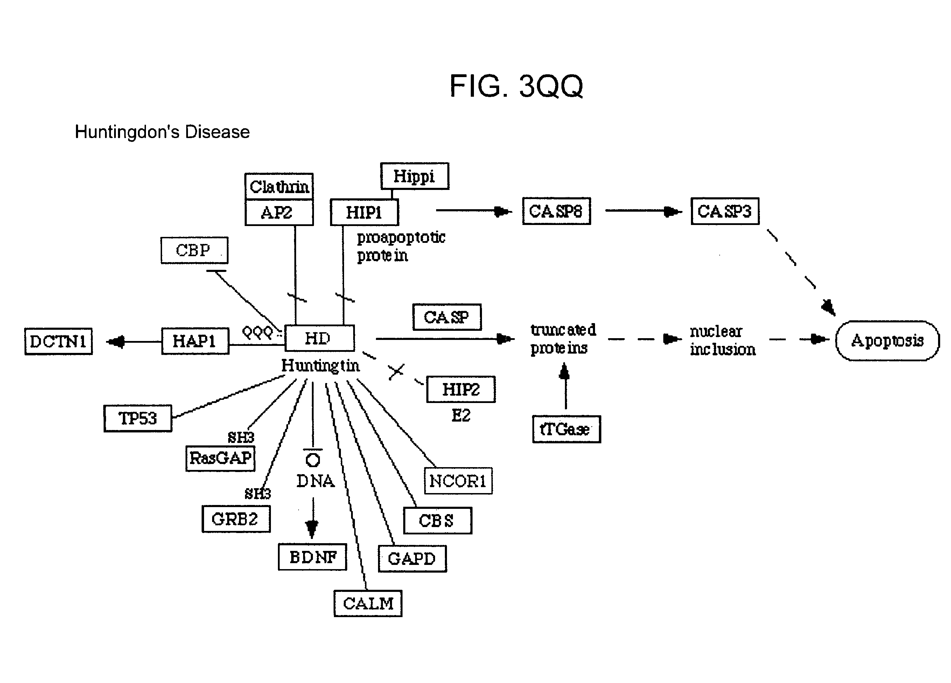

Figure 3QQ depicts the interactions relating to Huntington's Disease.

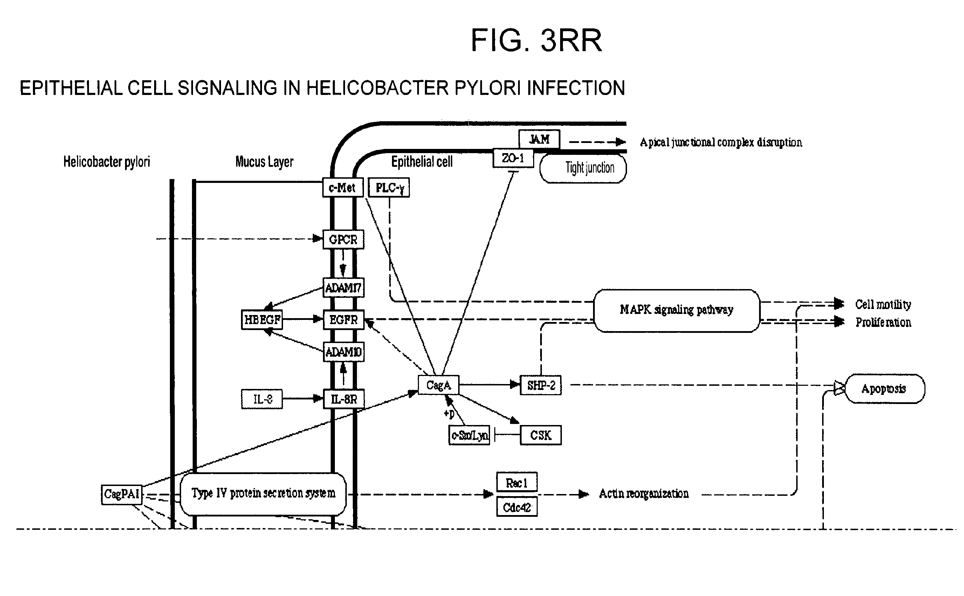

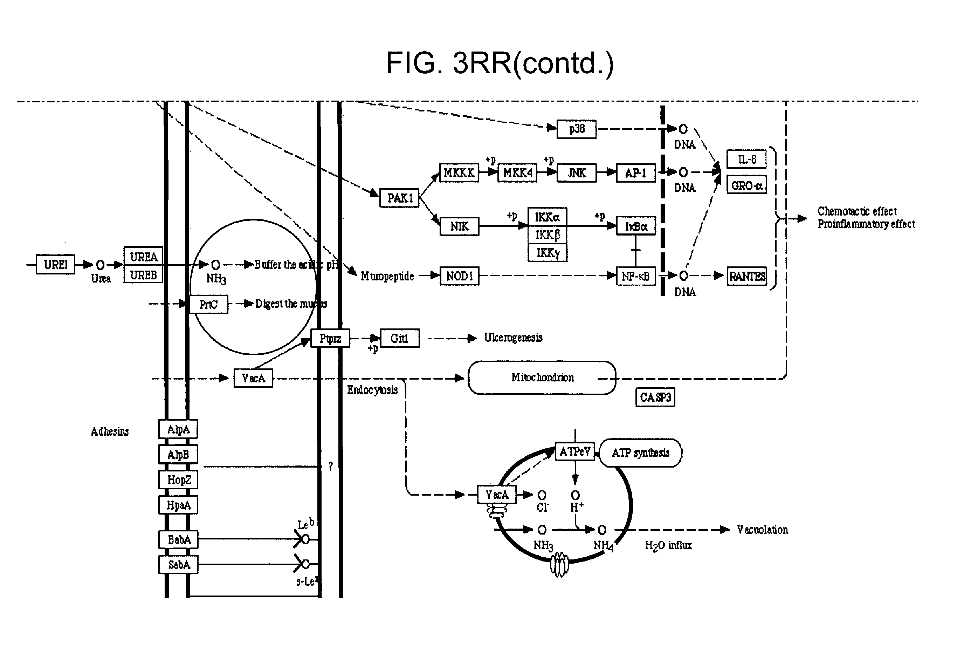

Figure 3RR shows the signaling pathways involved in *Helicobacter. pylori* infection.

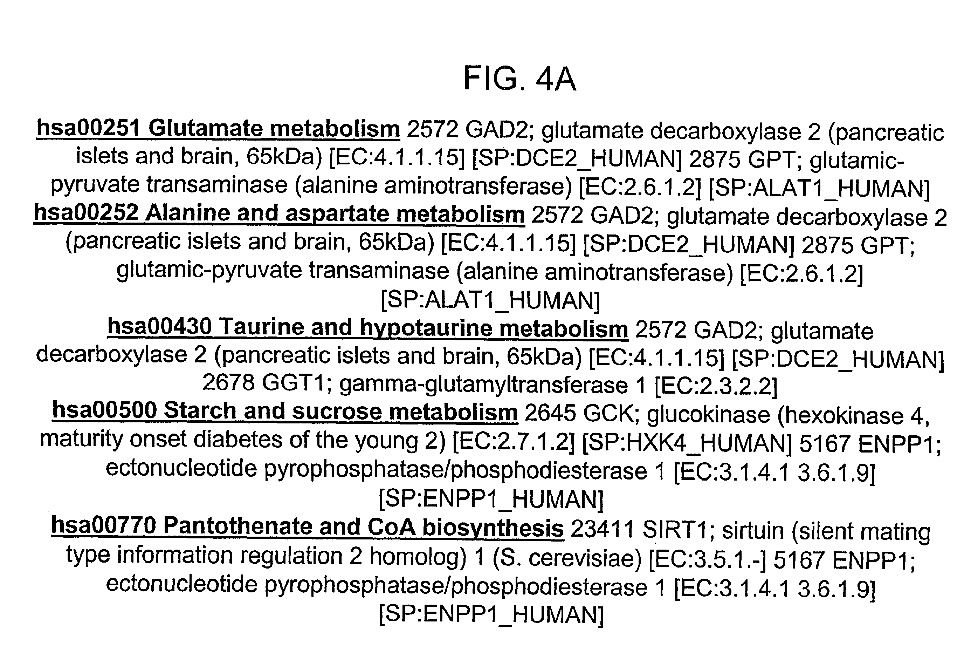

Figures 4A - 4F are listings of KEGG pathways with only one or two DBRISKMARKERS each within them.

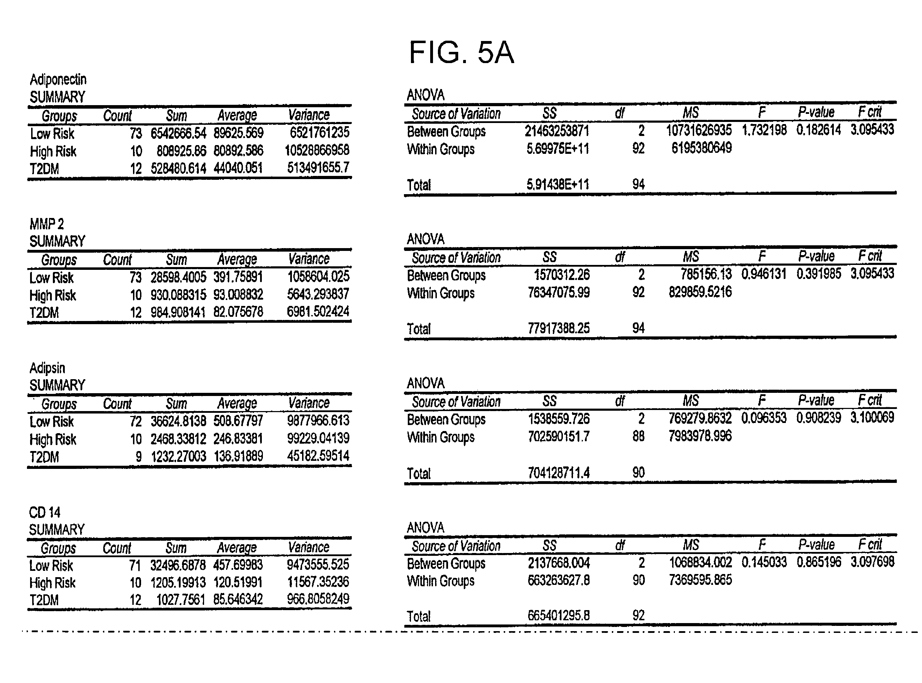

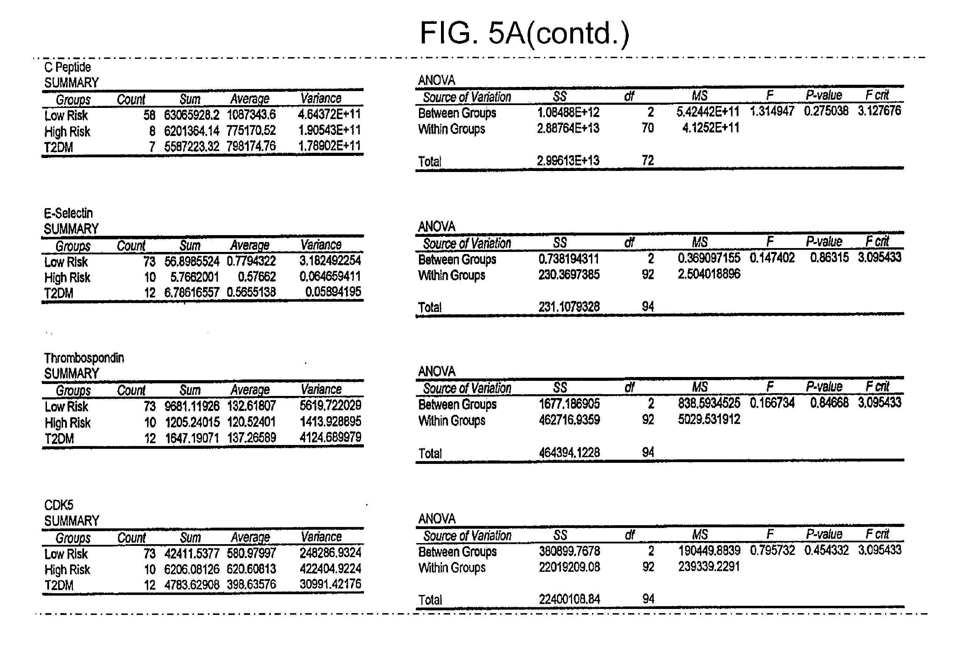

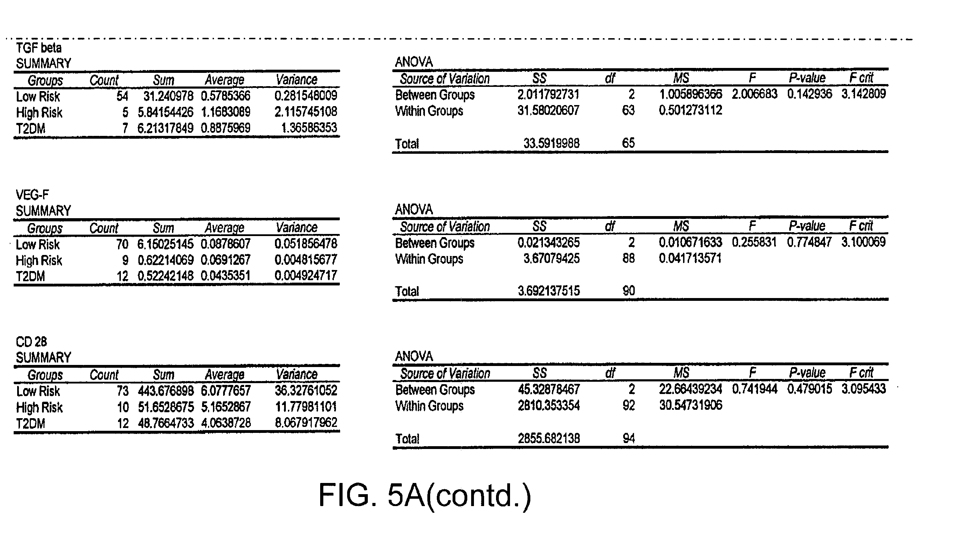

Figures 5A - 5E are examples of Pre-Diabetes classification performance characteristics of selected individual DBRISKMARKERS as shown in ANOVA analysis of said markers between patient samples from Normal, Pre-Diabetes, and Diabetes cohorts.

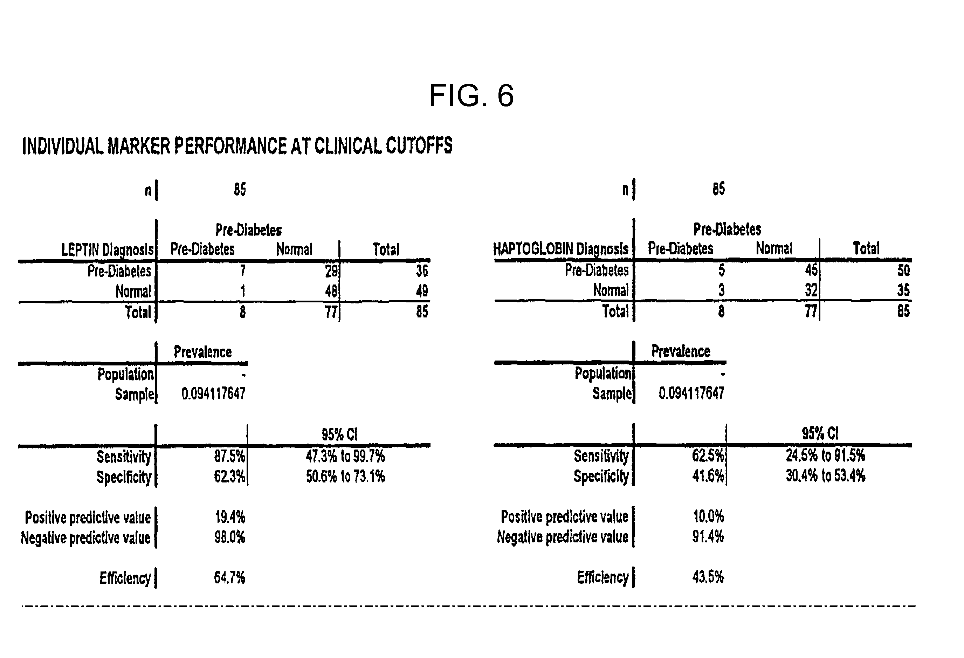

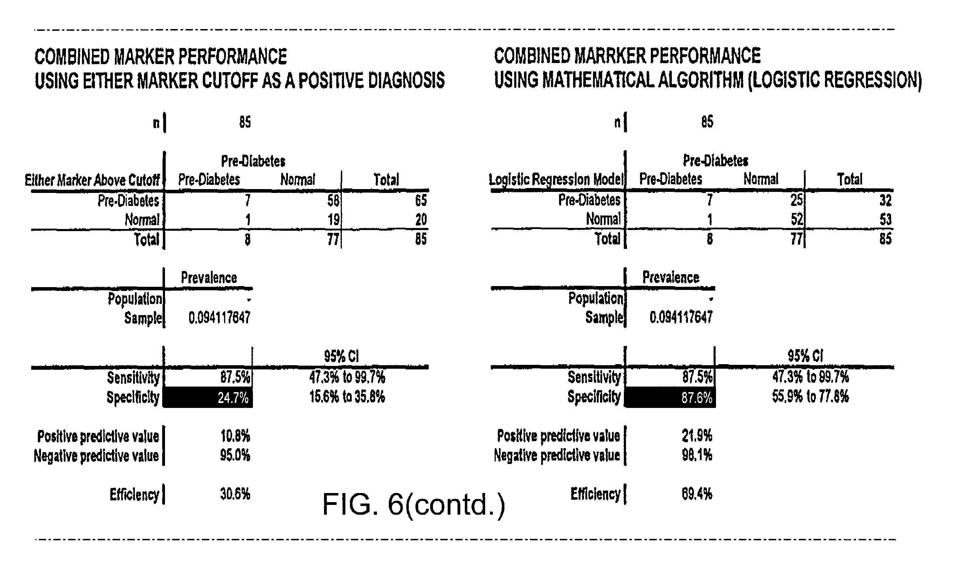

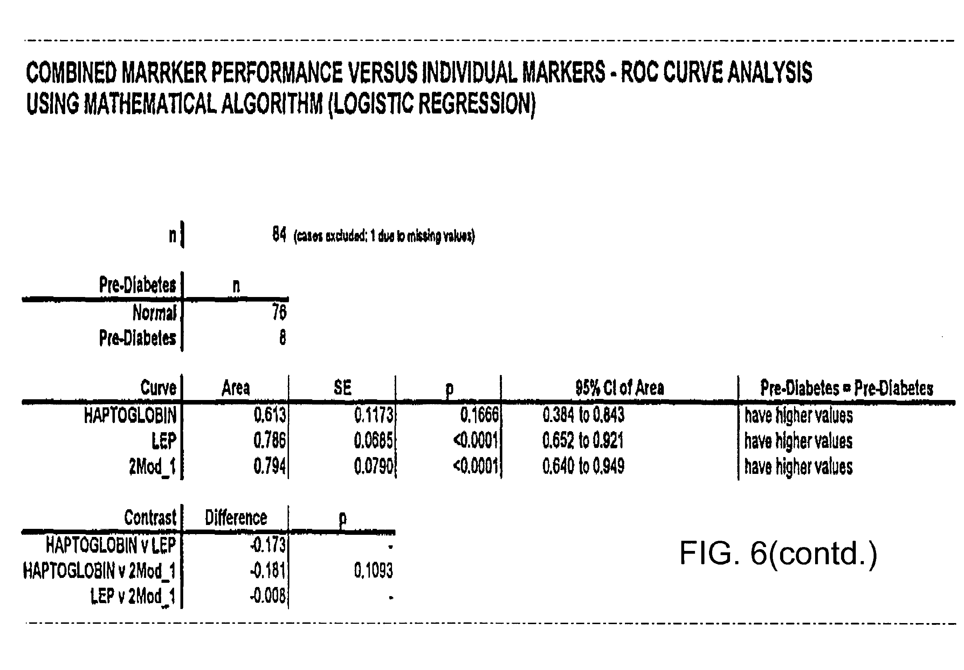

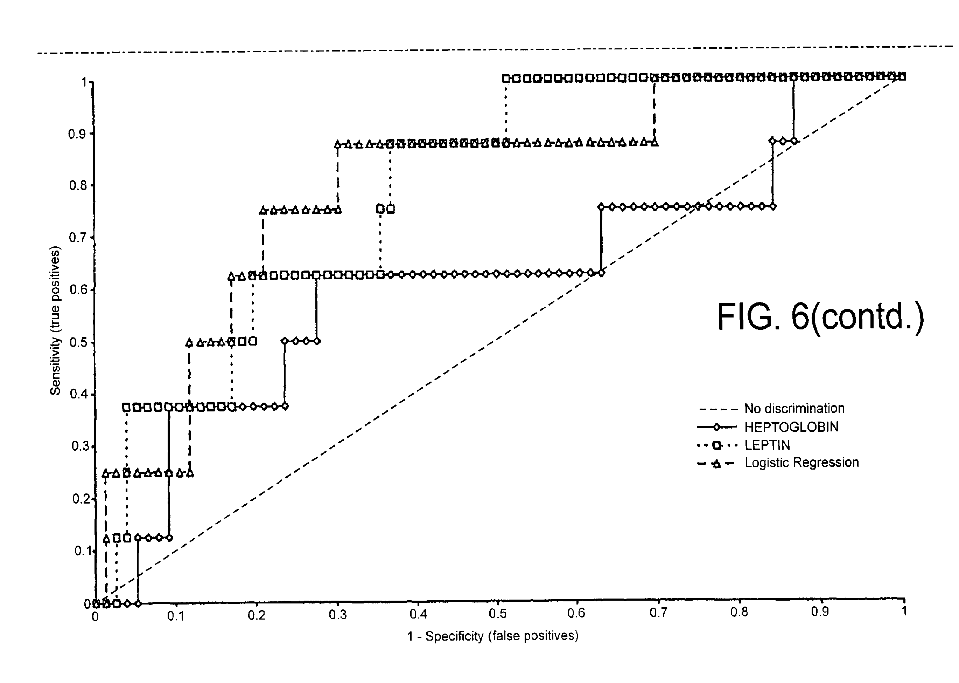

Figure 6 is a tabular example depicting the additive ROC performance characteristics of pairs of DBRISKMARKERS in classification of pre-Diabetes from normal cohorts absent a mathematical algorithm indicating the tradeoff of increased sensitivity at the cost of reduced specificity.

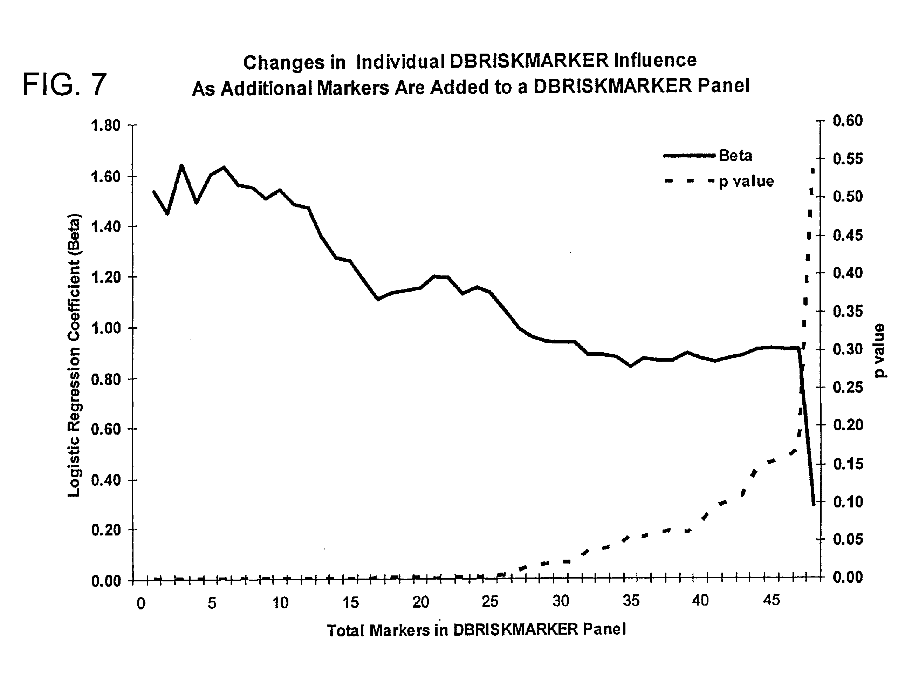

Figure 7 is a graph depicting the change in classification algorithm performance, as measured by $R^2$ versus the Reference Diabetes Conversion Risk with the addition of multiple DBRISDCRTRS utilizing a forward selection algorithm.

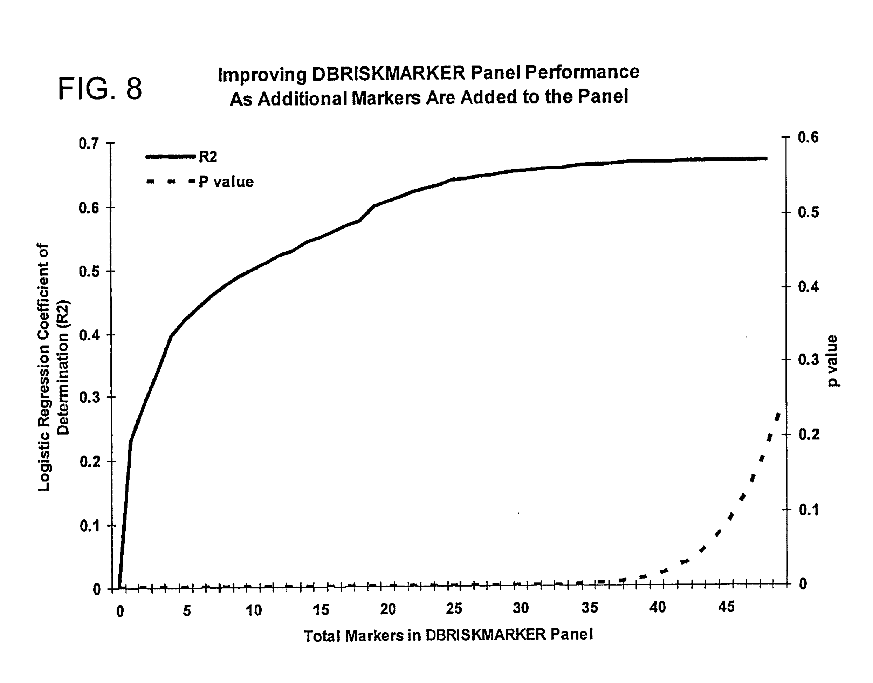

Figure 8 is a graph depicting a three-dimensional rendering of the performance characteristics of the entire set of possible three marker combinations of a group of 50 DBRISKMARKERS, highlighting the highest performing combinations.

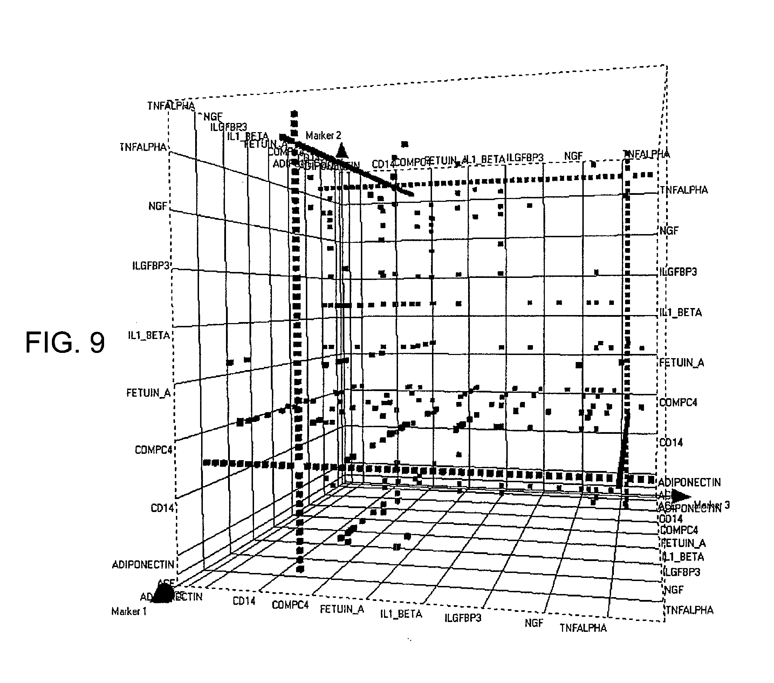

Figure 9 is a histogram depicting the distribution of the performance across the entire set of possible three marker combinations shown in Figure 6,

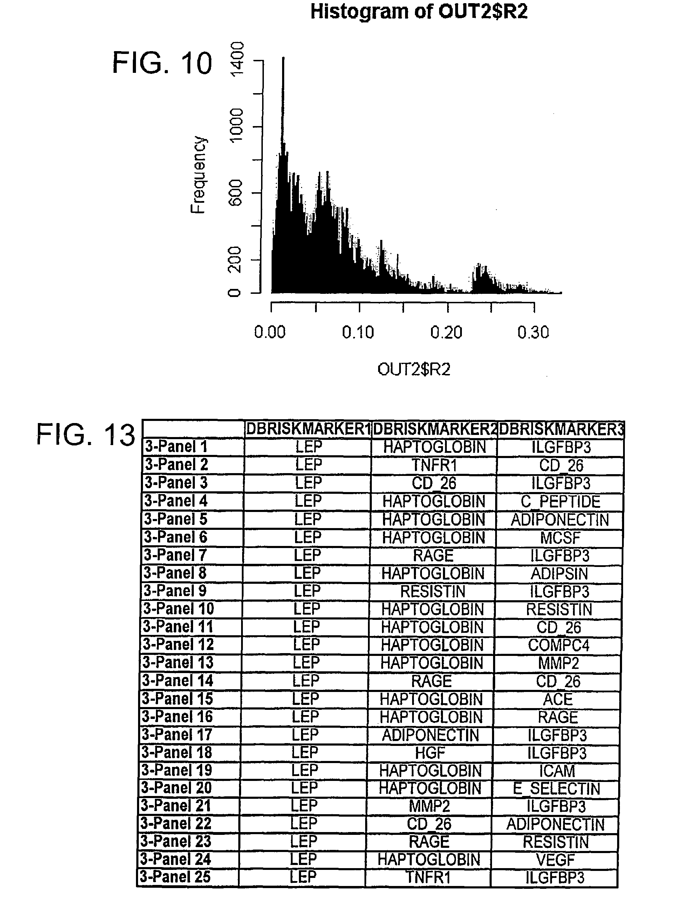

Figure 10 is a mathematical clustering and classification tree showing the Euclidean standardized distance the DBRISKMARKERS shown in Figure 6.

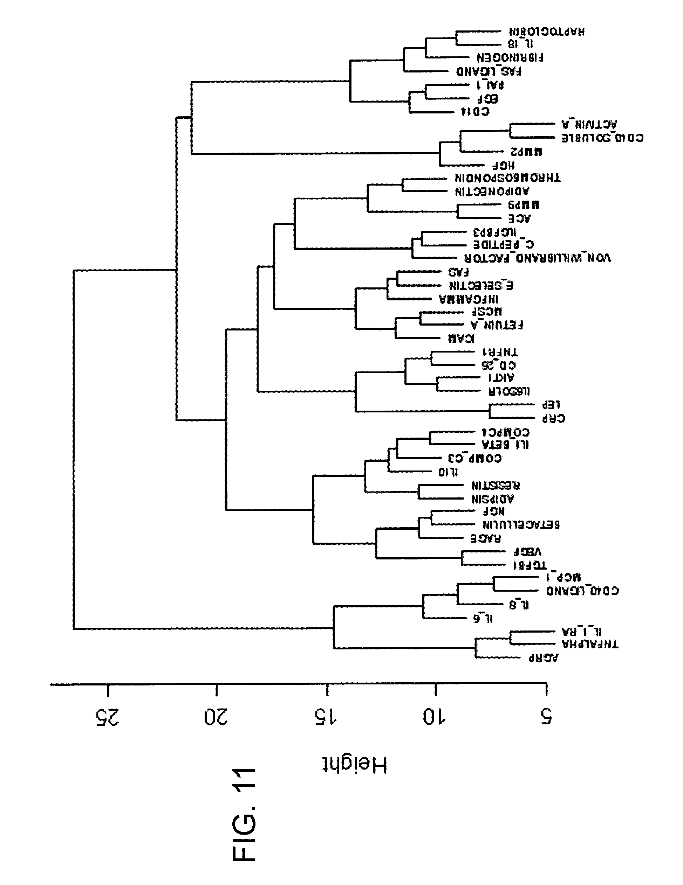

Figure 11 presents tables of selected DBRISKMARKERS by eight Position Categories useful for the construction of panels selecting DBRISKMARKERS according to the method disclosed herein.

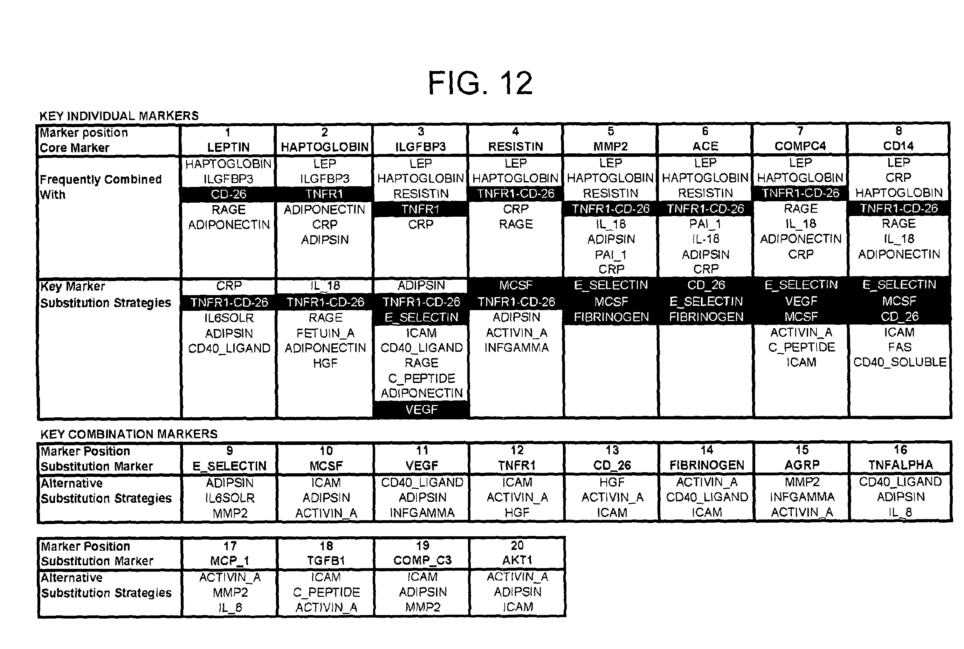

Figure 12 is a listing of 25 high performing DBRISKMARKER panels using three DBRISKMARKERS selected from Position Categories according to the method disclosed herein. Logistic regression algorithms using said panels had calculated R^2 values ranging from 0.300 to 0.329 when employed on samples in the described example and non-diabetic patient cohort.

Figure 13 is a listing of 25 high performing DBRISKMAKER panels using eight DBRISKMARKERS selected from Position Categories according to the method disclosed herein. Logistic regression algorithms using said panels had calculated R^2 values ranging from 0.310 to 0.475 when employed on samples in the described example and non-diabetic patient cohort.

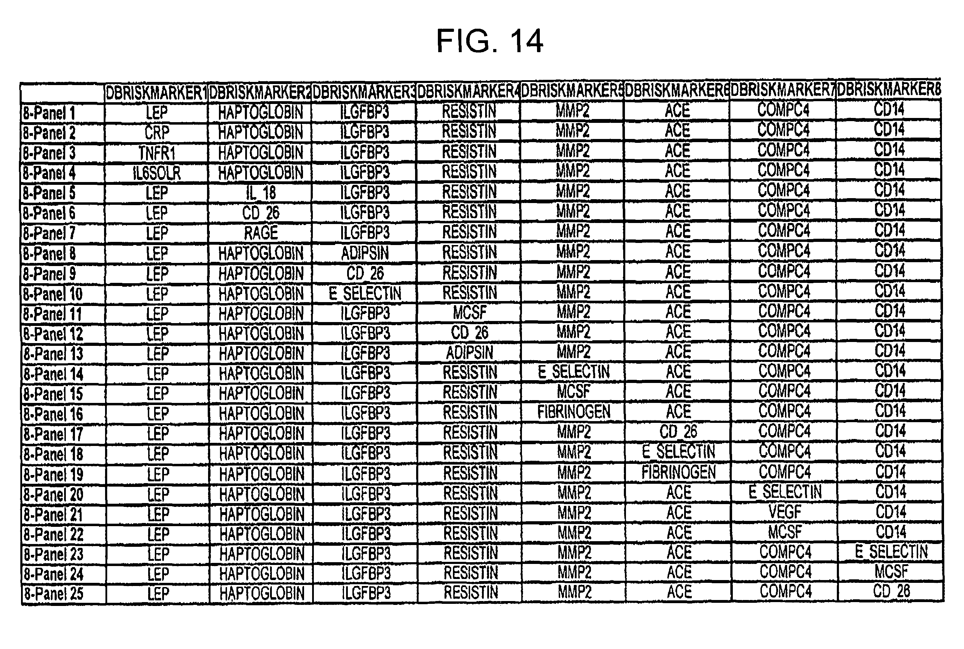

Figure 14 is a listing of 25 high performing DBRISKMAKER panels using eighteen DBRISKMARKERS selected from Position Categories according to the method disclosed herein. Logistic regression algorithms using said panels had calculated R^2 values ranging from 0.523 to 0.6105 when employed on samples in the described example and non-diabetic patient cohort.

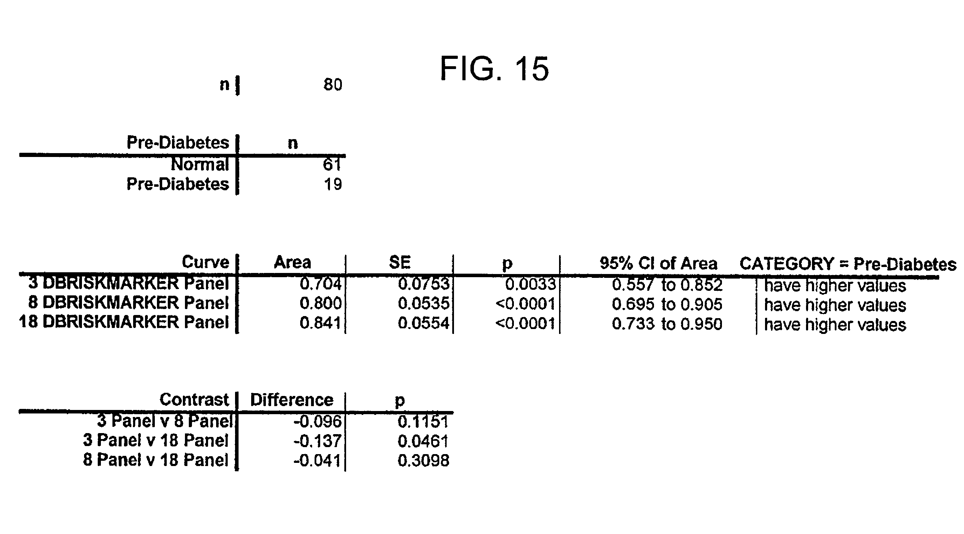

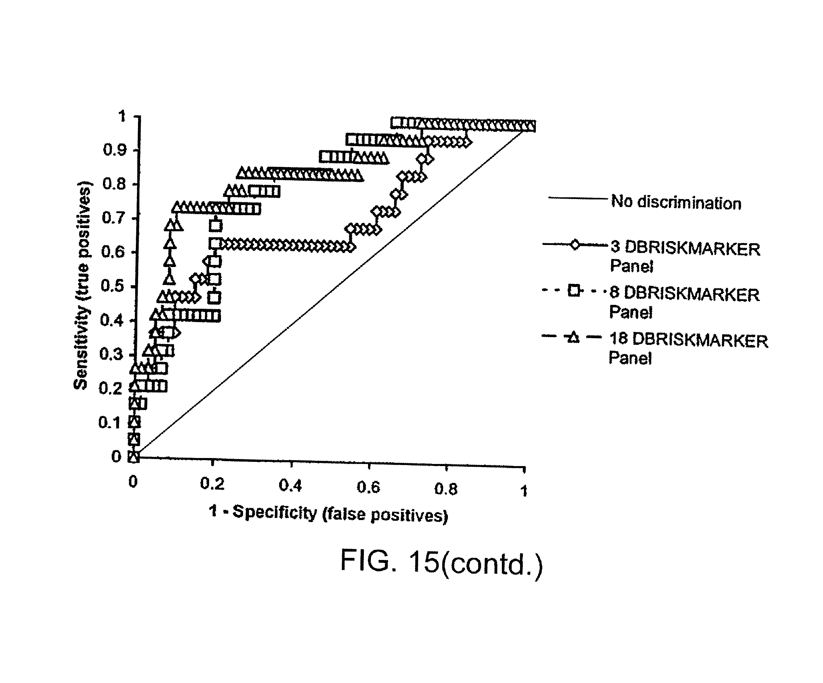

Figure 15 is a graph ROC curve and AUC statistics for the highest performing three, eight, and eighteen DBRISKMARKER panels respectively when employed on samples in the described example and non-diabetic patient cohort.

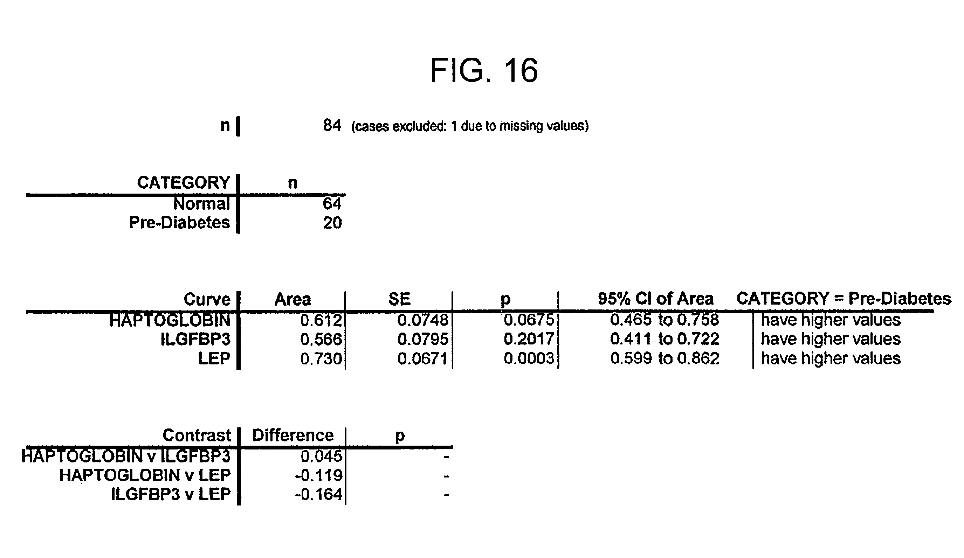

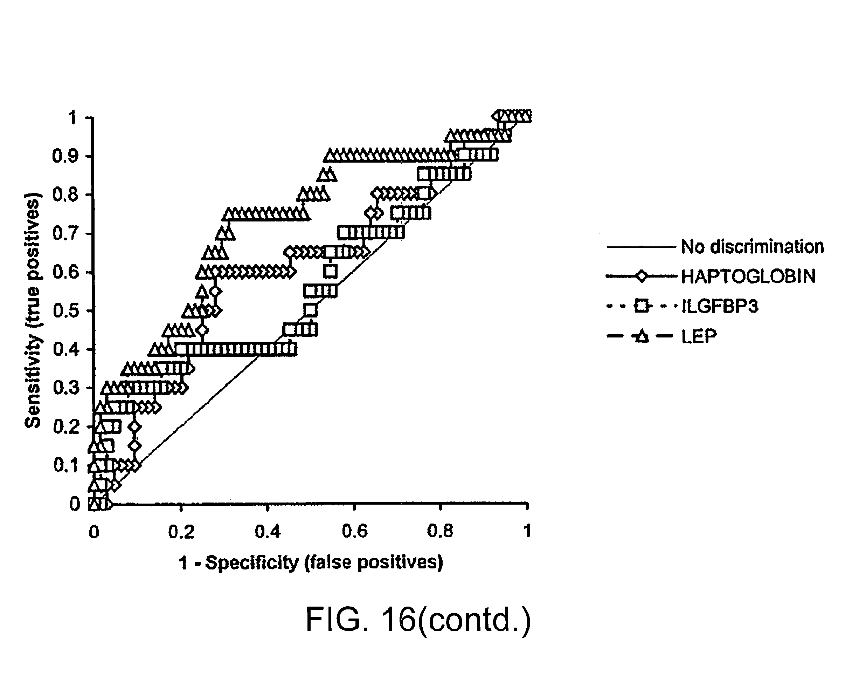

Figure 16 is an ROC curve and AUC statistics indicating the three relative highest performing individual DBRISKMARKERS markers when employed on samples in the described example and non-diabetic patient cohort.

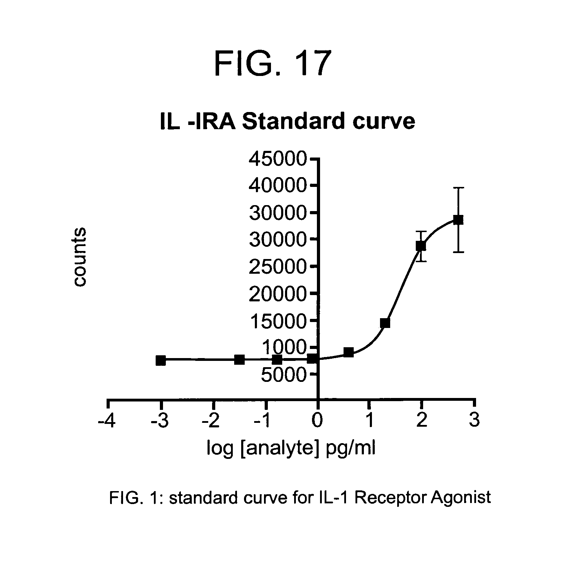

Figure 17 is a standard curve demonstrating a typical result from the methods of the present invention. Once a working standard curve is demonstrated, the assay is typically applied to 24 serum samples to determine the normal distribution of the target analyte across clinical samples.

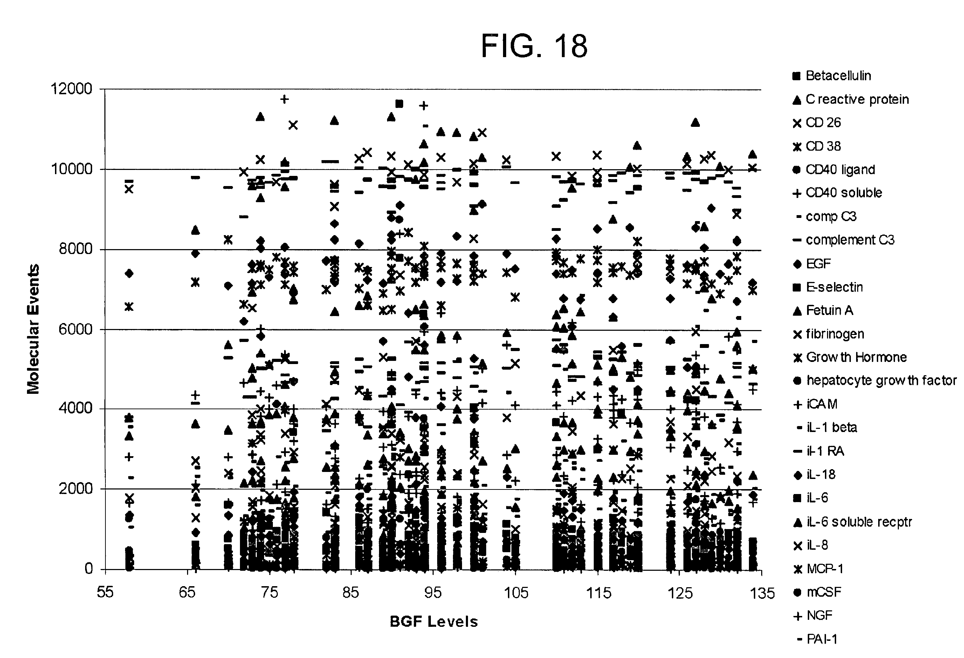

Figure 18 depicts a graph exemplifying single molecule detection data across 92 samples for 25 biomarkers.

## DETAILED DESCRIPTION OF THE INVENTION

The present invention relates to the identification of biomarkers associated with subjects having Diabetes or a pre-diabetic condition, or who are pre-disposed to developing Diabetes or a pre-diabetic condition. Accordingly, the present invention features methods for identifying subjects who are pre-disposed to developing Diabetes or a pre-diabetic condition, including those subjects who are asymptomatic for Diabetes or a pre-diabetic condition by detection of the biomarkers disclosed herein. These biomarkers are also useful for monitoring subjects undergoing treatments and therapies for Diabetes or pre-diabetic conditions, and for selecting therapies and treatments that would be efficacious in subjects having Diabetes or a pre-diabetic condition, wherein selection and use of such treatments and therapies slow the progression of Diabetes or pre-diabetic conditions, or substantially delay or prevent its onset.

"Diabetes Mellitus" in the context of the present invention encompasses Type 1 Diabetes, both autoimmune and idiopathic and Type 2 Diabetes (together, "Diabetes"). The World Health Organization defines the diagnostic value of fasting plasma glucose concentration to 7.0 mmol/l (126 mg/dl) and above for Diabetes Mellitus (whole blood 6.1 mmol/l or 110 mg/dl), or 2-hour glucose level ≥11.1 mmol/L (≥200 mg/dL). Other values suggestive of or indicating high risk for Diabetes Mellitus include elevated arterial pressure ≥ 140/90 mm Hg; elevated plasma triglycerides (≥1.7 mmol/L; 150 mg/dL) and/or low HDL-cholesterol (<0.9 mmol/L, 35 mg/dl for men; <1.0 mmol/L, 39 mg/dL women); central obesity (males: waist to hip ratio >0.90; females: waist to hip ratio > 0.85) and/or body mass index exceeding 30 kg/m$^2$; micro-albuminuria, where the urinary albumin excretion rate ≥20 µg/min or albumin:creatinine ratio ≥ 30 mg/g).

"Pre-diabetic condition" refers to a metabolic state that is intermediate between normal glucose homeostasis and metabolism and states seen in frank Diabetes Mellitus. Prediabetic conditions include, without limitation, Metabolic Syndrome ("Syndrome X"), Impaired Glucose Tolerance (IGT), and Impaired Fasting Glycemia (IFG). IGT refers to post-prandial abnormalities of glucose regulation, while IFG refers to abnormalities that are measured in a fasting state. The World Health Organization defines values for IFG as a fasting plasma glucose concentration of 6.1 mmol/L (100 mg/dL) or greater (whole blood 5.6 mmol/L; 100 mg/dL), but less than 7.0 mmol/L (126 mg/dL)(whole blood 6.1 mmol/L; 110 mg/dL). Metabolic syndrome according to the National Cholesterol Education Program (NCEP) criteria are defined as having at least three of the following: blood pressure ≥130/85 mm Hg; fasting plasma glucose ≥6.1 mmol/L; waist circumference >102 cm (men) or >88 cm (women); triglycerides ≥1.7 mmol/L; and HDL cholesterol <1.0 mmol/L (men) or 1.3 mmol/L (women).

"Pre-Diabetes" in the context of the present invention indicates the physiological state, in an individual or in a population, of having a higher than normal expected rate of disease conversion to frank Type 2 diabetes mellitus. Such absolute expected rate of conversion to frank Type 2 diabetes in Pre-Diabetes populations may be up to 1 percent or more per annum, and preferably 2 percent per annum or more. It may also be stated in terms of a relative risk from normal between quartiles of risk or as a likelihood ratio between differing biomarker and index scores, including those coming from the invention. Unless otherwise noted, and without limitation, when a categorical positive diagnosis of Pre-Diabetes is stated here, it is defined experimentally by the group of patients with an expected conversion rate to Type 2 Diabetes of two percent (2%) per annum over the coming 7.5 years, or fifteen percent (15%) of those testing at a given threshold value (the selected Pre-Diabetes clinical cutoff). When a continuous measure of Pre-Diabetes conversion risk is produced, having a "pre-diabetic condition" encompasses any expected annual rate of conversion above that seen in a normal reference or general unselected normal prevalence population.

"Impaired glucose tolerance" (IGT) is defined as having a blood glucose level that is higher than normal, but not high enough to be classified as Diabetes Mellitus. A subject with IGT will have two-hour glucose levels of 140 to 199 mg/dL (7.8 to 11.0 mmol) on the 75-g oral glucose tolerance test. These glucose levels are above normal but below the level that is diagnostic for Diabetes. Subjects with impaired glucose tolerance or impaired fasting glucose have a significant risk of developing Diabetes and thus are an important target group for primary prevention.

"Insulin resistance" refers to a condition in which the cells of the body become resistant to the effects of insulin, that is, the normal response to a given amount of insulin is reduced. As a result, higher levels of insulin are needed in order for insulin to exert its effects.

"Normal glucose levels" is used interchangeably with the term "normoglycemic" and refers to a fasting venous plasma glucose concentration of less than 6.1 mmol/L (110 mg/dL). Although this amount is arbitrary, such values have been observed in subjects with proven normal glucose tolerance, although some may have IGT as measured by oral glucose tolerance test (OGTT).

Two hundred and sixty biomarkers have been identified as being found to have altered or modified presence or concentration levels in subjects who have Diabetes, or who exhibit symptoms characteristic of a pre-diabetic condition, or have Pre-Diabetes (as defined herein) such as those subjects who are insulin resistant, have altered beta cell function or at risk of developing Diabetes based upon known clinical parameters or risk factors, such as family history of Diabetes, low activity level, poor diet, excess body weight (especially around the waist), age greater than 45 years, high blood pressure, high levels of triglycerides, HDL cholesterol of less than 35, previously identified impaired glucose tolerance, previous Diabetes during pregnancy ("gestational Diabetes Mellitus") or giving birth to a baby weighing more than nine pounds, and ethnicity.

The biomarkers and methods of the present invention allow one of skill in the art to identify, diagnose, or otherwise assess those subjects who do not exhibit any symptoms of Diabetes or a pre-diabetic condition, but who nonetheless may be at risk for developing Diabetes or experiencing symptoms characteristic of a pre-diabetic condition.

The term "biomarker" in the context of the present invention encompasses, without limitation, proteins, nucleic acids, polymorphisms of proteins and nucleic acids, elements, metabolites, and other analytes. Biomarkers can also include mutated proteins or mutated nucleic acids. The term "analyte" as used herein can mean any substance to be measured and can encompass electrolytes and elements, such as calcium. Finally, biomarkers can also refer to non-analyte physiological markers of health status encompassing other clinical characteristics such as, without limitation, age, ethnicity, diastolic and systolic blood pressure, body-mass index, and resting heart rate.

Proteins, nucleic acids, polymorphisms, and metabolites whose levels are changed in subjects who have Diabetes or a pre-diabetic condition, or are predisposed to developing Diabetes or a pre-diabetic condition are summarized in Table 1 and are collectively referred to herein as, *inter alia*, "Diabetes risk-associated proteins", "DBRISKMARKER polypeptides", or "DBRISKMARKER proteins". The corresponding nucleic acids encoding the polypeptides are referred to as "Diabetes risk-associated nucleic acids", "Diabetes risk-associated genes", "DBRISKMARKER nucleic acids", or "DBRISKMARKER genes". Unless indicated otherwise, "DBRISKMARKER", "Diabetes risk-associated proteins", "Diabetes risk-associated nucleic acids" are meant to refer to any of the sequences disclosed herein. The corresponding metabolites of the DBRISKMARKER proteins or nucleic acids can also be measured, as well as any of the aforementioned conventional risk marker metabolites previously disclosed, including, without limitation, such metabolites as dehydroepiandrosterone sulfate (DHEAS); c-peptide; cortisol; vitamin D3; 5-hydroxytryptamine (5-HT; serotonin); oxyntomodulin; estrogen; estradiol; and digitalis-like factor, herein referred to as "DBRISKMARKER metabolites". Non-analyte physiological markers of health status (e.g., such as age, ethnicity, diastolic or systolic blood pressure, body-mass index, and other non-analyte measurements commonly used as conventional risk factors) are referred to as "DBRISKMARKER physiology". Calculated indices created from mathematically combining measurements of one or more, preferably two or more of the aforementioned classes of DBRISKMARKERS are referred to as "DBRISKMARKER indices". Proteins, nucleic acids, polymorphisms, mutated proteins and mutated nucleic acids, metabolites, and other analytes are, as well as common physiological measurements and indices constructed from any of the preceding entities, are included in the broad category of "DBRISKMARKERS".

A "subject" in the context of the present invention is preferably a mammal. The mammal can be a human, non-human primate, mouse, rat, dog, cat, horse, or cow, but are not limited to these examples. Mammals other than humans can be advantageously used as subjects that represent animal models of Diabetes Mellitus or pre-Diabetes conditions. A subject can be male or female. A subject can be one who has been previously diagnosed or identified as having Diabetes or a pre-diabetic condition, and optionally has already undergone treatment for the Diabetes or pre-diabetic condition. Alternatively, a subject can also be one who has not been previously diagnosed as having Diabetes or a pre-diabetic condition. For example, a subject can be one who exhibits one or more risk factors for Diabetes or a pre-diabetic condition, or a subject who does not exhibit Diabetes risk factors, or a subject who is asymptomatic for Diabetes or pre-Diabetes. A subject can also be one who is suffering from or at risk of developing Diabetes or a pre-diabetic condition.

A "sample" in the context of the present invention is a biological sample isolated from a subject and can include, for example, serum, blood plasma, blood cells, endothelial cells, tissue biopsies, lymphatic fluid, ascites fluid, interstitital fluid (also known as "extracellular fluid" and encompasses the fluid found in spaces between cells, including, *inter alia,* gingival crevicular fluid), bone marrow, sputum, or urine.

One or more, preferably two or more DBRISKMARKERS can be detected in the practice of the present invention. For example, two (2), five (5), ten (10), fifteen (15), twenty (20), forty (40), fifty (50), seventy-five (75), one hundred (100), one hundred and twenty five (125), one hundred and fifty (150), one hundred and seventy-five (175), two hundred (200), two hundred and ten (210), two hundred and twenty (220), two hundred and thirty (230), two hundred and forty (240), two hundred and fifty (250) or more DBRISKMARKERS can be detected. In some aspects, all 260 DBRISKMARKERS disclosed herein can be detected. Preferred ranges from which the number of DBRISKMARKERS can be detected include ranges bounded by any minimum selected from between one and 260, particularly two, five, ten, twenty, fifty, seventy-five, one hundred, one hundred and twenty five, one hundred and fifty, one hundred and seventy-five, two hundred, two hundred and ten, two hundred and twenty, two hundred and thirty, two hundred and forty, two hundred and fifty, paired with any maximum up to the total known DBRISKMARKERS, particularly five, ten, twenty, fifty, and seventy-five. Particularly preferred ranges include two to five (2-5), two to ten (2-10), two to fifty (2-50), two to seventy-five (2-75), two to one hundred (2-100), five to ten (5-10), five to twenty (5-20), five to fifty (5-50), five to seventy-five (5-75), five to one hundred (5-100), ten to twenty (10-20), ten to fifty (10-50), ten to seventy-five (10-75), ten to one hundred (10-100), twenty to fifty (20-50), twenty to seventy-five (20-75), twenty to one hundred (20-100), fifty to seventy-five (50-75), fifty to one hundred (50-100), one hundred to one hundred and twenty-five (100-125), one hundred and twenty-five to one hundred and fifty (125-150), one hundred and fifty to one hundred and seventy five (150-175), one hundred and seventy-five to two hundred (175-200), two hundred to two hundred and ten (200-210), two hundred and ten to two hundred and twenty (210-220), two hundred and twenty to two hundred and thirty (220-230), two hundred and thirty to two hundred and forty (230- 240), two hundred and forty to two hundred and fifty (240-250), and two hundred and fifty to more than two hundred and fifty (250+).

**Diagnostic and Prognostic Methods**

The risk of developing Diabetes or Pre-Diabetes can be detected with a "predetermined level of predictability" by examining an "effective amount" of DBRISKMARKER proteins, nucleic acids, polymorphisms, metabolites, and other analytes in a test sample (e.g., a subject derived sample) and comparing the effective amounts to reference or index values, often utilizing mathematical algorithms in order to combine information from results of multiple individual DBRISKMARKERS into a single measurement or index. Subjects identified as having an increased risk of Diabetes or a prediabetic condition can optionally be selected to receive treatment regimens, such as administration of prophylactic or therapeutic compounds such as "diabetes-modulating drugs" as defined herein, or implementation of exercise regimens or dietary supplements to prevent or delay the onset of Diabetes or Pre-Diabetes. A sample isolated from the subject can comprise, for example, blood, plasma, blood cells, endothelial cells, tissue biopsies, lymphatic fluid, serum, bone marrow, ascites fluid, interstitial fluid (including, for example, gingival crevicular fluid), urine, sputum, or other bodily fluids.

The amount of the DBRISKMARKER protein, nucleic acid, polymorphism, metabolite, or other analyte can be measured in a test sample and compared to the normal control level. The term "normal control level", means the level of a DBRISKMARKER protein, nucleic acid, polymorphism, metabolite, or other analyte, or DBRISKMARKER physiology or indices, typically found in a subject not suffering from Diabetes or a prediabetic condition and not likely to have Diabetes or a pre-diabetic condition, e.g., relative to samples collected from young subjects who were monitored until advanced age and were found not to develop Diabetes or a pre-diabetic condition. Alternatively, the normal control level can mean the level of a DBRISKMARKER protein, nucleic acid, polymorphism, metabolite, or other analyte typically found in a subject suffering from Diabetes or a prediabetic condition. The normal control level can be a range or an index. Alternatively, the normal control level can be a database of patterns from previously tested subjects. A change in the level in the subject-derived sample of a DBRISKMARKER protein, nucleic acid, polymorphism, metabolite, or other analyte compared to the normal control level can indicate that the subject is suffering from or is at risk of developing Diabetes or a pre-diabetic condition. In contrast, when the methods are applied prophylactically, a similar level compared to the normal control level in the subject-derived sample of a DBRISKMARKER protein, nucleic acid, polymorphism, metabolite, or other analyte can indicate that the subject is not suffering from, is not at risk or is at low risk of developing Diabetes or a pre-diabetic condition.

The difference in the level of DBRISKMARKERS is preferably statistically significant. By "statistically significant", it is meant that the alteration is greater than what might be expected to happen by chance alone. Statistical significance can be determined by any method known in the art. For example, statistical significance can be determined by $p$-value. The $p$-value is a measure of probability that a difference between groups during an experiment happened by chance. (P(z>zobserved)). For example, a $p$-value of 0.01 means that there is a 1 in 100 chance the result occurred by chance. The lower the $p$-value, the more likely it is that the difference between groups was caused by treatment. An alteration is statistically significant if the $p$-value is at least 0.05. Preferably, the $p$-value is 0.04, 0.03, 0.02, 0.01, 0.005, 0.001 or less. As noted below, and without any limitation of the invention, achieving statistical significance generally but not always requires that combinations of several DBRISKMARKERS be used together in panels and combined with mathematical algorithms in order to achieve a statistically significant DBRISKMARKER index.

The "diagnostic accuracy" of a test, assay, or method concerns the ability of the test, assay, or method to distinguish between subjects having Diabetes or a pre-diabetic condition, or at risk for Diabetes or a pre-diabetic condition is based on whether the subjects have a "clinically significant presence" or a "clinically significant alteration" in the levels of a DBRISKMARKER. By "clinically significant presence" or "clinically significant alteration", it is meant that the presence of the DBRISKMARKER (e.g., mass, such as milligrams, nanograms, or mass per volume, such as milligrams per deciliter or copy number of a transcript per unit volume) or an alteration in the presence of the DBRISKMARKER in the subject (typically in a sample from the subject) is higher than the predetermined cut-off point (or threshold value) for that DBRISKMARKER and therefore indicates that the subject has Diabetes or a pre-diabetic condition for which the sufficiently high presence of that protein, nucleic acid, polymorphism, metabolite or analyte is a marker.

The present invention may be used to make categorical or continuous measurements of the risk of conversion to Type 2 Diabetes, thus diagnosing a category of subjects defined as Pre-Diabetic.

In the categorical scenario, the methods of the present invention can be used to discriminate between Normal and Pre-Diabetes subject cohorts. In this categorical use of the invention, the terms "high degree of diagnostic accuracy" and "very high degree of diagnostic accuracy" refer to the test or assay for that DBRISKMARKER (or DBRISKMARKER index; wherein DBRISKMARKER value encompasses any individual measurement whether from a single DBRISKMARKER or derived from an index of DBRISKMARKERS) with the predetermined cut-off point correctly (accurately) indicating the presence or absence of Pre-Diabetes. A perfect test would have perfect accuracy. Thus, for subjects who have Pre-Diabetes, the test would indicate only positive test results and would not report any of those subjects as being "negative" (there would be no "false negatives"). In other words, the "sensitivity" of the test (the true positive rate) would be 100%. On the other hand, for subjects who did not have Pre-Diabetes, the test would indicate only negative test results and would not report any of those subjects as being "positive" (there would be no "false positives"). In other words, the "specificity" (the true negative rate) would be 100%. See, e.g., O'Marcaigh AS, Jacobson RM, "Estimating The Predictive Value Of A Diagnostic Test, How To Prevent Misleading Or Confusing Results," Clin. Ped. 1993, 32(8): 485-491, which discusses specificity, sensitivity, and positive and negative predictive values of a test, e.g., a clinical diagnostic test. In other embodiments, the present invention may be used so as to discriminate Pre-Diabetes from Diabetes, or Diabetes from Normal. Such use may require a different DBRISKMARKER panel, mathematical algorithm, and/or cut-off point, but be subject to the same aforementioned measurements of diagnostic accuracy for the intended use.

In the categorical diagnosis of a disease, changing the cut point or threshold value of a test (or assay) usually changes the sensitivity and specificity, but in a qualitatively inverse relationship. For example, if the cut point is lowered, more subjects in the population tested will typically have test results over the cut point or threshold value. Because subjects who have test results above the cut point are reported as having the disease, condition, or syndrome for which the test is conducted, lowering the cut point will cause more subjects to be reported as having positive results (e.g., that they have Diabetes, Pre-Diabetes, or a prediabetic condition). Thus, a higher proportion of those who have Diabetes or Pre-Diabetes will be indicated by the test to have it. Accordingly, the sensitivity (true positive rate) of the test will be increased. However, at the same time, there will be more false positives because more people who do not have the disease, condition, or syndrome (e.g., people who are truly "negative") will be indicated by the test to have DBRISKMARKER values above the cut point and therefore to be reported as positive (e.g., to have the disease, condition, or syndrome) rather than being correctly indicated by the test to be negative. Accordingly, the specificity (true negative rate) of the test will be decreased. Similarly, raising the cut point will tend to decrease the sensitivity and increase the specificity. Therefore, in assessing the accuracy and usefulness of a proposed medical test, assay, or method for assessing a subject's condition, one should always take both sensitivity and specificity into account and be mindful of what the cut point is at which the sensitivity and specificity are being reported because sensitivity and specificity may vary significantly over the range of cut points.

There is, however, an indicator that allows representation of the sensitivity and specificity of a test, assay, or method over the entire range of test (or assay) cut points with just a single value. That indicator is derived from a Receiver Operating Characteristics ("ROC") curve for the test, assay, or method in question. See, e.g., Shultz, "Clinical Interpretation Of Laboratory Procedures," chapter 14 in Teitz, Fundamentals of Clinical Chemistry, Burtis and Ashwood (eds.), 4th edition 1996, W.B. Saunders Company, pages 192-199; and Zweig et al., "ROC Curve Analysis: An Example Showing The Relationships Among Serum Lipid And Apolipoprotein Concentrations In Identifying Subjects With Coronory Artery Disease," Clin. Chem., 1992, 38(8): 1425-1428.

An ROC curve is an x-y plot of sensitivity on the y-axis, on a scale of zero to one (e.g., 100%), against a value equal to one minus specificity on the x-axis, on a scale of zero to one (e.g., 100%). In other words, it is a plot of the true positive rate against the false positive rate for that test, assay, or method. To construct the ROC curve for the test, assay, or method in question, subjects can be assessed using a perfectly accurate or "gold standard" method that is independent of the test, assay, or method in question to determine whether the subjects are truly positive or negative for the disease, condition, or syndrome (for example, coronary angiography is a gold standard test for the presence of coronary atherosclerosis). The subjects can also be tested using the test, assay, or method in question, and for varying cut points, the subjects are reported as being positive or negative according to the test, assay, or method. The sensitivity (true positive rate) and the value equal to one minus the specificity (which value equals the false positive rate) are determined for each cut point, and each pair of x-y values is plotted as a single point on the x-y diagram. The "curve" connecting those points is the ROC curve.

The ROC curve is often used in order to determine the optimal single clinical cut-off or treatment threshold value where sensitivity and specificity are maximized; such a situation represents the point on the ROC curve which describes the upper left corner of the single largest rectangle which can be drawn under the curve.

The total area under the curve ("AUC") is the indicator that allows representation of the sensitivity and specificity of a test, assay, or method over the entire range of cut points with just a single value. The maximum AUC is one (a perfect test) and the minimum area is one half (e.g. the area where there is no discrimination of normal versus disease). The closer the AUC is to one, the better is the accuracy of the test. It should be noted that implicit in all ROC and AUC is the definition of the disease and the post-test time horizon of interest.

By a "high degree of diagnostic accuracy", it is meant a test or assay (such as the test of the invention for determining the clinically significant presence of DBRISKMARKERS, which thereby indicates the presence of Diabetes or a pre-diabetic condition) in which the AUC (area under the ROC curve for the test or assay) is at least 0.70, desirably at least 0.75, more desirably at least 0.80, preferably at least 0.85, more preferably at least 0.90, and most preferably at least 0.95.

By a "very high degree of diagnostic accuracy", it is meant a test or assay in which the AUC (area under the ROC curve for the test or assay) is at least 0.80, desirably at least 0.85, more desirably at least 0.875, preferably at least 0.90, more preferably at least 0.925, and most preferably at least 0.95.

Alternatively, in low disease prevalence tested populations (defined as those with less than 1% rate of occurrences per annum), ROC and AUC can be misleading as to the clinical utility of a test, and absolute and relative risk ratios as defined elsewhere in this disclosure can be employed to determine the degree of diagnostic accuracy. Populations of subjects to be tested can also be categorized into quartiles, where the top quartile (25% of the population) comprises the group of subjects with the highest relative risk for developing or suffering from Diabetes or a pre-diabetic condition and the bottom quartile comprising the group of subjects having the lowest relative risk for developing Diabetes or a pre-diabetic condition. Generally, values derived from tests or assays having over 2.5 times the relative risk from top to bottom quartile in a low prevalence population are considered to have a "high degree of diagnostic accuracy," and those with five to seven times the relative risk for each quartile are considered to have a very high degree of diagnostic accuracy. Nonetheless, values derived from tests or assays having only 1.2 to 2.5 times the relative risk for each quartile remain clinically useful are widely used as risk factors for a disease; such is the case with total cholesterol and for many inflammatory markers with respect to their prediction of future cardiovascular events.

The predictive value of any test depends on the sensitivity and specificity of the test, and on the prevalence of the condition in the population being tested. This notion, based on Bayes' theorem, provides that the greater the likelihood that the condition being screened for is present in a subject or in the population (pre-test probability), the greater the validity of a positive test and the greater the likelihood that the result is a true positive. Thus, the problem with using a test in any population where there is a low likelihood of the condition being present is that a positive result has limited value (i.e., more likely to be a false positive). Similarly, in populations at very high risk, a negative test result is more likely to be a false negative. By defining the degree of diagnostic accuracy, i.e., cut points on a ROC curve, defining an acceptable AUC value, and determining the acceptable ranges in relative concentration of what constitutes an effective amount of the DBRISKMARKERS of the invention allows for one of skill in the art to use the DBRISKMARKERS to diagnose or identify subjects with a pre-determined level of predictability.

Alternative methods of determining diagnostic accuracy must be used with continuous measurements of risk, which are commonly used when a disease category or risk category (such as Pre-Diabetes) has not yet been clearly defined by the relevant medical societies and practice of medicine.

"Risk" in the context of the present invention can mean "absolute" risk, which refers to that percentage probability that an event will occur over a specific time period. Absolute risk can be measured with reference to either actual observation post-measurement for the relevant time cohort, or with reference to index values developed from statistically valid historical cohorts that have been followed for the relevant time period. "Relative" risk refers to the ratio of absolute risks of a subject's risk compared either to low risk cohorts or average population risk, which can vary by how clinical risk factors are assessed. Odds ratios, the proportion of positive events to negative events for a given test result, are also commonly used (odds are according to the formula $p/(1-p)$ where p is the probability of event and $(1-p)$ is the probability of no event) to no-conversion. Alternative continuous measures which may be assessed in the context of the presentt invention include time to Diabetes conversion and therapeutic Diabetes conversion risk reduction ratios.

For such continuous measures, measures of diagnostic accuracy for a calculated index are typically based on linear regression curve fits between the predicted continuous value and the actual observed values (or historical index calculated value) and utilize measures such as R squared, p values and confidence intervals. It is not unusual for predicted values using such algorithms to be reported including a confidence interval (usually 90% or 95% CI) based on a historical observed cohort's predictions, as in the test for risk of future breast cancer recurrence commercialized by Genomic Health (Redwood City, California).

The ultimate determinant and gold standard of true risk conversion to Diabetes is a actual conversions within a sufficiently large population and observed over the length of time claimed. However, this is problematic, as it is necessarily a retrospective point of view, coming after any opportunity for preventive interventions. As a result, subjects suffering from or at risk of developing Diabetes or a pre-diabetic condition are commonly diagnosed or identified by methods known in the art, and future risk is estimated based on historical experience and registry studies. Such methods include, but are not limited to, measurement of systolic and diastolic blood pressure, measurements of body mass index, *in vitro* determination of total cholesterol, LDL, HDL, insulin, and glucose levels from blood samples, oral glucose tolerance tests, stress tests, measurement of human serum C-reactive protein (hsCRP), electrocardiogram (ECG), c-peptide levels, anti-insulin antibodies, anti-beta cell-antibodies, and glycosylated hemoglobin ($HbA_{1c}$). Additionally, any of the aforementioned methods can be used separately or in combination to assess if a subject has shown an "improvement in Diabetes risk factors." Such improvements include, without limitation, a reduction in body mass index (BMI), a reduction in blood glucose levels, an increase in HDL levels, a reduction in systolic and/or diastolic blood pressure, an increase in insulin levels, or combinations thereof.

The oral glucose tolerance test (OGTT) is principally used for diagnosis of Diabetes Mellitus or pre-diabetic conditions when blood glucose levels are equivocal, during pregnancy, or in epidemiological studies (Definition, Diagnosis and Classification of Diabetes Mellitus and its Complications, Part 1, World Health Organization, 1999). The OGTT should be administered in the morning after at least 3 days of unrestricted diet (greater than 150 g of carbohydrate daily) and usual physical activity. A reasonable (30-50 g) carbohydrate-containing meal should be consumed on the evening before the test. The test should be preceded by an overnight fast of 8-14 hours, during which water may be consumed. After collection of the fasting blood sample, the subject should drink 75 g of anhydrous glucose or 82.5 g of glucose monohydrate in 250-300 ml of water over the course of 5 minutes. For children, the test load should be 1.75 g of glucose per kg body weight up to a total of 75 g of glucose. Timing of the test is from the beginning of the drink. Blood samples must be collected 2 hours after the test load. As previously noted, a diagnosis of impaired glucose tolerance (IGT) has been noted as being only 50% sensitive, with a >10% false positive rate, for a 7.5 year conversion to diabetes when used at the WHO cut-off points. This is a significant problem for the clinical utility of the test, as even relatively high risk ethnic groups have only a 10% rate of conversion to Diabetes over such a period unless otherwise enriched by other risk factors; in an unselected general population, the rate of conversion over such periods is typically estimated at 5-6%, or less than 1% per annum.

Other methods of measuring glucose in blood include reductiometric methods known in the art such as, but not limited to, the Somogyi-Nelson method, methods using hexokinase and glucose dehydrogenase, immobilized glucose oxidase electrodes, the o-toluidine method, the ferricyanide method and the neocuprine autoanalyzer method. Whole blood glucose values are usually about 15% lower than corresponding plasma values in patients with a normal hematocrit reading, and arterial values are generally about 7% higher than corresponding venous values. Subjects taking insulin are frequently requested to build up a "glycemic profile" by self-measurement of blood glucose at specific times of the day. A "7-point profile" is useful, with samples taken before and 90 minutes after each meal, and just before going to bed.

A subject suffering from or at risk of developing Diabetes or a pre-diabetic condition may also be suffering from or at risk of developing arteriovascular disease, hypertension or obesity. Type 2 Diabetes in particular and arteriovascular disease have many risk factors in common, and many of these risk factors are highly correlated with one another. The relationships among these risk factors may be attributable to a small number of physiological phenomena, perhaps even a single phenomenon. Subjects suffering from or at risk of developing Diabetes, arteriovascular disease, hypertension or obesity are identified by methods known in the art. For example, Diabetes is frequently diagnosed by measuring fasting blood glucose levels or insulin. Normal adult glucose levels are 60-126 mg/dl. Normal insulin levels are 7 mU/mL $\pm$ 3mU. Hypertension is diagnosed by a blood pressure consistently at or above 140/90. Risk of arteriovascular disease can also be diagnosed by measuring cholesterol levels. For example, LDL cholesterol above 137 or total cholesterol above 200 is indicative of a heightened risk of arteriovascular disease. Obesity is diagnosed for example, by body mass index. Body mass index (BMI) is measured (kg/m$^2$ (or lb/in$^2$ X 704.5)). Alternatively, waist circumference (estimates fat distribution), waist-to-hip ratio (estimates fat distribution), skinfold thickness (if measured at several sites, estimates fat distribution), or bioimpedance (based on principle that lean mass conducts current better than fat mass (i.e. fat mass impedes current), estimates % fat) is measured. The parameters for normal, overweight, or obese individuals is as follows: Underweight: BMI <18.5; Normal: BMI 18.5 to 24.9; Overweight: BMI = 25 to 29.9. Overweight individuals are characterized as having a waist circumference of >94 cm for men or >80 cm for women and waist to hip ratios of $\geq$ 0.95 in men and $\geq$ 0.80 in women. Obese individuals are characterized as having a BMI of 30 to 34.9, being greater than 20% above "normal" weight for height, having a body fat percentage > 30% for women and 25% for men, and having a waist circumference >102 cm (40 inches) for men or 88 cm (35 inches) for women. Individuals with severe or morbid obesity are characterized as having a BMI of $\geq$ 35. Because of the interrelationship between Diabetes and arteriovascular disease, some or all of the individual DBRISKMARKERS and DBRISKMARKER panels of the present invention may overlap or be encompassed by biomarkers of arteriovascular disease, and indeed may be useful in the diagnosis of the risk of arteriovascular disease.

Risk prediction for Diabetes Mellitus or a pre-diabetic condition can also encompass risk prediction algorithms and computed indices that assess and estimate a subject's absolute risk for developing Diabetes or a pre-diabetic condition with reference to a historical cohort. Risk assessment using such predictive mathematical algorithms and computed indices has increasingly been incorporated into guidelines for diagnostic testing and treatment, and encompass indices obtained from and validated with, *inter alia,* multi-stage, stratified samples from a representative population. A plurality of conventional Diabetes risk factors are incorporated into predictive models. A notable example of such algorithms include the Framingham Heart Study (Kannel, W.B., et al, (1976) Am. J. Cardiol. 38: 46-51) and modifications of the Framingham Study, such as the National Cholesterol Education Program Expert Panel on Detection, Evaluation, and Treatment of High Blood Cholesterol in Adults (Adult Treatment Panel III), also know as NCEP/ATP III, which incorporates a patient's age, total cholesterol concentration, HDL cholesterol concentration, smoking status, and systolic blood pressure to estimate a person's 10-year risk of developing arteriovascular disease, which is commonly found in subjects suffering from or at risk for developing Diabetes Mellitus, or a pre-diabetic condition. The same Framingham algorithm has been found to be modestly predictive of the risk for developing Diabetes Mellitus, or a pre-diabetic condition.

Other Diabetes risk prediction algorithms include, without limitation, the San Antonio Heart Study (Stem, M.P. et al, (1984) Am. J. Epidemiol. 120: 834-851; Stem, M.P. et al, (1993) Diabetes 42: 706-714; Burke, J.P. et al, (1999) Arch. Intern. Med. 159: 1450-1456), Archimedes (Eddy, D.M. and Schlessinger, L. (2003) Diabetes Care 26(11): 3093-3101; Eddy, D.M. and Schlessinger, L. (2003) Diabetes Care 26(11): 3102-3110), the Finnish-based Diabetes Risk Score (Lindström, J. and Tuomilehto, J. (2003) Diabetes Care 26(3): 725-731), and the Ely Study (Griffin, S.J. et al, (2000) Diabetes Metab. Res. Rev. 16: 164-171), the contents of which are expressly incorporated herein by reference.

Archimedes is a mathematical model of Diabetes that simulates the disease state person-by-person, object-by-object and comprises biological details that are continuous in reality, such as the pertinent organ systems, more than 50 continuously interacting biological variables, and the major symptoms, tests, treatments, and outcomes commonly associated with Diabetes.

Archimedes includes many diseases simultaneously and interactively in a single integrated physiology, enabling it to address features such as co-morbidities, syndromes, treatments and other multiple effects. The Archimedes model includes Diabetes and its complications, such as coronary artery disease, congestive heart failure, and asthma. The model is written in differential equations, using object-oriented programming and a construct called "features". The model comprises the anatomy of a subject (all simulated subjects have organs, such as hearts, livers, pancreases, gastrointestinal tracts, fat, muscles, kidneys, eyes, limbs, circulatory systems, brains, skin, and peripheral nervous systems), the "features" that determine the course of the disease and representing real physical phenomena (e.g., the number of milligrams of glucose in a deciliter of plasma, behavioral phenomena, or conceptual phenomena (e.g., the "progression" of disease), risk factors, incidence, and progression of the disease, glucose metabolism, signs and tests, diagnosis, symptoms, health outcomes of glucose metabolism, treatments, complications, deaths from Diabetes and its complications, deaths from other causes, care processes, and medical system resources. For a typical application of the model, there are thousands of simulated subjects, each with a simulated anatomy and physiology, who will get simulated diseases, can seek care at simulated health care facilities, will be seen by simulated health care personnel in simulated facilities, will be given simulated tests and treatments, and will have simulated outcomes. As in reality, each of the simulated patients is different, with different characteristics, physiologies, behaviors, and responses to treatments, all designed to match the individual variations seen in reality.

The model is built by development of a non-quantitative or conceptual description of the pertinent biology and pathology - the variables and relationships - as best they are understood with current information. Studies are then identified that pertain to the variables and relationships, and typically comprise basic research, epidemiological, and clinical studies that experts in the field identify as the foundations of their own understanding of the disease. That information is used to develop differential equations that relate the variables. The development of any particular equation in the Archimedes model involves finding the form and coefficients that best fit the available information about the variables, after which the equations are programmed into an object-oriented language. This is followed by a series of exercises in which the parts of the model are tested and debugged, first one at a time, and then in appropriate combinations, using inputs that have known outputs. The entire model can then be used to simulate a complex trial, which demonstrates not only the individual parts of the model, but also the connections between all the parts. The Archimedes calculations are performed using distributed computing techniques. Archimedes has been validated as a realistic representation of the anatomy, pathophysiology, treatments and outcomes pertinent to Diabetes and its complications (Eddy, D.M. and Schlessinger, L. (2003) Diabetes Care 26(11) 3102-3110).

The Finland-based Diabetes Risk Score is designed as a screening tool for identifying high-risk subjects in the population and for increasing awareness of the modifiable risk factors and healthy lifestyle. The Diabetes Risk Score was determined from a random population sample of 35- to 64-year old Finnish men and women with no anti-diabetic drug treatment at baseline, and followed for 10 years. Multivariate logistic regression model coefficients were used to assign each variable category a score. The Diabetes Risk Score comprises the sum of these individual scores and validated in an independent population survey performed in 1992 with a prospective follow-up for 5 years. Age, BMI, waist circumference, history of anti-hypertensive drug treatment and high blood glucose, physical activity, and daily consumption of fruits, berries, or vegetables were selected as categorical variables.

The Finland-based Diabetes Risk Score values are derived from the coefficients of the logistic model by classifying them into five categories. The estimated probability (p) of drug-treated Diabetes over a 10-year span of time for any combination of risk factors can be calculated from the following coefficients:

$$p(Diabetes) = \frac{e^{(\beta_0 + \beta_{1x1} + \beta_{2x2} + ...)}}{1 + e^{(\beta 0 + \beta_{1x1} + \beta_{2x2} + ...)}}$$

where $\beta_0$ is the intercept and $\beta_1$, $\beta_2$, and so on represent the regression coefficients of the various categories of the risk factors $x_1$, $x_2$, and so on.

The sensitivity relates to the probability that the test is positive for subjects who will get drug-treated Diabetes in the future and the specificity reflects the probability that the test is negative for subjects without drug-treated Diabetes. The sensitivity and the specificity with 95% confidence interval (CI) were calculated for each Diabetes Risk Score level in differentiating the subjects who developed drug-treated Diabetes from those who did not. ROC curves were plotted for the Diabetes Risk score, the sensitivity was plotted on the y-axis and the false-positive rate (1-specificity) was plotted on the x-axis. The more accurately discriminatory the test, the steeper the upward portion of the ROC curve, and the higher the AUC, the optimal cut point being the peak of the curve.

Statistically significant independent predictors of future drug-treated Diabetes in the Diabetes Risk Score are age, BMI, waist circumference, antihypertensive drug therapy, and history of high blood glucose levels. The Diabetes Risk Score model comprises a concise model that includes only these statistically significant variables and a full model, which includes physical activity and fruit and vegetable consumption.

The San Antonio Heart Study is a long-term, community-based prospective observational study of Diabetes and arteriovascular disease in Mexican Americans and non-Hispanic Caucasians. The study initially enrolled 3,301 Mexican-American and 1,857 non-Hispanic Caucasian men and non-pregnant women in two phases between 1979 and 1988. Participants were 25-64 years of age at enrollment and were randomly selected from low, middle, and high-income neighborhoods in San Antonio, Texas. A 7-8 year follow-up exam followed approximately 73% of the surviving individuals initially enrolled in the study. Baseline characteristics such as medical history of Diabetes, age, sex, ethnicity, BMI, systolic and diastolic blood pressure, fasting and 2-hour plasma glucose levels, fasting serum total cholesterol, LDL, and HDL cholesterol levels, as well as triglyceride levels, were compiled and assessed. A multiple logistic regression model with incident Diabetes as the dependent variable and the aforementioned baseline characteristics were applied as independent variables. Using this model, univariate odds ratios can be computed for each potential risk factor for men and women separately and for both sexes combined. For continuous risk factors, the odds ratios can be presented for a 1-SD increment. A multivariate predicting model with both sexes combined can be developed using a stepwise logistic regression procedure in which the variables that had shown statistically significant odds ratios when examined individually were allowed to enter the model. This multivariable model is then analyzed by ROC curves and 95% CIs of the areas under the ROC curves estimated by non-parametric algorithms such as those described by DeLong (DeLong E.R. et al, (1988) Biometrics 44: 837-45). The results of the San Antonio Heart Study indicate that pre-diabetic subjects have an atherogenic pattern of risk factors (possibly caused by obesity, hyperglycemia, and especially hyperinsulinemia), which may be present for many years and may contribute to the risk of macrovascular disease as much as the duration of clinical Diabetes itself.

Despite the numerous studies and algorithms that have been used to assess the risk of Diabetes or a prediabetic condition, the evidence-based, multiple risk factor assessment approach is only moderately accurate for the prediction of short- and long-term risk of manifesting Diabetes or a pre-diabetic condition in individual asymptomatic or otherwise healthy subjects. Such risk prediction algorithms can be advantageously used in combination with the DBRISKMARKERS of the present invention to distinguish between subjects in a population of interest to determine the risk stratification of developing Diabetes or a prediabetic condition. The DBRISKMARKERS and methods of use disclosed herein provide tools that can be used in combination with such risk prediction algorithms to assess, identify, or diagnose subjects who are asymptomatic and do not exhibit the conventional risk factors.

The data derived from risk prediction algorithms and from the methods of the present invention can be compared by linear regression. Linear regression analysis models the relationship between two variables by fitting a linear equation to observed data. One variable is considered to be an explanatory variable, and the other is considered to be a dependent variable. For example, values obtained from the Archimedes or San Antonio Heart analysis can be used as a dependent variable and analyzed against levels of one or more DBRISKMARKERS as the explanatory variables in an effort to more fully define the underlying biology implicit in the calculated algorithm score (see Examples). Alternatively, such risk prediction algorithms, or their individual inputs, which are generally DBRISKMARKERS themselves, can be directly incorporated into the practice of the present invention, with the combined algorithm compared against actual observed results in a historical cohort.

A linear regression line has an equation of the form $Y = a + bX$, where X is the explanatory variable and Y is the dependent variable. The slope of the line is b, and a is the intercept (the value of y when x = 0). A numerical measure of association between two variables is the "correlation coefficient," or R, which is a value between -1 and 1 indicating the strength of the association of the observed data for the two variables. This is also often reported as the square of the correlation coefficient, as the "coefficient of determination" or $R^2$; in this form it is the proportion of the total variation in Y explained by fitting the line. The most common method for fitting a regression line is the method of least-squares. This method calculates the best-fitting line for the observed data by minimizing the sum of the squares of the vertical deviations from each data point to the line (if a point lies on the fitted line exactly, then its vertical deviation is 0). Because the deviations are first squared, then summed, there are no cancellations between positive and negative values.

After a regression line has been computed for a group of data, a point which lies far from the line (and thus has a large residual value) is known as an outlier. Such points may represent erroneous data, or may indicate a poorly fitting regression line. If a point lies far from the other data in the horizontal direction, it is known as an influential observation. The reason for this distinction is that these points have may have a significant impact on the slope of the regression line. Once a regression model has been fit to a group of data, examination of the residuals (the deviations from the fitted line to the observed values) allows one of skill in the art to investigate the validity of the assumption that a linear relationship exists. Plotting the residuals on the y-axis against the explanatory variable on the x-axis reveals any possible non-linear relationship among the variables, or might alert the skilled artisan to investigate "lurking variables." A "lurking variable" exists when the relationship between two variables is significantly affected by the presence of a third variable which has not been included in the modeling effort.

Linear regression analyses can be used, *inter alia,* to predict the risk of developing Diabetes or a pre-diabetic condition based upon correlating the levels of DBRISKMARKERS in a sample from a subject to that subjects' actual observed clinical outcomes, or in combination with, for example, calculated Archimedes risk scores, San Antonio Heart risk scores, or other known methods of diagnosing or predicting the prevalence of Diabetes or a pre-diabetic condition. Of particular use, however, are non-linear equations and analyses to determine the relationship between known predictive models of Diabetes and levels of DBRISKMARKERS detected in a subject sample. Of particular interest are structural and synactic classification algorithms, and methods of risk index construction, utilizing pattern recognition features, including established techniques such as the Kth-Nearest Neighbor, Boosting, Decision Trees, Neural Networks, Bayesian Networks, Support Vector Machines, and Hidden Markov Models. Most commonly used are classification algorithms using logistic regression, which are the basis for the Framingham, Finnish, and San Antonio Heart risk scores. Furthermore, the application of such techniques to panels of multiple DBRISKMARKERS is encompassed by or within the ambit of the present invention, as is the use of such combination to create single numerical "risk indices" or "risk scores" encompassing information from multiple DBRISKMARKER inputs. An example using logistic regression is described herein in the Examples.

Factor analysis is a mathematical technique by which a large number of correlated variables (such as Diabetes risk factors) can be reduced to fewer "factors" that represent distinct attributes that account for a large proportion of the variance in the original variables (Hanson, R.L. et al, (2002) Diabetes 51: 3120-3127). Thus, factor analysis is well suited for identifying components of Diabetes Mellitus and pre-diabetic conditions such as IGT, IFG, and Metabolic Syndrome. Epidemiological studies of factor "scores" from these anlyses can further determine relations between components of the metabolic syndrome and incidence of Diabetes. The premise underlying factor analysis is that correlations observed among a set of variables can be explained by a small number of unique unmeasured variables, or "factors". Factor analysis involves two procedures: 1) factor extraction to estimate the number of factors, and 2) factor rotation to determine constituents of each factor in terms of the original variables.

Factor extraction can be conducted by the method of principal components. These components are linear combinations of the original variables that are constructed so that each component has a correlation of zero with each of the other components. Each principal component is associated with an "eigen-value," which represents the variance in the original variables explained by that component (with each original variable standardized to have a variance of 1). The number of principal components that can be constructed is equal to the number of original variables. In factor analysis, the number of factors is customarily determined by retention of only those components that account for more of the total variance than any single original variable (i.e., those components with eigen-values of >1).

Once the number of factors has been established, then factor rotation is conducted to determine the composition of factors that has the most parsimonious interpretation in terms of the original variables. In factor rotation, "factor loadings," which represent correlations of each factor with the original variables, are changed so that these factor loadings are made as close to 0 or 1 as possible (with the constraint that the total amount of variance explained by the factors remains unchanged). A number of methods for factor rotation have been developed and can be distinguished by whether they require the final set of factors to remain uncorrelated with one another (also known as "orthogonal methods") or by whether they allow factors to be correlated ("oblique methods"). In interpretation of factor analysis, the pattern of factor loadings is examined to determine which original variables represent primary constituents of each factor. Conventionally, variables that have a factor loading of >0.4 (or less than -0.4) with a particular factor are considered to be its major constituents. Factor analysis can be very useful in constructing DBRISKMARKER panels from their constituent components, and in grouping substitutable groups of markers.

Levels of an effective amount of DBRISKMARKER proteins, nucleic acids, polymorphisms, metabolites, or other analytes also allows for the course of treatment of Diabetes or a pre-diabetic condition to be monitored. In this method, a biological sample can be provided from a subject undergoing treatment regimens, e.g., drug treatments, for Diabetes. Such treatment regimens can include, but are not limited to, exercise regimens, dietary supplementation, surgical intervention, and treatment with therapeutics or prophylactics used in subjects diagnosed or identified with Diabetes or a pre-diabetic condition. If desired, biological samples are obtained from the subject at various time points before, during, or after treatment. Levels of an effective amount of DBRISKMARKER proteins, nucleic acids, polymorphisms, metabolites, or other analytes can then be determined and compared to a reference value, e.g. a control subject or population whose diabetic state is known or an index value or baseline value. The reference sample or index value or baseline value may be taken or derived from one or more subjects who have been exposed to the treatment, or may be taken or derived from one or more subjects who are at low risk of developing Diabetes or a pre-diabetic condition, or may be taken or derived from subjects who have shown improvements in Diabetes risk factors as a result of exposure to treatment. Alternatively, the reference sample or index value or baseline value may be taken or derived from one or more subjects who have not been exposed to the treatment. For example, samples may be collected from subjects who have received initial treatment for Diabetes or a pre-diabetic condition and subsequent treatment for Diabetes or a pre-diabetic condition to monitor the progress of the treatment. A reference value can also comprise a value derived from risk prediction algorithms or computed indices from population studies such as those disclosed herein.

The DBRISKMARKERS of the present invention can thus be used to generate a "reference expression profile" of those subjects who do not have Diabetes or a pre-diabetic condition such as impaired glucose tolerance, and would not be expected to develop Diabetes or a pre-diabetic condition. The DBRISKMARKERS disclosed herein can also be used to generate a "subject expression profile" taken from subjects who have Diabetes or a prediabetic condition like impaired glucose tolerance. The subject expression profiles can be compared to a reference expression profile to diagnose or identify subjects at risk for developing Diabetes or a pre-diabetic condition, to monitor the progression of disease, as well as the rate of progression of disease, and to monitor the effectiveness of Diabetes or pre-Diabetes treatment modalities. The reference and subject expression profiles of the present invention can be contained in a machine-readable medium, such as but not limited to, analog tapes like those readable by a VCR, CD-ROM, DVD-ROM, USB flash media, among others. Such machine-readable media can also contain additional test results, such as, without limitation, measurements of conventional Diabetes risk factors like systolic and diastolic blood pressure, blood glucose levels, insulin levels, BMI indices, and cholesterol (LDL and HDL) levels. Alternatively or additionally, the machine-readable media can also comprise subject information such as medical history and any relevant family history. The machine-readable media can also contain information relating to other Diabetes-risk algorithms and computed indices such as those described herein.

Differences in the genetic makeup of subjects can result in differences in their relative abilities to metabolize various drugs, which may modulate the symptoms or risk factors of Diabetes or a pre-diabetic condition. Subjects that have Diabetes or a pre-diabetic condition, or at risk for developing Diabetes or a pre-diabetic condition can vary in age, ethnicity, body mass index (BMI), total cholesterol levels, blood glucose levels, blood pressure, LDL and HDL levels, and other parameters. Accordingly, use of the DBRISKMARKERS disclosed herein allow for a pre-determined level of predictability that a putative therapeutic or prophylactic to be tested in a selected subject will be suitable for treating or preventing Diabetes or a pre-diabetic condition in the subject.

To identify therapeutics or drugs that are appropriate for a specific subject, a test sample from the subject can be exposed to a therapeutic agent or a drug, and the level of one or more DBRISKMARKER proteins, nucleic acids, polymorphisms, metabolites or other analytes can be determined. The level of one or more DBRISKMARKERS can be compared to sample derived from the subject before and after treatment or exposure to a therapeutic agent or a drug, or can be compared to samples derived from one or more subjects who have shown improvements in Diabetes or pre-Diabetes risk factors as a result of such treatment or exposure. Examples of such therapeutics or drugs frequently used in Diabetes treatments, and may modulate the symptoms or risk factors of Diabetes include, but are not limited to, sulfonylureas like glimepiride, glyburide (also known in the art as glibenclamide), glipizide, gliclazide; biguanides such as metformin; insulin (including inhaled formulations such as Exubera), and insulin analogs such as insulin lispro (Humalog), insulin glargine (Lantus), insulin detemir, and insulin glulisine; peroxisome proliferator-activated receptor-γ (PPAR-γ) agonists such as the thiazolidinediones including troglitazone (Rezulin), pioglitazone (Actos), rosiglitazone (Avandia), and isaglitzone (also known as netoglitazone); dual-acting PPAR agonists such as BMS-298585 and tesaglitazar; insulin secretagogues including metglitinides such as repaglinide and nateglinide; analogs of glucagon-like peptide-1 (GLP-1) such as exenatide (AC-2993) and liraglutide (insulinotropin); inhibitors of dipeptidyl peptidase IV like LAF-237; pancreatic lipase inhibitors such as orlistat; α-glucosidase inhibitors such as acarbose, migitol, and voglibose; and combinations thereof, particularly metformin and glyburide (Glucovance), metformin and rosiglitazone (Avandamet), and metformin and glipizide (Metaglip). Such therapeutics or drugs have been prescribed for subjects diagnosed with Diabetes or a pre-diabetic condition, and may modulate the symptoms or risk factors of Diabetes or a pre-diabetic condition.

A subject sample can be incubated in the presence of a candidate agent and the pattern of DBRISKMARKER expression in the test sample is measured and compared to a reference profile, e.g., a Diabetes reference expression profile or a non-Diabetes reference expression profile or an index value or baseline value. The test agent can be any compound or composition or combination thereof. For example, the test agents are agents frequently used in Diabetes treatment regimens and are described herein.

Table 1 comprises the two-hundred and sixty (260) DBRISKMARKERS of the present invention. One skilled in the art will recognize that the DBRISKMARKERS presented herein encompasses all forms and variants, including but not limited to, polymorphisms, isoforms, mutants, derivatives, precursors including nucleic acids, receptors (including soluble and transmembrane receptors), ligands, and post-translationally modified variants, as well as any multi-unit nucleic acid, protein, and glycoprotein structures comprised of any of the DBRISKMARKERS as constituent subunits of the fully assembled structure.

Table 1: DBRISKMARKERS

| DERISKMARKER | Official Name | Common Name | Entrez Gene Link |
|---|---|---|---|
| 1 | ATP-binding cassette, sub-family C (CFTR/MRP), member 8 | sulfonylurea receptor (SUR1), HI; SUR; HHF1; MRP8; PHHI; SUR1; ABC36; HRINS | ABCC8 |
| 2 | ATP-binding cassette, sub-family C (CFTR/MRP), member 9 | sulfonylurea receptor (SUR2a), SUR2; ABC37; CMD1O; FLJ36852 | ABCC9 |

(continued)

| DERISKMARKER | Official Name | Common Name | Entrez Gene Link |
|---|---|---|---|
| 3 | angiotensin I converting enzyme (peptidyl-dipeptidase A) 1 | angiotensin-converting enzyme (ACE) - ACE1, CD143, DCP, DCP1, CD143 antigen; angiotensin I converting enzyme; angiotensin converting enzyme, somatic isoform; carboxycathepsin; dipeptidyl carboxypeptidase I; kininase II; peptidase P; peptidyldipeptidase A; testicular ECA | ACE |
| 4 | adenylate cyclase activating polypeptide 1 (pituitary) | adenylate cyclase activating polypeptide | ADCYAP1 |
| 5 | adiponectin, C1Q and collagen domain containing | Adiponectin - ACDC, ACRP30, APM-1, APM1, GBP28, adiponectin, adipocyte, C1Q and collagen domain containing; adipocyte, C1Q and collagen domain-containing; adiponectin; adipose most abundant gene transcript 1; gelatin-binding protein 28 | ADIPOQ |
| 6 | adiponectin receptor 1 | G Protein Coupled Receptor AdipoR1 - ACDCR1, CGI-45, PAQR1, TESBP1A | ADIPOR1 |
| 7 | adiponectin receptor 2 | G Protein Coupled Receptor AdipoR2 - ACDCR2, PAQR2 | ADIPOR2 |
| 8 | adrenomedullin | adrenomedullin - AM, preproadrenomedullin | ADM |
| 9 | adrenergic, beta-2-, receptor, surface | G Protein- Coupled Beta-2 Adrenoceptor - ADRB2R, ADRBR, B2AR, BAR, BETA2AR, beta-2 adrenergic receptor; beta-2 adrenoceptor; catecholamine receptor | ADRB2 |
| 10 | advanced glycosylation end product-specific receptor | RAGE - advanced glycosylation end product-specific receptor RAGE3; advanced glycosylation end product-specific receptor variant sRAGE1; advanced glycosylation end product-specific receptor variant sRAGE2; receptor for advanced glycosylation end-products; soluble receptor | AGER |
| 11 | agouti related protein homolog (mouse) | AGRT, ART, ASIP2, & Agouti-related transcript, mouse, homolog of; agouti (mouse) related protein; agouti related protein homolog | AGRP |

(continued)

| DERISKMARKER | Official Name | Common Name | Entrez Gene Link |
|---|---|---|---|
| 12 | angiotensinogen (serpin peptidase inhibitor, clade A, member 8) | angiotensin I; pre-angiotensinogen; angiotensin II precursor; angiotensinogen (serine (or cysteine) peptidase inhibitor, clade A, member 8); angiotensinogen (serine (or cysteine) proteinase inhibitor, clade A (alpha-1 antiproteinase, antitrypsin), member 8) | AGT |
| 13 | angiotensin II receptor, type 1 | G protein-Coupled Receptor AGTR1A - AG2S, AGTR1A, AGTR1B, AT1, AT1B, AT2R1, AT2R1A, AT2R1B, HAT1R, angiotensin receptor 1; angiotensin receptor 1B; type-1B angiotensin II receptor | AGTR1 |
| 14 | angiotensin II receptor-associated protein | angiotensin II - ATRAP, ATI receptor-associated protein; angiotensin II, type I receptor-associated protein | AGTRAP |
| 15 | alpha-2-HS-glycoprotein | A2HS, AHS, FETUA, HSGA, Alpha-2HS-glycoprotein; fetuin-A | AHSG |
| 16 | v-akt murine thymoma viral oncogene homolog 1 | Ser/Thr kinase Akt - PKB, PRKBA, RAC, RAC-ALPHA, RAC-alpha serine/threonine-protein kinase; murine thymoma viral (v-akt) oncogene homolog-1; protein kinase B; rac protein kinase alpha | AKT1 |
| 17 | v-akt murine thymoma viral oncogene homolog 2 | PKBBETA, PRKBB, RAC-BETA, Murine thymoma viral (v-akt) homolog-2; rac protein kinase beta | AKT2 |
| 18 | albumin | Ischemia-modified albumin (IMA) - cell growth inhibiting protein 42; growth-inhibiting protein 20; serum albumin | ALB |
| 19 | Alstrom syndrome 1 | ALSS | ALMS1 |
| 20 | archidonate 12-lipoxygenase | LOG12, 12(S)-lipoxygenase; platelet-type 12-lipoxygenase/arachidonate 12-lipoxygenase | ALOX12 |
| 21 | ankyrin repeat domain 23 | DARP, MARP3, Diabetes related ankyrin repeat protein; muscle ankyrin repeat protein 3 | ANKRD23 |
| 22 | apelin, AGTRL 1 Ligand | XNPEP2, apelin, peptide ligand for APJ receptor | APLN |
| 23 | apolipoprotein A-I | apolipoproteins A-I and B, amyloidosis; apolipoprotein A-I, preproprotein; apolipoprotein A1; preproapolipoprotein | APOA1 |
| 24 | apolipoprotein A-II | Apolipoprotein A-II | APOA2 |

(continued)

| DERISKMARKER | Official Name | Common Name | Entrez Gene Link |
|---|---|---|---|
| 25 | apolipoprotein B (including Ag(x) antigen) | apolipoproteins A-1 and B -Apolipoprotein B, FLDB, apoB-100; apoB-48; apolipoprotein B; apolipoprotein B48 | APOB |
| 26 | apolipoprotein E | APO E - AD2, apoprotein, Alzheimer disease 2 (APOE*E4-associated, late onset); apolipoprotein E precursor; apolipoprotein E3 | APOE |
| 27 | aryl hydrocarbon receptor nuclear translocator | dioxin receptor, nuclear translocator; hypoxia-inducible factor 1, beta subunit | ARNT |
| 28 | Aryl hydrocarbon receptor nuclear translocator-like | Bmal1, TIC; JAP3; MOP3; BMAL1; PASD3; BMAL1c; bHLH-PAS protein JAP3; member of PAS superfamily 3; ARNT-like protein 1, brain and muscle; basic-helix-loop-helix-PAS orphan MOP3 | ARNTL |
| 29 | arrestin, beta 1 | beta arrestin - ARB1, ARRI, arrestin beta 1 | ARRB1 |
| 30 | arginine vasopressin (neurophysin II, antidiuretic hormone, Diabetes insipidus, neurohypophyseal) | copeptin - ADH, ARVP, AVP-NPII, AVRP, VP, arginine vasopressin-neurophysin II; vasopressin-neurophysin II-copeptin, vasopressin | AVP |
| 31 | bombesin receptor subtype 3 | G-protein coupled receptor; bombesin receptor subtype 3 | BRS3 |
| 32 | betacellulin | betacellulin | BTC |
| 33 | benzodiazepine receptor (peripheral) | PBR - DBI, IBP, MBR, PBR, PKBS, PTBR, mDRC, pk18, benzodiazepine peripheral binding site; mitochondrial benzodiazepine receptor; peripheral benzodiazapine receptor; peripheral benzodiazepine receptor; peripheral-type benzodiazepine receptor | BZRP |
| 34 | complement component 3 | complement C3 - acylation-stimulating protein cleavage product; complement component C3, ASP; CPAMD1 | C3 |
| 35 | complement component 4A (Rodgers blood group) | complement C4 - C4A anaphylatoxin; Rodgers form of C4; acidic C4; c4 propeptide; complement component 4A; complement component C4B | C4A |

(continued)

| DERISKMARKER | Official Name | Common Name | Entrez Gene Link |
|---|---|---|---|
| 36 | complement component 4B (Childo blood group) | C4A, C4A13, C4A91, C4B1, C4B12, C4B2, C4B3, C4B5, C4F, CH, CO4, CPAMD3, C4 complement C4d region; Chido form of C4; basic C4; complement C4B; complement component 4B; complement component 4B, centromeric; complement component 4B, telomeric; complement component C4B | C4B |
| 37 | complement component 5 | anaphylatoxin C5a analog-CPAMD4 | C5 |
| 38 | Calpain-10 | calcium-activated neutral protease | CAPN10 |
| 39 | cholecystokinin | cholecystokinin | CCK |
| 40 | cholecystokinin (CCK)-A receptor | CCK-A;CCK-A;CCKRA;CCK1-R; cholecystokinin-1 receptor; cholecystokinin type-A receptor | CCKAR |
| 41 | chemokine (C-C motif) ligand 2 | Monocyte chemoattractant protein-1 (MCP-1) - GDCF-2, GDCF-2 HC11,HC11, HSMCR30, MCAF, MCP-1, MCP1, SCYA2, SMC-CF, monocyte chemoattractant protein-1; monocyte chemotactic and activating factor; monocyte chemotactic protein 1, homologous to mouse Sig-je; monocyte secretory protein JE; small inducible cytokine A2; small inducible cytokine A2 (monocyte chemotactic protein 1, homologous to mouse Sig-je); small inducible cytokine subfamily A (Cys-Cys), member 2 | CCL2 |
| 42 | CD14 molecule | CD14 antigen - monocyte receptor | CD14 |
| 43 | CD163 molecule | CD163 - M130, MM130-CD163 antigen; macrophage-associated antigen, macrophage-specific antigen | CD163 |

(continued)

| DERISKMARKER | Official Name | Common Name | Entrez Gene Link |
|---|---|---|---|
| 44 | CD36 molecule (thrombospondin receptor) | fatty acid translocase, FAT; GP4; GP3B; GPIV; PASIV; SCARB3, PAS-4 protein; collagen type I; glycoprotein IIIb; cluster determinant 36; fatty acid translocase; thrombospondin receptor; collagen type I receptor; platelet glycoprotein IV; platelet collagen receptor; scavenger receptor class B, member 3; leukocyte differentiation antigen CD36; CD36 antigen (collagen type I receptor, thrombospondin receptor) | CD36 |
| 45 | CD38 molecule | T10; CD38 antigen (p45); cyclic ADP-ribose hydrolase; ADP-ribosyl cyclase/cyclic ADP-ribose hydrolase | CD38 |
| 46 | CD3d molecule, delta (CD3-TCR complex) | CD3-DELTA, T3D, CD3D antigen, delta polypeptide; CD3d antigen, delta polypeptide (TiT3 complex); T-cell receptor T3 delta chain | CD3D |
| 47 | CD3g molecule, gamma (CD3-TCR complex) | T3G; CD3-GAMMA, T3G, CD3G gamma; CD3g antigen, gamma polypeptide (TiT3 complex); T-cell antigen receptor complex, gamma subunit of T3; T-cell receptor T3 gamma chain; T-cell surface glycoprotein CD3 gamma chain precursor | CD3G |
| 48 | CD40 molecule, TNF receptor superfamily member 5 | Bp50, CDW40, TNFRSF5, p50, B cell surface antigen CD40; B cell-associated molecule; CD40 antigen; CD40 antigen (TNF receptor superfamily member 5); CD40 type II isoform; CD40L. receptor; nerve growth factor receptor-related B-lymphocyte activation molecule; tumor necrosis factor receptor superfamily, member 5 | CD40 |

(continued)

| DERISKMARKER | Official Name | Common Name | Entrez Gene Link |
|---|---|---|---|
| 49 | CD40 ligand (TNF superfamily, member 5, hyper-IgM syndrome) | CD40 Ligand (CD40L) (also called soluble CD40L vs. platelet-bound CD40L), CD154, CD40L, HIGM1, IGM, IMD3, T-BAM, TNFSF5, TRAP, gp39, hCD40L, CD40 antigen ligand; CD40 ligand; T-B cell-activating molecule; TNF-related activation protein; tumor necrosis factor (ligand) superfamily member 5; tumor necrosis factor (ligand) superfamily, member 5 (hyper-IgM syndrome); tumor necrosis factor ligand superfamily member 5 | CD40LG |
| 50 | CD68 molecule | GP110; SCARD1; macrosialin; CD68 antigen; macrophage antigen CD68; scavenger receptor class D, member 1 | CD68 |
| 51 | cyclin-dependent kinase 5 | PSSALRE; cyclin-dependent kinase 5 | CDK5 |
| 52 | complement factor D (adipsin) | ADN, DF, PFD, C3 convertase activator; D component of complement (adipsin); adipsin; complement factor D; properdin factor D | CFD |
| 53 | CASP8 and FADD-like apoptosis regulator | FLIP - caspase 8 inhibitor, CASH; FLIP; MRIT; CLARP; FLAME; Casper; c-FLIP; FLAME-1; I-FLICE; USURPIN; c-FLIPL; c-FLIPR; c-FLIPS; CASP8AP1, usurpin beta; FADD-like anti-apoptotic molecule; Inhibitor of FLICE; Caspase-related inducer of apoptosis; Caspase homolog; Caspase-like apoptosis regulatory protein | CFLAR |
| 54 | Clock homolog (mouse) | clock protein; clock (mouse) homolog; circadian locomoter output cycles kaput protein | CLOCK |
| 55 | chymase 1, mast cell | chymase 1 - CYH, MCT1, chymase 1 preproprotein transcript E; chymase 1 preproprotein transcript I; chymase, heart; chymase, mast cell; mast cell protease 1 | CMA1 |
| 56 | cannabinoid receptor 1 (brain) | cannabinoid receptor 1 - CANN6, CB-R, CB1, CB1A, CB1K5, CNR, central cannabinoid receptor | CNR1 |
| 57 | cannabinoid receptor 2 (macrophage) | cannabinoid receptor 2 (macrophage), CB2, CX5 | CNR2 |

(continued)

| DERISKMARKER | Official Name | Common Name | Entrez Gene Link |
|---|---|---|---|
| 58 | cortistatin | CST-14; CST-17; CST-29; cortistatin-14; cortistatin-17; cortistatin-29; preprocortistatin | CORT |
| 59 | carnitine palmitoyltransferase I | CPT1; CPT1-L; L-CPT1, carnitine palmitoyltransferase I; liver | CPT1A |
| 60 | carnitine palmitoyltransferase II | CPT1, CPTASE | CPT2 |
| 61 | complement component (3b/4b) receptor 1 | complement receptor CR1; KN; C3BR; CD35; CD35 antigen; C3b/C4b receptor; C3-binding protein; Knops blood group antigen; complement component receptor 1; complement component (3b/4b) receptor 1, including Knops blood group system | CR1 |
| 62 | complement component (3d/ Epstein Barr virus) receptor 2 | complement receptor CR2; C3DR; CD21 | CR2 |
| 63 | CREB binding protein (Rubinstein-Taybi syndrome) | Cbp; CBP; RTS; RSTS, CREB-binding protein | CREBBP |
| 64 | C-reactive protein, pentraxin-related | C-Reactive Protein, CRP, PTX1 | CRP |
| 65 | CREB regulated transcription coactivator 2 | Torc2 (transcriptional coactivator); transducer of regulated cAMP response element-binding protein (CREB) 2 | CRTC2 |
| 66 | colony stimulating factor 1 (macrophage) | M-CSF - colony stimulating factor 1; macrophage colony stimulating factor | CSF1 |
| 67 | cathepsin B | cathepsin B - procathepsin B, APPS; CPSB, APP secretase; amyloid precursor protein secretase; cathepsin B1; cysteine protease; preprocathepsin B | CTSB |
| 68 | cathepsin L | CATL, MEP, major excreted protein | CTSL |
| 69 | cytochrome P450, family 19, subfamily A, polypeptide 1 | ARO, ARO1, CPV1, CYAR, CYP19, P-450AROM, aromatase; cytochrome P450, family 19; cytochrome P450, subfamily XIX (aromatization of androgens); estrogen synthetase; flavoprotein-linked monooxygenase; microsomal monooxygenase | CYP19A1 |
| 70 | Dio-2, death inducer-obliterator 1 | death associated transcription factor 1; BYE1; DIO1; DATF1; DIDO2; DIDO3; DIO-1 | DIDO1 |

(continued)

| DERISKMARKER | Official Name | Common Name | Entrez Gene Link |
|---|---|---|---|
| 71 | dipeptidyl-peptidase 4 (CD26, adenosine deaminase complexing protein 2) | dipeptidylpeptidase IV -ADABP, ADCP2, CD26, DPPIV, TP103, T-cell activation antigen CD26; adenosine deaminase complexing protein 2; dipeptidylpeptidase IV; dipeptidylpeptidase IV (CD26, adenosine deaminase complexing protein 2) | DPP4 |
| 72 | epidermal growth factor (beta-urogastrone) | URG - urogastrone | EGF |
| 73 | early growth response 1 | zinc finger protein 225; transcription factor ETR103; early growth response protein 1; nerve growth factor-induced protein A | EGR1 |
| 74 | epididymal sperm binding protein 1 | E12, HE12, epididymal secretory protein | ELSPBP1 |
| 75 | ectonucleotide pyrophosphatase/ phosphodiesterase 1 | ENPP1 - M6S1, NPP1, NPPS, PC-1, PCA1, PDNP1, Ly-41 antigen; alkaline phosphodiesterase 1; membrane component, chromosome 6, surface marker 1; phosphodiesterase I/nucleotide pyrophosphatase 1; plasma-cell membrane glycoprotein 1 | ENPP1 |
| 76 | E1A binding protein p300 | p300, E1A binding protein p300, E1A-binding protein, 300kD; E1A-associated protein p300 | EP300 |
| 77 | coagulation factor XIII, A1 polypeptide | Coagulation Factor XIII -Coagulation factor XIII A chain; Coagulation factor XIII, A polypeptide; TGase; (coagulation factor XIII, A1 polypeptide); coagulation factor XIII A1 subunit; factor XIIIa, coagulation factor XIII A1 subunit | F13A1 |
| 78 | coagulation factor VIII, procoagulant component (hemophilia A) | Factor VIII, AHF, F8 protein, F8B, F8C, FVIII, HEMA, coagulation factor VIII; coagulation factor VIII, isoform b; coagulation factor VIIIc; factor VIII F8B; procoagulant component, isoform b | F8 |
| 79 | fatty acid binding protein 4, adipocyte | fatty acid binding protein 4, adipocyte - A-FABP | FABP4 |

(continued)

| DERISKMARKER | Official Name | Common Name | Entrez Gene Link |
|---|---|---|---|
| 80 | Fas (TNF receptor superfamily, member 6) | soluble Fas/APO-1 (sFas), ALPS1A, APO-1, APT1, Apo-1 Fas, CD95, FAS1, FASTM, TNFRSF6, APO-1 cell surface antigen; CD95 antigen; Fas antigen; apoptosis antigen 1; tumor necrosis factor receptor superfamily, member 6 | FAS |
| 81 | Fas ligand (TNF superfamily, member 6) | Fas ligand (sFasL), APTILG1, CD178, CD95L, FASL, TNFSF6, CD95 ligand; apoptosis (APO-1) antigen ligand 1; fas ligand; tumor necrosis factor (ligand) superfamily, member 6 | FASLG |
| 82 | free fatty acid receptor 1 | G protein-coupled receptor 40 -FFA1R, GPR40, G protein-coupled receptor 40 | FFARI |
| 83 | fibrinogen alpha chain | Fibrin, Fib2, fibrinogen, A alpha polypeptide; fibrinogen, alpha chain, isoform alpha preproprotein; fibrinogen, alpha polypeptide | FGA |
| 84 | forkhead box A2 | (Foxa2); HNF3B; TCF3B; hepatic nuclear factor-3-beta; hepatocyte nuclear factor 3, beta | FOXA2 |
| 85 | forkhead box O1A | FKH1; FKHR; FOXO1; forkhead (Drosophila) homolog 1 (rhabdomyosarcoma); forkhead, Drosophila, homolog of, in rhabdomyosarcoma | FOXO1A |
| 86 | ferritin | FTH; PLIF; FTHL6; PIG15; apoferritin; placenta immunoregulatory factor; proliferation-inducing protein 15 | FTH1 |
| 87 | glutamate decarboxylase 2 | glutamic acid decarboxylase (GAD65) antibodies; Glutamate decarboxylase-2 (pancreas); glutamate decarboxylase 2 (pancreatic islets and brain, 65kD) | GAD2 |
| 88 | galanin | GALN; GLNN; galanin-related peptide | GAL |
| 89 | gastrin | gastrin - GAS | GAST |
| 90 | glucagon | glucagon-like peptide-1, GLP-1, GLP2, GRPP, glicentin-related polypeptide; glucagon-like peptide 1; glucagon-like peptide 2 | GCG |
| 91 | glucokinase | hexokinase 4, maturity to onset Diabetes of the young 2; GK; GLK; HK4; HHF3; HKIV; HXKP; MODY2 | GCK |

(continued)

| DERISKMARKER | Official Name | Common Name | Entrez Gene Link |
|---|---|---|---|
| 92 | gamma-glutamyltransferase 1 | GGT; GTG; CD224; glutamyl transpeptidase; gamma-glutamyl transpeptidase | GGT1 |
| 93 | growth hormone 1 | growth hormone - GH, GH-N, GHN, hGH-N, pituitary growth hormone | GH1 |
| 94 | ghrelin/obestatin preprohormone | ghrelin - MTLRP, ghrelin, obestatin, ghrelin; ghrelin precursor; ghrelin, growth hormone secretagogue receptor ligand; motilin-related peptide | GHRL |
| 95 | gastric inhibitory polypeptide | glucose-dependent insulinotropic peptide | GIP |
| 96 | gastric inhibitory polypeptide receptor | GIP Receptor | GIPR |
| 97 | glucagon-like peptide 1 receptor | glucagon-like peptide 1 receptor | GLP1R |
| 98 | guanine nucleotide binding protein (G protein), beta polypeptide 3 | G-protein beta-3 subunit - G protein, beta-3 subunit; GTP-binding regulatory protein beta-3 chain; guanine nucleotide-binding protein G(I)/G(S)/G(T) beta subunit 3; guanine nucleotide-binding protein, beta-3 subunit; hypertension associated protein; transducin beta chain 3 | GNB3 |
| 99 | glutamic-pyruvate transaminase (alanine aminotransferase) | glutamic-pyruvate transaminase (alanine aminotransferase), AAT1, ALT1, GPT1 | GPT |
| 100 | gastrin releasing peptide (bombesin) | bombesin; BN; GRP-10; proGRP; preproGRP; neuromedin C; pre-progastrin releasing peptide | GRP |
| 101 | gelsolin (amyloidosis, Finnish type) | gelsolin | GSN |
| 102 | hemoglobin | CD31; alpha-1 globin; alpha-1-globin; alpha-2 globin; alpha-2-globin; alpha one globin; hemoglobin alpha 2; hemoglobin alpha-2; hemoglobin alpha-1 chain; hemoglobin alpha 1 globin chain, NCBI Reference Sequences(RefSeq) | HBA1 |
| 103 | hemoglobin, beta | HBD, beta globin | HBB |
| 104 | hypocretin (orexin) neuropeptide precursor | orexin A; OX; PPOX | HCRT |

(continued)

| DERISKMARKER | Official Name | Common Name | Entrez Gene Link |
|---|---|---|---|
| 105 | hepatocyte growth factor (hepapoietin A; scatter factor) | Hepatocyte growth factor (HGF) - F-TCF, HGFB, HPTA, SF, fibroblast-derived tumor cytotoxic factor; hepatocyte growth factor; hepatopoietin A; lung fibroblast-derived mitogen; scatter factor | HGF |
| 106 | hepatocyte nuclear factor 4, alpha | hepatocyte nuclear factor 4 -HNF4, HNF4a7, HNF4a8, HNF4a9, MODY, MODY1, NR2A1, NR2A21, TCF, TCF14, HNF4-alpha; hepatic nuclear factor 4 alpha; hepatocyte nuclear factor 4 alpha; transcription factor-14 | HNF4A |
| 107 | haptoglobin | haptoglobin - hp2-alpha | HP |
| 108 | hydroxysteroid (11-beta) dehydrogenase 1 | Corticosteroid 11-beta-dehydrogenase, isozyme 1; HDL; 11-DH; HSD11; HSD118; HSD11L; 11-beta-HSD1 | HSD11B1 |
| 109 | heat shock 70kDa protein 1B | HSP70-2, heat shock 70kD protein 1B | HSPA1B |
| 110 | islet amyloid polypeptide | Amylin - DAP, IAP, Islet amyloid polypeptide (Diabetes-associated peptide; amylin) | IAPP |
| 111 | intercellular adhesion molecule 1 (CD54), human rhinovirus receptor | soluble intercellular adhesion molecule-1, BB2, CD54, P3.58, 60 bp after segment 1; cell surface glycoprotein; cell surface glycoprotein P3.58; intercellular adhesion molecule 1 | ICAM1 |
| 112 | interferon, gamma | IFNG: IFG; IFI | IFNG |
| 113 | insulin-like growth factor 1 (somatomedin C) | IGF-1: somatomedin C. insulin-like growth factor-1 | IGF1 |
| 114 | insulin-like growth factor 2 (somatomedin A) | IGF-II polymorphisms (somatomedin A) - C11 orf43, INSIGF, pp9974, insulin-like growth factor 2; insulin-like growth factor II; insulin-like growth factor type 2; putative insulin-like growth factor II associated protein | IGF2 |

(continued)

| DERISKMARKER | Official Name | Common Name | Entrez Gene Link |
|---|---|---|---|
| 115 | insulin-like growth factor binding protein 1 | insulin-like growth factor binding protein-1 (IGFBP-1)-AFBP, IBP1, IGF-BP25, PP12, hIGFBP-1, IGF-binding protein 1; alpha-pregnancy-associated endometrial globulin; amniotic fluid binding protein; binding protein-25; binding protein-26; binding protein-28; growth hormone independent-binding protein; placental protein 12 | IGFBP1 |
| 116 | insulin-like growth factor binding protein 3 | insulin-like growth factor binding protein 3: IGF-binding protein 3 - BP-53, IBP3, IGF-binding protein 3; acid stable subunit of the 140 K IGF complex; binding protein 29; binding protein 53; growth hormone-dependent binding protein | IGFBP3 |
| 117 | inhibitor of kappa light polypeptide gene enhancer in B-cells, kinase beta | ikk-beta; IKK2; IKKB; NFKBIKB; IKK-beta; nuclear factor NF-kappa-B inhibitor kinase beta; inhibitor of nuclear factor kappa B kinase beta subunit | IKBKB |
| 118 | interleukin 10 | IL-10, CSIF, IL-10, IL10A, TGIF, cytokine synthesis inhibitory factor | IL10 |
| 119 | interleukin 18 (interferon-gamma-inducing factor) | IL-18 - IGIF, IL-18, IL-1g, IL1F4, IL-1 gamma; interferon-gamma-inducing factor; interleukin 18; interleukin-1 gamma; interleukin-18 | IL18 |
| 120 | interleukin I, alpha | IL 1 - IL-1A, IL1, IL1-ALPHA, IL1F1, IL1A (IL1F1); hematopoietin-1; preinterleukin 1 alpha; pro-interleukin-1-alpha | IL1A |
| 121 | interleukin 1, beta | interleukin-1 beta (IL-1 beta) -IL-1, IL1-BETA, IL1F2, catabolin; preinterleukin 1 beta; pro-interleukin-1-beta | ILIB |
| 122 | interleukin 1 receptor antagonist | interleukin-1 receptor antagonist (IL-1Ra) - ICIL-1RA, IL-1ra3, IL1F3, IL1RA, IRAP, IL1RN (IL1F3); intracellular IL-1 receptor antagonist type II; intracellular interleukin-1 receptor antagonist (icIL-1ra); type II interleukin-1 receptor antagonist | IL1RN |

(continued)

| DERISKMARKER | Official Name | Common Name | Entrez Gene Link |
|---|---|---|---|
| 123 | interleukin 2 | interleukin-2 (IL-2) - IL-2, TCGF, lymphokine, T cell growth factor; aldesleukin; interleukin-2; involved in regulation of T-cell clonal expansion | IL2 |
| 124 | interleukin 6 (interferon, beta 2) | Interleukin-6 (IL-6), BSF2, HGF, HSF, IFNB2, IL-6 | IL6 |
| 125 | interleukin 6 receptor | interleukin-6 receptor, soluble (sIL-6R) - CD126, IL-6R-1, IL-6R-alpha, IL6RA, CD126 antigen; interleukin 6 receptor alpha subunit | IL6R |
| 126 | interleukin 8 | Interleukin-8 (IL-8), 3-10C, AMCF-I, CXCL8, GCP-1, GCP1, IL-8, K60, LECT, LUCT, LYNAP, MDNCF, MONAP, NAF, NAP-1, NAP1, SCYB8, TSG-1, b-ENAP, CXC chemokine ligand 8; LUCT/ interleukin-8; T cell chemotactic factor; beta-thromboglobulin-like protein; chemokine (C-X-C motif) ligand 8; emoctakin; granulocyte chemotactic protein 1; lymphocyte-derived neutrophil-activating factor; monocyte derived neutrophil-activating protein; monocyte-derived neutrophil chemotactic factor; neutrophil-activating factor; neutrophil-activating peptide 1; neutrophil-activating protein 1; protein 3-10C; small inducible cytokine subfamily B, member 8 | IL8 |
| 127 | inhibin, beta A (activin A, activin AB alpha polypeptide) | activin A - EDF, FRP, Inhibin, beta-1; inhibin beta A | INHBA |
| 128 | insulin | insulin, proinsulin | INS |
| 129 | insulin receptor | CD220, HHF5 | INSR |
| 130 | insulin promoter factor-1 | IPF-1, PDX-1 (pancreatic and duodenal homeobox factor-1) | IPF1 |
| 131 | insulin receptor substrate 1 | HIRS-1 | IRS1 |
| 132 | insulin receptor substrate-2 | IRS2 | IRS2 |
| 133 | potassium inwardly-rectifying channel, subfamily J, member 11 | ATP gated K+ channels, Kir 6.2; BIR; HHF2; PHHI; IKATP; KIR6.2 | KCNJ1 |
| 134 | potassium inwardly-rectifying channel, subfamily J, member 8 | ATP gated K+ channels, Kir 6.1 | KCNJ8 |
| 135 | klotho | klotho | KL |

(continued)

| DERISKMARKER | Official Name | Common Name | Entrez Gene Link |
|---|---|---|---|
| 136 | kallikrein B, plasma (Fletcher factor) 1 | kallikrein 3 - KLK3 - Kallikrein, plasma; kallikrein 3, plasma; kallikrein B plasma; kininogenin; plasma kallikrein B1 | KLKB1 |
| 137 | leptin (obesity homolog, mouse) | leptin - OB, OBS, leptin; leptin (murine obesity homolog); obesity; obesity (murine homolog, leptin) | LEP |
| 138 | leptin receptor | leptin receptor, soluble - CD295, OBR, OB receptor | LEPR |
| 139 | legumain | putative cysteine protease 1 - AEP, LGMN1, PRSC1, asparaginyl endopeptidase; cysteine protease 1; protease, cysteine, 1 (legumain) | LGMN |
| 140 | lipoprotein, Lp(a) | lipoprotein (a) [Lp(a)], AK38, APOA, LP, Apolipoprotein Lp(a); antiangiogenic AK38 protein; apolipoprotein(a) | LPA |
| 141 | lipoprotein lipase | LPL - LIPD | LPL |
| 142 | v-maf musculoaponeurotic fibrosarcoma oncogene homolog A (avian) | MafA (transcription factor) - RIPE3b1, hMafA, v-maf musculoaponeurotic fibrosarcoma oncogene homolog A | MAFA |
| 143 | mitogen-activated protein kinase 8 interacting protein 1 | IB1, JIP-1, JIP1, PRKM8IP, JNK-interacting protein 1; PRKM8 interacting protein; islet-brain 1 | MAPK8IP1 |
| 144 | mannose-binding lectin (protein C) 2, soluble (opsonic defect) | COLEC1, HSMBPC, MBL, MBP, MBP1, Mannose-binding lectin 2, soluble (opsonic defect); mannan-binding lectin; mannan-binding protein; mannose binding protein; mannose-binding protein C; soluble mannose-binding lectin | MBL2 |
| 145 | melanocortin 4 receptor | G protein coupled receptor MC4 | MC4R |
| 146 | melanin-concentrating hormone receptor I | G Protein-Coupled Receptor 24 - GPR24, MCHIR, SLCI, G protein-coupled receptor 24; G-protein coupled receptor 24 isoform 1, GPCR24 | MCHR1 |
| 147 | matrix metallopeptidase 12 (macrophage elastase) | Matrix Metalloproteinases (MMP), HME, MME, macrophage elastase; macrophage metalloelastase; matrix metalloproteinase 12; matrix metalloproteinase 12 (macrophage elastase) | MMP12 |

(continued)

| DERISKMARKER | Official Name | Common Name | Entrez Gene Link |
|---|---|---|---|
| 148 | matrix metallopeptidase 14 (membrane-inserted) | Matrix Metalloproteinases (MMP), MMP-X1, MT1-MMP, MTMMP1, matrix metalloproteinase 14; matrix metalloproteinase 14 (membrane-inserted); membrane type 1 metalloprotease; membrane-type matrix metalloproteinase 1; membrane-type-1 matrix metalloproteinase | MMP14 |
| 149 | matrix metallopeptidase 2 (gelatinase A, 72kDa gelatinase, 72kDa type IV collagenase) | Matrix Metalloproteinases (MMP), MMP-2, CLG4, CLG4A, MMP-II, MONA, TBE-1, 72kD type IV collagenase; collagenase type IV-A; matrix metalloproteinase 2; matrix metalloproteinase 2 (gelatinase A, 72kD gelatinase, 72kD type IV collagenase); matrix metalloproteinase 2 (gelatinase A, 72kDa gelatinase, 72kDa type IV collagenase); matrix metalloproteinase-II; neutrophil gelatinase | MMP2 |
| 150 | matrix metallopeptidase 9 (gelatinase B, 92kDa gelatinase, 92kDa type IV collagenase) | Matrix Metalloproteinases (MMP), MMP-9, CLG4B, GELB, 92kD type IV collagenase; gelatinase B; macrophage gelatinase; matrix metalloproteinase 9; matrix metalloproteinase 9 (gelatinase B, 92kD gelatinase, 92kD type IV collagenase); matrix metalloproteinase 9 (gelatinase B, 92kDa gelatinase, 92kDa type IV collagenase); type V collagenase | MMP9 |
| 151 | nuclear receptor co-repressor 1 | NCoR; thyroid hormone- and retinoic acid receptor-associated corepressor 1 | NCOR1 |
| 152 | neurogenic differentiation 1 | neuroD (transcription factor) -BETA2, BHF-1, NEUROD | NEUROD1 |
| 153 | nuclear factor of kappa light polypeptide gene enhancer in B-cells 1(p105) | nuclear factor, kappa B (NFKB); DNA binding factor KBF1; nuclear factor NF-kappa-B p50 subunit; nuclear factor kappa-B DNA binding subunit | NFKB1 |
| 154 | nerve growth factor, beta polypeptide | B-type neurotrophic growth factor (BNGF) - beta-nerve growth factor; nerve growth factor, beta subunit | NGFB |
| 155 | non-insulin-dependent Diabetes Mellitus (common, type 2) 1 | NIDDMI | NIDDM1 |
| 156 | non-insulin-dependent Diabetes Mellitus (common, type 2) 2 | NIDDM2 | NIDDM2 |

(continued)

| DERISKMARKER | Official Name | Common Name | Entrez Gene Link |
|---|---|---|---|
| 157 | Noninsulin-dependent Diabetes Mellitus 3 | NIDDM3 | NIDDM3 |
| 158 | nischarin (imidazoline receptor) | imidazoline receptor; IRAS; I-1 receptor candidate protein; imidazoline receptor candidate; imidazoline receptor antisera selected | NISCH |
| 159 | NF-kappaB repressing factor | NRF; ITBA4 gene; transcription factor NRF; NF-kappa B repressing factor; NF-kappa B-repressing factor | NKRF |
| 160 | neuronatin | Peg5 | NNAT |
| 161 | nitric oxide synthase 2A | NOS, type II; nitric oxide synthase, macrophage | NOS2A |
| 162 | Niemann-Pick disease, type C2 | epididymal secreting protein 1 -HE1, NP-C2, epididymal secretory protein; epididymal secretory protein E1; tissue-specific secretory protein | NPC2 |
| 163 | natriuretic peptide precursor B | B-type Natriuretic Peptide (BNP), BNP, brain type natriuretic peptide, pro-BNP?, NPPB | NPPB |
| 164 | nuclear receptor subfamily 1, group D, member 1 | Human Nuclear Receptor NRIDI -EAR1, THRA1, THRAL, ear-1, hRev, Rev-erb-alpha; thyroid hormone receptor, alpha-like | NR1D1 |
| 165 | nuclear respiratory factor 1 | NRF1; ALPHA-PAL; alpha palindromic-binding protein | NRF1 |
| 166 | oxytocin, prepro- (neurophysin I) | oxytocin - OT, OT-NPI, oxytocin-neurophysin I; oxytocin-neurophysin I, preproprotein | OXT |
| 167 | purinergic receptor P2Y, G-protein coupled, 10 | G Protein Coupled Receptor P2Y10 - P2Y10, G-protein coupled purinergic receptor P2Y10; P2Y purinoceptor 10; P2Y-like receptor | P2RY10 |
| 168 | purinergic receptor P2Y, G-protein coupled, 12 | G Protein- Coupled Receptor P2Y12 - ADPG-R, HORK3, P2T (AC), P2Y(AC), P2Y(ADP), P2Y (cyc), P2Y 12, SP1999, ADP-glucose receptor; G-protein coupled receptor SP1999; Gi-coupled ADP receptor HORK3; P2Y purinoceptor 12; platelet ADP receptor; purinergic receptor P2RY12; purinergic receptor P2Y, G-protein coupled 12; purinergic receptor P2Y12; putative G-protein coupled receptor | P2RY12 |

(continued)

| DERISKMARKER | Official Name | Common Name | Entrez Gene Link |
|---|---|---|---|
| 169 | purinergic receptor P2Y, G-protein coupled, 2 | Purinoceptor 2 Type Y (P2Y2) - HP2U, P2RU1, P2U, P2U1, P2UR, P2Y2, P2Y2R, ATP receptor; P2U nucleotide receptor; P2U purinoceptor 1; P2Y purinoceptor 2; purinergic receptor P2Y2; purinoceptor P2Y2 | P2RY2 |
| 170 | progestagen-associated endometrial protein (placental protein 14, pregnancy-associated endometrial alpha-2-globulin, alpha uterine protein) | glycodelin-A; glycodelin-F; glycodelin-S; progesterone-associated endometrial protein | PAEP |
| 171 | paired box gene 4 | Pax4 (transcription factor) -paired domain gene 4 | PAX4 |
| 172 | pre-B-cell colony enhancing factor 1 | visfatin; nicotinamide phosphoribosyltransferase | PBEF1 |
| 173 | phosphoenolpyruvate carboxykinase 1 (PEPCK1) | PEPCK1; PEP carboxykinase; phosphopyruvate carboxylase; phosphoenolpyruvate carboxylase | PCK1 |
| 174 | proprotein convertase subtilisin/ kexin type 1 | proprotein convertase 1 (PC1, PC3, PCSK1, cleaves pro-insulin) | PCSK1 |
| 175 | placental growth factor, vascular endothelial growth factor-related protein | placental growth factor -PLGF, PlGF-2 | PGF |
| 176 | phosphoinositide-3-kinase, catalytic, alpha polypeptide | PI3K, p110-alpha, PI3-kinase p110 subunit alpha; PtdIns-3-kinase p110; phosphatidylinositol 3-kinase, catalytic, 110-KD, alpha; phosphatidylinositol 3-kinase, catalytic, alpha polypeptide; phosphatidylinositol-4,5-bisphosphate 3-kinase catalytic subunit, alpha isoform | PIK3CA |
| 177 | phosphoinositide-3-kinase, regulatory subunit 1 (p85 alpha) | phophatidylinositol 3-kinase; phosphatidylinositol 3-kinase, regulatory, 1; phosphatidylinositol 3-kinase-associated p-85 alpha; phosphoinositide-3-kinase, regulatory subunit, polypeptide 1 (p85 alpha); phosphatidylinositol 3-kinase, regulatory subunit, polypeptide 1 (p85 alpha) | PIK3R1 |
| 178 | phospholipase A2, group XIIA | PLA2G12, group XII secreted phospholipase A2; group XIIA secreted phospholipase A2 | PLA2G12A |
| 179 | phospholipase A2, group IID | phospholipase A2, secretory - SPLASH, sPLA2S, secretory phospholipase A2s | PLA2G2D |

(continued)

| DERISKMARKER | Official Name | Common Name | Entrez Gene Link |
|---|---|---|---|
| 180 | plasminogen activator, tissue | tissue Plasminogen Activator (tPA), T-PA, TPA, alteplase; plasminogen activator, tissue type; reteplase; t-plasminogen activator; tissue plasminogen activator (t-PA) | PLAT |
| 181 | patatin-like phospholipase domain containing 2 | Adipose tissue lipase, ATGL - ATGL, TTS-2.2, adipose triglyceride lipase; desnutrin; transport-secretion protein 2.2; triglyceride hydrolase | PNPLA2 |
| 182 | proopiomelanocortin (adrenocorticotropin/ beta-lipotropin/ alpha-melanocyte stimulating hormone/ beta-melanocyte stimulating hormone/ beta-endorphin) | proopiomelanocortin - beta-LPH; beta-MSH; alpha-MSH; gamma-LPH; gamma-MSH; corticotropin; beta-endorphin; met-enkephalin; lipotropin beta; lipotropin gamma; melanotropin beta; N-terminal peptide; melanotropin alpha; melanotropin gamma; pro-ACTH-endorphin; adrenocorticotropin; pro-opiomelanocortin; corticotropin-lipotrophin; adrenocorticotropic hormone; alpha-melanocyte-stimulating hormone; corticotropin-like intermediary peptide | POMC |
| 183 | paraoxonase 1 ESA, PON, Paraoxonase | paraoxonase - ESA, PON, Paraoxonase | PON1 |
| 184 | peroxisome proliferative activated receptor, alpha | Peroxisome proliferator-activated receptor (PPAR), NR1C1, PPAR, hPPAR, PPAR alpha | PPARA |
| 185 | peroxisome proliferative activated receptor, delta | Peroxisome proliferator-activated receptor (PPAR), FAAR, NR1C2, NUC1, NUCI, NUCII, PPAR-beta, PPARB, nuclear hormone receptor 1, PPAR Delta | PPARD |
| 186 | peroxisome proliferative activated receptor, gamma | Peroxisome proliferator-activated receptor (PPAR), HUMPPARG, NR1C3, PPARG1, PPARG2, PPAR gamma; peroxisome proliferative activated receptor gamma; peroxisome proliferator activated-receptor gamma; peroxisome proliferator-activated receptor gamma 1; ppar gamma2 | PPARG |
| 187 | peroxisome proliferative activated receptor, gamma, coactivator 1 | Pgc1 alpha; PPAR gamma coactivator-1; ligand effect modulator-6; PPAR gamma coactivator variant form3 | PPARGC1A |

(continued)

| DERISKMARKER | Official Name | Common Name | Entrez Gene Link |
|---|---|---|---|
| 188 | protein phosphatase 1, regulatory (inhibitor) subunit 3A (glycogen and sarcoplasmic reticulum binding subunit, skeletal muscle) | PP1G, PPPIR3, protein phosphatase 1 glycogen-associated regulatory subunit; protein phosphatase 1 glycogen-binding regulatory subunit 3; protein phosphatase type-1 glycogen targeting subunit; serine /threonine specific protein phosphatase; type-1 protein phosphatase skeletal muscle glycogen targeting subunit | PPP1R3A |
| 189 | protein phosphatase 2A, regulatory subunit B' (PR 53) | protein phosphatase 2A - PP2A, PR53, PTPA, PP2A, subunit B'; phosphotyrosyl phosphatase activator; protein phosphatase 2A, regulatory subunit B' | PPP2R4 |
| 190 | protein kinase, AMP-activated, beta 1 non-catalytic subunit | on list as adenosine monophosphate kinase? -AMPK, HAMPKb, 5'-AMP-activated protein kinase beta-1 subunit; AMP-activated protein kinase beta 1 non-catalytic subunit; AMP-activated protein kinase beta subunit; AMPK beta -1 chain; AMPK beta 1; protein kinase, AMP-activated, noncatalytic, beta-1 | PRKAB1 |
| 191 | protein kinase, cAMP-dependent, catalytic, alpha | PKA (kinase) - PKACA, PKA C-alpha; cAMP-dependent protein kinase catalytic subunit alpha; cAMP-dependent protein kinase catalytic subunit alpha, isoform 1; protein kinase A catalytic subunit | PRKACA |
| 192 | protein kinase C, epsilon | PKC-epsilon - PKCE, nPKC-epsilon | PRKCE |
| 193 | proteasome (prosome, macropain) 26S subunit, non-ATPase, 9 (Bridge-1) | Bridge-1; homolog of rat Bridge 1; 26S proteasome regulatory subunit p27; proteasome 26S non-ATPase regulatory subunit 9 | PSMD9 |
| 194 | prostaglandin E synthase | mPGES - MGST-IV, MGST1-L1, MGST1L1, PGES, PIG12, PP102, PP1294, TP53I12 Other Designations: MGST1-like 1; glutathione S-transferase 1-like 1; microsomal glutathione S-transferase 1-like 1; p53-induced apoptosis protein 12; p53-induced gene 12; tumor protein p53 inducible protein 12 | PTGES |

(continued)

| DERISKMARKER | Official Name | Common Name | Entrez Gene Link |
|---|---|---|---|
| 195 | prostaglandin-endoperoxide synthase 2 (prostaglandin G/H synthase and cyclooxygenase) | Cyclo-oxygenase-2 (COX-2) -COX-2, COX2, PGG/HS, PGHS-2, PHS-2, hCox-2, cyclooxygenase 2b; prostaglandin G/H synthase and cyclooxygenase; prostaglandin-endoperoxide synthase 2 | PTGS2 |
| 196 | protein tyrosine phosphatase, mitochondrial 1 | PTPMT1 - PLIP, PNAS-129, NB4 apoptosis/differentiation related protein; PTEN-like phosphatase | PTPMT1 |
| 197 | Peptide YY | PYY1 | PYY |
| 198 | retinol binding protein 4, plasma (RBP4) | RBP4; retinol-binding protein 4, plasma; retinol-binding protein 4, interstitial | RBP4 |
| 199 | regenerating islet-derived 1 alpha (pancreatic stone protein, pancreatic thread protein) | regenerating gene product (Reg); protein-X; lithostathine 1 alpha; pancreatic thread protein; regenerating protein I alpha; islet cells regeneration factor; pancreatic stone protein, secretory; islet of langerhans regenerating protein | REG1A |
| 200 | resistin | resistin - ADSF, FIZZ3, RETN1, RSTN, XCP1, C/EBP-epsilon regulated myeloid-specific secreted cysteine-rich protein precursor 1; found in inflammatory zone 3 | RETN |
| 201 | ribosomal protein S6 kinase, 90kDa, polypeptide 1 | S6-kinase 1 - HU-1, RSK, RSK1, S6K-alpha 1, (ribosomal protein S6 kinase, 90kD, polypeptide 1); p90-RSK 1; ribosomal protein S6 kinase alpha 1; ribosomal protein S6 kinase, 90kD, 1; ribosomal protein S6 kinase, 90kD, polypeptide 1 | RPS6KA1 |
| 202 | Ras-related associated with Diabetes | RAD, RADI, REM3, RAS (RAD and GEM) like GTP binding 3 | RRAD |
| 203 | serum amyloid A1 | Serum Amyloid A (SAA), PIG4, SAA, TP53I4, tumor protein p53 inducible protein 4 | SAA1 |
| 204 | selectin E (endothelial adhesion molecule 1) | E-selectin, CD62E, ELAM, ELAM1, ESEL, LECAM2, leukocyte endothelial cell adhesion molecule 2; selectin E, endothelial adhesion molecule 1 | SELE |

(continued)

| DERISKMARKER | Official Name | Common Name | Entrez Gene Link |
|---|---|---|---|
| 205 | serpin peptidase inhibitor, clade A (alpha-1 antiproteinase, antitrypsin), member 6 | corticosteroid-binding globulin; transcortin; corticosteroid binding globulin; serine (or cysteine) proteinase inhibitor, clade A (alpha-1 antiproteinase, antitrypsin), member 6 | SERPINA6 |
| 206 | serpin peptidase inhibitor, clade E (nexin, plasminogen activator inhibitor type 1), member 1 | plasminogen activator inhibitor-1 - PAI, PAI-1, PAI1, PLANH1, plasminogen activator inhibitor, type I; plasminogen activator inhibitor-1; serine (or cysteine) proteinase inhibitor, clade E (nexin, plasminogen activator inhibitor type 1), member 1 | SERPINE1 |
| 207 | serum/glucocorticoid regulated kinase | Serum/Glucocorticoid Regulated Kinase 1 - SGK1, serine/threonine protein kinase SGK; serum and glucocorticoid regulated kinase | SGK |
| 208 | sex hormone-binding globulin | sex hormone-binding globulin (SHBG) - ABP, Sex hormone-binding globulin (androgen binding protein) | SHBG |
| 209 | thioredoxin interacting protein | Sirt1; SIR2alpha; sir2-like 1; sirtuin type 1; sirtuin (silent mating type information regulation 2, S. cerevisiae, homolog) 1 | SIRT1 |
| 210 | solute carrier family 2, member 10 | glucose transporter 10 (GLUT10); ATS | SLC2A10 |
| 211 | solute carrier family 2, member 2 | glucose transporter 2 (GLUT2) | SLC2A2 |
| 212 | solute carrier family 2, member 4 | glucose transporter 4 (GLUT4) | SLC2A4 |
| 213 | solute carrier family 7 (cationic amino acid transporter, y+ system), member 1(ERR) | ERR - ATRC1, CAT-1, ERR, HCAT1, REC1L, amino acid transporter, cationic 1; ecotropic retroviral receptor | SLC7A1 |
| 214 | SNF1-like kinase 2 | Sik2; salt-inducible kinase 2; salt-inducible serine/threonine kinase 2 | SNF1LK2 |
| 215 | suppressor of cytokine signaling 3 | CIS3, Cish3, SOCS-3, SSI-3, SSI3, STAT induced STAT inhibitor 3; cytokine-induced SH2 protein 3 | SOCS3 |
| 216 | v-src sarcoma (Schmidt-Ruppin A-2) viral oncogene homolog (avian) | ASV, SRC1, c-SRC, p60-Src, proto-oncogene tyrosine-protein kinase SRC; protooncogene SRC, Rous sarcoma; tyrosine kinase pp60c-src; tyrosine-protein kinase SRC-1 | SRC |
| 217 | sterol regulatory element binding transcription factor | sterol regulatory element-binding protein 1c (SREBP-1c) | SREBF1 |

(continued)

| DERISKMARKER | Official Name | Common Name | Entrez Gene Link |
|---|---|---|---|
| 218 | solute carrier family 2, member 4 | SMST, somatostatin-14, somatostatin-28 | SST |
| 219 | somatostatin receptor 2 | somatostatin receptor subtype 2 | SSTR2 |
| 220 | somatostatin receptor 5 | somatostatin receptor 5 -somatostatin receptor subtype 5 | SSTR5 |
| 221 | transcription factor 1, hepatic; LF-B1, hepatic nuclear factor (TNF1) | HNF1$\alpha$; albumin proximal factor; hepatic nuclear factor 1; maturity onset Diabetes of the young 3; Interferon production regulator factor (HNF1) | TCF1 |
| 222 | transcription factor 2, hepatic; LF-B3; variant hepatic nuclear factor | hepatocyte nuclear factor 2 -FJHN, HNF1B, HNF1beta, HNF2, LFB3, MODY5, VHNF1, transcription factor 2 | TCF2 |
| 223 | transcription factor 7-like 2 (T-cell specific, HMG-box) | TCF7L2 - TCF-4, TCF4 | TCF7L2 |
| 224 | transforming growth factor, beta 1 (Camurati-Engelmann disease) | TGF-beta: TGF-beta 1 protein; diaphyseal dysplasia 1, progressive; transforming growth factor beta 1; transforming growth factor, beta 1; transforming growth factor-beta 1, CED, DPD1, TGFB | TGFB1 |
| 225 | transglutaminase 2 (C polypeptide, protein-glutamine-gamma-glutamyltransferase) | TG2, TGC, C polypeptide; TGase C; TGase-H; protein-glutamine-gamma-glutamyltransferase; tissue transglutaminase; transglutaminase 2; transglutaminase C | TGM2 |
| 226 | thrombospondin 1 | thrombospondin - THBS, TSP, TSP1, thrombospondin-1p180 | THBS1 |
| 227 | thrombospondin, type I, domain containing 1 | TMTSP, UNQ3010, thrombospondin type I domain-containing 1; thrombospondin, type I, domain 1; transmembrane molecule with thrombospondin module | THSD1 |
| 228 | tumor necrosis factor (TNF superfamily, member 2) | TNF-alpha (tumour necrosis factor-alpha) - DIF, TNF-alpha, TNFA, TNFSF2, APCI protein; TNF superfamily, member 2; TNF, macrophage-derived; TNF, monocyte-derived; cachectin; tumor necrosis factor alpha | TNF |

(continued)

| DERISKMARKER | Official Name | Common Name | Entrez Gene Link |
|---|---|---|---|
| 229 | tumor necrosis factor (TNF superfamily, member 2) | tumor necrosis factor receptor 2 - DIF, TNF-alpha, TNFA, TNFSF2, APCI protein; TNF superfamily, member 2; TNF, macrophage-derived; TNF, monocyte-derived; cachectin; tumor necrosis factor alpha | TNF |
| 230 | tumor necrosis factor receptor superfamily, member 1A | tumor necrosis factor receptor 1 gene R92Q polymorphism -CD120a, FPF, TBP1, TNF-R, TNF-R-I, TNF-R55, TNFAR, TNF1, TNFR55, TNFR60, p55, p55-R, p60, tumor necrosis factor binding protein 1; tumor necrosis factor receptor 1; tumor necrosis factor receptor type 1; tumor necrosis factor-alpha receptor | TNFRSFIA |
| 231 | tumor necrosis factor receptor superfamily, member 1B | soluble necrosis factor receptor - CD120b, TBPII, TNF-R-II, TNF-R75, TNFBR, TNFR2, TNFR80, p75, p75TNFR, p75 TNF receptor; tumor necrosis factor beta receptor; tumor necrosis factor binding protein 2; tumor necrosis factor receptor 2 | TNFRSF1B |
| 232 | tryptophan hydroxylase 2 | enzyme synthesizing serotonin; neuronal tryptophan hydroxylase, NTPH | TPH2 |
| 233 | thyrotropin-releasing hormone | thyrotropin-releasing hormone | TRH |
| 234 | transient receptor potential cation channel, subfamily V, member 1 | vanilloid receptor 1 - VRI, capsaicin receptor; transient receptor potential vanilloid Ia; transient receptor potential vanilloid 1b; vanilloid receptor subtype 1, capsaicin receptor; transient receptor potential vanilloid subfamily 1 (TRPV1) | TRPV1 |
| 235 | thioredoxin interacting protein | thioredoxin binding protein 2; upregulated by 1,25-dihydroxyvitamin D-3 | TXNIP |
| 236 | thioredoxin reductase 2 | TR; TR3; SELZ; TRXR2; TR-BETA; selenoprotein Z; thioredoxin reductase 3; thioredoxin reductase beta | TXNRD2 |
| 237 | urocortin 3 (stresscopin) | archipelin, urocortin III, SCP, SPC, UCNIII, stresscopin; urocortin 3 | UCN3 |
| 238 | uncoupling protein 2 (mitochondrial, proton carrier) | UCPH, uncoupling protein 2; uncoupling protein-2 | UCP2 |
| 239 | upstream transcription factor 1 | major late transcription factor 1 | USF1 |

(continued)

| DERISKMARKER | Official Name | Common Name | Entrez Gene Link |
|---|---|---|---|
| 240 | urotensin 2 | PRO1068, U-II, UCN2, UII | UTS2 |
| 241 | vascular cell adhesion molecule 1 | (soluble) vascular cell adhesion molecule-1, CD106, INCAM-100, CD106 antigen, VCAM-1 | VCAM1 |
| 242 | vascular endothelial growth factor | VEGF - VEGFA, VPF, vascular endothelial growth factor A; vascular permeability factor | VEGF |
| 243 | vimentin | vimentin | VIM |
| 244 | vasoactive intestinal peptide | vasoactive intestinal peptide -PHM27 | VIP |
| 245 | vasoactive intestinal peptide receptor 1 | vasoactive intestinal peptide receptor 1 - HVR1, II, PACAP-R-2, RCDI, RDC1, VIPR, VIRG, VPAC1, PACAP type II receptor; VIP receptor, type I; pituitary adenylate cyclase activating polypeptide receptor, type II | VIPR1 |
| 246 | vasoactive intestinal peptide receptor 2 | Vasoactive Intestinal Peptide Receptor 2 - VPAC2 | VIPR2 |
| 247 | von Willebrand factor | von Willebrand factor, FSVWF, VWD, coagulation factor VIII VWF | VWF |
| 248 | Wolfram syndrome 1 (wolframin) | DFNA14, DFNA38, DFNA6, DIDMOAD, WFRS, WFS, WOLFRAMIN | WFS1 |
| 249 | X-ray repair complementing defective repair in Chinese hamster cells 6 | Ku autoantigen, 70kDa; Ku autoantigen p70 subunit; thyroid-lupus autoantigen p70; CTC box binding factor 75 kDa subunit; thyroid autoantigen 70kD (Ku antigen); thyroid autoantigen 70kDa (Ku antigen); ATP-dependent DNA helicase II, 70 kDa subunit | XRCC6 |
| 250 | c-peptide | c-peptide | |
| 251 | cortisol | cortisol - hydrocortisone is the synthetic form | |
| 252 | vitamin D3 | vitamin D3 | |
| 253 | estrogen | estrogen | |
| 254 | estradiol | estradiol | |
| 255 | digitalis-like factor | digitalis-like factor | |
| 256 | oxyntomodulin | oxyntomodulin | |
| 257 | dehydroepiandrosterone sulfate (DHEAS) | dehydroepiandrosterone sulfate (DHEAS) | |
| 258 | serotonin (5-hydroxytryptamine) | serotonin (5-hydroxytryptamine) | |
| 259 | anti-CD38 autoantibodies | anti-CD38 autoantibodies | |
| 260 | gad65 autoantibody | gad65 autoantibody epitopes | |

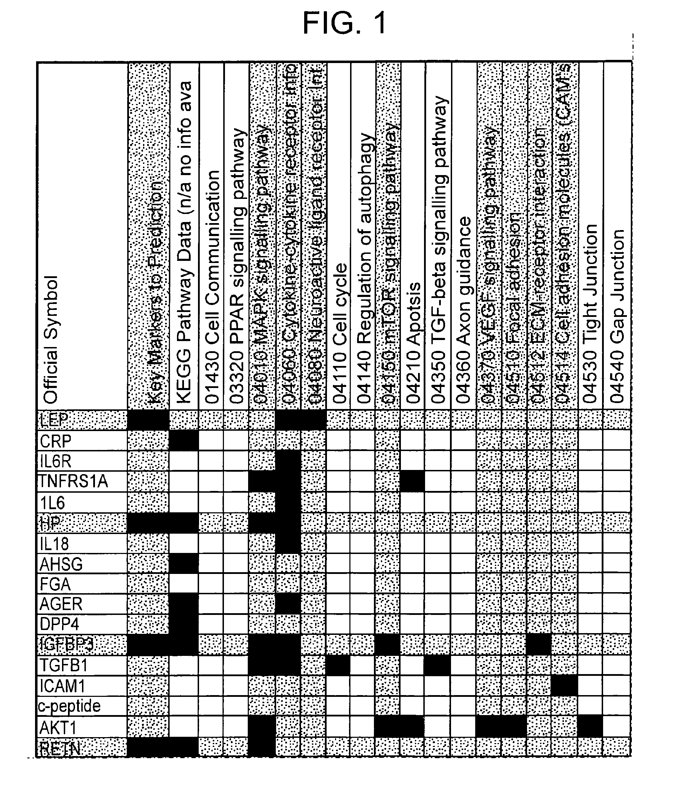

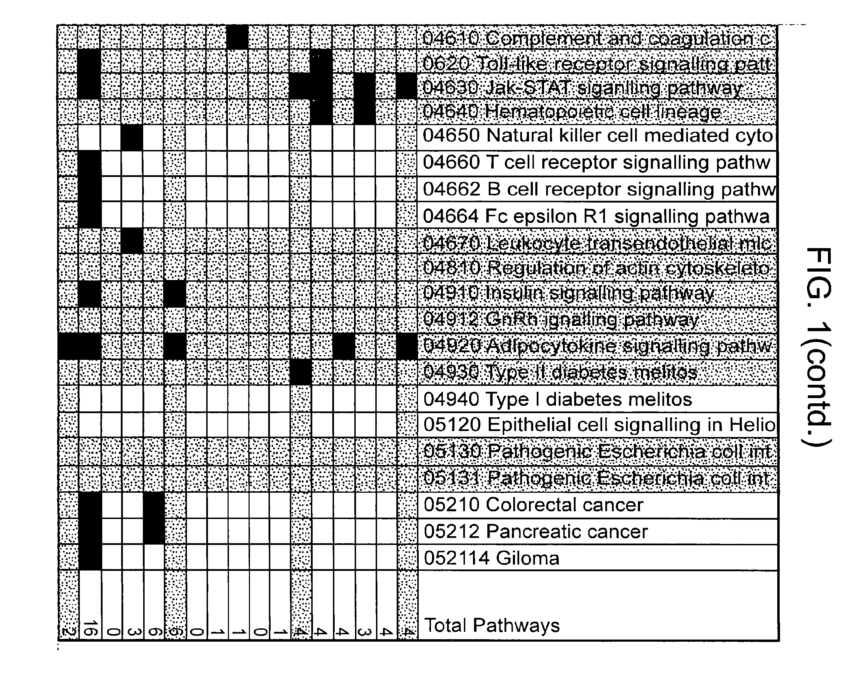

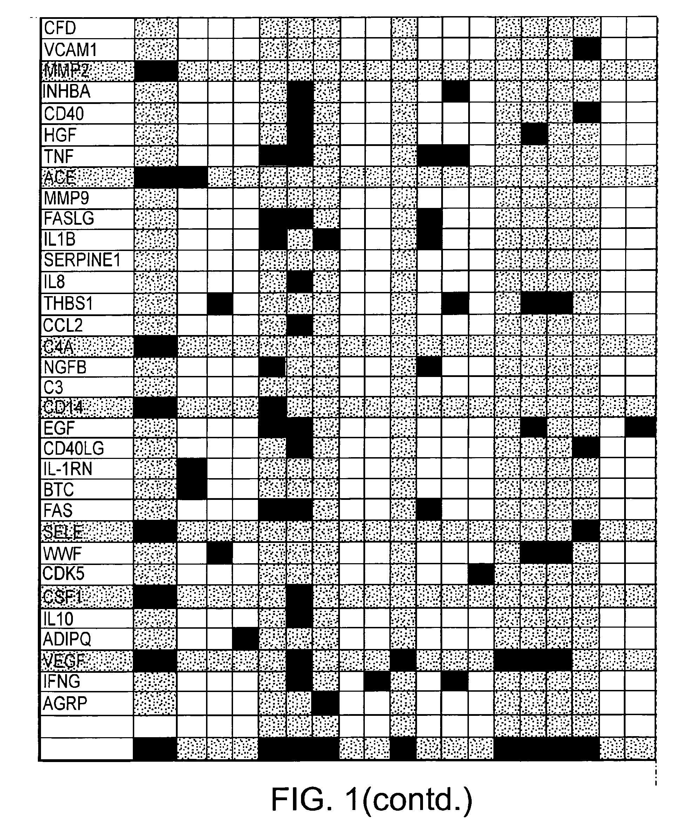

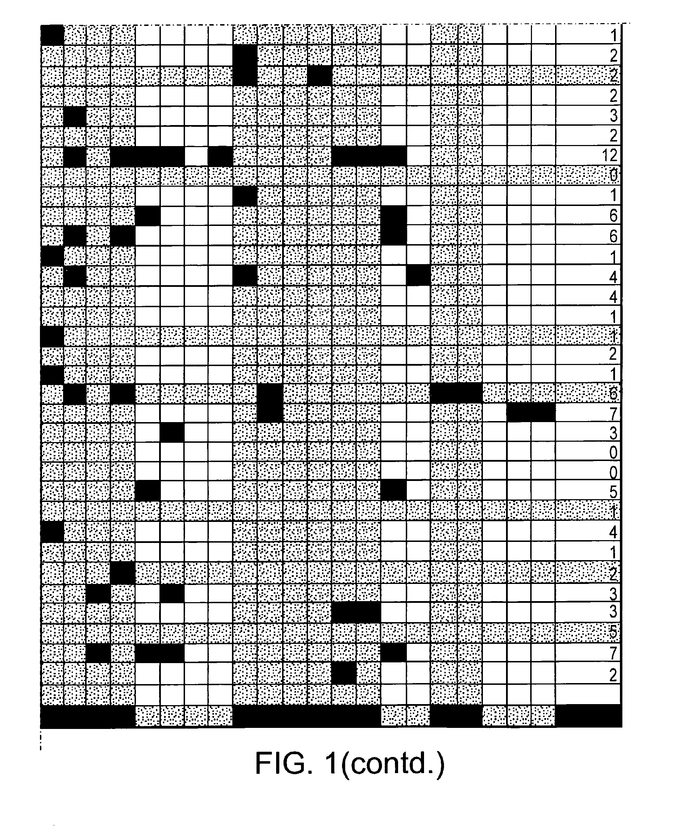

One skilled in the art will note that the above listed DBRISKMARKERS come from a diverse set of physiological and biological pathways, including many which are not commonly accepted to be related to diabetes. For convenience and ease of analysis, a representative subset of approximately fifty of the disclosed DBRISKMARKERS was studied in depth in order to elucidate the more important pathways. Figure 1 is a matrix depicting DBRISKMARKER physiological and biological pathways and categories, with reference to the Kyoto University Encyclopedia of Genes and Genomes (KEGG) pathway numbers and descriptions. These database inquiries to KEGG (and subsequent literature searches to update that database) were combined with experimental work interrogating actual human serum samples from relevant populations cohorts, as detailed below in the Examples section. This was done in order to ascertain the actual levels of expression, translation and blood serum precense of this representative group of DBRISKMARKERS, so as to calibrate the DBRISKMARKER results with respect to Normal, Pre-Diabetes, and Diabetes cohorts.

In Figure 1, the highlighted horizontal rows of the matrix indicate the most significant biomarker signals and algorithm contributors to the DBRISKMARKER panels that constitute the invention. The highlighted vertical columns indicate the KEGG pathways numbers and their descriptions which have representation by the most statistically significant DBRISKMARKERS for the classification of individuals or cohorts with Pre-Diabetes, or prediabetic conditions, from those within Normal non-diabetic populations. The total counts in the bottom row of the figure indicate mentions only due to the highlighted DBRISKMARKERS. Although there was broad general representation across most of the listed pathways by one or another DBRISKMARKERS, a differing concentration of pathways appear evident in the more statistically significant DBRISKMARKERS versus less significant DBRISKMARKERS. As will be detailed below, these groupings of different DBRISKMARKERS even within those high significance segments may presage differing signals of the stage or rate of the progression of the disease.

The strongest signal comes from inflammatory markers concentrated on the cytokine-cytokine receptor and adipocytokine signaling pathways, and significantly the Jak-STAT signalling pathway, which is concentrated in a group of markers including LEP (Leptin) and HP (Haptoglobin). Another overlapping signal also covers the MAPK and insulin signaling pathways and, interestingly, the mTOR signaling pathway, coming from DBRISKMARKERS including ILGFBP3 (Insulin-like growth factor binding protein 3) and such DBRISKMARKERS as VEGF. This group also has the overlapping involvement of ECM-receptor interaction and cell adhesion molecule (CAMs) pathways, together with complement and coagulation cascades and hematopoietic cell lineages and toll-like receptor pathways, perhaps indicating endothelial and vascular changes, and is further represented by CD14 and CSF1 (M-CSF). A final signal, involving the DBRISKMARKERS such as VEGF and SELE (E-Selectin), is concentrated on focal adhesion, ECM and other pathways related to vascular and endothelial remodeling. The kinetics of these expression relative to status of pre-diabetic risk remains to be ascertained and validated, but it is believed that such distinct patterns may allow a more biologically detailed and clinically useful signal from the DBRISKMARKERS as well as opportunities for pattern recognition within the DBRISKMARKER panel algorithms combining the biomarker signals.

The above discussion for convenience focuses on a subset of the DBRISKMARKERS; other DBRISKMARKERS and even biomarkers which are not listed in the above table but related to these physiological and biological pathways may prove to be useful given the signal and information provided from these studies. To the extent that other participants within the total list of DBRISKMARKERS are also relevant pathway participants in Pre-Diabetes they may be functional equivalents to the biomarkers thus far disclosed. DBRISKMARKERS provided they additionally share certain defined characteristics of a good biomarker, which would include both this biological process involvement and also analytically important characterisitics such as the bioavailability of said markers at a useful signal to noise ration, and in a useful sample matrix such as blood serum. Such requirements typically limit the usefulness of many members of a biological KEGG pathway, as this is unlikely to be generally the case, and frequently occurs only in pathway members that constitute secretory substances, those accessible on the plasma membranes of cells, as well as those that are released into the serum upon cell death, due to apotosis or for other reasons such as endothelial remodeling or other cell turnover or cell necrotic processes, whether or not said is related to the disease progression of Pre-Diabetes and Diabetes. However, the remaining and future biomarkers that meet this high standard for DBRISKMARKERS are likely to be quite valuable. Our invention encompasses such functional and statistical equivalents to the aforelisted DBRISKMARKERS. Furthermore, the statistical utility of such additional DBRISKMARKERS is substantially dependent on the cross-correlation between markers and new markers will often be required to operate within a panel in order to elaborate the meaning of the underlying biology.

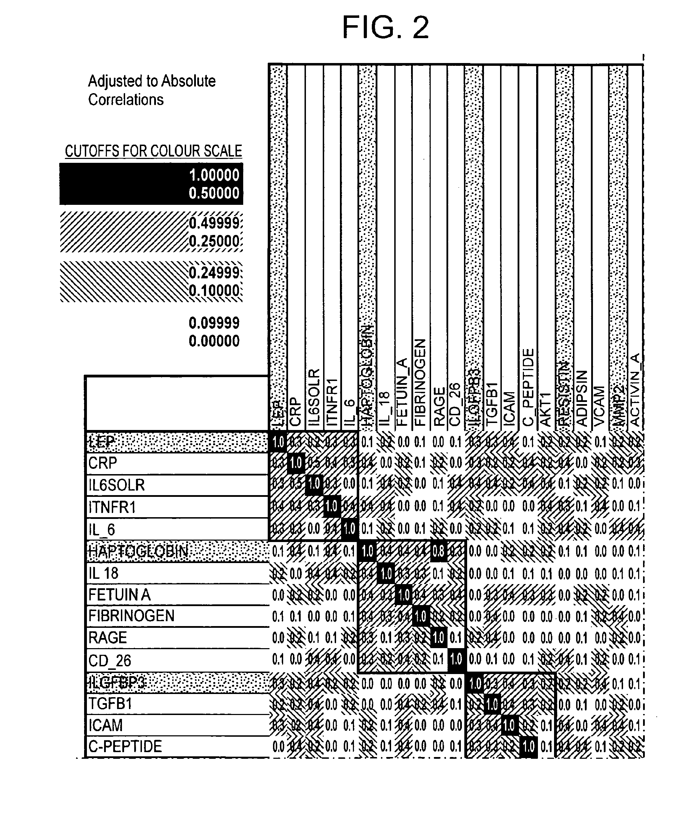

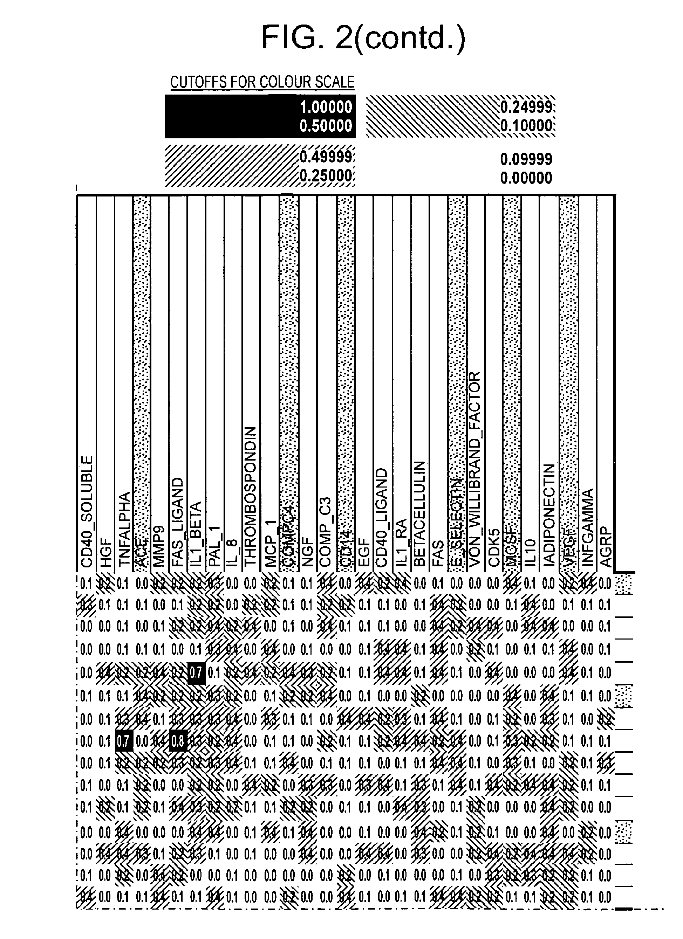

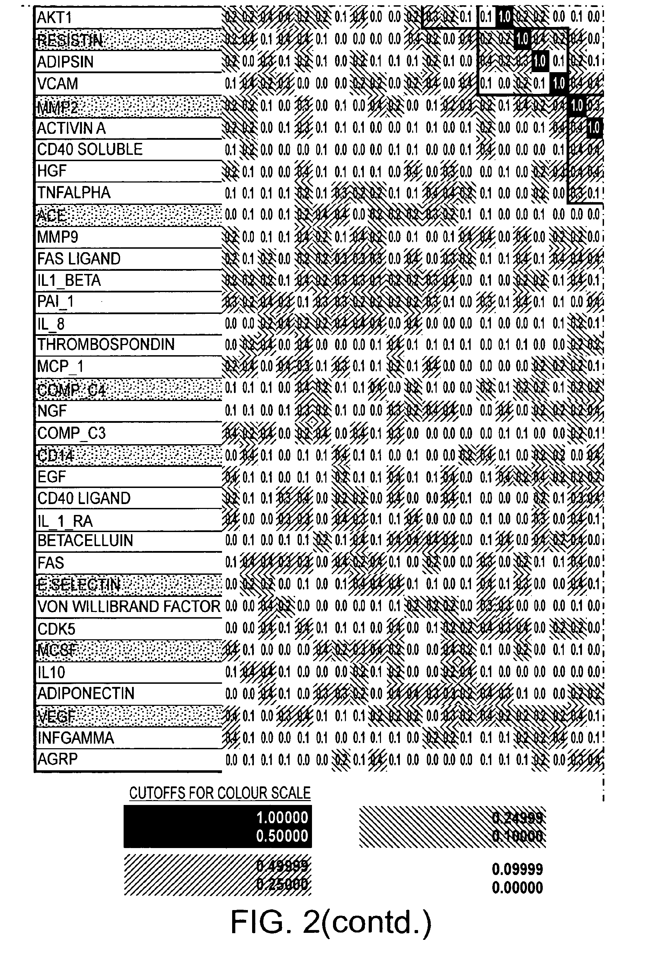

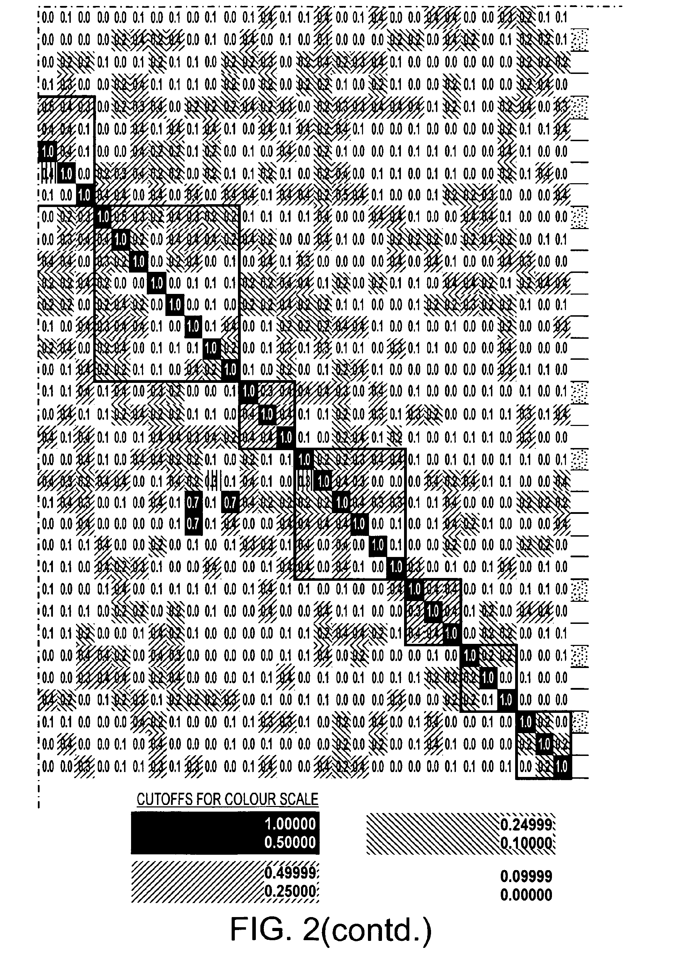

As is shown in Figure 2, many DBRISKMARKERS within the aforementioned representative set of fifty (50) are closely correlated and clustered in groups that thus rise or fall in their concentration with each other (or in opposite directions to each other). While this may offer multiple opportunities for new and useful DBRISKMARKERS within known and previously disclosed biological pathways, our invention hereby anticipates and claims such useful biomarkers that are functional or statistical equivalents to those listed, and such correlations and DBRISKMARKER concentrations are disclosed hereby referenced and disclosed herein, as are the potential identies of other biological pathway members in.

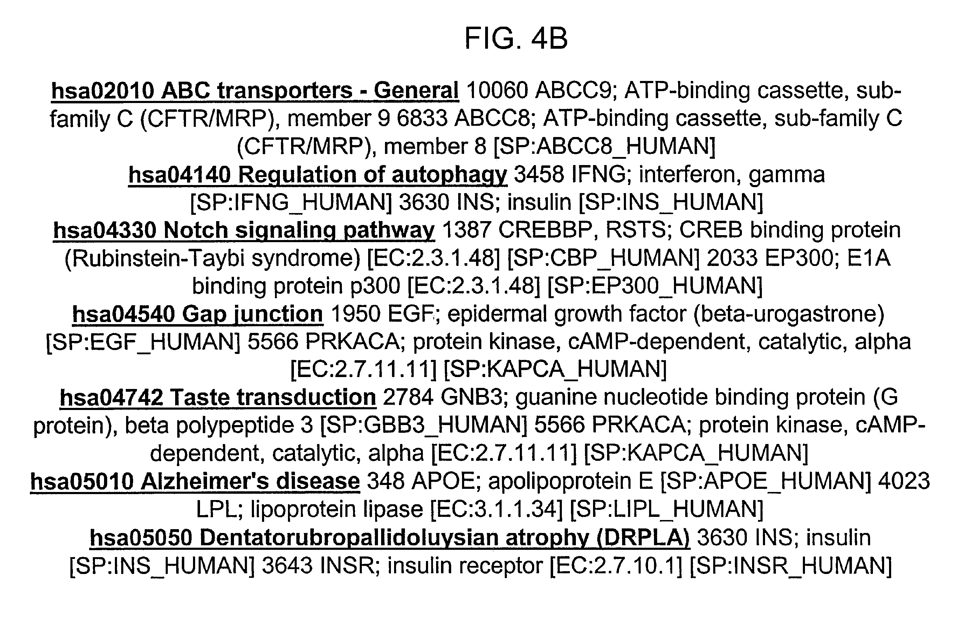

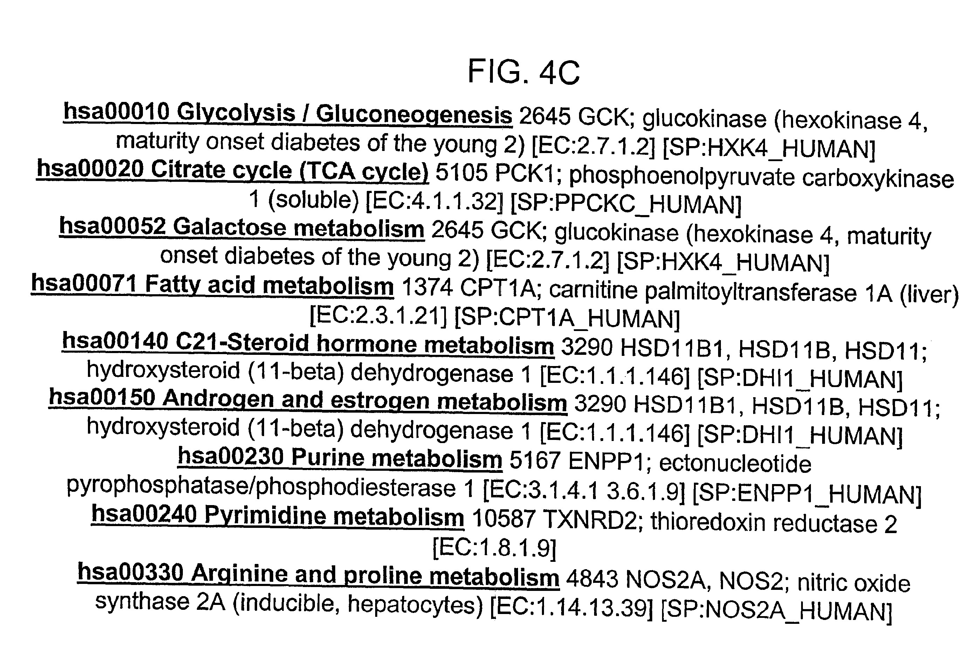

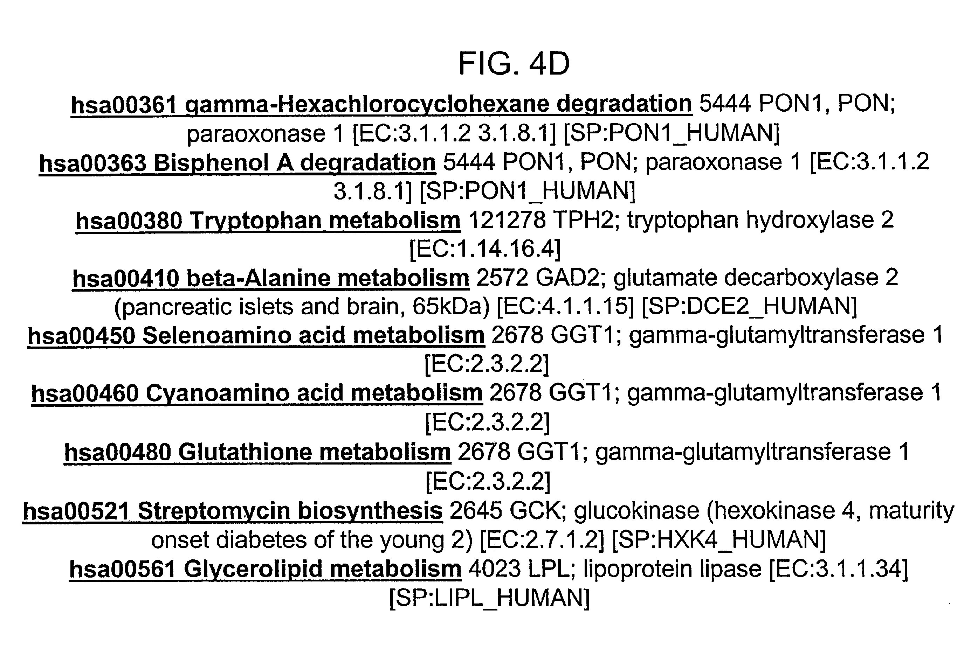

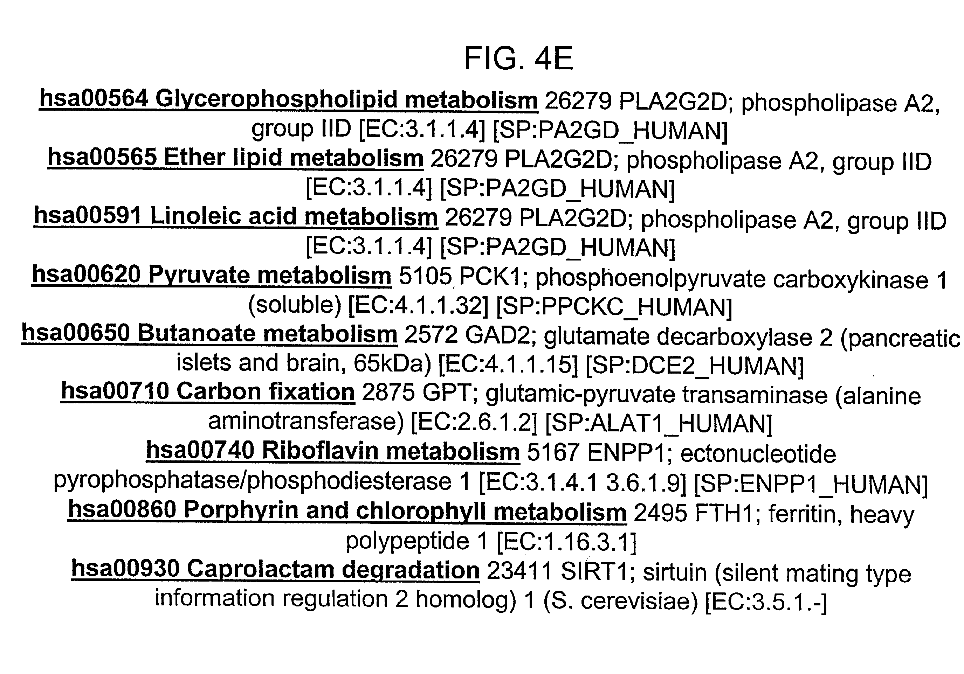

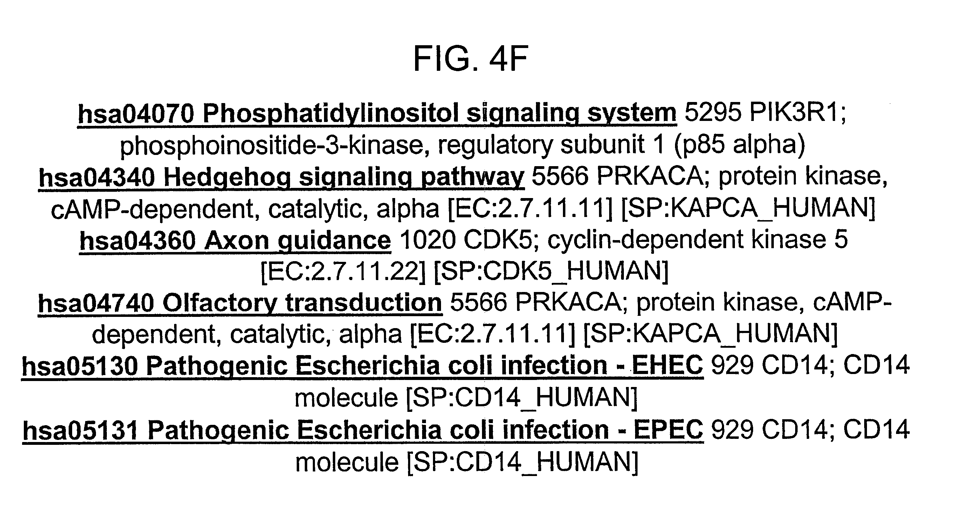

Figure 2 also illustrates several differing patterns of markers that are useful in the diagnosis in subjects of Pre-Diabetes and Diabetes from Normal; several specific clusters of markers are clearly observable from the aforementioned human sample data and in the figure. As earlier mentioned, individual DBRISKMARKERS provide differing pathway and physiological information, and one aspect of the invention are methods of arriving at DBRISKMARKER panels which provide sufficient information for improvements in performance over traditional risk assessment techniques. Figure 3 and 4 encompasses the listing of the KEGG pathways with three or more (in the case of Figure 3), or only one or two (in the case of Figure 4) of the DBRISKMARKERS listed above respectively highlighted within the relevant pathways as colored icons.

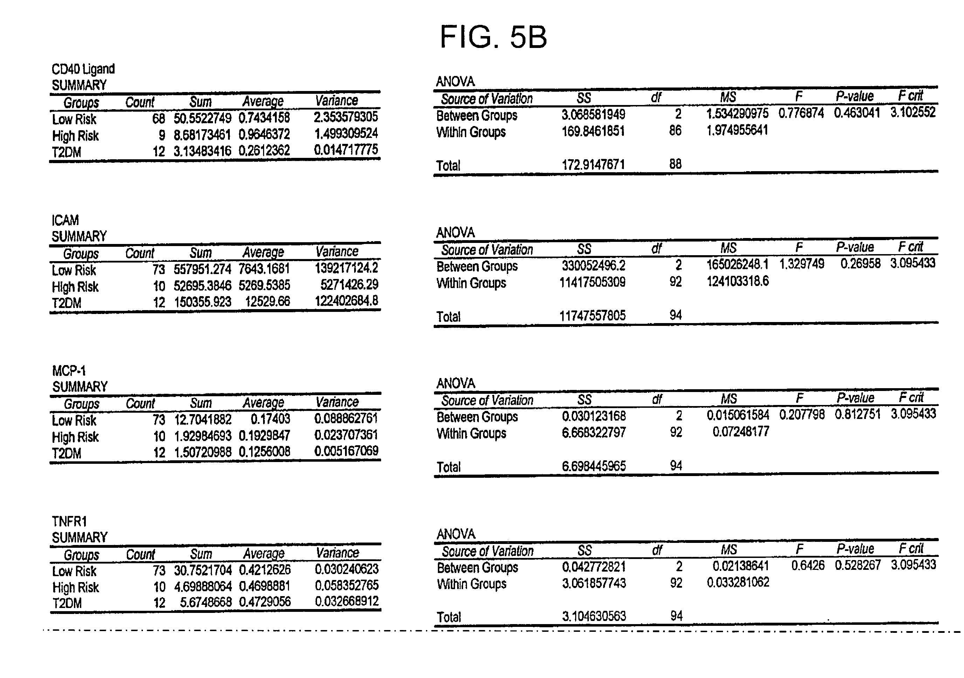

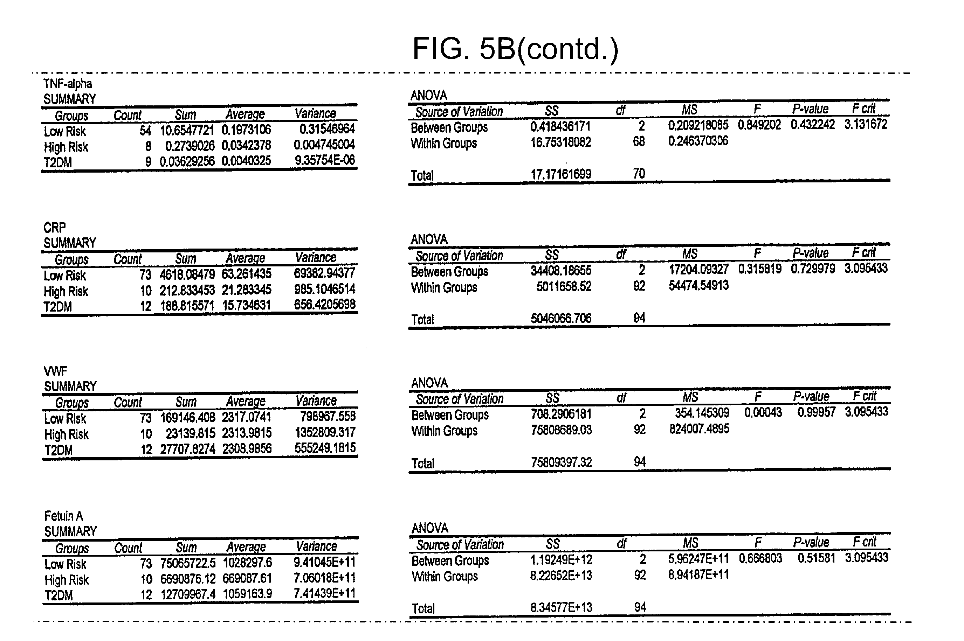

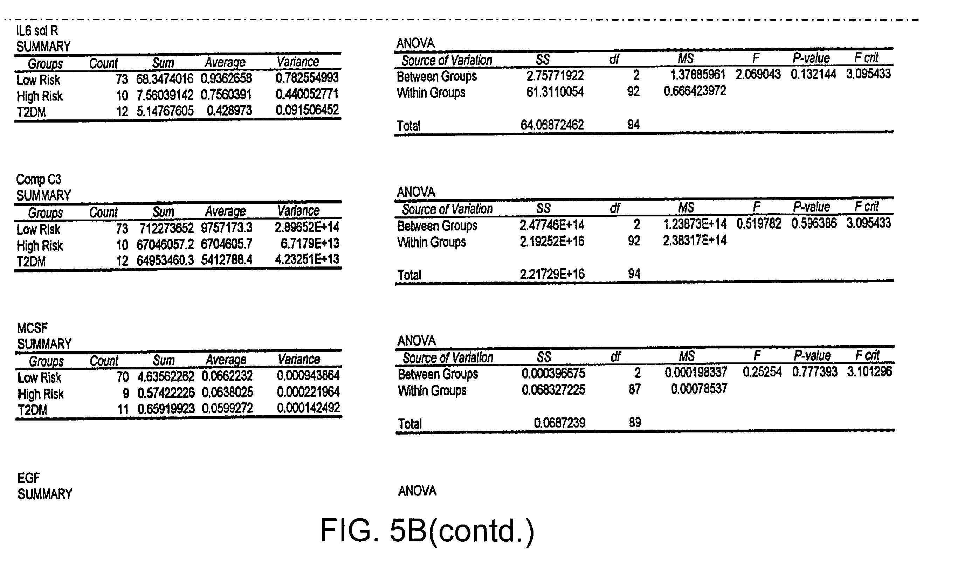

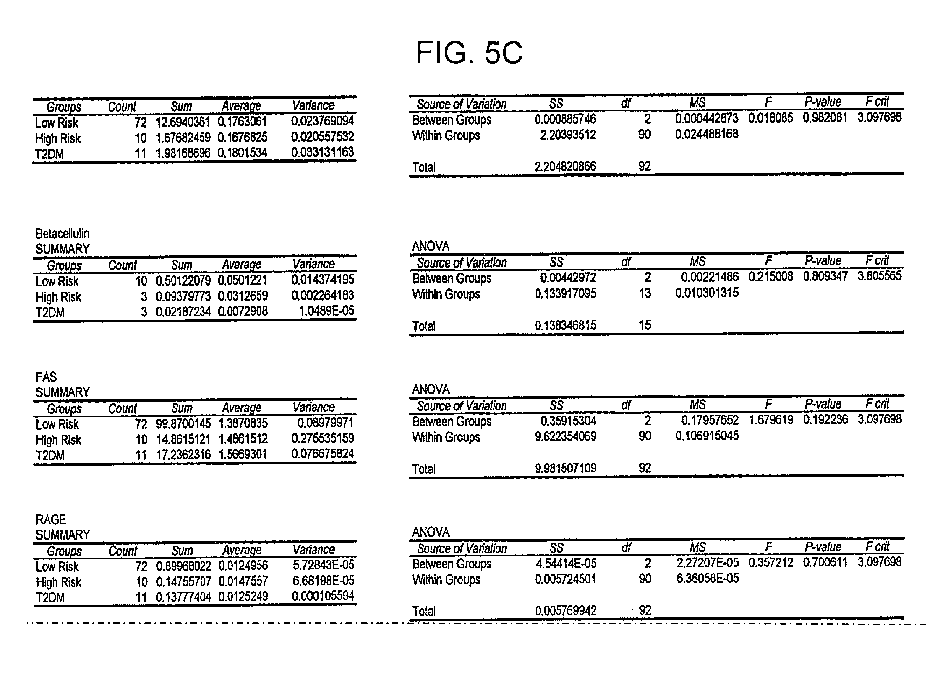

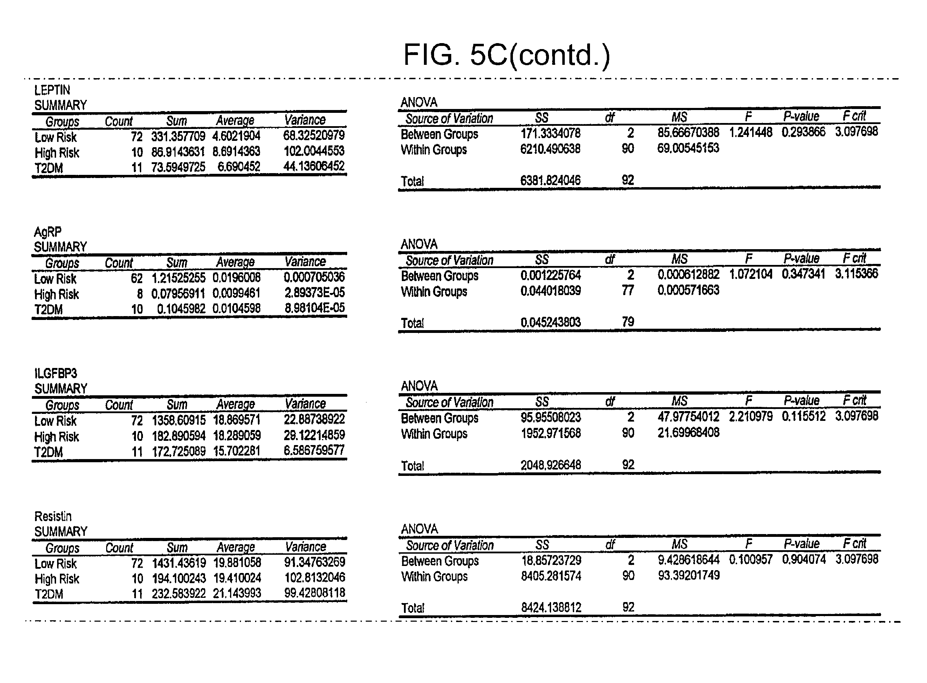

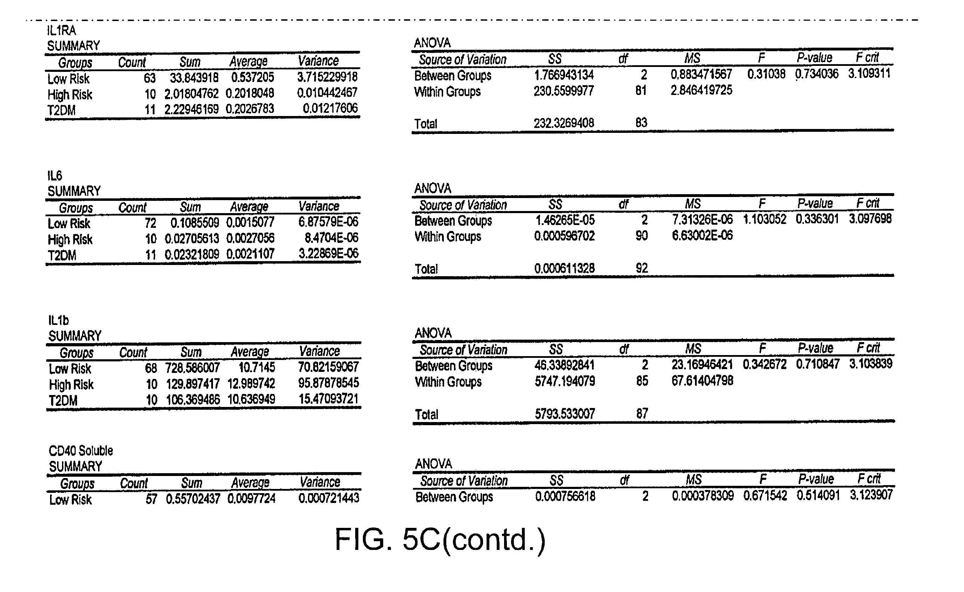

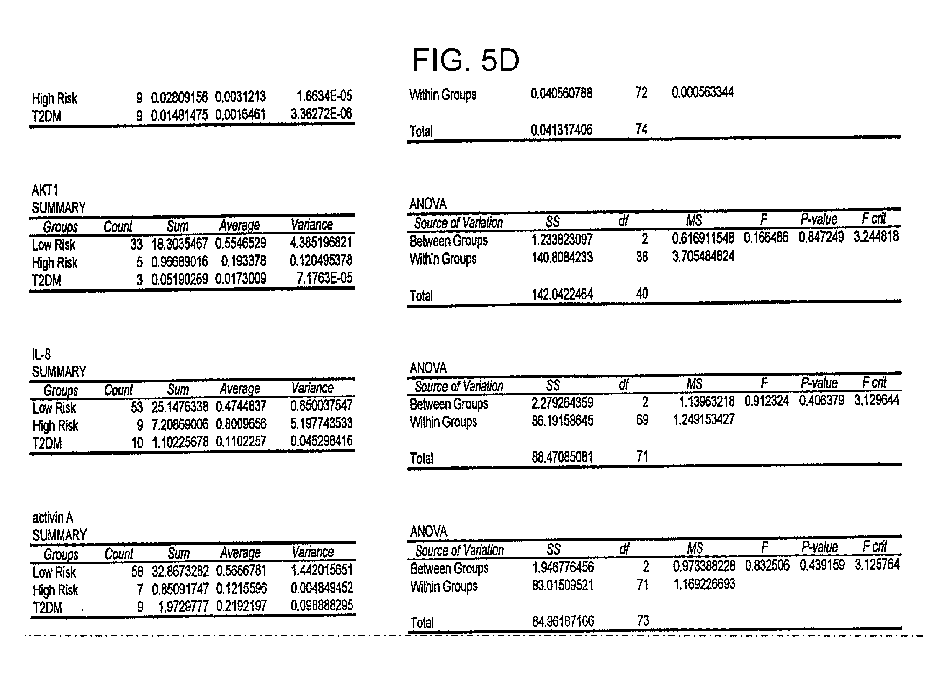

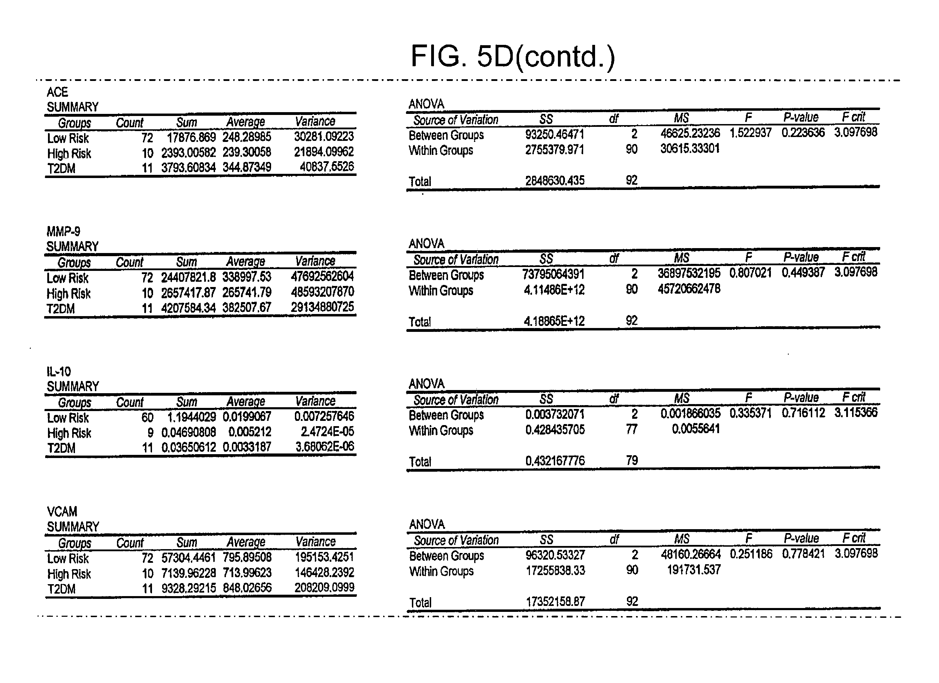

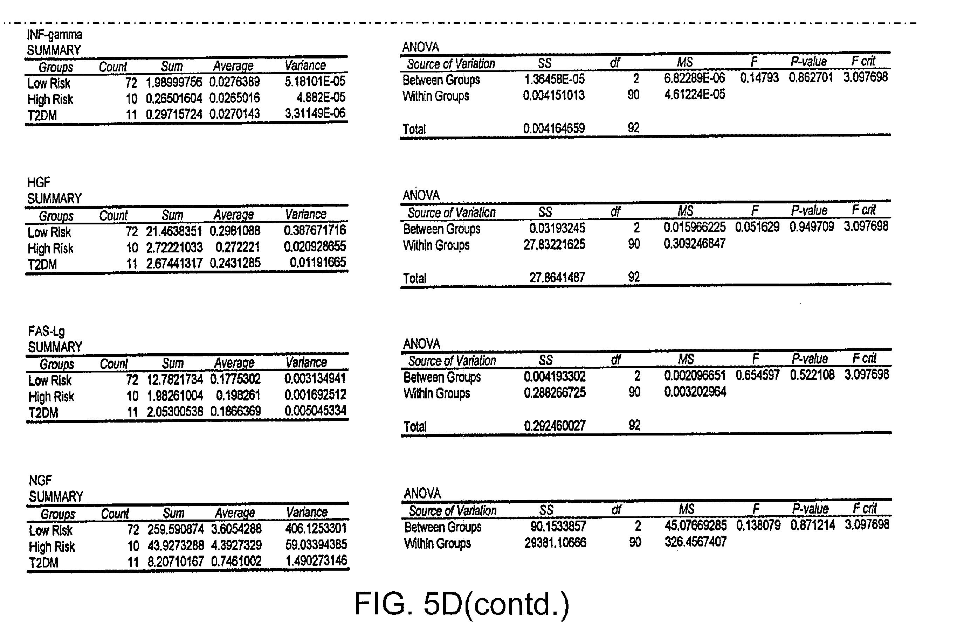

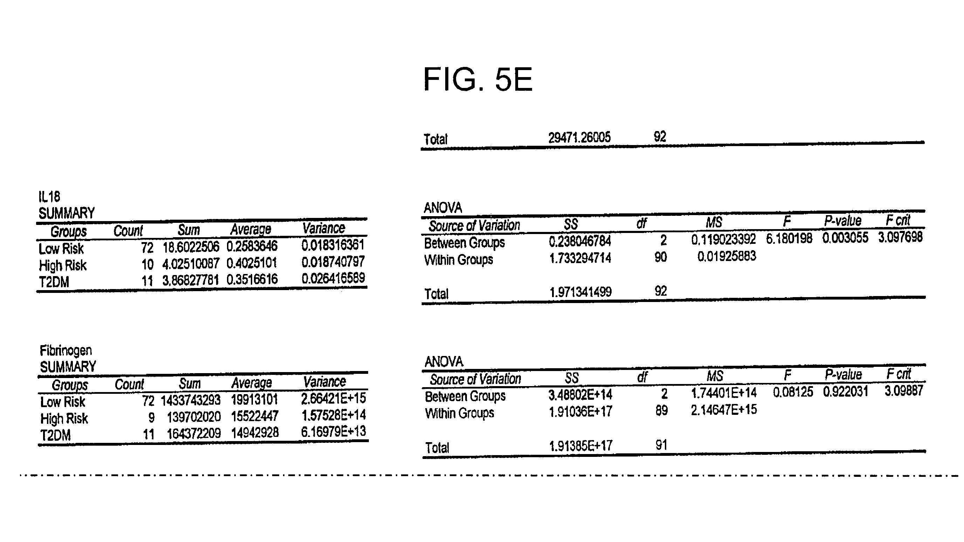

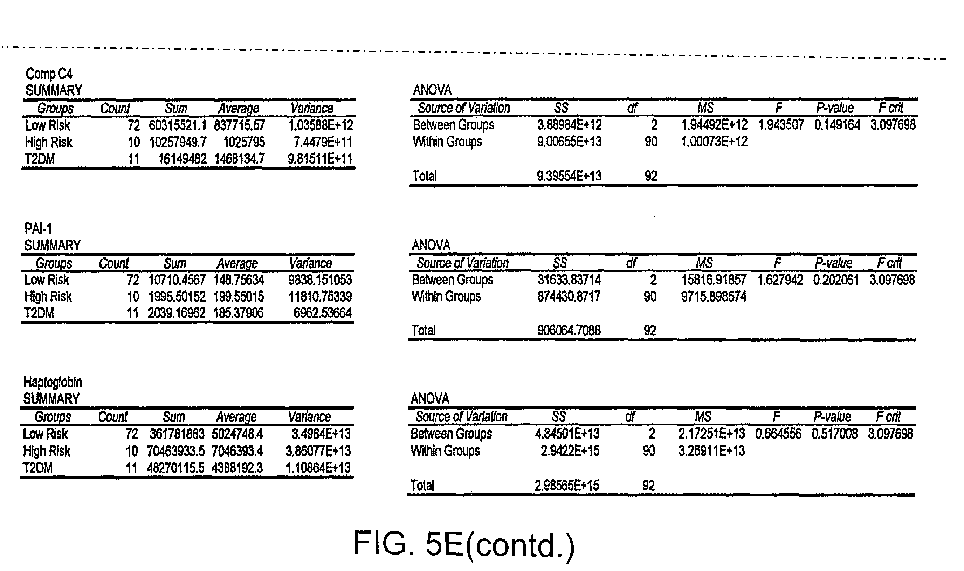

It was previously noted that many of the individual markers listed, when used alone and not as a member of a multi-marker panel of DBRISKMARKERS, have little or no statistically significant differences in their concentration levels between Normal, Pre-Diabetes, and Diabetes populations, and thus cannot reliably be used alone in classifying any patient between those three states (Normal, Pre-Diabetes, or Diabetes). As also previously mentioned, a common measure of statistical significance is the p value, which indicates the probability that an observation has arisen by chance rather than correlation or causation; preferably, such p values are 0.05 or less, representing a 95% chance that the observation of interest arose by other than chance. Figure 5 details such statistical analysis for our entire representative list of fifty DBRISKMARKERS, disclosing the DBRISKMARKER concentrations and studying the variances between and within patient samples across all three subject populations, based on established one -factor ANOVA (analysis of variance) statistical techniques. It is particularly noteworthy that only one (IL-18) of the fifty studied DBRISKMARKERS has a p value under 0.05 indicating reliable utility in disease classification; in many cases the p values indicate very significant odds of random chance having had the predominant role in describing the observed concentration variances between and within the subject populations. It can be concluded that when taken individually such DBRISKMARKERS are of limited use in the diagnosis of Diabetes or Pre-Diabetes.

Despite this individual marker performance, it is the subject matter of our invention that certain specific combinations of two or more DBRISKMARKERS of the present invention can also be used as multi-marker panels comprising combinations of DBRISKMARKERS that are known to be involved in one or more physiological or biological pathways, and that such information can be combined and made clinically useful through the use of various statistical classification algorithms, including those commonly used such as logistic regression. In fact, it is the further detailed subject matter of the invention, that such algorithms, when optimized for their best clinical classification performance (as measured by line fitting statistics such as $R^2$) across a reasonably large group of potentially contributing DBRISKMARKERS as continuous measurements of the risk of conversion to Type 2 Diabetes, will commonly share one of of a discrete number of multimarker components motifs and combinations. These include, solely within the representative group of DBRISKMARKERS previously assayed, strong significance around groupings aound the marker LEPTIN (LEP), and in particular the various permutations and component combinations of LEPTIN, HAPTOGLOBIN (HP), INSULIN-LIKE GROWTH FACTOR BINDING PROTEIN 3 (IGFBP3), and RESISTIN (RETN) including, without limitation, the various subsets of two or more of each of the foregoing markers, and the combination of those sets with additional markers. An alternative general strategy to that of using LEPTIN and its supporting cluster of partner markers involves the use of TNFR1 and CD26, typically together as a cluster, but either alone or with other markers (including with the use of LEPTIN and any of the other individually mentioned family of markers in panels of three or more DBRISKMARKERS). A third, generally lower performing strategy than that of LEP is to use more generalized markers of inflammation, such as C-REACTIVE PROTEIN (CRP), RECEPTOR FOR ADVANCED GLYCOSYLATION ENDPRODUCTS (RAGE, now AGER), and general cytokines, adipocytokines, and complement and coagulation cascade members such as IL-18, ADIPONECTIN (ADIPOQ), ADIPISIN (aka COMPLEMENT FACTOR D or CFD), and PAI-1 (SERPINE1), among the others disclosed, in larger numbers or in combination with more specific DBRISKMARKERS.

The general concept of how two less specific or lower performing biomarkers are combined into novel and more useful combinations for the purpose of diagnosing PRE-DIABETES, is a subject and key aspect of the invention. An illustrative example, Figure 6 presents individual marker performance for LEPTIN and HAPTOGLOBIN in the top two panels showing each marker alone. In the lower left panel, the two tests are shown used together combined in a simple clinical classification rule, where the tested subject is considered a positive panel test for disease if either marker is above its individual ROC defined clinical cut-off level (those used in the previous panels). This type of "either A or B" rule is very commonplace in medicine; for example, a patient is considered dylipidemic if any one of the three total cholesterol, HDL or triglycerides measurements are above certain individual cut-offs for each test.

As the lower left panel indicates, while the test has maintained its sensitivity (a larger patient cohort might show an improvement, but LEPTIN had excellent starting performance, and only one false negative remains). However, specificity has declined dramatically, to a level worse than either marker alone, due to the higher number of false positives called (58 together versus 29 for LEPTIN alone or 45 for HAPTOGLOBIN alone). More typically, an improvement in sensitivity at the cost of a drop in specificity is expected when two markers are used in this way together.

In contrast, in the lower right panel, the same two markers are tested together when combined using a standard logistic regression algorithm. In this scenario, sensitivity remains maintained, but specificity has increased to a higher level than either marker is capable of alone. The logistic algorithm scenario is shown across all cut-offs in the following ROC curve, and has the a higher AUC than either marker alone (unfortunately, again due to the small sample size of the disease cohort, this AUC difference does not quite make statistical significance; however, it is clear from the preceeding categorical analysis that the combination is a superior test, with a lower false positive rate and false negative rate)).

This example illustrated several concepts. The first is that multiple markers can often yield better performance than the individual components when proper mathematical and clinical algorithms are used; this is often evident in both sensitivity and specificity, and results in a greater AUC. The second key concept is that there is often novel unperceived information in existing markers, as was necessary in order to achieve the new algorithm combined level of specificity. The final concept is that this hidden information may hold true even for markers which are generally regarded to have suboptimal clinical performance on their own, as did the HAPTOGLOBIN in the example, at only 62.5% sensitivity and 41.5% specificity, a conclusion which would not be obvious prior to testing the two markers together with an algorithm. In fact, the suboptimal performance in terms of high false positive rates on the individual test in may very well be the indicator that some important additional information is contained within the tests results - information which would not be elucidated absent the combination with a second marker and a mathematical algorithm. The example in Figure 6 was shown using actual patient marker data and calculated diabetes risk outcomes.

Figure 7 is a further demonstration of the synergy and often unforeseeable benefits and impacts of multi-marker approach. It demonstrates that a marker which is perceived as a valuable and heavily weighted determinant when used alone, or even with one or several other markers, may significantly change in its contribution with the addition of new information in the form of additional biomarkers. The graph depicts the change in the logistic regression coefficient (or marker loading) for the first marker as a second through fourty-eighth marker is added. It indicates that the weighing of the marker has changed with the provision of more markers and more information to the re-optimized algorithm. This is again using an actual example of how the inventors developed several of the multimarker approaches disclosed here by using a search algorithm which seeks the best additional marker from the group of fifty to add to the algorithm at each step, improving the algorithm output or clinical index measurement with each additional marker. Figure 8 presents that same improved performance at each DBRISKMARKER addition, as measured by $R^2$ versus the calculated reference Diabetes expected conversion rate curve, through the addition of multiple DBRISKMARKERS step by step utilizing a "forward selection" algorithm comparing all possible remaining additions to the panel at each given panel size, and then choosing the one with the highest improvement in performance.

The disadvantages of such forward selection techniques is the possibility of non-step wise solutions, where synergistic information can be gained by also testing a "step backwards" in order to reassess each existing markers remaining contribution (as noted, the beta coefficients do change) and to test for such synergies that might be cloaked by the legacy steps taken to get to the current panel size. This forward and backwards technique can be combined with a balancing factor providing input as to when the additional complexity of more markers outweighs the incremental gain to further marker additions, a searching technique commonly called a "stepwise." searching algorithm. It is clear from the $R^2$ graph in Figure 8 that the return to additional markers decreases over time if each step is taken in an optimal manner- eventually, there just is no more relevant clinical information to be fed to the algorithm, and additional markers largely bring complexity and redundant information, decreasing algorithm usability and reliability.

Several techniques can be used to generate such best marker addition algorithms, building the optimal DBRISKMARKER additions at each step. Figure 9 is a depiction of a non-stepwise technique - total enumeration of ALL of the possibilities, which is increasingly possible given advances in computer power - but not typically employed for problems over a certain size and complexity. The graph depicts a three dimensional cube with a list of all the markers on each of the x, y , and axes. Marker combinations are depicted by interior cubes within the interior of the base cube; an interactive user interface allows the viewer to highlight algorithms with specific members or levels of performance. The cube pictured represented a total of over two hundred thousand individual logistic regression calculations covering all possible combinations of approximately sixty DBRISKMARKERS, each with intercept, coefficients and $R^2$ calculated. The "rods" suspended within the cube represent high contribution markers, such as LEPTIN, TNFR1, and CD26, which for one or more second marker partners, have a much higher than average algorithm performance, irrespective of their third partners, thus describing a straight line through the cube. Such a technique, which is an inherent part of the invention, comprising a mechanism for selecting the best DBRISKMARKER combination, has the added benefit of allowing complete trends to be seen across the entire space of probabilities - trends which may be continued in larger panels and enable deeper insight into marker interrelationships and biology, ultimately and allowing higher effectiveness in DBRISKMARKER panel construction.

Figure 10 is a histogram depicting the distribution of the $R^2$ performance measure across the entire set of possible three marker combinations shown previously in Figure 9. Clearly there is a division of a relatively small minority of high performance algorithms, regardless of the technique used in panel construction.

Other statistical tools such as factor and cross-marker correlation/covariance analysis allow more rationale approaches to panel construction. Figure 11 is a mathematical clustering and classification tree showing the Euclidean standardized distance between the DBRISKMARKERS as shown in Figure 1 and 2. While such grouping may or may not give direct insight into the biology and desired informational content targets for ideal Pre-Diabetes algorithms, it is the result of a method of factor analysis intended to group collections of markers with similar information content (see Examples below for more statistical techniques commonly employed).

Figure 12 presents tables of selected DBRISKMARKERS dividing them into two key classes of Key Individual Markers and Key Combination Markers, useful in constructing various categories of DBRISKMARKER Panels. As previously noted, the position of the individual DBRISKMARKER on the panel is closely related to its provision of incremental information content for the algorithm, so the order of contribution is highly dependent on the other constituent DBRISKMARKERS in the panel.

A DBRISKMARKER panel is comprised of a series of individual DBRISKMARKER components. Within our study using 50 representative DBRISKMARKERS, there are three core marker approaches which can be used independently or, when larger panels are desired, in combination in order to achieve high performance in a DBRISKMARKER panel: the first, which we term the Key Individual Marker approach in Figure 11, centers first on LEPTIN as the core marker with the highest individual contribution to $R^2$ and continues through a rank ordering of other Key Indivdual Marker positions such as HAPTOGLOBIN, ILGFBP, RESISTIN, MMP2, ACE, COMPC4, and CD14. While substitution is possible with this approach, several forward search algorithms have demonstrated and confirmed the order of the core markers shown below as a high relative contribution to $R^2$ in representative populations for the diagnoses of Pre-Diabetes from Normal. In general, for smaller panels, the higher performing panels are generally chosen first from the core marker listed under Key Individual Markers, with highest levels of performance when each of the eight marker positions is occupied. The earlier positions such as Marker Position 1 (LEPTIN) have the typically have the potential for the highest contributions.

An second alternative approach is to begin building a DBRISKMARKER panel using what we have defined in Figure 11 as Key Combination Markers, which achieve high performance primarily through their close interaction in sets, most particularly the TNFR1 - CD26 pairing, but also pairing and supporting various members of the Key Individual Markers, where they are identified as common substitution strategies that still arrive at high overall DBRISKMARKER panel performance, In fact, as a group, some substitutions of Key Individual Markers for Key Combination Markers is beneficial for panels over a certain size, particularly when Key Marker substitution has already occurred, or when panel size is beyond the core eight Key Marker positions.

Key Combination Markers do not have a set order of hierarchy or order beyond the common upfront pairing of TNFR1 and CD26, and of several of the other members with Key Individual Markers, notably E-Selctin, MCSF, and VEGF. Often the Key Combination Markers are added late in a DBRISKMARKER panel construction approach, of when factor and information redundancy makes multiple statistically similar high performance solutions to the optimal DBRISKMARKER panel possible.

A final, third approach is to work within the group of more generalized inflammation cytokine, adipokine and coagulation markers, including CRP, RAGE, IL-18, ADIPONECTIN, ACTIVIN_A, and ADIPISIN, This is a common fill-in strategy for approaches begun with Key Individual or Key Combination Markers, as the more generalized and broad information content of some of these multi-potent markers (such as CRP and RAGE in particular) makes them amenable to being added to many different panel combinations without creating information redundancy.

Examples of specific DBRISKMARKER panel construction using the above general techniques are also disclosed herein, without limitation of the foregoing, our techniques of marker panel construction, or the applicability of alternative DBRISKMARKERS or biomarkers from functionally equivalent classes which are also involved in the same constituent physiological and biological pathways.

**KEY INDIVIDUAL MARKERS**

| Marker position / Core Marker | 1 LEPTIN | 2 HAPTOGLOBIN | 3 ILGFBP3 | 4 RESISTIN | 5 MMP2 | 6 ACE | 7 COMPC4 | 8 CD14 |
|---|---|---|---|---|---|---|---|---|
| Frequently Combined With | HAPTOGLOBIN ILGFBP3 CD-26 RAGE ADIPONECTIN | LEP ILGFBP3 TNFR1 ADIPONECTIN CRP ADIPSIN | LEP HAPTOGLOBIN RESISTIN TNFR1 CRP | LEP HAPTOGLOBIN TNFR1-CD-26 CRP RAGE | LEP HAPTOGLOBIN RESISTIN TNFR1-CD-26 IL_18 ADIPSIN PAI_1 CRP | LEP HAPTOGLOBIN RESISTIN TNFR1-CD-26 PAI_1 IL-18 ADIPSIN CRP | LEP HAPTOGLOBIN TNFR1-CD-26 RAGE IL_18 ADIPONECTIN CRP | LEP HAPTOGLOBIN TNFR1-CD-26 CRP RAGE IL_18 ADIPONECTIN |
| Key Marker Substitution Strategies | TNFR1-CD-26 CRP IL6SOLR ADIPSIN CD40_LIGAND | TNFR1-CD-26 IL_18 RAGE FETUIN_A ADIPONECTIN HGF | TNFR1-CD-26 E_SELECTIN VEGF ADIPSIN ICAM CD40_LIGAND RAGE C_PEPTIDE ADIPONECTIN | MCSF TNFR1-CD-26 ADIPSIN ACTIVIN_A INFGAMMA | E_SELECTIN MCSF FIBRINOGEN | CD_26 E_SELECTIN FIBRINOGEN | E_SELECTIN VEGF MCSF ACTIVIN_A C_PEPTIDE ICAM | E_SELECTIN MCSF CD_26 ICAM FAS CD40_SOLUBLE |

**KEY COMBINATION MARKERS**

| Marker Position / Substitution Marker | 9 E_SELECTIN | 10 MCSF | 11 VEGF | 12 TNFR1 | 13 CD_26 | 14 FIBRINOGEN | 15 AGRP | 16 TNFALPHA |
|---|---|---|---|---|---|---|---|---|
| Alternative Substitution Strategies | ADIPSIN IL6SOLR MMP2 | ICAM ADIPSIN ACTIVIN_A | CD40_LIGAND ADIPSIN INFGAMMA | ICAM ACTIVIN_A HGF | HGF ACTIVIN_A ICAM | ACTIVIN_A CD40_LIGAND ICAM | MMP2 INFGAMMA ACTIVIN_A | CD40_LIGAND ADIPSIN IL_8 |

| Marker Position / Substitution Marker | 17 MCP_1 | 18 TGFB1 | 19 COMP_C3 | 20 AKT1 |
|---|---|---|---|---|
| Alternative Substitution Strategies | ACTIVIN_A MMP2 IL_6 | ICAM C_PEPTIDE ACTIVIN_A | ICAM ADIPSIN MMP2 | ACTIVIN_A ADIPSIN ICAM |

Figure 12 is a listing of 25 high performing DBRISKMARKER panels using three DBRISKMARKERS selected from Position Categories according to the method disclosed herein. Logistic regression algorithms using said panels had calculated R^2 values ranging from 0.300 to 0.329 when employed on samples in the described example and non-diabetic patient cohort.

Figure 13 is a listing of 25 high performing DBRISKMAKER panels using eight DBRISKMARKERS selected from Position Categories according to the method disclosed herein, using single marker substitution from a base set of markers assembled using a backwards seeking algorithm with an AIC feedback loop (see methods below). Logistic regression algorithms using said panels had calculated $R^2$ values ranging from 0.310 to 0.475 when employed on samples in the described example and non-diabetic patient cohort.

Figure 14 is a listing of 55 high performing DBRISKMAKER panels using eighteen DBRISKMARKERS selected from Position Categories according to the method disclosed herein, using single marker substitution with three options from a starting set of markers previously assembled using a backwards seeking algorithm with an AIC feedback loop (see methods below). Logistic regression algorithms using said panels had calculated $R^2$ values ranging from 0.523 to 0.6105 when employed on samples in the described example and non-diabetic patient cohort.

Levels of the DBRISKMARKERS can be determined at the protein or nucleic acid level using any method known in the art. For example, at the nucleic acid level, Northern and Southern hybridization analysis, as well as ribonuclease protection assays using probes which specifically recognize one or more of these sequences can be used to determine gene expression. Alternatively, expression can be measured using reverse-transcription-based PCR assays (RT-PCR), e.g., using primers specific for the differentially expressed sequence of genes. Expression can also be determined at the protein level, e.g., by measuring the levels of peptides encoded by the gene products described herein, or activities thereof. Such methods are well known in the art and include, e.g., immunoassays based on antibodies to proteins encoded by the genes, aptamers or molecular imprints.. Any biological material can be used for the detection/quantification of the protein or its activity. Alternatively, a suitable method can be selected to determine the activity of proteins encoded by the marker genes according to the activity of each protein analyzed.

The DBRISKMARKER proteins, polypeptides, mutations, and polymorphisms thereof can be detected in any suitable manner, but is typically detected by contacting a sample from the subject with an antibody which binds the DBRISKMARKER protein, polypeptide, mutation, or polymorphism and then detecting the presence or absence of a reaction product. The antibody may be monoclonal, polyclonal, chimeric, or a fragment of the foregoing, as discussed in detail above, and the step of detecting the reaction product may be carried out with any suitable immunoassay. The sample from the subject is typically a biological fluid as described above, and may be the same sample of biological fluid used to conduct the method described above.

Immunoassays carried out in accordance with the present invention may be homogeneous assays or heterogeneous assays. In a homogeneous assay the immunological reaction usually involves the specific antibody (e.g., anti-DBRISKMARKER protein antibody), a labeled analyte, and the sample of interest. The signal arising from the label is modified, directly or indirectly, upon the binding of the antibody to the labeled analyte. Both the immunological reaction and detection of the extent thereof can be carried out in a homogeneous solution. Immunochemical labels which may be employed include free radicals, radioisotopes, fluorescent dyes, enzymes, bacteriophages, or coenzymes.

In a heterogeneous assay approach, the reagents are usually the sample, the antibody, and means for producing a detectable signal. Samples as described above may be used. The antibody can be immobilized on a support, such as a bead (such as protein A and protein G agarose beads), plate or slide, and contacted with the specimen suspected of containing the antigen in a liquid phase. The support is then separated from the liquid phase and either the support phase or the liquid phase is examined for a detectable signal employing means for producing such signal. The signal is related to the presence of the analyte in the sample. Means for producing a detectable signal include the use of radioactive labels, fluorescent labels, or enzyme labels. For example, if the antigen to be detected contains a second binding site, an antibody which binds to that site can be conjugated to a detectable group and added to the liquid phase reaction solution before the separation step. The presence of the detectable group on the solid support indicates the presence of the antigen in the test sample. Examples of suitable immunoassays are oligonucleotides, immunoblotting, immunofluorescence methods, chemiluminescence methods, electrochemiluminescence or enzyme-linked immunoassays.

Those skilled in the art will be familiar with numerous specific immunoassay formats and variations thereof which may be useful for carrying out the method disclosed herein. See generally E. Maggio, Enzyme-Immunoassay, (1980) (CRC Press, Inc., Boca Raton, Fla.); see also U.S. Pat. No. 4,727,022 to Skold et al. titled "Methods for Modulating Ligand-Receptor Interactions and their Application," U.S. Pat. No. 4,659,678 to Forrest et al., titled "Immunoassay of Antigens," U.S. Pat. No. 4,376,110 to David et al., titled "Immunometric Assays Using Monoclonal Antibodies," U.S. Pat. No. 4,275,149 to Litman et al., titled "Macromolecular Environment Control in Specific Receptor Assays," U.S. Pat. No. 4,233,402 to Maggio et al., titled "Reagents and Method Employing Channeling," and U.S. Pat. No. 4,230,767 to Boguslaski et al., titled "Heterogenous Specific Binding Assay Employing a Coenzyme as Label."

Antibodies can be conjugated to a solid support suitable for a diagnostic assay (e.g., beads such as protein A or protein G agarose, microspheres, plates, slides or wells formed from materials such as latex or polystyrene) in accordance with known techniques, such as passive binding. Antibodies as described herein may likewise be conjugated to detectable labels or groups such as radiolabels (e.g., $^{35}$S, $^{125}$I, $^{131}$I), enzyme labels (e.g., horseradish peroxidase, alkaline phosphatase), and fluorescent labels (e.g., fluorescein, Alexa, green fluorescent protein) in accordance with known techniques.

Antibodies can also be useful for detecting post-translational modifications of DBRISKMARKER proteins, polypeptides, mutations, and polymorphisms, such as tyrosine phosphorylation, threonine phosphorylation, serine phosphorylation, glycosylation (e.g., O-GlcNAc). Such antibodies specifically detect the phosphorylated amino acids in a protein or proteins of interest, and can be used in immunoblotting, immunofluorescence, and ELISA assays described herein. These antibodies are well-known to those skilled in the art, and commercially available. Post-translational modifications can also be determined using metastable ions in reflector matrix-assisted laser desorption ionization-time of flight mass spectrometry (MALDI-TOF) (Wirth, U. et al. (2002) Proteomics 2(10): 1445-51).

For DBRISKMARKER proteins, polypeptides, mutations, and polymorphisms known to have enzymatic activity, the activities can be determined *in vitro* using enzyme assays known in the art. Such assays include, without limitation, kinase assays, phosphatase assays, reductase assays, among many others. Modulation of the kinetics of enzyme activities can be determined by measuring the rate constant $K_M$ using known algorithms, such as the Hill plot, Michaelis-Menten equation, linear regression plots such as Lineweaver-Burk analysis, and Scatchard plot.

Using sequence information provided by the database entries for the DBRISKMARKER sequences, expression of the DBRISKMARKER sequences can be detected (if present) and measured using techniques well known to one of ordinary skill in the art. For example, sequences within the sequence database entries corresponding to DBRISKMARKER sequences, or within the sequences disclosed herein, can be used to construct probes for detecting DBRISKMARKER RNA sequences in, e.g., Northern blot hybridization analyses or methods which specifically, and, preferably, quantitatively amplify specific nucleic acid sequences. As another example, the sequences can be used to construct primers for specifically amplifying the DBRISKMARKER sequences in, e.g., amplification-based detection methods such as reverse-transcription based polymerase chain reaction (RT-PCR). When alterations in gene expression are associated with gene amplification, deletion, polymorphisms, and mutations, sequence comparisons in test and reference populations can be made by comparing relative amounts of the examined DNA sequences in the test and reference cell populations.

Expression of the genes disclosed herein can be measured at the RNA level using any method known in the art. For example, Northern hybridization analysis using probes which specifically recognize one or more of these sequences can be used to determine gene expression. Alternatively, expression can be measured using reverse-transcription-based PCR assays (RT-PCR), e.g., using primers specific for the differentially expressed sequences.

Alternatively, DBRISKMARKER protein and nucleic acid metabolites can be measured. The term "metabolite" includes any chemical or biochemical product of a metabolic process, such as any compound produced by the processing, cleavage or consumption of a biological molecule (e.g., a protein, nucleic acid, carbohydrate, or lipid). Metabolites can be detected in a variety of ways known to one of skill in the art, including the refractive index spectroscopy (RI), ultraviolet spectroscopy (UV), fluorescence analysis, radiochemical analysis, near-infrared spectroscopy (near-IR), nuclear magnetic resonance spectroscopy (NMR), light scattering analysis (LS), mass spectrometry, pyrolysis mass spectrometry, nephelometry, dispersive Raman spectroscopy, gas chromatography combined with mass spectrometry, liquid chromatography combined with mass spectrometry, matrix-assisted laser desorption ionization-time of flight (MALDI-TOF) combined with mass spectrometry, ion spray spectroscopy combined with mass spectrometry, capillary electrophoresis, NMR and IR detection. (See, WO 04/056456 and WO 04/088309, each of which are hereby incorporated by reference in their entireties) In this regard, other DBRISKMARKER analytes can be measured using the above-mentioned detection methods, or other methods known to the skilled artisan.

## Kits

The invention also includes a DBRISKMARKER-detection reagent, e.g., nucleic acids that specifically identify one or more DBRISKMARKER nucleic acids by having homologous nucleic acid sequences, such as oligonucleotide sequences, complementary to a portion of the DBRISKMARKER nucleic acids or antibodies to proteins encoded by the DBRISKMARKER nucleic acids packaged together in the form of a kit. The oligonucleotides can be fragments of the DBRISKMARKER genes. For example the oligonucleotides can be 200, 150, 100, 50, 25, 10 or less nucleotides in length. The kit may contain in separate containers a nucleic acid or antibody (either already bound to a solid matrix or packaged separately with reagents for binding them to the matrix), control formulations (positive and/or negative), and/or a detectable label. Instructions (e.g., written, tape, VCR, CD-ROM, etc.) for carrying out the assay may be included in the kit. The assay may for example be in the form of a Northern hybridization or a sandwich ELISA as known in the art.

For example, DBRISKMARKER detection reagents can be immobilized on a solid matrix such as a porous strip to form at least one DBRISKMARKER detection site. The measurement or detection region of the porous strip may include a plurality of sites [0157] Alternatively, the kit contains a nucleic acid substrate array comprising one or more nucleic acid sequences. The nucleic acids on the array specifically identify one or more nucleic acid sequences represented by DBRISKMARKERS 1-260. In various embodiments, the expression of 2, 3,4, 5, 6, 7, 8, 9, 10, 15, 20, 25, 40, 50, 100, 125, 150, 175, 200, 210, 220, 230, 240 or more of the sequences represented by DBRISKMARKERS 1-260 can be identified by virtue of binding to the array. The substrate array can be on, e.g., a solid substrate, e.g., a "chip" as described in U.S. Patent No.5,744,305. Alternatively, the substrate array can be a solution array, e.g., xMAP (Luminex, Austin, TX), Cyvera (Illumina, San Diego, CA), CellCard (Vitra Bioscience, Mountain View, CA) and Quantum Dots' Mosaic (Invitrogen, Carlsbad, CA).

The skilled artisan can routinely make antibodies, nucleic acid probes, e.g., oligonucleotides, aptamers, siRNAs, antisense oligonucleotides, against any of the DBRISKMARKERS in Table 1.

**EXAMPLES**

Example 1

The protein biomarker panels were determined by analyzing 64 proteins in human serum samples derived from a group of 96 normal, pre-diabetic, and diabetic persons.

Source Reagents: A large and diverse array of vendors that were used to source immunoreagents as a starting point for assay development, such as, but not limited to, Abazyme, Abnova, Affinity Biologicals, AntibodyShop, Biogenesis, Biosense Laboratories, Calbiochem, Cell Sciences, Chemicon International, Chemokine, Clontech, Cytolab, DAKO, Diagnostic BioSystems, eBioscience, Endocrine Technologies, Enzo Biochem, Eurogentec, Fusion Antibodies, Genesis Biotech, GloboZymes, Haematologic Technologies, Immunodetect, Immunodiagnostik, Immunometrics, Immunostar, Immunovision, Biogenex, Invitrogen, Jackson ImmunoResearch Laboratory, KMI Diagnostics, Koma Biotech, LabFrontier Life Science Institute, Lee Laboratories, Lifescreen, Maine Biotechnology Services, Mediclone, MicroPharm Ltd., ModiQuest, Molecular Innovations, Molecular Probes, Neoclone, Neuromics, New England Biolabs, Novocastra, Novus Biologicals, Oncogene Research Products, Orbigen, Oxford Biotechnology, Panvera, PerkinElmer Life Sciences, Pharmingen, Phoenix Pharmaceuticals, Pierce Chemical Company, Polymun Scientific, Polysiences, Inc., Promega Corporation, Proteogenix, Protos Immunoresearch, QED Biosciences, Inc., R&D Systems, Repligen, Research Diagnostics, Roboscreen, Santa Cruz Biotechnology, Seikagaku America, Serological Corporation, Serotec, SigmaAldrich, StemCell Technologies, Synaptic Systems GmbH, Technopharm, Terra Nova Biotechnology, TiterMax, Trillium Diagnostics, Upstate Biotechnology, US Biological, Vector Laboratories, Wako Pure Chemical Industries, and Zeptometrix. A search for capture antibodies, detection antibodies, and analytes was performed to configure a working sandwich immunoassay. The reagents were ordered and received into inventory.

Immunoassays were developed in three steps: Prototyping, Validation, and Kit Release. Prototyping was conducted using standard ELISA formats when the two antibodies used in the assay were from different host species. Using standard conditions, anti-host secondary antibodies conjugated with horse radish peroxidase were evaluated in a standard curve. If a good standard curve was detected, the assay proceeded to the next step. Assays that had the same host antibodies went directly to the next step (e.g., mouse monoclonal sandwich assays).

Validation of a working assay was performed using the Zeptosense detection platform from Singulex, Inc. (St. Louis, MO). The detection antibody was first conjugated to the fluorescent dye Alexa 647. The conjugations used standard NHS ester chemistry, for example, according to the manufacturer. Once the antibody was labeled, the assay was tested in a sandwich assay format using standard conditions. Each assay well was solubilized in a denaturing buffer, and the material was read on the Zeptosense platform.

FIG. 1 shows a typical result for a working standard curve. Once a working standard curve was demonstrated, the assay was typically applied to 24 serum samples to determine the normal distribution of the target analyte across clinical samples. The amount of serum required to measure the biomarker within the linear dynamic range of the assay was determined, and the assay proceeded to kit release. For the initial 39 validated assays, 0.004 microliters were used per well on average.

Each component of the kit including manufacturer, catalog numbers, lot numbers, stock and working concentrations, standard curve, and serum requirements were compiled into a standard operating procedures for each biomarker assay. This kit was then released for use to test clinical samples.

Samples were collected from several sources. In all cases, sufficient clinical annotations were available to calculate risk factors using the model developed by Stem et al. (2002). Typically, a minimum of the following clinical annotations were available from each study: Date of collection, age, sex, height, weight, waist, BMI, ethnicity, family history, diastolic and systolic blood pressure, fasting glucose levels, cholesterol. The samples were collected using standard protocols, and were stored at -80C from the time of collection.

Clinical samples arrived frozen on dry ice, and each sample was stored at -80C. Each sample typically had many clinical annotations associated with it. The clinical annotations associated with each sample set were brought into a standardized nomenclature prior to import. All of the clinical annotations associated with each sample were then imported into a relational database.

The frozen aliquots of clinical samples were thawed and aliquotted for use in the laboratory. Each clinical sample was thawed on ice, and aliquots were dispensed into barcoded tubes (daughter tubes). Each daughter tube was stored at -80C until it was needed for the immunoassays. The daughter tubes were then arrayed into sample plates. Each barcoded daughter tube to be assayed was arrayed into barcoded 96 or 384 well plates (sample plates). The daughter tube to sample plate well mapping was tracked by the relational database.

Each sample plate was prepared for immunoassay analysis. The 384 well barcoded assay plates were dedicated to one biomarker per plate. Typically, 4-12 assay plates were derived from each sample plate depending upon the amount of serum required for each assay. The sample plate went through a series of dilutions to ensure that the clinical samples were at an appropriate dilution for each immunoassay. The clinical samples were then deposited into the assay plate wells in triplicate for each marker. Again, tracking of each sample plate well to assay plate well was tracked in the relational database. The assays were then be processed using standard immunoassay procedures, and the assay plate was read on the Zeptosense instrument. Each run contained data for a single biomarker across about 384 clinical samples. The resulting data files were then imported back into the relational database, where standard curves were calculated and the concentration values for each biomarker for each sample were calculated. FIG. 2 shows an example of single molecule detection data across 92 samples for 25 biomarkers.

The biomarker values assigned to each clinical sample were reassociated with the original clinical annotations. The quantitative biomarker data were correlated to the clinical annotations associated with each sample. Diabetes risk over 7.5 years was calculated using the model developed by Stem et al. (2002). The clinical model is of the form of a logistic equation

$$p = 1/(1 + e^{-x}),$$

where

$$x = -13.415 + 0.028(\text{age}) + 0.661(\text{sex}) + 0.412(\text{MA}) + 0.079(\text{FG}) + 0.018(\text{SBP}) - 0.039(\text{HDL}) + 0.070(\text{BMI}) + 0.481(\text{family history}).$$

In this equation, $p$ = the probability of developing diabetes over the 7.5 year follow-up period; age is in years; sex = 1 if female, 0 if male; *MA* = 1 if Mexican American, 0 if non-Hispanic white; *FG* = fasting glucose in mg/dL; *SBP* = systolic blood pressure in mm Hg; *HDL* = high-density lipoprotein cholesterol level in mg/dL; *BMI* = body mass index in kg/m2; and family history = 1 if at least one parent or sibling has diabetes or 0 if not (Stem et al. 2002).

In order to estimate risk for the cohort patient samples, the following modifications were made to these parameters. First, African Americans and Hispanics were included in the high risk group with Mexican Americans and patients with a diagnosis of hypertension were assumed to have a SBP=150 and patients without an SBP=125. The rest of the data were available in the clinical record. Raw concentration data for each marker were $\log_{10}$ transformed and used as the inputs for several linear regression models on the logit transfom of risk (x in the above equation).

Linear regression ofx on the $\log_{10}$ biomarker concentration on each univariate, bivariate, and tri-variate basis by marker sets was performed via a complete search of all combinations. The quality of models was judged on the basis of the coefficient of determination, $R^2$.

Models larger than three markers were developed using forward, backward, and stepwise selection based on Akaike Information Criterion (AIC). Alternatives to these marker sets were identified by eliminating each marker and searching the remaining set for the best replacement, where 'best' is the marker with the highest $R^2$ value.

A full model was also created by adding a single variable to the null model one by one until all markers were used. Each marker was selected based on the coefficient of determination of the complete marker set being used up to that point. Selected fits of these models were used to calculate sensitivity and specificity of any individual model.

The uniqueness/similarity of biomarker concentrations was investigated using principle components analysis (PCA), Hierarchical clustering, and simple correlation. The results of the PCA were evaluated graphically using scree plots, bi-plots, and sample projections to quantify how much independent variation existed among these markers. Hierarchical clustering, using the standardized (mean=0, sd=1) concentrations, was based on euclidian distance as a distance metric and Ward's method as the means of agglomeration. Clusters were used to identify markers behaving similarly.

The following is an illustrative example of a method that was used in developing protein biomarker tests in accordance with the invention. Assay Analyte: C-Reactive Protein

Table 2: Components

| Component | Vendor | Catalog Number | Lot Number |
|---|---|---|---|
| C-Reactive Protein | US Biologicals | C7907-26A | L5042910 |
| Capture Antibody | US Biologicals | C7907-09 | L4030562 |
| Detection Antibody | US Biologicals | C7907-10 | L2121306M |

Each individual well on a NUNC Maxisorp 384-well plate was coated with 20 µl of capture antibody diluted in coating buffer (0.05 M carbonate, pH 9.6; diluted to 1 µg/mL and prepared immediately before use) and incubated overnight at room temperature. The plate was then washed three times in 100 µl of Wash buffer A (PBS with 0.1% Tween 20), and blocked in 30 µl PBS buffer containing 1% BSA, 5% sucrose, 0.05% NaN$_3$ for analyte capture for at least two hours at room temperature. After incubation, blocking buffer was removed and the blocked plates air-dried for at least 5 hours at room temperature and prepared for storage at 4°C or for Zeptosense assay.

Samples were diluted 1:400 in Assay Buffer (BS buffer containing 1% BSA, 0.1% Triton X-100. To the blocked and dried plate, 20 µl/well of standards and diluted unknown samples were added and allowed to incubate overnight at room temperature. After incubation, the plate was washed five times in wash buffer B (BS buffer with 0.02% Triton X-100 and 0.0001% BSA), detection antibody A647 was diluted to 50 ng/ml in assay buffer and was added to the wells in an amount of 20 µl/well. The detection antibody was allowed to bind for 2 hours at room temperature, after which the plate was washed five times in 100 µl of wash buffer B. A standard curve was generated using a control diluted to 100 ng/ml in a calibrator. Serial dilutions from 100 ng/ml to 0.01 pg/ml in calibrator diluent (assay buffer + additional 5% BSA) were prepared. FIG 1 is a representative standard curve using IL-1 receptor antagonist. Elution buffer (4 M urea, 1X BS with 0.02% Triton X-100, and 0.001% BSA) was added in an amount of 20 µl/well and incubated for half an hour at room temperature, after which the samples were analyzed on a Zeptosense instrument.

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

**EMBODIMENTS OF THE INVENTION**

1. A first embodiment of the invention is a method with a predetermined level of predictability for assessing a risk of development of Diabetes Mellitus or a prediabetic condition in a subject comprising:

   a. measuring the level of an effective amount of two or more DBRISKMARKERS selected from the group consisting of DBRISKMARKERS 1-260 in a sample from the subject, and
   b. measuring a clinically significant alteration in the level of the two or more DBRISKMARKERS in the sample, wherein the alteration indicates an increased risk of developing Diabetes Mellitus or a prediabetic condition in the subject.

2. Another embodiment of the invention is the method of the first embodiment, wherein the Diabetes Mellitus comprises Type 1 Diabetes, Type 2 Diabetes, or gestational Diabetes.

3. Another embodiment of the invention is the method of the first embodiment, wherein the pre-diabetic condition comprises IFG, IGT, Metabolic Syndrome, or Syndrome X.

4. Another embodiment of the invention is the method of the first embodiment, wherein the level of DBRISKMARKERS is measured electrophoretically or immunochemically.

5. Another embodiment of the invention is the method of the fourth embodiment, wherein the immunochemical detection is by radioimmunoassay, immunofluorescence assay or by an enzyme-linked immunosorbent assay.

6. Another embodiment of the invention is the method of the first embodiment, wherein the subject has not been previously diagnosed or identified as having the Diabetes Mellitus or the pre-diabetic condition.

7. Another embodiment of the invention is the method of the first embodiment, wherein the subject is asymptomatic for the Diabetes Mellitus or the pre-diabetic condition.

8. Another embodiment of the invention is the method of the first embodiment, wherein the sample is serum, blood plasma, blood cells, endothelial cells, tissue biopsies, ascites fluid, bone marrow, interstitial fluid, sputum, or urine.

9. Another embodiment of the invention is the method of the first embodiment, wherein the level of expression of five or more DBRISKMARKERS is measured.

10. Another embodiment of the invention is the method of the first embodiment, wherein the level of expression of ten or more DBRISKMARKERS is measured.

11. Another embodiment of the invention is the method of the first embodiment, wherein the level of expression of twenty-five or more DBRISKMARKERS is measured.

12. Another embodiment of the invention is the method of the first embodiment, wherein the level of expression of fifty or more DBRISKMARKERS is measured.

13. A thirteenth embodiment of the invention is a method with a predetermined level of predictability for diagnosing or identifying a subject having Diabetes Mellitus or a pre-diabetic condition comprising:

a. measuring the level of an effective amount of two or more DBRISKMARKERS selected from the group consisting of DBRISKMARKERS 1-260 in a sample from the subject, and
b. comparing the level of the effective amount of the two or more DBRISKMARKERS to a reference value.

14. Another embodiment of the invention is the method of the thirteenth embodiment, wherein the Diabetes Mellitus comprises Type 1 Diabetes, Type 2 Diabetes, or gestational Diabetes.

15. Another embodiment of the invention is the method of the thirteenth embodiment, wherein the pre-diabetic condition comprises IFG, IGT, Metabolic Syndrome, or Syndrome X.

16. Another embodiment of the invention is the method of the thirteenth embodiment, wherein the reference value is an index value.

17. Another embodiment of the invention is the method of the thirteenth embodiment, wherein the reference value is derived from one or more risk prediction algorithms or computed indices for the Diabetes or pre-diabetic condition.

18. Another embodiment of the invention is the method of the thirteenth embodiment, wherein the sample is serum, blood plasma, blood cells, endothelial cells, tissue biopsies, ascites fluid, bone marrow, interstitial fluid, sputum, or urine.

19. A nineteenth embodiment of the invention is a method with a predetermined level of predictability for assessing a risk of impaired glucose tolerance in a subject comprising:

a. measuring the level of an effective amount of two or more DBRISKMARKERS selected from the group consisting of DBRISKMARKERS 1-260 in a sample from the subject, and
b. measuring a clinically significant alteration in the level of the two or more DBRISKMARKERS in the sample, wherein the alteration indicates an increased risk of impaired glucose tolerance in the subject.

20. Another embodiment of the invention is the method of the nineteenth embodiment, wherein the level of DBRISKMARKERS is measured electrophoretically or immunochemically.

21. Another embodiment of the invention is the method of the nineteenth embodiment, wherein the level of DBRISKMARKERS is measured by specific oligonucleotide hybridization.

22. Another embodiment of the invention is the method of the twentieth embodiment, wherein the immunochemical detection is by radioimmunoassay, immunofluorescence assay or by an enzyme-linked immunosorbent assay.

23. Another embodiment of the invention is the method of the nineteenth embodiment, wherein the subject has not been previously diagnosed as having impaired glucose tolerance.

24. Another embodiment of the invention is the method of the nineteenth embodiment, wherein the subject is asymptomatic for the impaired glucose tolerance.

25. Another embodiment of the invention is the method of the nineteenth embodiment, wherein the sample is serum, blood plasma, blood cells, endothelial cells, tissue biopsies, ascites fluid, bone marrow, interstitial fluid, sputum, or urine.

26. Another embodiment of the invention is the method of the nineteenth embodiment, wherein the level of expression of five or more DBRISKMARKERS is measured.

27. Another embodiment of the invention is the method of the nineteenth embodiment, wherein the level of expression of ten or more DBRISKMARKERS is measured.

28. Another embodiment of the invention is the method of the nineteenth embodiment, wherein the level of expression of twenty-five or more DBRISKMARKERS is measured.

29. Another embodiment of the invention is the method of the nineteenth embodiment, wherein the level of expression of fifty or more DBRISKMARKERS is measured.

30. A thirtieth embodiment of the invention is a method with a predetermined level of predictability for diagnosing or identifying a subject having impaired glucose tolerance comprising:

a. measuring the level of an effective amount of two or more DBRISKMARKERS selected from the group consisting of DBRISKMARKERS 1-260 in a sample from the subject, and
b. comparing the level of the effective amount of the two or more DBRISKMARKERS to a reference value.

31. Another embodiment of the invention is the method of the thirtieth embodiment, wherein the sample is serum, blood plasma, blood cells, endothelial cells, tissue biopsies, ascites fluid, bone marrow, interstitial fluid, sputum, or urine.

32. Another embodiment of the invention is the method of the thirtieth embodiment, wherein the reference value is an index value.

33. Another embodiment of the invention is the method of the thirtieth embodiment, wherein the reference value is derived from one or more risk prediction algorithms or computed indices for impaired glucose tolerance.

34. A thirty fourth embodiment of the invention is a method with a predetermined level of predictability for assessing the progression of Diabetes Mellitus or a prediabetic condition in a subject, comprising:

a. detecting the level of an effective amount of two or more DBRISKMARKERS selected from the group consisting of DBRISKMARKERS 1-260 in a first sample from the subject at a first period of time;
b. detecting the level of an effective amount of two or more DBRISKMARKERS in a second sample from the subject at a second period of time;
c. comparing the level of the effective amount of the two or more DBRISKMARKERS detected in step (a) to the amount detected in step (b), or to a reference value.

35. Another embodiment of the invention is the method of the thirty fourth embodiment, wherein the Diabetes Mellitus comprises Type 1 Diabetes, Type 2 Diabetes, or gestational Diabetes.

36. Another embodiment of the invention is the method of the thirty fourth embodiment, wherein the pre-diabetic condition comprises IFG, IGT, Metabolic Syndrome, or Syndrome X.

37. Another embodiment of the invention is the method of the thirty fourth embodiment, wherein the subject has previously been diagnosed or identified as suffering from the Diabetes Mellitus or the pre-diabetic condition.

38. Another embodiment of the invention is the method of the thirty fourth embodiment, wherein the subject has previously been treated for the Diabetes Mellitus or the pre-diabetic condition.

39. Another embodiment of the invention is the method of the thirty fourth embodiment, wherein the subject has not been previously diagnosed or identified as suffering from the Diabetes Mellitus or the pre-diabetic condition.

40. Another embodiment of the invention is the method of the thirty fourth embodiment, wherein the subject is asymptomatic for the Diabetes Mellitus or the pre-diabetic condition.

41. Another embodiment of the invention is the method of the thirty fourth embodiment, wherein the first sample is taken from the subject prior to being treated for the Diabetes Mellitus or the pre-diabetic condition.

42. Another embodiment of the invention is the method of the thirty fourth embodiment, wherein the second sample is taken from the subject after being treated for the Diabetes Mellitus or the pre-diabetic condition.

43. Another embodiment of the invention is the method of the thirty fourth embodiment, wherein the reference value is derived from one or more subjects who have suffered from Diabetes Mellitus or a pre-diabetic condition.

44. A forty fourth embodiment of the invention is a method with a predetermined level of predictability for assessing the progression of impaired glucose tolerance associated with Diabetes Mellitus or a pre-diabetic condition in a subject comprising:

> a. detecting the level of an effective amount of two or more DBRISKMARKERS selected from the group consisting of DBRISKMARKERS 1-260 in a first sample from the subject at a first period of time;
> b. detecting the level of an effective amount of two or more DBRISKMARKERS in a second sample from the subject at a second period of time;
> c. comparing the level of the effective amount of the two or more DBRISKMARKERS detected in step (a) to the amount detected in step (b), or to a reference value.

45. Another embodiment of the invention is the method of the forty fourth embodiment, wherein the subject is suffering from the Diabetes Mellitus or the prediabetic condition.

46. Another embodiment of the invention is the method of the forty fourth embodiment, wherein the subject has previously been treated for the Diabetes Mellitus or the pre-diabetic condition.

47. Another embodiment of the invention is the method of the forty fourth embodiment, wherein the subject has not been previously diagnosed or identified as having impaired glucose tolerance or suffering from the Diabetes Mellitus or the pre-diabetic condition.

48. Another embodiment of the invention is the method of the forty fourth embodiment, wherein the subject is asymptomatic for the impaired glucose tolerance, or is asymptomatic for the Diabetes Mellitus or the pre-diabetic condition.

49. Another embodiment of the invention is the method of the forty fourth embodiment, wherein the first sample is taken from the subject prior to being treated for the impaired glucose tolerance, Diabetes Mellitus, or the pre- diabetic condition.

50. Another embodiment of the invention is the method of the forty fourth embodiment, wherein the second sample is taken from the subject after being treated for the impaired glucose tolerance, Diabetes Mellitus, or the pre- diabetic condition.

51. Another embodiment of the invention is the method of the forty fourth embodiment, wherein the reference value is derived from one or more subjects who have suffered from impaired glucose tolerance, Diabetes Mellitus, or a pre-diabetic condition.

52. A fifty second embodiment of the invention is a method with a predetermined level of predictability for monitoring the effectiveness of treatment for Diabetes Mellitus or a pre-diabetic condition comprising:

a. detecting the level of an effective amount of two or more DBRISKMARKERS selected from the group consisting of DBRISKMARKERS 1-260 in a first sample from the subject at a first period of time;
b. detecting the level of an effective amount of two or more DBRISKMARKERS in a second sample from the subject at a second period of time;
c. comparing the level of the effective amount of the two or more DBRISKMARKERS detected in step (a) to the amount detected in step (b), or to a reference value, wherein the effectiveness of treatment is monitored by a change in the level of the effective amount of two or more DBRISKMARKERS from the subject.

53. Another embodiment of the invention is the method of the fifty second embodiment, wherein the Diabetes Mellitus comprises Type 1 Diabetes, Type 2 Diabetes, or gestational Diabetes.

54. Another embodiment of the invention is the method of the fifty second embodiment, wherein the pre-diabetic condition comprises IFG, IGT, Metabolic Syndrome, or Syndrome X.

55. Another embodiment of the invention is the method of the fifty second embodiment, wherein the subject is suffering from the Diabetes Mellitus or the prediabetic condition.

56. Another embodiment of the invention is the method of the fifty second embodiment, wherein the subject has previously been treated for the Diabetes Mellitus or the pre-diabetic condition.

57. Another embodiment of the invention is the method of the fifty second embodiment, wherein the first sample is taken from the subject prior to being treated for the Diabetes Mellitus or the pre-diabetic condition.

58. Another embodiment of the invention is the method of the fifty second embodiment, wherein the second sample is taken from the subject after being treated for the Diabetes Mellitus or the pre-diabetic condition.

59. Another embodiment of the invention is the method of the fifty second embodiment, wherein the treatment for the Diabetes Mellitus or the pre- diabetic condition comprises exercise regimens, dietary supplements, therapeutic agents, surgical intervention, and prophylactic agents.

60. Another embodiment of the invention is the method of the fifty second embodiment, wherein the reference value is derived from one or more subjects who show an improvement in Diabetes risk factors as a result of one or more treatments for the Diabetes Mellitus or the pre-diabetic condition.

61. Another embodiment of the invention is the method of the fifty second embodiment, wherein the effectiveness of treatment is additionally monitored by detecting changes in body mass index (BMI), insulin levels, blood glucose levels, HDL levels, systolic and/or diastolic blood pressure, or combinations thereof.

62. Another embodiment of the invention is the method of the sixty first embodiment, wherein changes in blood glucose levels are detected by an oral glucose tolerance test.

63. A sixty third embodiment of the invention is a method with a predetermined level of predictability for selecting a treatment regimen for a subject diagnosed with or at risk for Diabetes Mellitus or a pre- diabetic condition comprising:

a. detecting the level of an effective amount of two or more DBRISKMARKERS selected from the group consisting of DBRISKM ARKERS 1-260 in a first sample from the subject at a first period of time;
b. optionally detecting the level of an effective amount of two or more DBRISKMARKERS in a second sample from the subject at a second period of time;
c. comparing the level of the effective amount of the two or more DBRISKMARKERS detected in step (a) to a reference value, or optionally, to the amount detected in step (b).

64. Another embodiment of the invention is the method of the sixty third embodiment, wherein the Diabetes Mellitus comprises Type 1 Diabetes, Type 2 Diabetes, or gestational Diabetes.

65. Another embodiment of the invention is the method of the sixty third embodiment, wherein the pre-diabetic condition comprises IFG, IGT, Metabolic Syndrome, or Syndrome X.

66. Another embodiment of the invention is the method of the sixty third embodiment, wherein the subject is suffering from the Diabetes Mellitus or the prediabetic condition.

67. Another embodiment of the invention is the method of the sixty third embodiment, wherein the subject has previously been treated for the Diabetes Mellitus or the pre-diabetic condition.

68. Another embodiment of the invention is the method of the sixty third embodiment, wherein the subject has not been previously diagnosed or identified as suffering from Diabetes Mellitus or the pre-diabetic condition.

69. Another embodiment of the invention is the method of the sixty third embodiment, wherein the first sample is taken from the subject prior to being treated for the Diabetes Mellitus or the pre-diabetic condition.

70. Another embodiment of the invention is the method of the sixty third embodiment, wherein the second sample is taken from the subject after being treated for the Diabetes Mellitus or the pre-diabetic condition.

71. Another embodiment of the invention is the method of the sixty third embodiment, wherein the treatment for the Diabetes Mellitus or the pre- diabetic condition comprises exercise regimens, dietary supplements, therapeutic agents, surgical intervention, and prophylactic agents.

72. Another embodiment of the invention is the method of the sixty third embodiment, wherein the reference value is derived from one or more subjects who show an improvement in Diabetes risk factors as a result of one or more treatments for the Diabetes Mellitus or the pre-diabetic condition.

73. Another embodiment of the invention is the method of the seventy second embodiment, wherein the improvement is monitored by detecting a reduction in body mass index (BMI), a reduction in blood glucose levels, an increase in insulin levels, an increase in HDL levels, a reduction in systolic and/or diastolic blood pressure, or combinations thereof.

74. Another embodiment of the invention is the method of the seventy third embodiment, wherein the reduction in blood glucose levels is measured by oral glucose tolerance test.

75. A seventy fifth embodiment of the invention is a Diabetes Mellitus reference expression profile, comprising a pattern of marker levels of an effective amount of two or more markers selected from the group consisting of DBRISKMARKERS 1-260, taken from one or more subjects who do not have the Diabetes Mellitus.

76. Another embodiment of the invention is the profile of the seventy fifth embodiment, wherein the Diabetes Mellitus comprises Type 1 Diabetes, Type 2 Diabetes, or gestational Diabetes.

77. A seventy seventh embodiment of the invention is an impaired glucose tolerance reference expression profile, comprising a pattern of marker levels of an effective amount of two or more markers selected from the group consisting of DBRISKMARKERS 1-260, taken from one or more subjects who do not have impaired glucose tolerance.

78. A seventy eighth embodiment of the invention is a Diabetes Mellitus subject expression profile, comprising a pattern of marker levels of an effective amount of two or more markers selected from the group consisting of DBRISKMARKERS 1-260 taken from one or more subjects who have the Diabetes Mellitus, are at risk for developing the Diabetes Mellitus, or are being treated for the Diabetes Mellitus.

79. Another embodiment of the invention is the profile of the seventy eighth embodiment, wherein the Diabetes Mellitus comprises Type 1 Diabetes, Type 2 Diabetes, or gestational Diabetes.

80. An eightieth embodiment of the invention is an impaired glucose tolerance subject expression profile, comprising a pattern of marker levels of an effective amount of two ore more markers selected from the group consisting of DBRISKMARKERS 1-260 taken from one or more subjects who have impaired glucose tolerance, are at risk for developing impaired glucose tolerance, or are being treated for impaired glucose tolerance.

81. An eighty first embodiment of the invention is a kit comprising a plurality of DBRISKMARKER detection reagents that detect the corresponding DBRISKMARKERS selected from the group consisting of DBRISKMARKERS 1-260, sufficient to generate the profiles of embodiments 75, 77, 78, or 80.

82. Another embodiment of the invention is the kit of the eighty first embodiment, wherein the detection reagent comprises one or more antibodies or fragments thereof.

83. Another embodiment of the invention is the kit of the eighty first embodiment, wherein the detection reagent comprises one or more oligonucleotides.

84. Another embodiment of the invention is the kit of the eighty first embodiment, wherein the detection reagent comprises one or more aptamers.

85. An eighty fifth embodiment of the invention is a machine readable media containing one or more Diabetes Mellitus reference expression profiles according to embodiment 75, or one or more Diabetes Mellitus subject expression profiles according to embodiment 78, and optionally, additional test results and subject information.

86. An eighty sixth embodiment of the invention is a machine readable media containing one or more impaired glucose tolerance reference expression profiles according to embodiment 77, or one or more impaired glucose tolerance subject expression profiles according to embodiment 80, and optionally, additional test results and subject information.

87. An eighty seventh embodiment of the invention is a DBRISKMARKER panel comprising one or more DBRISKMARKERS that are indicative of a physiological or biochemical pathway associated with Diabetes Mellitus or a pre-diabetic condition.

88. Another embodiment of the invention is the panel of the eighty seventh embodiment, wherein the physiological or biochemical pathway comprises autoimmune regulation, inflammation and endothelial function, focal adhesions, leukocyte transendothelial migration, natural killer cell mediated cytotoxicity, regulation of the actin cytoskeleton, adherens/tight/gap junctions, and extracellular matrix-receptor interaction, adipocyte development and maintenance, hematopoietic cell lineage, complement and coagulation cascades, intra- and extracellular cell signaling pathways.

89. Another embodiment of the invention is the panel of the eighty seventh embodiment, wherein the Diabetes Mellitus comprises Type 1 Diabetes, Type 2 Diabetes, or gestational Diabetes.

90. Another embodiment of the invention is the panel of the eighty seventh embodiment, wherein the pre-diabetic condition comprises IFG, IGT, Metabolic Syndrome, or Syndrome X.

91. A ninety first embodiment of the invention is a DBRISKMARKER panel comprising one or more DBRISKMARKERS that are indicative of a site associated with Diabetes Mellitus or a pre-diabetic condition.

92. Another embodiment of the invention is the panel of the ninetieth embodiment, wherein the site comprises beta cells, endothelial cells, skeletal and smooth muscle, or peripheral, cardiovascular, or cerebrovascular arteries.

93. Another embodiment of the invention is the panel of the ninetieth embodiment, wherein the Diabetes Mellitus comprises Type 1 Diabetes, Type 2 Diabetes, or gestational Diabetes.

94. Another embodiment of the invention is the panel of the ninetieth embodiment, wherein the pre-diabetic condition comprises IFG, IGT, Metabolic Syndrome, or Syndrome X.

95. A ninety fifth embodiment of the invention is a DBRISKMARKER panel comprising one or more DBRISKMARKERS that are indicative of the progression of Diabetes Mellitus or a pre-diabetic condition.

96. Another embodiment of the invention is the panel of the ninety fifth embodiment, wherein the Diabetes Mellitus comprises Type 1 Diabetes, Type 2 Diabetes, or gestational Diabetes.

97. Another embodiment of the invention is the panel of the ninety fifth embodiment, wherein the pre-diabetic condition comprises IFG, IGT, Metabolic Syndrome, or Syndrome X.

98. A ninety eighth embodiment of the invention is a DBRISKMARKER panel comprising one or more DBRISKMARKERS that are indicative of the speed of progression of Diabetes Mellitus or a pre-diabetic condition.

99. Another embodiment of the invention is the panel of the ninety eighth embodiment, wherein the Diabetes Mellitus comprises Type 1 Diabetes, Type 2 Diabetes, or gestational Diabetes.

100. Another embodiment of the invention is the panel of the ninety eighth embodiment, wherein the pre-diabetic condition comprises IFG, IGT, Metabolic Syndrome, or Syndrome X.

101. A one hundred and first embodiment of the invention is a DBRISKMARKER panel comprising one or more DBRISKMARKERS that are specific to one or more types of Diabetes Mellitus.

102. Another embodiment of the invention is the panel of the one hundred and first embodiment, wherein the Diabetes Mellitus comprises Type 1 Diabetes, Type 2 Diabetes, or gestational Diabetes.

103. A one hundred and third embodiment of the invention is a DBRISKMARKER panel comprising one or more DBRISKMARKERS that are specific to a prediabetic condition.

104. Another embodiment of the invention is the panel of the one hundred and third embodiment, wherein the pre-diabetic condition comprises IFG, IGT, Metabolic Syndrome, or Syndrome X.

105. A one hundred and fifth embodiment of the invention is a DBRISKMARKER panel comprising two or more DBRISKMARKERS selected from the group consisting of: Leptin (LEP), Haptoglobin (HP), Insulin-like growth factor binding protein 3 (ILGFBP3), Resistin (RETN), Matrix Metallopeptidase 2 (MMP-2), Angiotensin I converting enzyme (peptidyl dipeptidase A)-I (ACE), complement component 4A (C4A), CD 14 molecule (CD 14), selectin E (SELE), colony stimulating factor 1 (macrophage; CSF1), and vascular endothelial growth factor (VEGF), c-reactive protein (pentraxin-related;CRP), Tumor Necrosis Factor Receptor Superfamily Member IA (TNFRSF1A), RAGE (Advanced Glycosylation End Product-specific Receptor [AGER]), and CD26 (dipeptidyl peptidase 4; DPP4).

106. A one hundred and sixth embodiment of the invention is a method for treating one or more subjects at risk for developing Diabetes Mellitus or a pre-diabetic condition, comprising:

   a. detecting the presence of increased levels of at least two different DBRISKMARKERS present in a sample from the one or more subjects; and
   b. treating the one or more subjects with one or more Diabetes-modulating drags until altered levels of the at least two different DBRISKMARKERS return to a baseline value measured in one or more subjects at low risk for developing the Diabetes Mellitus or the pre-diabetic condition, or a baseline value measured in one or more subjects who show improvements in Diabetes risk markers as a result of treatment with one or more Diabetes-modulating drags.

107. Another embodiment of the invention is the method of the one hundred and sixth embodiment, wherein the Diabetes Mellitus comprises Type 1 Diabetes, Type 2 Diabetes, or gestational Diabetes.

108. Another embodiment of the invention is the method of the one hundred and sixth embodiment, wherein the pre-diabetic condition comprises IFG, IGT, Metabolic Syndrome, or Syndrome X.

109. Another embodiment of the invention is the method of the one hundred and sixth embodiment, wherein the Diabetes-modulating drags comprise sulfonylureas; biguanides; insulin, insulin analogs; peroximsome proliferator-activated receptor-$\gamma$ (PPAR-$\gamma$) agonists; dual-acting PPAR agonists; insulin secretagogues; analogs of glucagon-like peptide- 1 (GLP-I); inhibitors of dipeptidyl peptidase IV; pancreatic lipase inhibitors; $\alpha$-glucosidase inhibitors; and combinations thereof.

110. Another embodiment of the invention is the method of the one hundred and sixth embodiment, wherein the improvements in Diabetes risk markers as a result of treatment with one or more Diabetes-modulating drags comprise a reduction in body mass index (BMI), a reduction in blood glucose levels, an increase in insulin levels, an increase in HDL levels, a reduction in systolic and/or diastolic blood pressure, or combinations thereof.

111. A one hundred and eleventh embodiment of the invention is a method of evaluating changes in the risk of impaired glucose tolerance in a subject diagnosed with or at risk for developing a pre-diabetic condition, comprising:

   a. detecting the level of an effective amount of two or more DBRISKMARKERS selected from the group consisting

61

of DBRISKMARKERS 1-260 in a first sample from the subject at a first period of time;

b. optionally detecting the level of an effective amount of two or more DBRISKMARKERS in a second sample from the subject at a second period of time;

c. comparing the level of the effective amount of the two or more DBRISKMARKERS detected in step (a) to a reference value, or optionally, the amount in step (b).

112. Another embodiment of the invention is the method of the one hundred and tenth embodiment, wherein the pre-diabetic condition comprises IFG, IGT, Metabolic Syndrome, or Syndrome X.

113. Another embodiment of the invention is the method of the one hundred and tenth embodiment, wherein the subject is suffering from the pre-diabetic condition.

114. Another embodiment of the invention is the method of the one hundred and tenth embodiment, wherein the subject has previously been treated for the prediabetic condition.

115. Another embodiment of the invention is the method of the one hundred and tenth embodiment, wherein the subject has not been previously diagnosed or identified as suffering from the pre-diabetic condition.

116. Another embodiment of the invention is the method of the one hundred and tenth embodiment, wherein the subject is asymptomatic for the pre-diabetic condition.

117. Another embodiment of the invention is the method of the one hundred and tenth embodiment, wherein the first sample is taken from the subject prior to being treated for the pre-diabetic condition.

118. Another embodiment of the invention is the method of the one hundred and tenth embodiment, wherein the second sample is taken from the subject after being treated for the pre-diabetic condition.

119. Another embodiment of the invention is the method of the one hundred and tenth embodiment, wherein the treatment for the pre-diabetic condition comprises exercise regimens, dietary supplements, therapeutic agents, surgical intervention, and prophylactic agents.

120. Another embodiment of the invention is the method of the one hundred and tenth embodiment, wherein the reference value is derived from one or more subjects who have suffered from impaired glucose tolerance.

121. A one hundred and twenty first embodiment of the invention is a method of differentially diagnosing disease states associated with Diabetes Mellitus or a prediabetic condition in a subject comprising:

a. detecting the level of an effective amount of two or more DBRISKMARKERS selected from the group consisting of DBRISKMARKERS 1-260 in a sample from the subject; and

b. comparing the level of the effective amount of the two or more DBORISKMARKERS detected in step (a) to the Diabetes Mellitus disease subject expression profile of embodiment 78, to the impaired glucose tolerance subject expression profile of embodiment 80, or to a reference value.

122. Another embodiment of the invention is the method of the one hundred and twentieth embodiment, wherein the Diabetes Mellitus comprises Type 1 Diabetes, Type 2 Diabetes, or gestational Diabetes.

123. Another embodiment of the invention is the method of the one hundred and twentieth embodiment, wherein the pre-diabetic condition comprises IFG, IGT, Metabolic Syndrome, or Syndrome X.

124. Another embodiment of the invention is the method of the one hundred and twentieth embodiment, wherein the subject has not previously been diagnosed or identified as suffering from the Diabetes Mellitus or the pre-diabetic condition.

125. Another embodiment of the invention is the method of the one hundred and twentieth embodiment, wherein the subject has not been previously treated for the Diabetes Mellitus or the pre-diabetic condition.

126. Another embodiment of the invention is the method of the one hundred and twentieth embodiment, wherein the subject has been previously treated for the Diabetes Mellitus or the pre-diabetic condition.

127. Another embodiment of the invention is the method of the one hundred and twentieth embodiment, wherein the subject is asymptomatic for the Diabetes Mellitus or the pre-diabetic condition.

128. A one hundred and twenty eighth embodiment of the invention is a method of diagnosing or identifying a subject at risk for developing Diabetes or a pre-diabetic condition by analyzing Diabetes risk factors, the method comprising:

a. measuring the level of an effective amount of two or more DBRISKMARKERS selected from the group consisting of DBRISKMARKERS 1-260 in a sample from the subject, and

b. measuring a clinically significant alteration in the level of the two or more DBRISKMARKERS in the sample, wherein the alteration indicates an increased risk of developing Diabetes Mellitus or a prediabetic condition in the subject.

129. A one hundred and twenty ninth embodiment of the invention is a method of diagnosing or identifying a subject at risk for developing Diabetes or a pre-diabetic condition by analyzing Diabetes risk factors, the method comprising:

a. measuring the level of an effective amount of one or more DBRISKMARKERS selected from the group consisting of: Leptin (LEP), Haptoglobin (HP), Insulin-like growth factor binding protein 3 (ILGFBP3), Resistin (RETN), Matrix Metallopeptidase 2 (MMP-2), Angiotensin I converting enzyme (peptidyl dipeptidase A)-I (ACE), complement component 4A (C4A), CD 14 molecule (CD 14), selectin E (SELE), colony stimulating factor 1 (macrophage; CSF1), and vascular endothelial growth factor (VEGF), c-reactive protein (pentraxin-related; CRP), Tumor Necrosis Factor Receptor Superfamily Member IA (TNFRSF1A), RAGE (Advanced Glycosylation End Product-specific Receptor [AGER]), and CD26 (dipeptidyl peptidase 4; DPP4), and

b. measuring a clinically significant alteration in the level of the one or more DBRISKMARKERS in the sample, wherein the alteration indicates an increased risk of developing Diabetes Mellitus or a prediabetic condition in the subject.

130. A one hundred and thirtieth embodiment of the invention is a method of diagnosing or identifying a subject at risk for developing Diabetes or a pre-diabetic condition by analyzing Diabetes risk factors, the method comprising:

a. measuring the level of an effective amount of two or more DBRISKMARKERS selected from the group consisting of: Leptin (LEP), Haptoglobin (HP), Insulin-like growth factor binding protein 3 (ILGFBP3), Resistin (RETN), Matrix Metallopeptidase 2 (MMP-2), Angiotensin I converting enzyme (peptidyl dipeptidase A)-I (ACE), complement component 4A (C4A), CD 14 molecule (CD 14), selectin E (SELE), colony stimulating factor 1 (macrophage; CSF1), and vascular endothelial growth factor (VEGF), c-reactive protein (pentraxin-related; CRP), Tumor Necrosis Factor Receptor Superfamily Member IA (TNFRSF1A), RAGE (Advanced Glycosylation End Product-specific Receptor [AGER]), and CD26 (dipeptidyl peptidase 4; DPP4), and

b. measuring a clinically significant alteration in the level of the two or more DBRISKMARKERS in the sample, wherein the alteration indicates an increased risk of developing Diabetes Mellitus or a prediabetic condition in the subject.

**REFERENCES**

1. Aalto, K., L. Korhonen, et al. (2002). "Nerve growth factor in serum of children with systemic lupus erythematosus is correlated with disease activity." Cytokine 20(3): 136-9.

2. Abate, N., M. Chandalia, et al. (2005). "ENPP1/PC-1 K121Q polymorphism and genetic susceptibility to type 2 Diabetes." Diabetes 54(4): 1207-13.

3. Adam, T. C., J. Jocken, et al. (2005). "Decreased glucagon-like peptide 1 release after weight loss in overweight/ obese subjects." Obes Res 13(4): 710-6.

4. Aguilar-Bryan, L., C. G. Nichols, et al. (1995). "Cloning of the beta cell high-affinity sulfonylurea receptor: a regulator of insulin secretion." Science 268(5209): 423-6.

5. Al-Khalili, L., M. Forsgren, et al. (2005). "Enhanced insulin-stimulated glycogen synthesis in response to insulin, metformin or rosiglitazone is associated with increased mRNA expression of GLUT4 and peroxisomal proliferator activator receptor gamma co-activator 1." Diabetologia 48(6): 1173-9.

6. Arosio, M., S. Porretti, et al. (2003). "Elevated circulating somatostatin levels in acromegaly." J Endocrinol Invest 26(6): 499-502.

7. Astaneie, F., M. Afshari, et al. (2005). "Total antioxidant capacity and levels of epidermal growth factor and nitric oxide in blood and saliva of insulin-dependent diabetic patients." Arch Med Res 36(4): 376-81.

8. Authier, F., M. Kouach, et al. (2005). "Endosomal proteolysis of insulin-like growth factor-I at its C-terminal D-domain by cathepsin B." FEBS Lett 579(20): 4309-16.

9. Baranowska, B., E. Wolinska-Witort, et al. (2005). "Plasma orexin A, orexin B, leptin, neuropeptide Y (NPY) and insulin in obese women." Neuro Endocrinol Lett 26(4): 293-6.

10. Baratta, R., R. Di Paola, et al. (2003). "Evidence for genetic epistasis in human insulin resistance: the combined effect of PC-1 (K121Q) and PPARgamma2 (P12A) polymorphisms." J Mol Med 81(11): 718-23.

11. Beale, E. G., R. E. Hammer, et al. (2004). "Disregulated glyceroneogenesis: PCK1 as a candidate Diabetes and obesity gene." Trends Endocrinol Metab 15(3): 129-35.

12. Bell, C. G., D. Meyre, et al. (2005). "Association of melanin-concentrating hormone receptor 1 5' polymorphism with early-onset extreme obesity." Diabetes 54(10): 3049-55.

13. Beranek, M., K. Kankova, et al. (2002). "Polymorphisms in the von Willebrand factor gene are not associated with proliferative retinopathy in non-insulin-dependent Diabetes Mellitus." Ophthalmic Res 34(5): 327-30.

14. Bernassola, F., M. Federici, et al. (2002). "Role of transglutaminase 2 in glucose tolerance: knockout mice studies and a putative mutation in a MODY patient." Faseb J 16(11): 1371-8.

15. Bertherat, J., F. Tenenbaum, et al. (2003). "Somatostatin receptors 2 and 5 are the major somatostatin receptors in insulinomas: an in vivo and in vitro study." J Clin Endocrinol Metab 88(11): 5353-60.

16. Bluher, M., R. Unger, et al. (2002). "Relation between glycaemic control, hyperinsulinaemia and plasma concentrations of soluble adhesion molecules in patients with impaired glucose tolerance or Type II Diabetes." Diabetoloaia 45(2): 210-6.

17. Boucher, J., B. Masri, et al. (2005). "Apelin, a newly identified adipokine upregulated by insulin and obesity." Endocrinology 146(4): 1764-71.

18. Boutin, P., C. Dina, et al. (2003). "GAD2 on chromosome 10p12 is a candidate gene for human obesity." PLoS Biol 1(3): E68.

19. Brackenridge, A., E. R. Pearson, et al. (2006). "Contrasting insulin sensitivity of endogenous glucose production rate in subjects with hepatocyte nuclear factor-lbeta and -1 alpha mutations." Diabetes 55(2): 405-11.

20. Bruun, J. M., C. Verdich, et al. (2003). "Association between measures of insulin sensitivity and circulating levels of interleukin-8, interleukin-6 and tumor necrosis factor-alpha. Effect of weight loss in obese men." Eur J Endocrinol 148(5): 535-42.

21. Bullmann, C., J. Kotzka, et al. (2002). "Identification of a novel mutation in the arginine vasopressin-neurophysin II gene in familial central Diabetes insipidus." Exp Clin Endocrinol Diabetes 110(3): 134-7.

22. Carroll, J. S., E. W. Seely, et al. (2001). "Digitalis-like factor response to hyperinsulinemia accompanying a euglycemic hyperinsulinemic clamp or oral glucose tolerance test." Life Sci 69(7): 829-37.

23. Chen, M. B., A. J. McAinch, et al. (2005). "Impaired activation of AMP-kinase and fatty acid oxidation by globular adiponectin in cultured human skeletal muscle of obese type 2 diabetics." J Clin Endocrinol Metab 90(6): 3665-72.

24. Chen, M. P., F. M. Chung, et al. (2006). "Elevated plasma level of visfatin/pre-B cell colony-enhancing factor in patients with type 2 Diabetes Mellitus." J Clin Endocrinol Metab 91(1): 295-9.

25. Cho, Y. M., M. D. Ritchie, et al. (2004). "Multifactor-dimensionality reduction shows a two-locus interaction associated with Type 2 Diabetes Mellitus." Diabetologia 47(3): 549-54.

26. Chouchane, L., J. Danguir, et al. (2001). "Genetic variation in the stress protein hsp70-2 gene is highly associated with obesity." Int J Obes Relat Metab Disord 25(4): 462-6.

27. Clemmons, D. R., M. Sleevi, et al. (2005). "Recombinant, nonglycosylated human insulin-like growth factor-binding protein-3 (IGFBP-3) is degraded preferentially after administration to type II diabetics, resulting in increased endogenous glycosylated IGFBP-3." J Clin Endocrinol Metab 90(12): 6561-8.

28. Collin, G. B., J. D. Marshall, et al. (2002). "Mutations in ALMS1 cause obesity, type 2 Diabetes and neurosensory degeneration in Alstrom syndrome." Nat Genet 31(1): 74-8.

29. Cosson, E., A. F. Bringuier, et al. (2005). "Fas/Fas-Ligand pathway is impaired in patients with type 2 Diabetes. Influence of hypertension and insulin resistance." Diabetes Metab 31(1): 47-54.

30. Cryns, K., T. A. Sivakumaran, et al. (2003). "Mutational spectrum of the WFS1 gene in Wolfram syndrome, nonsyndromic hearing impairment, Diabetes Mellitus, and psychiatric disease." Hum Mutat 22(4): 275-87.

31. Cummings, D. E., J. Q. Purnell, et al. (2001). "A preprandial rise in plasma ghrelin levels suggests a role in meal initiation in humans." Diabetes 50(8): 1714-9.

32. Dantz, D., J. Bewersdorf, et al. (2002). "Vascular endothelial growth factor: a novel endocrine defensive response to hypoglycemia." J Clin Endocrinol Metab 87(2): 835-40.

33. Delplanque, J., F. Vasseur, et al. (2002). "Mutation screening of the urocortin gene: identification of new single nucleotide polymorphisms and association studies with obesity in French Caucasians." J Clin Endocrinol Metab 87 (2): 867-9.

34. Dempfle, A., A.. Hinney, et al. (2004). "Large quantitative effect of melanocortin-4 receptor gene mutations on body mass index." J Med Genet 41(10): 795-800.

35. Du, K., S. Herzig, et al. (2003). "TRB3: a tribbles homolog that inhibits Akt/PKB activation by insulin in liver." Science 300(5625): 1574-7.

36. Dzienis-Straczkowska, S., M. Straczkowski, et al. (2003). "Soluble tumor necrosis factor-alpha receptors in young obese subjects with normal and impaired glucose tolerance." Diabetes Care 26(3): 875-80.

37. Esposito, K., R. Marfella, et al. (2004). "Plasma interleukin-18 concentrations are elevated in type 2 Diabetes." Diabetes Care 27(1): 272.

38. Faraj, M., P. J. Havel, et al. (2003). "Plasma acylation-stimulating protein, adiponectin, leptin, and ghrelin before and after weight loss induced by gastric bypass surgery in morbidly obese subjects." J Clin Endocrinol Metab 88 (4): 1594-602.

39. Feng, X., Z. Guo, et al. (2002). "Identification of a negative response element in the human inducible nitric-oxide synthase (hiNOS) promoter: The role of NF-kappa B-repressing factor (NRF) in basal repression of the hiNOS gene." Proc Natl Acad Sci U S A 99(22): 14212-7.

40. Feng, Y., Q. Wang, et al. (2005). "SGK1-mediated fibronectin formation in diabetic nephropathy." Cell Physiol Biochem 16(4-6): 237-44.

41. Fernandez-Real, J. M., M. Broch, et al. (2003). "CD14 monocyte receptor, involved in the inflammatory cascade, and insulin sensitivity." J Clin Endocrinol Metab 88(4): 1780-4.

42. Fernandez-Real, J. M., M. Pugeat, et al. (2002). "Serum corticosteroid-binding globulin concentration and insulin resistance syndrome: a population study." J Clin Endocrinol Metab 87(10): 4686-90.

43. Fontaine, C., G. Dubois, et al. (2003). "The orphan nuclear receptor Rev-Erbalpha is a peroxisome proliferator-activated receptor (PPAR) gamma target gene and promotes PPARgamma-induced adipocyte differentiation." J Biol Chem 278(39): 37672-80.

44. Forbes, J. M., S. R. Thorpe, et al. (2005). "Modulation of soluble receptor for advanced glycation end products by angiotensin-converting enzyme-1 inhibition in diabetic nephropathy." J Am Soc Nephrol 16(8): 2363-72.

45. Fukui, M., Y. Kitagawa, et al. (2005). "Association between alcohol consumption and serum dehydroepiandrosterone sulphate concentration in men with Type 2 Diabetes: a link to decreased cardiovascular risk." Diabet Med 22(10): 1446-50.

46. Gavrila, A., J. L. Chan, et al. (2005). "Circulating melanin-concentrating hormone, agouti-related protein, and alpha-melanocyte-stimulating hormone levels in relation to body composition: alterations in response to food deprivation and recombinant human leptin administration." J Clin Endocrinol Metab 90(2): 1047-54.

47. George, S., J. J. Rochford, et al. (2004). "A family with severe insulin resistance and Diabetes due to a mutation in AKT2." Science 304(5675): 1325-8.

48. Ghosh, S., E. R. Hauser, et al. (1998). "A large sample of finnish diabetic sib-pairs reveals no evidence for a non-insulin-dependent Diabetes Mellitus susceptibility locus at 2qter." J Clin Invest 102(4): 704-9.

49. Ghosh, S., R. M. Watanabe, et al. (1999). "Type 2 Diabetes: evidence for linkage on chromosome 20 in 716 Finnish affected sib pairs." Proc Natl Acad Sci U S A 96(5): 2198-203.

50. Gonzalez, M., C. Flores, et al. (2004). "Cell signalling-mediating insulin increase of mRNA expression for cationic amino acid transporters-1 and -2 and membrane hyperpolarization in human umbilical vein endothelial cells." Pflugers Arch 448(4): 383-94.

51. Gouni-Berthold, I., E. Giannakidou, et al. (2004). "Association between the PPARalpha L162V polymorphism, plasma lipoprotein levels, and atherosclerotic disease in patients with Diabetes Mellitus type 2 and in nondiabetic controls." Am Heart J 147(6): 1117-24.

52. Grant, S. F., G. Thorleifsson, et al. (2006). "Variant of transcription factor 7-like 2 (TCF7L2) gene confers risk of type 2 Diabetes." Nat Genet 38(3): 320-3.

53. Gunton, J. E., R. N. Kulkarni, et al. (2005). "Loss of ARNT/HIF1beta mediates altered gene expression and pancreatic-islet dysfunction in human type 2 Diabetes." Cell 122(3): 337-49.

54. Gylvin, T., R. Nolsoe, et al. (2004). "Mutation analysis of suppressor of cytokine signalling 3, a candidate gene in Type 1 Diabetes and insulin sensitivity." Diabetologia 47(7): 1273-7.

55. Haap, M., C. Thamer, et al. (2002). "Metabolic characterization of a woman homozygous for the Ser113Leu missense mutation in carnitine palmitoyl transferase II." J Clin Endocrinol Metab 87(5): 2139-43.

56. Hamid, Y. H., S. A. Urhammer, et al. (2004). "Variation in the interleukin-6 receptor gene associates with type 2 Diabetes in Danish whites." Diabetes 53(12): 3342-5.

57. Hansen, S. K., C. S. Rose, et al. (2005). "Variation near the hepatocyte nuclear factor (HNF)-4alpha gene associates with type 2 Diabetes in the Danish population." Diabetologia 48(3): 452-8.

58. Hara, K., M. Horikoshi, et al. (2005). "Absence of an association between the polymorphisms in the genes encoding adiponectin receptors and type 2 Diabetes." Diabetologia 48(7): 1307-14.

59. Hart, L. M., R. P. Stolk, et al. (1999). "Prevalence of variants in candidate genes for type 2 Diabetes Mellitus in The Netherlands: the Rotterdam study and the Hoorn study." J Clin Endocrinol Metab 84(3): 1002-6.

60. Hasegawa, G., M. Ohta, et al. (2005). "Increased serum resistin levels in patients with type 2 Diabetes are not linked with markers of insulin resistance and adiposity." Acta Diabetol 42(2): 104-9.

61. Hauache, O. M., A. F. Reis, et al. (2005). "Estimation of Diabetes risk in Brazilian population by typing for polymorphisms in HLA-DR-DQ, INS and CTLA-4 genes." Dis Markers 21(3): 139-45.

62. He, Z. H. and L. H. Ma (2005). "The aerobic fitness (VO2 peak) and alpha-fibrinogen genetic polymorphism in obese and non-obese Chinese boys." Int J Sports Med 26(4): 253-7.

63. Herlihy, O. M., B. A. Barrow, et al. (2002). "Hyperglycaemic siblings of Type II (non-insulin-dependent) diabetic patients have increased PAI-1, central obesity and insulin resistance compared with their paired normoglycaemic sibling." Diabetologia 45(5): 635-41.

64. Hernandez, C., E. Carrasco, et al. (2005). "Somatostatin molecular variants in the vitreous fluid: a comparative study between diabetic patients with proliferative diabetic retinopathy and nondiabetic control subjects." Diabetes Care 28(8): 1941-7.

65. Hirai, S., M. Yamanaka, et al. (2005). "Activin A inhibits differentiation of 3T3-L1 preadipocyte." Mol Cell Endocrinol 232(1-2): 21-6.

66. Horike, N., H. Takemori, et al. (2003). "Adipose-specific expression, phosphorylation of Ser794 in insulin receptor substrate-1, and activation in diabetic animals of salt-inducible kinase-2." J Biol Chem 278(20): 18440-7.

67. Hotamisligil, G. S. and B. M. Spiegelman (1994). "Tumor necrosis factor alpha: a key component of the obesity-Diabetes link." Diabetes 43(11): 1271-8.

68. Hristova, M. and L. Aloe (2006). "Metabolic syndrome--neurotrophic hypothesis." Med Hypotheses 66(3): 545-9.

69. Humpert, P. M., S. Kopf, et al. (2006). "Plasma sRAGE is independently associated with urinary albumin excretion in type 2 Diabetes." Diabetes Care 29(5): 1111-3.

70. Ide, A., E. Kawasaki, et al. (2002). "Genetic association between interleukin-10 gene promoter region polymorphisms and type 1 Diabetes age-at-onset." Hum Immunol 63(8): 690-5.

71. Illig, T., F. Bongardt, et al. (2004). "Significant association of the interleukin-6 gene polymorphisms C-174G and A-598G with type 2 Diabetes." J Clin Endocrinol Metab 89(10): 5053-8.

72. Itoh, Y., Y. Kawamata, et al. (2003). "Free fatty acids regulate insulin secretion from pancreatic beta cells through GPR40." Nature 422(6928): 173-6.

73. Jayagopal, V., E. S. Kilpatrick, et al. (2004). "The biological variation of sex hormone-binding globulin in type 2 Diabetes: implications for sex hormone-binding globulin as a surrogate marker of insulin resistance." Diabetes Care 27(1): 278-80.

74. Jinchuan, Y., W. Zonggui, et al. (2004). "Upregulation of CD40--CD40 ligand system in patients with Diabetes Mellitus." Clin Chim Acta 339(1-2): 85-90.

75. Johannesen, J., A. Pie, et al. (2001). "Linkage of the human inducible nitric oxide synthase gene to type 1 Diabetes." J Clin Endocrinol Metab 86(6): 2792-6.

76. Johansen, T., C. Laurino, et al. (2005). "Reduction of adiposity with prolonged growth hormone treatment in old obese rats: effects on glucose handling and early insulin signaling." Growth Horm IGF Res 15(1): 55-63.

77. Kalina, A., C. Szalai, et al. (2002). "Association of plasma lipid levels with apolipoprotein E polymorphism in Type 2 Diabetes." Diabetes Res Clin Pract 56(1): 63-8.

78. Kalmar, T., I. Seres, et al. (2005). "Correlation between the activities of lipoprotein lipase and paraoxonase in type 2 Diabetes Mellitus." Diabetes Metab 31(6): 574-80.

79. Kaneto, H., T. A. Matsuoka, et al. (2005). "A crucial role of MafA as a novel therapeutic target for Diabetes." J Biol Chem 280(15): 15047-52.

80. Kim, H. S., S. Yumkham, et al. (2005). "C-terminal part of AgRP stimulates insulin secretion through calcium release in pancreatic beta Rin5mf cells." Neuropeptides 39(4): 385-93.

81. Kim, J. H., H. D. Shin, et al. (2005). "Peroxisome proliferator-activated receptor gamma coactivator 1 alpha promoter polymorphisms are associated with early-onset type 2 Diabetes Mellitus in the Korean population." Diabetologia 48(7): 1323-30.

82. Kim, M. J., M. Maachi, et al. (2006). "Increased adiponectin receptor-1 expression in adipose tissue of impaired glucose-tolerant obese subjects during weight loss." Eur J Endocrinol 155(1): 161-5.

83. Koka, V., W. Wang, et al. (2006). "Advanced glycation end products activate a chymase-dependent angiotensin II-generating pathway in diabetic complications." Circulation 113(10): 1353-60.

84. Konheim, Y. L. and J. K. Wolford (2003). "Association of a promoter variant in the inducible cyclooxygenase-2 gene (PTGS2) with type 2 Diabetes Mellitus in Pima Indians." Hum Genet 113(5): 377-81.

85. Koo, S. H., L. Flechner, et al. (2005). "The CREB coactivator TORC2 is a key regulator of fasting glucose metabolism." Nature 437(7062): 1109-11.

86. Kotnik, P., J. Nielsen, et al. (2005). "Altered expression of COX-1, COX-2, and mPGES in rats with nephrogenic and central Diabetes insipidus." Am J Physiol Renal Physiol 288(5): F1053-68.

87. Lakka, T. A., H. M. Lakka, et al. (2005). "Effect of exercise training on plasma levels of C-reactive protein in healthy adults: the HERITAGE Family Study." Eur Heart J 26(19): 2018-25.

88. Laukkanen, O., J. Lindstrom, et al. (2005). "Polymorphisms in the SLC2A2 (GLUT2) gene are associated with the conversion from impaired glucose tolerance to type 2 Diabetes: the Finnish Diabetes Prevention Study." Diabetes 54(7): 2256-60.

89. Laukkanen, O., J. Pihlajamaki, et al. (2004). "Polymorphisms of the SUR1 (ABCC8) and Kir6.2 (KCNJ11) genes predict the conversion from impaired glucose tolerance to type 2 Diabetes. The Finnish Diabetes Prevention Study." J Clin Endocrinol Metab 89(12): 6286-90.

90. Lee, S. W., K. E. Song, et al. (2005). "Alterations in peripheral blood levels of TIMP-1, MMP-2, and MMP-9 in patients with type-2 Diabetes." Diabetes Res Clin Pract 69(2): 175-9.

91. Legakis, I., T. Mantzouridis, et al. (2005). "Positive correlation of galanin with glucose in type 2 Diabetes." Diabetes Care 28(3): 759-60.

92. Lewitt, M. S., K. Hall, et al. (2005). "Altered response of insulin-like growth factor-binding protein 1 to nutritional deprivation in type 2 Diabetes Mellitus." Metabolism 54(3): 275-80.

93. Lim, H. S., A. D. Blann, et al. (2004). "Soluble CD40 ligand, soluble P-selectin, interleukin-6, and tissue factor in Diabetes Mellitus: relationships to cardiovascular disease and risk factor intervention." Circulation 109(21): 2524-8.

94. Lindgren, C. M., M. M. Mahtani, et al. (2002). "Genomewide search for type 2 Diabetes Mellitus susceptibility loci in Finnish families: the Botnia study." Am J Hum Genet 70(2): 509-16.

95. Lo, J. C., X. Zhao, et al. (2006). "The association of genetic polymorphisms in sex hormone biosynthesis and action with insulin sensitivity and Diabetes Mellitus in women at midlife." Am J Med 119(9 Suppl 1): S69-78.

96. Maedler, K., A. Fontana, et al. (2002). "FLIP switches Fas-mediated glucose signaling in human pancreatic beta cells from apoptosis to cell replication." Proc Natl Acad Sci U S A 99(12): 8236-41.

97. Magnusson, M., O. Melander, et al. (2004). "Elevated plasma levels ofNt-proBNP in patients with type 2 Diabetes without overt cardiovascular disease." Diabetes Care 27(8): 1929-35.

98. Mallone, R. and P. C. Perin (2006). "Anti-CD38 autoantibodies in type? Diabetes." Diabetes Metab Res Rev 22 (4): 284-94.

99. Mannucci, E., L. Pala, et al. (2005). "Hyperglycaemia increases dipeptidyl peptidase IV activity in Diabetes Mellitus." Diabetologia 48(6): 1168-72.

100. Marchal-Victorion, S., N. Vionnet, et al. (2002). "Genetic, pharmacological and functional analysis of cholecystokinin-1 and cholecystokinin-2 receptor polymorphism in type 2 Diabetes and obese patients." Pharmacogenetics 12(1): 23-30.

101. Marculescu, R., G. Endler, et al. (2002). "Interleukin-1 receptor antagonist genotype is associated with coronary atherosclerosis in patients with type 2 Diabetes." Diabetes 51(12): 3582-5.

102. Marques, R. G., M. J. Fontaine, et al. (2004). "C-peptide: much more than a byproduct of insulin biosynthesis." Pancreas 29(3): 231-8.

103. Marselli, L., L. Trincavelli, et al. (2004). "The role of peripheral benzodiazepine receptors on the function and survival of isolated human pancreatic islets." Eur J Endocrinol 151(2): 207-14.

104. Mathieu, C., C. Gysemans, et al. (2005). "Vitamin D and Diabetes." Diabetologia 48(7): 1247-57.

105. Miele, C., A. Riboulet, et al. (2003). "Human glycated albumin affects glucose metabolism in L6 skeletal muscle cells by impairing insulin-induced insulin receptor substrate (IRS) signaling through a protein kinase C alpha-mediated mechanism." J Biol Chem 278(48): 47376-87.

106. Migdalis, I., T. Thomaides, et al. (2001). "Changes of gastric emptying rate and gastrin levels are early indicators of autonomic neuropathy in type II diabetic patients." Clin Auton Res 11(4): 259-63.

107. Milewicz, A., E. Mikulski, et al. (2000). "Plasma insulin, cholecystokinin, galanin, neuropeptide Y and leptin levels in obese women with and without type 2 Diabetes Mellitus." Int J Obes Relat Metab Disord 24 Suppl 2: S 152-3.

108. Minn, A. H., C. Hafele, et al. (2005). "Thioredoxin-interacting protein is stimulated by glucose through a carbohydrate response element and induces beta-cell apoptosis." Endocrinology 146(5): 2397-405.

109. Mironczuk, K., A. Okruszko, et al. (2005). "Interleukin 18 and sICAM-1 serum levels in families with type 1 Diabetes Mellitus." Rocz Akad Med Bialymst 50: 151-4.

110. Mitamura, Y., A. Tashimo, et al. (2002). "Vitreous levels of placenta growth factor and vascular endothelial growth factor in patients with proliferative diabetic retinopathy." Diabetes Care 25(12): 2352.

111. Miyake, Y., H. Eguchi, et al. (2003). "Glucose intolerance and serum aminotransferase activities in Japanese men." J Hepatol 38(1): 18-23.

112. Miyawaki, K., Y. Yamada, et al. (1999). "Glucose intolerance caused by a defect in the entero-insular axis: a study in gastric inhibitory polypeptide receptor knockout mice." Proc Natl Acad Sci U S A 96(26): 14843-7.

113. Mohlke, K. L., A. D. Skol, et al. (2005). "Evaluation of SLC2A10 (GLUT10) as a candidate gene for type 2 Diabetes and related traits in Finns." Mol Genet Metab 85(4): 323-7.

114. Mustafa, S., T. Vukovich, et al. (2004). "Haptoglobin phenotype and gestational Diabetes." Diabetes Care 27 (9): 2103-7.

115. Nair, S., Y. H. Lee, et al. (2004). "11beta-Hydroxysteroid dehydrogenase Type 1: genetic polymorphisms are associated with Type 2 Diabetes in Pima Indians independently of obesity and expression in adipocyte and muscle." Diabetologia 47(6): 1088-95.

116. Nakagawa, T., H. Furuta, et al. (2005). "Molecular scanning of the betacellulin gene for mutations in type 2 diabetic patients." Diabetes Res Clin Pract 68(3): 188-92.

117. Nomura, S., A. Shouzu, et al. (2005). "5-HT2A receptor antagonist increases circulating adiponectin in patients with type 2 Diabetes." Blood Coagul Fibrinolysis 16(6): 423-8.

118. Noushmehr, H., E. D'Amico, et al. (2005). "Fatty acid translocase (FAT/CD36) is localized on insulin-containing granules in human pancreatic betacells and mediates fatty acid effects on insulin secretion." Diabetes 54(2): 472-81.

119. O'Connell, T. and D. R. Clemmons (2002). "IGF-I/IGF-binding protein-3 combination improves insulin resistance by GH-dependent and independent mechanisms." J Clin Endocrinol Metab 87(9): 4356-60.

120. Ohki-Hamazaki, H., K. Watase, et al. (1997). "Mice lacking bombesin receptor subtype-3 develop metabolic defects and obesity." Nature 390(6656): 165-9.

121. Okazawa, K., Y. Yoshimasa, et al. (2005). "The haplotypes of the IRS-2 gene affect insulin sensitivity in Japanese patients with type 2 Diabetes." Diabetes Res Clin Pract 68(1): 39-48.

122. Pagliarini, D. J., S. E. Wiley, et al. (2005). "Involvement of a mitochondrial phosphatase in the regulation of ATP production and insulin secretion in pancreatic beta cells." Mol Cell 19(2): 197-207.

123. Patti, M. E., A. J. Butte, et al. (2003). "Coordinated reduction of genes of oxidative metabolism in humans with insulin resistance and Diabetes: Potential role of PGC1 and NRF1." Proc Natl Acad Sci U S A 100(14): 8466-71.

124. Repaske, D. R., J. A. Phillips, 3rd, et al. (1990). "Molecular analysis of autosomal dominant neurohypophyseal Diabetes insipidus." J Clin Endocrinol Metab 70(3): 752-7.

125. Rodgers, J. T., C. Lerin, et al. (2005). "Nutrient control of glucose homeostasis through a complex of PGC-1alpha and SIRT1." Nature 434(7029): 113-8.

126. Rose, C. S., J. Ek, et al. (2005). "A -30G>A polymorphism of the beta-cell-specific glucokinase promoter associates with hyperglycemia in the general population of whites." Diabetes 54(10): 3026-31.

127. Rosmond, R., M. Chagnon, et al. (2003). "Increased abdominal obesity, insulin and glucose levels in nondiabetic subjects with a T29C polymorphism of the transforming growth factor-beta1 gene." Horm Res 59(4): 191-4.

128. Roth, S. M., M. A. Schrager, et al. (2002). "IGF2 genotype and obesity in men and women across the adult age span." Int J Obes Relat Metab Disord 26(4): 585-7.

129. Rudofsky, G., Jr., P. Reismann, et al. (2004). "Reduction of postprandial hyperglycemia in patients with type 2 Diabetes reduces NF-kappaB activation in PBMCs." Horm Metab Res 36(9): 630-8.

130. Salopuro, T., J. Lindstrom, et al. (2004). "Common variants in beta2- and beta3-adrenergic receptor genes and uncoupling protein 1 as predictors of the risk for type 2 Diabetes and body weight changes. The Finnish Diabetes Prevention Study." Clin Genet 66(4): 365-7.

131. Sano, H., S. C. Liu, et al. (2002). "Insulin receptor substrate 4 associates with the protein IRAS." J Biol Chem 277(22): 19439-47.

132. Sauvaget, D., V. Chauffeton, et al. (2004). "In vitro transcriptional induction of the human apolipoprotein A-II gene by glucose." Diabetes 53(3): 672-8.

133. Scassa, M. E., A. S. Guberman, et al. (2004). "Hepatic nuclear factor 3 and nuclear factor 1 regulate 5-aminolevulinate synthase gene expression and are involved in insulin repression." J Biol Chem 279(27): 28082-92.

134. Schlosser, M., J. P. Banga, et al. (2005). "Dynamic changes of GAD65 autoantibody epitope specificities in individuals at risk of developing type 1 Diabetes." Diabetologia 48(5): 922-30.

135. Several (2006). "Nature Medicine Special Issue on Metabolic Syndrome." Nature Medicine 12(1): 26-80.

136. Several (2006). "Third Annual World Congress on the Insulin Resistance Syndrome." Diabetes Care 29: 2165-2174.

137. Shaat, N., M. Ekelund, et al. (2005). "Association of the E23K polymorphism in the KCNJ11 gene with gestational Diabetes Mellitus." Diabetologia 48(12): 2544-51.

138. Shimomura, I., R. E. Hammer, et al. (1998). "Insulin resistance and Diabetes Mellitus in transgenic mice expressing nuclear SREBP-1c in adipose tissue: model for congenital generalized lipodystrophy." Genes Dev 12 (20): 3182-94.

139. Shumay, E., X. Song, et al. (2002). "pp60Src mediates insulin-stimulated sequestration of the beta(2)-adrenergic receptor: insulin stimulates pp60Src phosphorylation and activation." Mol Biol Cell 13(11): 3943-54.

140. Siddiq, A., F. Lepretre, et al. (2005). "A synonymous coding polymorphism in the alpha2-Heremans-schmid glycoprotein gene is associated with type 2 Diabetes in French Caucasians." Diabetes 54(8): 2477-81.

141. Sivitz, W. I., S. M. Wayson, et al. (2003). "Leptin and body fat in type 2 Diabetes and monodrug therapy." J Clin Endocrinol Metab 88(4): 1543-53.

142. Song, K. H., S. H. Ko, et al. (2005). "Prospective study of lipoprotein(a) as a risk factor for deteriorating renal function in type 2 diabetic patients with overt proteinuria." Diabetes Care 28(7): 1718-23.

143. Sosa-Pineda, B. (2004). "The gene Pax4 is an essential regulator of pancreatic beta-cell development." Mol Cells 18(3): 289-94.

144. Southgate, R. J., C. R. Bruce, et al. (2005). "PGC-1 alpha gene expression is down-regulated by Akt- mediated phosphorylation and nuclear exclusion of FoxO1 in insulin-stimulated skeletal muscle." Faseb J 19(14): 2072-4.

145. Spranger, J., A. Kroke, et al. (2003). "Inflammatory cytokines and the risk to develop type 2 Diabetes: results of the prospective population-based European Prospective Investigation into Cancer and Nutrition (EPIC)-Potsdam Study." Diabetes 52(3): 812-7.

146. Taniguchi, A., M. Fukushima, et al. (2005). "Soluble E-selectin, leptin, triglycerides, and insulin resistance in nonobese Japanese type 2 diabetic patients." Metabolism 54(3): 376-80.

147. Tayebjee, M. H., H. S. Lim, et al. (2004). "Matrix metalloproteinase-9 and tissue inhibitor of metalloproteinase-1 and -2 in type 2 Diabetes: effect of 1 year's cardiovascular risk reduction therapy." Diabetes Care 27(8): 2049-51.

148. Terazono, K., H. Yamamoto, et al. (1988). "A novel gene activated in regenerating islets." J Biol Chem 263(5): 2111-4.

149. Theodorakis, M. J., O. Carlson, et al. (2004). "Elevated plasma glucose-dependent insulinotropic polypeptide associates with hyperinsulinemia in impaired glucose tolerance." Diabetes Care 27(7): 1692-8.

150. Thomas, G. N., J. A. Critchley, et al. (2003). "Renin-angiotensin system gene polymorphisms and retinopathy in chinese patients with type 2 Diabetes." Diabetes Care 26(5): 1643-4.

151. Thomas, M. K., K. M. Yao, et al. (1999). "Bridge-1, a novel PDZ-domain coactivator of E2A-mediated regulation of insulin gene transcription." Mol Cell Biol 19(12): 8492-504.

152. Tohjima, T., N. Honda, et al. (1998). "Decreased activity of arachidonate 12-lipoxygenase in platelets of Japanese patients with non-insulin-dependent Diabetes Mellitus." Metabolism 47(3): 257-63.

153. Tokuyama, Y., K. Matsui, et al. (2004). "Five missense mutations in glucagon-like peptide 1 receptor gene in Japanese population." Diabetes Res Clin Pract 66(1): 63-9.

154. Torres, S. H., J. B. De Sanctis, et al. (2004). "Inflammation and nitric oxide production in skeletal muscle of type 2 diabetic patients." J Endocrinol 181(3): 419-27.

155. Tsiavou, A., E. Hatziagelaki, et al. (2005). "Correlation between intracellular interferon-gamma (IFN-gamma) production by CD4+ and CD8+ lymphocytes and IFN-gamma gene polymorphism in patients with type 2 Diabetes Mellitus and latent autoimmune Diabetes of adults (LADA)." Cytokine 31(2): 135-41.

156. Tsuchida, H., M. Bjornholm, et al. (2002). "Gene expression of the p85alpha regulatory subunit of phosphatidylinositol 3-kinase in skeletal muscle from type 2 diabetic subjects." Pflugers Arch 445(1): 25-31.

157. Ukkola, O., M. Chagnon, et al. (2003). "Genetic variation at the adipsin locus and response to long-term overfeeding." Eur J Clin Nutr 57(9): 1073-8.

158. Ukkola, O., J. Salonen, et al. (2005). "Role of candidate genes in the lipid responses to intensified treatment in Type 2 Diabetes." J Endocrinol Invest 28(10): 871-5.

159. Um, S. H., F. Frigerio, et al. (2004). "Absence of S6KI protects against age-and diet-induced obesity while enhancing insulin sensitivity." Nature 431(7005): 200-5.

160. Vanttinen, M., P. Nuutila, et al. (2005). "Single nucleotide polymorphisms in the peroxisome proliferator-activated receptor delta gene are associated with skeletal muscle glucose uptake." Diabetes 54(12): 3587-91.

161. Vettor, R., G. Milan, et al. (2005). "Review article: adipocytokines and insulin resistance." Aliment Pharmacol Ther 22 Suppl 2: 3-10.

162. Wahab, N. A., L. Schaefer, et al. (2005). "Glomerular expression of thrombospondin-1, transforming growth factor beta and connective tissue growth factor at different stages of diabetic nephropathy and their interdependent roles in mesangial response to diabetic stimuli." Diabetologia 48(12): 2650-60.

163. Wakabayashi, M., H. Ohi, et al. (2006). "Acquired Loss of Erythrocyte Complement Receptor Type 1 in Patients with Diabetic Nephropathy Undergoing Hemodialysis." Nephron Exp Nephrol 104(3): e89-e95.

164. Wautier, M. P., E. Boulanger, et al. (2004). "AGEs, macrophage colony stimulating factor and vascular adhesion molecule blood levels are increased in patients with diabetic microangiopathy." Thromb Haemost 91(5): 879-85.

165. Wei, F. Y., K. Nagashima, et al. (2005). "Cdk5-dependent regulation of glucose-stimulated insulin secretion." Nat Med 11(10): 1104-8.

166. Wolford, J. K., K. A. Yeatts, et al. (2005). "Sequence variation in PPARG may underlie differential response to troglitazone." Diabetes 54(11): 3319-25.

167. Wu, B., J. Takahashi, et al. (2005). "Variants of calpain-10 gene and its association with type 2 Diabetes Mellitus in a Chinese population." Diabetes Res Clin Pract 68(2): 155-61.

168. Xue, Y. M., L. Zhou, et al. (2002). "Correlation between angiotensin II type 1 receptor gene polymorphism and type 2 Diabetes Mellitus complicated by hypertension." Di Yi Jun Yi Da Xue Xue Bao 22(5): 444-6.

169. Yagui, K., F. Shimada, et al. (1998). "A missense mutation in the CD38 gene, a novel factor for insulin secretion: association with Type II Diabetes Mellitus in Japanese subjects and evidence of abnormal function when expressed in vitro." Diabetologia 41(9): 1024-8.

170. Yang, Q., T. E. Graham, et al. (2005). "Serum retinol binding protein 4 contributes to insulin resistance in obesity and type 2 Diabetes." Nature 436(7049): 356-62.

171. Yang, Y., E. K. Chung, et al. (2003). "Diversity in intrinsic strengths of the human complement system: serum C4 protein concentrations correlate with C4 gene size and polygenic variations, hemolytic activities, and body mass index." J Immunol 171(5): 2734-45.

172. Ye, L., Y. Xu, et al. (2002). "[Association of polymorphism in neurogenic differentiation factor 1 gene with type 2 Diabetes]." Zhonghua Yi Xue Yi Chuan Xue Za Zhi 19(6): 484-7.

173. Zacharova, J., J. L. Chiasson, et al. (2005). "Leptin receptor gene variation predicts weight change in subjects with impaired glucose tolerance." Obes Res 13(3): 501-6.

174. Zeggini, E., J. Parkinson, et al. (2004). "Association studies of insulin receptor substrate 1 gene (IRS1) variants in type 2 Diabetes samples enriched for family history and early age of onset." Diabetes 53(12): 3319-22.

175. Zhang, J., R. I. Holt, et al. (2005). "Plasma adiponectin concentrations are independently predicted by fat insulin sensitivity in women and by muscle insulin sensitivity in men." Diabetes Care 28(3): 755-6.

176. Zhu, F., L. Ji, et al. (2002). "[The role of urotensin II gene in the genetic susceptibility to type 2 Diabetes in Chinese population]." Zhonghua Yi Xue Za Zhi 82(21): 1473-5.

177. Zimmermann, R., J. G. Strauss, et al. (2004). "Fat mobilization in adipose tissue is promoted by adipose triglyceride lipase." Science 306(5700): 1383-6.

**Claims**

1. A method with a predetermined level of predictability for assessing a risk of development of Diabetes Mellitus or a pre-diabetic condition in a subject, comprising:

   a. measuring the level of an effective amount of two or more DBRISKMARKERS selected from the group consisting of: adiponectin (C1Q and collagen domain containing, ADIPOQ), c-reactive protein (pentraxin-related; CRP), ferritin (FTH1), and insulin (INS) in a sample from the subject, and
   b. measuring a clinically significant alteration in the level of the two or more DBRISKMARKERS in the sample, wherein the alteration indicates an increased risk of developing Diabetes Mellitus or a pre-diabetic condition in the subject.

2. A method with a predetermined level of predictability for assessing a risk of development of Diabetes Mellitus or a pre-diabetic condition in a subject, comprising:

   a. measuring the level of an effective amount of two or more DBRISKMARKERS selected from the group consisting of DBRISKMARKERS 1-260 in a sample from the subject, and
   b. measuring a clinically significant alteration in the level of the two or more DBRISKMARKERS in the sample, wherein the alteration indicates an increased risk of developing Diabetes Mellitus or a pre-diabetic condition in the subject.

3. A method with a predetermined level of predictability for diagnosing or identifying a subject having Diabetes Mellitus or a pre-diabetic condition comprising:

   a. measuring the level of an effective amount of two or more DBRISKMARKERS selected from the group consisting of DBRISKMARKERS 1-260 in a sample from the subject, and
   b. comparing the level of the effective amount of the two or more DBRISKMARKERS to a reference value.

4. A method with a predetermined level of predictability for assessing the progression of Diabetes Mellitus or a pre-diabetic condition in a subject, comprising:

   a. detecting the level of an effective amount of two or more DBRISKMARKERS selected from the group consisting of DBRISKMARKERS 1-260 in a first sample from the subject at a first period of time;
   b. detecting the level of an effective amount of two or more DBRISKMARKERS in a second sample from the subject at a second period of time;
   c. comparing the level of the effective amount of the two or more DBRISKMARKERS detected in step (a) to the amount detected in step (b), or to a reference value.

5. A method with a predetermined level of predictability for monitoring the effectiveness of treatment for Diabetes Mellitus or a pre-diabetic condition comprising:

> a. detecting the level of an effective amount of two or more DBRISKMARKERS selected from the group consisting of DBRISKMARKERS 1-260 in a first sample from the subject at a first period of time;
> b. detecting the level of an effective amount of two or more DBRISKMARKERS in a second sample from the subject at a second period of time;
> c. comparing the level of the effective amount of the two or more DBRISKMARKERS detected in step (a) to the amount detected in step (b), or to a reference value,

wherein the effectiveness of treatment is monitored by a change in the level of the effective amount of two or more DBRISKMARKERS from the subject.

6.  A kit comprising a plurality of DBRISKMARKER detection reagents that detect the corresponding DBRISKMARKERS selected from the group consisting of DBRISKMARKERS 1-260, sufficient to generate:

> a. a Diabetes Mellitus reference expression profile, comprising a pattern of marker levels of an effective amount of two or more markers selected from the group consisting of DBRISKMARKERS 1-260, taken from one or more subjects who do not have the Diabetes Mellitus
> b. an impaired glucose tolerance reference expression profile, comprising a pattern of marker levels of an effective amount of two or more markers selected from the group consisting of DBRISKMARKERS 1-260, taken from one or more subjects who do not have impaired glucose tolerance
> c. a Diabetes Mellitus subject expression profile, comprising a pattern of marker levels of an effective amount of two or more markers selected from the group consisting of DBRISKMARKERS 1-260 taken from one or more subjects who have the Diabetes Mellitus, are at risk for developing the Diabetes Mellitus, or are being treated for the Diabetes Mellitus; or
> d. an impaired glucose tolerance subject expression profile, comprising a pattern of marker levels of an effective amount of two or more markers selected from the group consisting of DBRISKMARKERS 1-260 taken from one or more subjects who have impaired glucose tolerance, are at risk for developing impaired glucose tolerance, or are being treated for impaired glucose tolerance.

7.  The method or kit of any preceding claim, wherein the level of expression of three DBRISKMARKERS is measured.

8.  The method or kit of any preceding claim, wherein the level of expression of five or more DBRISKMARKERS is measured.

9.  The method or kit of any of claims 2-8, wherein the DBRISKMARKERS are selected from the group consisting of: adiponectin (C1Q and collagen domain containing, ADIPOQ), c-reactive protein (pentraxin-related; CRP), ferritin (FTH1), and insulin (INS).

10. The method or kit of any one of claims 2 to 8, wherein the DBRISKMARKERS are selected from the group consisting of: adiponectin (ADIPOQ), c-reactive protein (CRP), receptor for advanced glycosylation endproducts (RAGE), IL-18, adipisin, and PAI-I (SERPINE1).

11. The method of any preceding claim, wherein the sample is serum, blood plasma, blood cells, endothelial cells, tissue biopsies, ascites fluid, bone marrow, interstitial fluid, sputum, or urine.

12. The method of any preceding claim, wherein the level of DBRISKMARKERS is measured electrophoretically or immunochemically (e.g. by radioimmunoassay, immunofluorescence assay or by an enzyme-linked immunosorbent assay) or by specific oligonucleotide hybridization.

13. The method of claim 3, 4 or 5, or claims 7-12 when dependent on claim 3, 4 or 5, wherein the reference value is (i) derived from one or more subjects who have suffered from Diabetes Mellitus or the pre-diabetic condition, (ii) derived from one or more risk prediction algorithms or computed indices for the Diabetes or pre-diabetic condition, (iii) derived from samples from one or more subjects who have shown an improvement in Diabetes risk factors as a result of one or more treatments for the Diabetes Mellitus or the pre-diabetic condition, or (iv) an index value.

14. The method of claim 4 or 5, or claims 7-13 when dependent on claim 4 or 5, wherein the first sample has been obtained from the subject prior to treatment for the Diabetes Mellitus or the prediabetic condition and/or the second sample has been obtained from the subject after treatment for the Diabetes Mellitus or the pre-diabetic condition.

**15.** The kit of any one of claims 5 to 10, wherein the detection reagent comprises: (i) one or more antibodies or fragments thereof; (ii) one or more oligonucleotides; or (iii) one or more aptamers.

**16.** The method or kit of any preceding claim wherein: (i) the Diabetes Mellitus comprises Type 2 Diabetes, Type 1 Diabetes, or gestational Diabetes; or (ii) the pre-diabetic condition comprises IFG, IGT, Metabolic Syndrome, or Syndrome X.

# FIG. 1

| Official Symbol | Key Markers to Prediction | KEGG Pathway Data (n/a no info ava | 01430 Cell Communication | 03320 PPAR signalling pathway | 04010 MAPK signalling pathway | 04060 Cytokine-cytokine receptor info | 04080 Neuroactive ligand receptor Int | 04110 Cell cycle | 04140 Regulation of autophagy | 04150 mTOR signalling pathway | 04210 Apotsis | 04350 TGF-beta signalling pathway | 04360 Axon guidance | 04370 VEGF signalling pathway | 04510 Focal adhesion | 04512 ECM receptor interaction | 04514 Cell adhesion molecules (CAMs) | 04530 Tight Junction | 04540 Gap Junction |
|---|---|---|---|---|---|---|---|---|---|---|---|---|---|---|---|---|---|---|---|
| LEP | ■ | | | | ■ | ■ | | | | | | | | | | | | | |
| CRP | | ■ | | | | | | | | | | | | | | | | | |
| IL6R | | | | | | ■ | | | | | | | | | | | | | |
| TNFRS1A | | | | | ■ | ■ | | | | | ■ | | | | | | | | |
| 1L6 | | | | | | ■ | | | | | | | | | | | | | |
| HP | ■ | ■ | | | ■ | ■ | | | | | | | | | | | | | |
| IL18 | | | | | | ■ | | | | | | | | | | | | | |
| AHSG | | ■ | | | | | | | | | | | | | | | | | |
| FGA | | | | | | | | | | | | | | | | | | | |
| AGER | | ■ | | | ■ | | | | | | | | | | | | | | |
| DPP4 | | ■ | | | | | | | | | | | | | | | | | |
| IGFBP3 | ■ | ■ | | | ■ | | | ■ | | | | | | | | ■ | | | |
| TGFB1 | | | | | ■ | | | ■ | | | | ■ | | | | | | | |
| ICAM1 | | | | | | | | | | | | | | | | | ■ | | |
| c-peptide | | | | | | | | | | | | | | | | | | | |
| AKT1 | | | | | ■ | | | | | ■ | | | ■ | | | | ■ | | |
| RETN | ■ | ■ | | | ■ | | | | | | | | | | | | | | |

# FIG. 1(contd.)

| Pathway | Total Pathways |
|---|---|
| 04610 Complement and coagulation c | 4 |
| 0620 Toll-like receptor signaling patt | 4 |
| 04630 Jak-STAT signaling pathway | 3 |
| 04640 Hematopoietic cell lineage | 4 |
| 04650 Natural killer cell mediated cyto | 4 |
| 04660 T cell receptor signalling pathw | 1 |
| 04662 B cell receptor signalling pathw | 0 |
| 04664 Fc epsilon R1 signalling pathwa | 1 |
| 04670 Leukocyte transendothelial mic | 1 |
| 04810 Regulation of actin cytoskeleto | 0 |
| 04910 Insulin signalling pathway | 6 |
| 04912 GnRh signalling pathway | 6 |
| 04920 Adipocytokine signalling pathw | 3 |
| 04930 Type II diabetes melitos | 0 |
| 04940 Type I diabetes melitos | 16 |
| 05120 Epithelial cell signalling in Helio | 2 |
| 05130 Pathogenic Escherichia coli inf | |
| 05131 Pathogenic Escherichia coli inf | |
| 05210 Colorectal cancer | |
| 05212 Pancreatic cancer | |
| 052114 Giloma | |

FIG. 1(contd.)

FIG. 1(contd.)

# FIG. 2

# FIG. 2(contd.)

AKT1
RESISTIN
ADIPSIN
VCAM
MMP2
ACTIVIN A
CD40 SOLUBLE
HGF
TNFALPHA
ACE
MMP9
FAS LIGAND
IL1_BETA
PAI_1
IL_8
THROMBOSPONDIN
MCP_1
COMP_C4
NGF
COMP_C3
CD14
EGF
CD40 LIGAND
IL_1_RA
BETACELLUIN
FAS
E_SELECTIN
VON WILLIBRAND FACTOR
CDK5
MCSF
IL10
ADIPONECTIN
VEGF
INFGAMMA
AGRP

CUTOFFS FOR COLOUR SCALE

| | | |
|---|---|---|
| 1.00000 | | 0.24999 |
| 0.50000 | | 0.10000 |
| 0.49999 | | 0.09999 |
| 0.25000 | | 0.00000 |

FIG. 2(contd.)

CUTOFFS FOR COLOUR SCALE

| | |
|---|---|
| 1.00000 | |
| 0.50000 | |
| 0.49999 | |
| 0.25000 | |

| | |
|---|---|
| 0.24999 | |
| 0.10000 | |
| 0.09999 | |
| 0.00000 | |

FIG. 2(contd.)

# FIG. 3A

## NEUROACTIVE LIGAND-RECPTOR INTERACTION

GPCRs

Class A Rhodopsin like
Amine

FIG. 3A — Neuroactive ligand-receptor interaction pathway diagram showing GPCR Class A Rhodopsin-like receptors.

# FIG. 3B

## CYTOKINE-CYTOKINE RECEPTOR INTERACTION

Chemokines

# FIG. 3B (contd.)

# FIG. 3B (contd.)

# FIG. 3B (contd.)

### TGF-b family

| | | |
|---|---|---|
| TGFB1 | TGFBR2 | Type II |
| TGFB2 | TGFBR1 | Type I |
| TGFB3 | | |

| | | |
|---|---|---|
| | ACVR2 | Type II |
| | ACVR1 | Type I |
| INHBA | ACVR2 | Type II |
| INHBB | ACVR1B | Type I |
| INHBC | ACVR2B | Type II |
| INHBE | ACVR1 | Type I |
| | ACVR2B | Type II |
| | ACVR1B | Type I |

| | | |
|---|---|---|
| AMH | AMHR2 | Type II |
| | ACVR1? | Type I |

| | | |
|---|---|---|
| | BMPR2 | Type II |
| | ACVR1 | Type I |
| BMP2 | BMPR2 | Type II |
| | BMPR1B | Type I |
| | BMPR2 | Type II |
| | BMPR1A | Type I |

| | | |
|---|---|---|
| BMP7 | ACVR2 | Type II |
| | BMPR1B | Type I |

| | | |
|---|---|---|
| GDF5 | ACVR2 | Type II |
| | BMPR1A | Type I |

### IL-17 family

| | |
|---|---|
| IL17A | IL17R |
| IL17B | IL17RB |
| IL17E | |

### IL-1 family

| | | |
|---|---|---|
| | IL1R1 | |
| IL1A | ILRAP | |
| IL1B | IL1R2 | No signal |
| | IL1RAP | transduction |
| IL18 | IL18R1 | |
| | IL18RAP | |

# Fig. 3C ADIPOCYTOKINE SIGNALING PATHWAY

EP 2 177 908 A2

# FIG. 3D

## MAPK SIGNALLING PATHWAY

# FIG. 3D(contd.)

FIG. 3E INSULIN SIGNALING PATHWAY

# FIG. 3F TYPE II DIABETES MELLITIUS

Adipocyte, hepatocyte, skeletal muscle cell

Obesity ---| ADIPO

Adipocytokine signaling pathway

GLUT4

Insulin signaling pathway

INS (Hyperinsulinism) → INSR

+py

IRS

IRS

PI3K

Type II diabetes mellitus

SOCS

+ps

ERK

Adipocytokine signaling pathway

IKK

Obesity ---→ FFA

JNK

TNFα

mTOR

PKCζ

PKCc1δ

Insulin resistance

Transient hyperglycemia Hyperinsulinism

Pancreatic β-cell

Glucose (Hyperglycemia)

Ca$^{2+}$-dependent

Apoptosis

Impaired insulin secretion

Glycation, hexsamine pathway,respiratory chain

JNK

PDX-1

ROS

MafA

DNA

INS

Maturity onset diabetes of the young

Prevention of membrane depolarization

VDDC

Ca$^{2+}$

SUR1

Kir62

K$^+$

Mitochondrion

Mitochondrial dysfunction

Glucose

GLUT2

GK

PYK

Pyruvate

EP 2 177 908 A2

# Fig. 3G  APOPTOSIS

# Fig. 3G (contd.)

# COPMPLEMENT AND COAGULATION CASCADES

FIG. 3H

FIG. 3H(contd.)

# FIG. 3I   JAK-STAT SIGNALING PATHWAY

Endocytosis

Lysosomal proteolysis

Ubiquitin mediated proteolysis

Growth, proliferation fate determination development, immunity

SOCS

Pim-1

CIS

IFNα

P48

CBP

c-Myc

CycD — Cell Cycle

Bc1XL — Antiapoptosis / Apoptosis

Spred

Sprouty

Cb1

STAM

STAT

+p

PIAS

Homo / hetero STAT dimers

STAT5

SHP2

GRB

SOS

Rasl MAPK — MAPK signaling pathway

P13K

AKT

+p

Antiapoptosis / Apoptosis

CytokineR

JAK

SHP1

-p

+p

+u

EPO

IFNIL10

IL2/3

IL6

UPD

Cytokine

Cytokine-cytokine receptor interaction

HEMATOPOIETIC CELL LINEAGE

# FIG. 3J

**Lymphoid Related Dendritic Cell**

IL-7

γδ T cll

**Thymuses**

CD8 T cell

SCF IL-7 → **Pro T cell (DN2)** → SCF IL-7 → **DN3** → (IL-7) → **DN4** → **Intermediate single-positive cell (ISP)** → **Double-positive cell (DP)** → CD4 T cell

**Pro T cell (DN2)**
(CD2) (CD5)
CD7 CD25
CD38 CD44
(CD71) CD117
CD127 TdT
HLA-DR

**DN3**
CD2 CD5
CD7 CD25
CD38 CD44
CD71 CD117
(CD127) TdT

**DN4**
CD1 (CD2)
(CD4) CD5
CD7 CD38
(CD44) (CD117)
TdT

**Intermediate single-positive cell (ISP)**
CD2 CD3
CD4or8 CD5
CD7 CD38

**Double-positive cell (DP)**
CD2 CD3
CD4or8 CD5
(CD7)

RegulatoryT cell

NK T cell

| SCF | IL-7 |
| --- | --- |

| HLA-DR | CD44 | CD117 | CD25 | CD127 | TdT | CD71 | CD38 | CD7 | CD2 | CD5 | CD1 | CD4 | CD8 | CD3 |
| --- | --- | --- | --- | --- | --- | --- | --- | --- | --- | --- | --- | --- | --- | --- |

**NK cell Precursor**

**NK cell**

SCF IL-7 → **Lymphoid stem cell, Double-negative cell (DN1)**
CD34
CD44
CD117
TdT
HLA-DR

IL7 → **Pro B cell**
(CD9) (CD10)
CD19 (CD20)
CD22 CD24
CD117 CD127
TdT HLA-DR

→ **Pre BI cell**
CD9 CD10
CD19 CD20
CD22 CD24
CD38 CD117
CD127 TdT
HLA-DR

→ **Pre B II cell**

→ **Immature B cell**
(CD9) CD19
CD20 CD21
CD22 CD24
CD37 HLA-DR
IgM

→ **B cell**
(CD5) CD9
CD19 CD20
CD21 CD22
(CD23) CD24
CD35 CD37
HLA-DR IgM
IgD

| IL-7 |
| --- |

| TdT | CD117 | CD10 | CD38 | CD127 | CD9 | HLA-DR | CD19 | CD22 | CD24 | CD25 | CD20 | CD21 | CD37 | IgM | CD23 | CD35 | IgD |
| --- | --- | --- | --- | --- | --- | --- | --- | --- | --- | --- | --- | --- | --- | --- | --- | --- | --- |

EP 2 177 908 A2

# FIG. 3J(contd.)

FIG. 3J(contd.)

**Myeloid**

Myeloid Stem Cell

| Flt3L | SCF | G-CSF | IL-3 | IL-6 | IL-11 |

Flt3L
SCF
GM-CSF
G-CSF
IL-3

IL-1
IL-3
IL-6
IL-11

CFU-GEMM
CD33
CD116
CD121
IL-9R
HLA-DR
CD34
CD117
CD123
EPOR

| Flt3L | SCF | G-CSF | IL-3 | IL-6 | IL-11 | GM-CSF |

GM-CSF
G-CSF
IL-3

CFU-GM
CD15
CD34
CD114
CD116
CD123
CD125
HLA-DR
CD33
CD64
CD115
CD121
CD124
CD126

Flt3L
SCF
GM-CSF

G-CSF
GM-CSF
IL-3

CFU-G
CD13
CD33
CD116
CD123
CD125
HLA-DR
CD15
CD114
CD121
CD124
CD126

| Flt3L | SCF | IL-3 | GM-CSF | G-CSF |
| IL-9R | CD34 | HLA-DR | CD116 | CD121 | CD114 | CD123 | CD124 | CD126 | CD33 | CD13 | CD125 | CD15 | CD11b |

GM-CSF
G-CSF

Myeloblast
CD13
CD33
CD116
CD123
CD125
CD15
CD114
CD121
CD124
CD126

GM-CSF
G-CSF

Neutrophilic Myelocyte
CD11b
CD33
CD15
CD116
CD123
CD125

GM-CSF
G-CSF

Neutrophil
CD11b
CD15
CD33

**BFU-E**
CD33
CD117
EPOR
CD34
CD123
HLA-DR

SCF
GM-CSF
IL-3

IL-3
IL-4
EPO

| Flt3L | SCF | GM-CSF | IL-3 | IL-4 | EPO | TPO |
| HLA-DR | EPOR | CD33 | CD34 | CD117 | CD123 | CD36 | CD235a | CD35 | CD44 | CD55 | CD59 |

CFU-E
CD36
CD235a

TPO
EPO

Proerythroblast
CD235a

EPO

Erythrocyte
CD35
CD55
CD235a
CD44
CD59

**BFU-MK**
CD33
CD116
CD126
HLA-DR
CD34
CD123
IL-11R

Flt3L
SCF
GM-CSF
IL-3

IL-6
IL-11
TPO

Flt3L
SCF
GM-CSF
IL-3

Meg-CSF
IL-3
IL-6

IL-11
TPO

| Flt3L | SCF | IL-3 | IL-6 | IL-11 | GM-CSF | Meg-CSF | TPO |
| HLA-DR | CD34 | CD33 | IL-11R | CD116 | CD123 | CD126 | CD9 | CD14 | CD36 | CD41 | CD42 | CD49 |

CFU-MK
CD61
CD116
CD122
CD126

SCF
GM-CSF
IL-3

IL-6
IL-11
TPO

Mega-karyocyte
CD9
CD36
CD42
CD116
CD126
CD14
CD41
CD61
CD123

IL-6
IL-11
TPO

Platelets
CD9
CD36
CD42
CD61
CD14
CD41
CD49
CD126

# FIG. 3K PPAR SIGNALING PATHWAY

Ligand                                    Transcription factor

# FIG. 3K (contd.)

Target gene

# Fig. 3L TOLL-LIKE RECEPTOR SIGNALING PATHWAY

# Fig. 3L  (contd.)

# Fig. 3L  (contd.)

Inflammatory cytokines

| TNFα |
| IL-1β |
| IL-6 | - - - - → Proinflammatory effects
| IL-12 |

| IL-8 |
| RANTES | - - - - → Chemotactic effects (Nutrophil, Immature DC NK cell)
| MIP-1α |
| MIP-1β |

Complement and Coagulation cascade

Costimulatory molecules

| CD40 |
| CD80 | - - - - → T cell stimulation
| CD86 |

Inflammatory cytokines

| IFN-α |
| IFN-β | - - - - → Antiviral effects

Cyokine-cytokine receptor interaction

| IP10 |
| MIG | - - - - → Chemotactic effects (T cell)
| I-TAC |

# FIG. 3M

T CELL RECEPTOR SIGNALLING PATHWAY

# FIG. 3M(contd.)

# FIG. 3N

FOCAL ADHESION

# FIG. 3N(contd.)

# FIG. 3O

EP 2 177 908 A2

# FIG. 3P

EP 2 177 908 A2

FIG. 3P(contd.)

# Fig. 3Q  mTOR SIGNALING PATHWAY

EP 2 177 908 A2

114

TGF-BETA SIGNALING PATHWAY          FIG. 3R

# FIG. 3R(contd.)

# Fig. 3S CALCIUM SIGNALING PATHWAY

# Fig. 3S  (contd.)

# FIG. 3T
## NATURAL KILLER CELL MEDIATED CYTOTOXICITY

# FIG. 3T(contd.)

# FIG. 3T(contd.)

Fig. 3U B CELL RECEPTOR SIGNALING PATHWAY

Fc EPSILON R1 SIGNALING PATHWAY

Mast cell

FIG. 3V

Phosphatidyl inositol signaling system

MAPK signaling pathway

IP$_3$

DAG

PLCγ

PDK1

MKK4/7

MKK3/6

MEK

Raf-1

PI3K

RAC

Ras

Btk

SLP-76

Vav

Sos

PIP$_3$

Gab2

Grb2

SHIP

Fyn

Syk

LAT

Lyn

FCεRIα  FCεRIβ  FCεRIγ

FCεRIα  FCεRIβ  FCεRIγ

IgE

IgE

Antigen

FIG. 3V(contd.)

**Degranulation**

Toxic to parasites

Histamine  Heparin

Increase vascular permeability

Cause smooth muscle contraction

$Ca^{2+}$

PKC

+p AKT

+p JNK

+p p38

+p ERK

DNA

cPLA2

Arachidoni acid

Arachidonic acid metabolism

**Cytokine gene transcription**

IL-4   IL-13

Stimulate and amplify Th2 cell response

IL-3   IL-5

GM-CSF

Promote eosinophil production and activation

TNFα

Promotes inflammation
Stimulates cytokine production by many cell types
Activates endothelium

**Generation of eicosanoids**

$LTC_4$   $LTD_4$
$LTE_4$

Cause smooth muscle contraction
Increase vascular permeability
Stimulate mucus secretion

$PGD_2$

Recruit effector cells

EP 2 177 908 A2

FIG. 3W

LEUKOCYTE TRANSENDOTHELIAL MIGRATION

ARACHIDONIC ACID METABOLISM

# FIG. 3X

EP 2 177 908 A2

# FIG. 3Y

## WNT SIGNALLING PATHWAY

Canonical pathway

Alzheimer's disease

PS-1

PKA

FRP

Idax

Proc

Dally

Wnt

Frizzled

CKIε

GBP

GSK-3β +p β-catenin

APC

CKIα

PP2A

Cer-1 Wif-1

LRP5/6

Dvl

CK2

Axam

Axin

Dkk

p53 Siah-1

SIP

APC Skp1

PAR-1 Nkd

Ebi1

Phosphorylation independence

Planar cell polarity (PCP) pathway

Stbm Prickle

Daam1 RhoA ROCK2

Wnt11 Frizzled Dvl

Rac JNK

Knypek

MAPK signaling pathway

Wnt/ Ca2+ pathway

CaMKII

Ca2+

Wnt5 Frizzled PLC O CaN

G protein? PKC

127

FIG. 3Y(contd.)

VEGF SIGNALING PATHWAY

FIG. 3Z

# FIG. 3AA

## CELL ADHESION MOLECULES

## NEURAL SYSTEM

FIG. 3AA(contd.)

NEURAL SYSTEM

Epithelial Cells

CLDN — CLDN
OCLN — OCLN
JAM1 — JAM1
PVRL1 ←→ PVRL1
CDHE ←→ CDHE
IGSF4 — IGSF4

Tight
Junction

Adherns
Junction

Sperma tid    Sertoli cell

CDHE — CDHE
CDH2 — CDH2
PVRL3 — PVRL2
          ITGA6
          ITGB1

FIG. 3AA(contd.)

Ciliary body
Pigment     Non pigment
epithelia     epithelia

PVRL1 — PVRL3
PVRL3 — PVRL1
CDH3 — CDH3

Myoblasts

CDH2 ←→ CDH2
CDH4 ←→ CDH4
CDH15 — CDH15
          NEO1

132

# FIG. 3BB
## REGULATION OF ACTIN CYTOSKELETON

FIG. 3BB (contd.)

FIG. 3CC

GLIOMA

FIG. 3CC (contd.)

# FIG. 3DD
## NICOTINATE AND NICOTINAMIDE MEATBOLISM

# FIG. 3DD(contd.)

# FIG. 3EE

ADHERNS JUNCTION

FIG. 3EE (contd.)

# FIG. 3FF

TIGHT JUNCTION

Paracellular space

Inside

Inside

Crumbs3

PAL S1

PATJ

PAR3  +p

Formation of lateral membrane domain

PKCs

CKII

Gα

Claudins | Claudins

ZAK

Tight junction formation

ZO-3  +p

MUPP1

YAP33

ZO-1

ZO-2

Occludin | Occludin

AF-6

Fodrin

7H6

Ras

JEAP

PP2A  CASK

PILT

-p

JAM | JAM

MAGI1

MAGI2/3

# FIG. 3FF(contd.)

# ANTIGEN PROCESSING AND PRESENTATION

MHC I pathway

FIG. 3GG

| INFα | TNFα |
|------|------|

↓

| PA28 | Immuno-
proteasome

Cytosolic antigens → Proteasome

HSP70

HSP90

TAP1/2

CD8

CD8 T cell

TCR → Killing of target cells

T cell receptor signaling pathway

Endoplasmic reticulum

| BiP |
| CANX |
| MHCI |

| ERp57 |
| CALR |
| MHCI |
| β2m |

TAPBP

| MHCI |
| β2m |

→ | MHCI |
| β2m |

NK cell

KIR → Regulation of NK cell activity

Natural killer cell mediated cytotoxicity

MHC II pathway

| GILT | AEP | CTSB |

Endocytosed antigens

Endosome

EP 2 177 908 A2

FIG. 3GG(contd.)

FIG. 3HH

LONG-TERM POTENTIATION

# FIG. 3II

## GnRH SIGNALING PATHWAY

Pituitary gonadotrope cell

# FIG. 3II(contd.)

# FIG. 3JJ
## COLORECTAL CANCER

Genetic alterations

Oncogenes :           β-catenin, K-Ras

Tumor suppressors : APC, DCC, TGFβRII, Smad2,
                    Smad4, Bax, p53

DNA repair genes :  hMLH1, hMSH2, hMSH3, hMSH6

Survivin — — ▶ Anti-apoptosis

TCF/LEF

DNA

c-Myc

CyclinD1 — — ▶ Proliferation

Cell cycle

c-Jun

+p    c-Fos — ▶O ▶ CyclinD1 — — ▶ Proliferation

DNA

c-Myc

# FIG. 3JJ(contd.)

MSI pathway
only

hMLH1

hMSH2

Loss of growth inhibitory
effects of TGFβ

DNA

TGFβRII

Genes with coding    hMSH3    hMSH6
microsatellites
DNA

Bax

DNA     Karyotypic instability
damage    p53    O — — ▶  Impaired G1 cycle arrest
                            Reduced apoptosis
DNA

**FIG. 3KK**

Wnt signaling pathway

TGF-beta signaling pathway

Cytokine-cytokine receptor interaction

Neuroactive ligand-receptor interaction

Phosphatidylinositol signaling system

Calcium signaling pathway

Regulation of actin cytoskeleton

Cell cycle

Apoptosis

MAPK signaling pathway

Nucleus

Actin

Actin

Actin

Tight junction — CLDN, OCLN, JAM

Adherens junction — Nectin, Cadherin

Gap junction — Connexin

Desmosome — Dsg, Dsc

IF

BP180, ITGA6, ITGB4

ITGA, ITGB — ECM-receptor interaction

ECM

Hemidesmosome

Focal adhesion

CELL JUNCTION

NEURODEGENERATIVE DISORDERS

# FIG. 3LL

MAPK signalling pathway

Ubiquitin mediated proteolysis

| PARK2 | SNCA | UCHL1 |
| PARK4 | PARK7 | PINK1 |

Parkinson's Disease    O NAC

Focal dhesion

Apoptosis

Insulin signaling pathway

FBXW7

MAPT

APP    Aβ42 O    Aβ40 O

APOE    PSEN

Alzheimer's Disease

APBA1

CASP1

BCL2L1

CASP7

GAPD

CASP3    BAX

BAD

NEFH

ALS2    SOD1

ALS

NGFR    APLP1    GFAP    HSPA5

BCL2

GRB2

CASP6

PrPᶜ    Prion Disease

CASP8

Jak-STAT signaling pathway

Glycolysis/ Gluconeogenesis

DRPLA    DRPLA

CREBBF

HD    Huntington's Disease

Cytokine-cytokine receptor interaction

Gap junction

Toll-like receptor signaling pathway

Notch signaling pathway

Wnt signaling pathway

TGF-beta signaling pathway

Calcium signaling pathway

Dorso-ventral axis formation

Adherens junction

EP 2 177 908 A2

FIG. 3MM

CELL CYCLE

ECM-RECEPTOR INTERACTION  FIG. 3NN

# FIG. 3NN(contd.)

FIG. 300

# FIG. 3PP

LONG-TERM DEPRESSION

FIG. 3PP(contd.)

# FIG. 3QQ

Huntingdon's Disease

EP 2 177 908 A2

# FIG. 3RR

## EPITHELIAL CELL SIGNALING IN HELICOBACTER PYLORI INFECTION

EP 2 177 908 A2

# FIG. 3RR(contd.)

# FIG. 4A

<u>hsa00251 Glutamate metabolism</u> 2572 GAD2; glutamate decarboxylase 2 (pancreatic islets and brain, 65kDa) [EC:4.1.1.15] [SP:DCE2_HUMAN] 2875 GPT; glutamic-pyruvate transaminase (alanine aminotransferase) [EC:2.6.1.2] [SP:ALAT1_HUMAN]

<u>hsa00252 Alanine and aspartate metabolism</u> 2572 GAD2; glutamate decarboxylase 2 (pancreatic islets and brain, 65kDa) [EC:4.1.1.15] [SP:DCE2_HUMAN] 2875 GPT; glutamic-pyruvate transaminase (alanine aminotransferase) [EC:2.6.1.2] [SP:ALAT1_HUMAN]

<u>hsa00430 Taurine and hypotaurine metabolism</u> 2572 GAD2; glutamate decarboxylase 2 (pancreatic islets and brain, 65kDa) [EC:4.1.1.15] [SP:DCE2_HUMAN] 2678 GGT1; gamma-glutamyltransferase 1 [EC:2.3.2.2]

<u>hsa00500 Starch and sucrose metabolism</u> 2645 GCK; glucokinase (hexokinase 4, maturity onset diabetes of the young 2) [EC:2.7.1.2] [SP:HXK4_HUMAN] 5167 ENPP1; ectonucleotide pyrophosphatase/phosphodiesterase 1 [EC:3.1.4.1 3.6.1.9] [SP:ENPP1_HUMAN]

<u>hsa00770 Pantothenate and CoA biosynthesis</u> 23411 SIRT1; sirtuin (silent mating type information regulation 2 homolog) 1 (S. cerevisiae) [EC:3.5.1.-] 5167 ENPP1; ectonucleotide pyrophosphatase/phosphodiesterase 1 [EC:3.1.4.1 3.6.1.9] [SP:ENPP1_HUMAN]

# FIG. 4B

**hsa02010 ABC transporters - General** 10060 ABCC9; ATP-binding cassette, sub-family C (CFTR/MRP), member 9 6833 ABCC8; ATP-binding cassette, sub-family C (CFTR/MRP), member 8 [SP:ABCC8_HUMAN]

**hsa04140 Regulation of autophagy** 3458 IFNG; interferon, gamma [SP:IFNG_HUMAN] 3630 INS; insulin [SP:INS_HUMAN]

**hsa04330 Notch signaling pathway** 1387 CREBBP, RSTS; CREB binding protein (Rubinstein-Taybi syndrome) [EC:2.3.1.48] [SP:CBP_HUMAN] 2033 EP300; E1A binding protein p300 [EC:2.3.1.48] [SP:EP300_HUMAN]

**hsa04540 Gap junction** 1950 EGF; epidermal growth factor (beta-urogastrone) [SP:EGF_HUMAN] 5566 PRKACA; protein kinase, cAMP-dependent, catalytic, alpha [EC:2.7.11.11] [SP:KAPCA_HUMAN]

**hsa04742 Taste transduction** 2784 GNB3; guanine nucleotide binding protein (G protein), beta polypeptide 3 [SP:GBB3_HUMAN] 5566 PRKACA; protein kinase, cAMP-dependent, catalytic, alpha [EC:2.7.11.11] [SP:KAPCA_HUMAN]

**hsa05010 Alzheimer's disease** 348 APOE; apolipoprotein E [SP:APOE_HUMAN] 4023 LPL; lipoprotein lipase [EC:3.1.1.34] [SP:LIPL_HUMAN]

**hsa05050 Dentatorubropallidoluysian atrophy (DRPLA)** 3630 INS; insulin [SP:INS_HUMAN] 3643 INSR; insulin receptor [EC:2.7.10.1] [SP:INSR_HUMAN]

# FIG. 4C

<u>hsa00010 Glycolysis / Gluconeogenesis</u> 2645 GCK; glucokinase (hexokinase 4, maturity onset diabetes of the young 2) [EC:2.7.1.2] [SP:HXK4_HUMAN]

<u>hsa00020 Citrate cycle (TCA cycle)</u> 5105 PCK1; phosphoenolpyruvate carboxykinase 1 (soluble) [EC:4.1.1.32] [SP:PPCKC_HUMAN]

<u>hsa00052 Galactose metabolism</u> 2645 GCK; glucokinase (hexokinase 4, maturity onset diabetes of the young 2) [EC:2.7.1.2] [SP:HXK4_HUMAN]

<u>hsa00071 Fatty acid metabolism</u> 1374 CPT1A; carnitine palmitoyltransferase 1A (liver) [EC:2.3.1.21] [SP:CPT1A_HUMAN]

<u>hsa00140 C21-Steroid hormone metabolism</u> 3290 HSD11B1, HSD11B, HSD11; hydroxysteroid (11-beta) dehydrogenase 1 [EC:1.1.1.146] [SP:DHI1_HUMAN]

<u>hsa00150 Androgen and estrogen metabolism</u> 3290 HSD11B1, HSD11B, HSD11; hydroxysteroid (11-beta) dehydrogenase 1 [EC:1.1.1.146] [SP:DHI1_HUMAN]

<u>hsa00230 Purine metabolism</u> 5167 ENPP1; ectonucleotide pyrophosphatase/phosphodiesterase 1 [EC:3.1.4.1 3.6.1.9] [SP:ENPP1_HUMAN]

<u>hsa00240 Pyrimidine metabolism</u> 10587 TXNRD2; thioredoxin reductase 2 [EC:1.8.1.9]

<u>hsa00330 Arginine and proline metabolism</u> 4843 NOS2A, NOS2; nitric oxide synthase 2A (inducible, hepatocytes) [EC:1.14.13.39] [SP:NOS2A_HUMAN]

# FIG. 4D

<u>hsa00361 gamma-Hexachlorocyclohexane degradation</u> 5444 PON1, PON; paraoxonase 1 [EC:3.1.1.2 3.1.8.1] [SP:PON1_HUMAN]

<u>hsa00363 Bisphenol A degradation</u> 5444 PON1, PON; paraoxonase 1 [EC:3.1.1.2 3.1.8.1] [SP:PON1_HUMAN]

<u>hsa00380 Tryptophan metabolism</u> 121278 TPH2; tryptophan hydroxylase 2 [EC:1.14.16.4]

<u>hsa00410 beta-Alanine metabolism</u> 2572 GAD2; glutamate decarboxylase 2 (pancreatic islets and brain, 65kDa) [EC:4.1.1.15] [SP:DCE2_HUMAN]

<u>hsa00450 Selenoamino acid metabolism</u> 2678 GGT1; gamma-glutamyltransferase 1 [EC:2.3.2.2]

<u>hsa00460 Cyanoamino acid metabolism</u> 2678 GGT1; gamma-glutamyltransferase 1 [EC:2.3.2.2]

<u>hsa00480 Glutathione metabolism</u> 2678 GGT1; gamma-glutamyltransferase 1 [EC:2.3.2.2]

<u>hsa00521 Streptomycin biosynthesis</u> 2645 GCK; glucokinase (hexokinase 4, maturity onset diabetes of the young 2) [EC:2.7.1.2] [SP:HXK4_HUMAN]

<u>hsa00561 Glycerolipid metabolism</u> 4023 LPL; lipoprotein lipase [EC:3.1.1.34] [SP:LIPL_HUMAN]

EP 2 177 908 A2

# FIG. 4E

<u>hsa00564 Glycerophospholipid metabolism</u> 26279 PLA2G2D; phospholipase A2, group IID [EC:3.1.1.4] [SP:PA2GD_HUMAN]

<u>hsa00565 Ether lipid metabolism</u> 26279 PLA2G2D; phospholipase A2, group IID [EC:3.1.1.4] [SP:PA2GD_HUMAN]

<u>hsa00591 Linoleic acid metabolism</u> 26279 PLA2G2D; phospholipase A2, group IID [EC:3.1.1.4] [SP:PA2GD_HUMAN]

<u>hsa00620 Pyruvate metabolism</u> 5105 PCK1; phosphoenolpyruvate carboxykinase 1 (soluble) [EC:4.1.1.32] [SP:PPCKC_HUMAN]

<u>hsa00650 Butanoate metabolism</u> 2572 GAD2; glutamate decarboxylase 2 (pancreatic islets and brain, 65kDa) [EC:4.1.1.15] [SP:DCE2_HUMAN]

<u>hsa00710 Carbon fixation</u> 2875 GPT; glutamic-pyruvate transaminase (alanine aminotransferase) [EC:2.6.1.2] [SP:ALAT1_HUMAN]

<u>hsa00740 Riboflavin metabolism</u> 5167 ENPP1; ectonucleotide pyrophosphatase/phosphodiesterase 1 [EC:3.1.4.1 3.6.1.9] [SP:ENPP1_HUMAN]

<u>hsa00860 Porphyrin and chlorophyll metabolism</u> 2495 FTH1; ferritin, heavy polypeptide 1 [EC:1.16.3.1]

<u>hsa00930 Caprolactam degradation</u> 23411 SIRT1; sirtuin (silent mating type information regulation 2 homolog) 1 (S. cerevisiae) [EC:3.5.1.-]

EP 2 177 908 A2

# FIG. 4F

**hsa04070 Phosphatidylinositol signaling system** 5295 PIK3R1;
phosphoinositide-3-kinase, regulatory subunit 1 (p85 alpha)
**hsa04340 Hedgehog signaling pathway** 5566 PRKACA; protein kinase,
cAMP-dependent, catalytic, alpha [EC:2.7.11.11] [SP:KAPCA_HUMAN]
**hsa04360 Axon guidance** 1020 CDK5; cyclin-dependent kinase 5
[EC:2.7.11.22] [SP:CDK5_HUMAN]
**hsa04740 Olfactory transduction** 5566 PRKACA; protein kinase, cAMP-
dependent, catalytic, alpha [EC:2.7.11.11] [SP:KAPCA_HUMAN]
**hsa05130 Pathogenic Escherichia coli infection - EHEC** 929 CD14; CD14
molecule [SP:CD14_HUMAN]
**hsa05131 Pathogenic Escherichia coli infection - EPEC** 929 CD14; CD14
molecule [SP:CD14_HUMAN]

# FIG. 5A

**Adiponectin**
SUMMARY

| Groups | Count | Sum | Average | Variance |
|---|---|---|---|---|
| Low Risk | 73 | 6542666.54 | 89625.569 | 6521761235 |
| High Risk | 10 | 808925.86 | 80892.586 | 10528866958 |
| T2DM | 12 | 528480.614 | 44040.051 | 513491655.7 |

ANOVA

| Source of Variation | SS | df | MS | F | P-value | F crit |
|---|---|---|---|---|---|---|
| Between Groups | 21463253871 | 2 | 10731626935 | 1.732198 | 0.182614 | 3.095433 |
| Within Groups | 5.69975E+11 | 92 | 6195380649 | | | |
| Total | 5.91438E+11 | 94 | | | | |

**MMP 2**
SUMMARY

| Groups | Count | Sum | Average | Variance |
|---|---|---|---|---|
| Low Risk | 73 | 28598.4005 | 391.75891 | 1058604.025 |
| High Risk | 10 | 930.088315 | 93.008832 | 5643.293837 |
| T2DM | 12 | 984.908141 | 82.075678 | 6981.502424 |

ANOVA

| Source of Variation | SS | df | MS | F | P-value | F crit |
|---|---|---|---|---|---|---|
| Between Groups | 1570312.26 | 2 | 785156.13 | 0.946131 | 0.391985 | 3.095433 |
| Within Groups | 76347075.99 | 92 | 829859.5216 | | | |
| Total | 77917388.25 | 94 | | | | |

**Adipsin**
SUMMARY

| Groups | Count | Sum | Average | Variance |
|---|---|---|---|---|
| Low Risk | 72 | 36624.8138 | 508.67797 | 9877966.613 |
| High Risk | 10 | 2468.33812 | 246.83381 | 99229.04139 |
| T2DM | 9 | 1232.27003 | 136.91889 | 45182.59514 |

ANOVA

| Source of Variation | SS | df | MS | F | P-value | F crit |
|---|---|---|---|---|---|---|
| Between Groups | 1538559.726 | 2 | 769279.8632 | 0.096353 | 0.908239 | 3.100069 |
| Within Groups | 702590151.7 | 88 | 7983978.996 | | | |
| Total | 704128711.4 | 90 | | | | |

**CD 14**
SUMMARY

| Groups | Count | Sum | Average | Variance |
|---|---|---|---|---|
| Low Risk | 71 | 32496.6878 | 457.69983 | 9473555.525 |
| High Risk | 10 | 1205.19913 | 120.51991 | 11567.35236 |
| T2DM | 12 | 1027.7561 | 85.646342 | 966.8058249 |

ANOVA

| Source of Variation | SS | df | MS | F | P-value | F crit |
|---|---|---|---|---|---|---|
| Between Groups | 2137668.004 | 2 | 1068834.002 | 0.145033 | 0.865196 | 3.097698 |
| Within Groups | 663263627.8 | 90 | 7369595.865 | | | |
| Total | 665401295.8 | 92 | | | | |

EP 2 177 908 A2

# FIG. 5A(contd.)

## C Peptide
### SUMMARY

| Groups | Count | Sum | Average | Variance |
|---|---|---|---|---|
| Low Risk | 58 | 63065928.2 | 1087343.6 | 4.64372E+11 |
| High Risk | 8 | 6201364.14 | 775170.52 | 1.90543E+11 |
| T2DM | 7 | 5587223.32 | 798174.76 | 1.78902E+11 |

### ANOVA

| Source of Variation | SS | df | MS | F | P-value | F crit |
|---|---|---|---|---|---|---|
| Between Groups | 1.08488E+12 | 2 | 5.42442E+11 | 1.314947 | 0.275038 | 3.127676 |
| Within Groups | 2.88764E+13 | 70 | 4.1252E+11 | | | |
| Total | 2.99613E+13 | 72 | | | | |

## E-Selectin
### SUMMARY

| Groups | Count | Sum | Average | Variance |
|---|---|---|---|---|
| Low Risk | 73 | 56.8985524 | 0.7794322 | 3.182492254 |
| High Risk | 10 | 5.7662001 | 0.57662 | 0.064659411 |
| T2DM | 12 | 6.78616557 | 0.5655138 | 0.05894195 |

### ANOVA

| Source of Variation | SS | df | MS | F | P-value | F crit |
|---|---|---|---|---|---|---|
| Between Groups | 0.738194311 | 2 | 0.369097155 | 0.147402 | 0.86315 | 3.095433 |
| Within Groups | 230.3697385 | 92 | 2.504018896 | | | |
| Total | 231.1079328 | 94 | | | | |

## Thrombospondin
### SUMMARY

| Groups | Count | Sum | Average | Variance |
|---|---|---|---|---|
| Low Risk | 73 | 9681.11926 | 132.61807 | 5619.722029 |
| High Risk | 10 | 1205.24015 | 120.52401 | 1413.928895 |
| T2DM | 12 | 1647.19071 | 137.26589 | 4124.689979 |

### ANOVA

| Source of Variation | SS | df | MS | F | P-value | F crit |
|---|---|---|---|---|---|---|
| Between Groups | 1677.186905 | 2 | 838.5934525 | 0.166734 | 0.84668 | 3.095433 |
| Within Groups | 462716.9359 | 92 | 5029.531912 | | | |
| Total | 464394.1228 | 94 | | | | |

## CDK5
### SUMMARY

| Groups | Count | Sum | Average | Variance |
|---|---|---|---|---|
| Low Risk | 73 | 42411.5377 | 580.97997 | 248286.9324 |
| High Risk | 10 | 6206.08126 | 620.60813 | 422404.9224 |
| T2DM | 12 | 4783.62908 | 398.63576 | 30991.42176 |

### ANOVA

| Source of Variation | SS | df | MS | F | P-value | F crit |
|---|---|---|---|---|---|---|
| Between Groups | 380899.7678 | 2 | 190449.8839 | 0.795732 | 0.454332 | 3.095433 |
| Within Groups | 22019209.08 | 92 | 239339.2291 | | | |
| Total | 22400108.84 | 94 | | | | |

EP 2 177 908 A2

**TGF beta**

**SUMMARY**

| Groups | Count | Sum | Average | Variance |
|---|---|---|---|---|
| Low Risk | 54 | 31.240978 | 0.5785366 | 0.281548009 |
| High Risk | 5 | 5.84154426 | 1.1683089 | 2.115745108 |
| T2DM | 7 | 6.21317849 | 0.8875969 | 1.36586353 |

**ANOVA**

| Source of Variation | SS | df | MS | F | P-value | F crit |
|---|---|---|---|---|---|---|
| Between Groups | 2.011792731 | 2 | 1.005896366 | 2.006683 | 0.142936 | 3.142809 |
| Within Groups | 31.58020607 | 63 | 0.501273112 | | | |
| Total | 33.5919988 | 65 | | | | |

**VEG-F**

**SUMMARY**

| Groups | Count | Sum | Average | Variance |
|---|---|---|---|---|
| Low Risk | 70 | 6.15025145 | 0.0878607 | 0.051856478 |
| High Risk | 9 | 0.62214069 | 0.0691267 | 0.004815677 |
| T2DM | 12 | 0.52242148 | 0.0435351 | 0.004924717 |

**ANOVA**

| Source of Variation | SS | df | MS | F | P-value | F crit |
|---|---|---|---|---|---|---|
| Between Groups | 0.021343265 | 2 | 0.010671633 | 0.255831 | 0.774847 | 3.100069 |
| Within Groups | 3.67079425 | 88 | 0.041713571 | | | |
| Total | 3.692137515 | 90 | | | | |

**CD 28**

**SUMMARY**

| Groups | Count | Sum | Average | Variance |
|---|---|---|---|---|
| Low Risk | 73 | 443.676898 | 6.0777657 | 36.32761052 |
| High Risk | 10 | 51.6528675 | 5.1652867 | 11.77981101 |
| T2DM | 12 | 48.7664733 | 4.0638728 | 8.067917962 |

**ANOVA**

| Source of Variation | SS | df | MS | F | P-value | F crit |
|---|---|---|---|---|---|---|
| Between Groups | 45.32878467 | 2 | 22.66439234 | 0.741944 | 0.479015 | 3.095433 |
| Within Groups | 2810.353354 | 92 | 30.54731906 | | | |
| Total | 2855.682138 | 94 | | | | |

# FIG. 5A(contd.)

EP 2 177 908 A2

# FIG. 5B

**CD40 Ligand**
**SUMMARY**

| Groups | Count | Sum | Average | Variance |
|---|---|---|---|---|
| Low Risk | 68 | 50.5522749 | 0.7434158 | 2.353579305 |
| High Risk | 9 | 8.68173461 | 0.9646372 | 1.499309524 |
| T2DM | 12 | 3.13483416 | 0.2612362 | 0.014717775 |

**ANOVA**

| Source of Variation | SS | df | MS | F | P-value | F crit |
|---|---|---|---|---|---|---|
| Between Groups | 3.068581949 | 2 | 1.534290975 | 0.776874 | 0.463041 | 3.102552 |
| Within Groups | 169.8461851 | 86 | 1.974955641 | | | |
| Total | 172.9147671 | 88 | | | | |

**ICAM**
**SUMMARY**

| Groups | Count | Sum | Average | Variance |
|---|---|---|---|---|
| Low Risk | 73 | 557951.274 | 7643.1681 | 139217124.2 |
| High Risk | 10 | 52695.3846 | 5269.5385 | 5271426.29 |
| T2DM | 12 | 150355.923 | 12529.66 | 122402684.8 |

**ANOVA**

| Source of Variation | SS | df | MS | F | P-value | F crit |
|---|---|---|---|---|---|---|
| Between Groups | 330052496.2 | 2 | 165026248.1 | 1.329749 | 0.26958 | 3.095433 |
| Within Groups | 11417505309 | 92 | 124103318.6 | | | |
| Total | 11747557805 | 94 | | | | |

**MCP-1**
**SUMMARY**

| Groups | Count | Sum | Average | Variance |
|---|---|---|---|---|
| Low Risk | 73 | 12.7041882 | 0.17403 | 0.088862761 |
| High Risk | 10 | 1.92984693 | 0.1929847 | 0.023707361 |
| T2DM | 12 | 1.50720988 | 0.1256008 | 0.005167069 |

**ANOVA**

| Source of Variation | SS | df | MS | F | P-value | F crit |
|---|---|---|---|---|---|---|
| Between Groups | 0.030123168 | 2 | 0.015061584 | 0.207798 | 0.812751 | 3.095433 |
| Within Groups | 6.668322797 | 92 | 0.07248177 | | | |
| Total | 6.698445965 | 94 | | | | |

**TNFR1**
**SUMMARY**

| Groups | Count | Sum | Average | Variance |
|---|---|---|---|---|
| Low Risk | 73 | 30.7521704 | 0.4212626 | 0.030240623 |
| High Risk | 10 | 4.69888064 | 0.4698881 | 0.058352765 |
| T2DM | 12 | 5.6748668 | 0.4729056 | 0.032668912 |

**ANOVA**

| Source of Variation | SS | df | MS | F | P-value | F crit |
|---|---|---|---|---|---|---|
| Between Groups | 0.042772821 | 2 | 0.02138641 | 0.6426 | 0.528267 | 3.095433 |
| Within Groups | 3.061857743 | 92 | 0.033281062 | | | |
| Total | 3.104630563 | 94 | | | | |

# FIG. 5B(contd.)

**TNF-alpha**
**SUMMARY**

| Groups | Count | Sum | Average | Variance |
|---|---|---|---|---|
| Low Risk | 54 | 10.6547721 | 0.1973106 | 0.31546964 |
| High Risk | 8 | 0.2739026 | 0.0342378 | 0.004745004 |
| T2DM | 9 | 0.03629256 | 0.0040325 | 9.35754E-06 |

**ANOVA**

| Source of Variation | SS | df | MS | F | P-value | F crit |
|---|---|---|---|---|---|---|
| Between Groups | 0.418436171 | 2 | 0.209218085 | 0.849202 | 0.432242 | 3.131672 |
| Within Groups | 16.75318082 | 68 | 0.246370306 | | | |
| Total | 17.17161699 | 70 | | | | |

**CRP**
**SUMMARY**

| Groups | Count | Sum | Average | Variance |
|---|---|---|---|---|
| Low Risk | 73 | 4618.08479 | 63.261435 | 69382.94377 |
| High Risk | 10 | 212.833453 | 21.283345 | 985.1046514 |
| T2DM | 12 | 188.815571 | 15.734631 | 656.4205698 |

**ANOVA**

| Source of Variation | SS | df | MS | F | P-value | F crit |
|---|---|---|---|---|---|---|
| Between Groups | 34408.18655 | 2 | 17204.09327 | 0.315819 | 0.729979 | 3.095433 |
| Within Groups | 5011658.52 | 92 | 54474.54913 | | | |
| Total | 5046066.706 | 94 | | | | |

**VWF**
**SUMMARY**

| Groups | Count | Sum | Average | Variance |
|---|---|---|---|---|
| Low Risk | 73 | 169146.408 | 2317.0741 | 798967.558 |
| High Risk | 10 | 23139.815 | 2313.9815 | 1352809.317 |
| T2DM | 12 | 27707.8274 | 2308.9856 | 555249.1815 |

**ANOVA**

| Source of Variation | SS | df | MS | F | P-value | F crit |
|---|---|---|---|---|---|---|
| Between Groups | 708.2906181 | 2 | 354.145309 | 0.00043 | 0.99957 | 3.095433 |
| Within Groups | 75808689.03 | 92 | 824007.4895 | | | |
| Total | 75809397.32 | 94 | | | | |

**Fetuin A**
**SUMMARY**

| Groups | Count | Sum | Average | Variance |
|---|---|---|---|---|
| Low Risk | 73 | 75065722.5 | 1028297.6 | 9.41045E+11 |
| High Risk | 10 | 6690876.12 | 669087.61 | 7.06018E+11 |
| T2DM | 12 | 12709967.4 | 1059163.9 | 7.41439E+11 |

**ANOVA**

| Source of Variation | SS | df | MS | F | P-value | F crit |
|---|---|---|---|---|---|---|
| Between Groups | 1.19249E+12 | 2 | 5.96247E+11 | 0.666803 | 0.51581 | 3.095433 |
| Within Groups | 8.22652E+13 | 92 | 8.94187E+11 | | | |
| Total | 8.34577E+13 | 94 | | | | |

EP 2 177 908 A2

**IL6 sol R**
SUMMARY

| Groups | Count | Sum | Average | Variance |
|---|---|---|---|---|
| Low Risk | 73 | 68.3474016 | 0.9362658 | 0.782554993 |
| High Risk | 10 | 7.56039142 | 0.7560391 | 0.440052771 |
| T2DM | 12 | 5.14767605 | 0.428973 | 0.091506452 |

ANOVA

| Source of Variation | SS | df | MS | F | P-value | F crit |
|---|---|---|---|---|---|---|
| Between Groups | 2.75771922 | 2 | 1.37885961 | 2.069043 | 0.132144 | 3.095433 |
| Within Groups | 61.3110054 | 92 | 0.666423972 | | | |
| Total | 64.06872462 | 94 | | | | |

**Comp C3**
SUMMARY

| Groups | Count | Sum | Average | Variance |
|---|---|---|---|---|
| Low Risk | 73 | 712273652 | 9757173.3 | 2.89652E+14 |
| High Risk | 10 | 67046057.2 | 6704605.7 | 6.7179E+13 |
| T2DM | 12 | 64953460.3 | 5412788.4 | 4.23251E+13 |

ANOVA

| Source of Variation | SS | df | MS | F | P-value | F crit |
|---|---|---|---|---|---|---|
| Between Groups | 2.47746E+14 | 2 | 1.23873E+14 | 0.519782 | 0.596386 | 3.095433 |
| Within Groups | 2.19252E+16 | 92 | 2.38317E+14 | | | |
| Total | 2.21729E+16 | 94 | | | | |

**MCSF**
SUMMARY

| Groups | Count | Sum | Average | Variance |
|---|---|---|---|---|
| Low Risk | 70 | 4.63562262 | 0.0662232 | 0.000943864 |
| High Risk | 9 | 0.57422226 | 0.0638025 | 0.000221964 |
| T2DM | 11 | 0.65919923 | 0.0599272 | 0.000142492 |

ANOVA

| Source of Variation | SS | df | MS | F | P-value | F crit |
|---|---|---|---|---|---|---|
| Between Groups | 0.000396675 | 2 | 0.000198337 | 0.25254 | 0.777393 | 3.101296 |
| Within Groups | 0.068327225 | 87 | 0.00078537 | | | |
| Total | 0.0687239 | 89 | | | | |

**EGF**
SUMMARY

ANOVA

# FIG. 5B(contd.)

EP 2 177 908 A2

# FIG. 5C

| Groups | Count | Sum | Average | Variance |
|---|---|---|---|---|
| Low Risk | 72 | 12.6940361 | 0.1763061 | 0.023769094 |
| High Risk | 10 | 1.67682459 | 0.1676825 | 0.020557532 |
| T2DM | 11 | 1.98168696 | 0.1801534 | 0.033131163 |

| Source of Variation | SS | df | MS | F | P-value | F crit |
|---|---|---|---|---|---|---|
| Between Groups | 0.000885746 | 2 | 0.000442873 | 0.018085 | 0.982081 | 3.097698 |
| Within Groups | 2.20393512 | 90 | 0.024488168 | | | |
| Total | 2.204820866 | 92 | | | | |

Betacellulin
SUMMARY

| Groups | Count | Sum | Average | Variance |
|---|---|---|---|---|
| Low Risk | 10 | 0.50122079 | 0.0501221 | 0.014374195 |
| High Risk | 3 | 0.09379773 | 0.0312659 | 0.002264183 |
| T2DM | 3 | 0.02187234 | 0.0072908 | 1.0489E-05 |

ANOVA

| Source of Variation | SS | df | MS | F | P-value | F crit |
|---|---|---|---|---|---|---|
| Between Groups | 0.00442972 | 2 | 0.00221486 | 0.215008 | 0.809347 | 3.805565 |
| Within Groups | 0.133917095 | 13 | 0.010301315 | | | |
| Total | 0.138346815 | 15 | | | | |

FAS
SUMMARY

| Groups | Count | Sum | Average | Variance |
|---|---|---|---|---|
| Low Risk | 72 | 99.8700145 | 1.3870835 | 0.08979971 |
| High Risk | 10 | 14.8615121 | 1.4861512 | 0.275535159 |
| T2DM | 11 | 17.2362316 | 1.5669301 | 0.076675824 |

ANOVA

| Source of Variation | SS | df | MS | F | P-value | F crit |
|---|---|---|---|---|---|---|
| Between Groups | 0.35915304 | 2 | 0.17957652 | 1.679619 | 0.192236 | 3.097698 |
| Within Groups | 9.622354069 | 90 | 0.106915045 | | | |
| Total | 9.981507109 | 92 | | | | |

RAGE
SUMMARY

| Groups | Count | Sum | Average | Variance |
|---|---|---|---|---|
| Low Risk | 72 | 0.89968022 | 0.0124956 | 5.72843E-05 |
| High Risk | 10 | 0.14755707 | 0.0147557 | 6.68198E-05 |
| T2DM | 11 | 0.13777404 | 0.0125249 | 0.000105594 |

ANOVA

| Source of Variation | SS | df | MS | F | P-value | F crit |
|---|---|---|---|---|---|---|
| Between Groups | 4.54414E-05 | 2 | 2.27207E-05 | 0.357212 | 0.700611 | 3.097698 |
| Within Groups | 0.005724501 | 90 | 6.36056E-05 | | | |
| Total | 0.005769942 | 92 | | | | |

EP 2 177 908 A2

# FIG. 5C(contd.)

LEPTIN
SUMMARY

| Groups | Count | Sum | Average | Variance |
|---|---|---|---|---|
| Low Risk | 72 | 331.357709 | 4.6021904 | 68.32520979 |
| High Risk | 10 | 86.9143631 | 8.6914363 | 102.0044553 |
| T2DM | 11 | 73.5949725 | 6.690452 | 44.13606452 |

ANOVA

| Source of Variation | SS | df | MS | F | P-value | F crit |
|---|---|---|---|---|---|---|
| Between Groups | 171.3334078 | 2 | 85.66670388 | 1.241448 | 0.293866 | 3.097698 |
| Within Groups | 6210.490638 | 90 | 69.00545153 | | | |
| Total | 6381.824046 | 92 | | | | |

AgRP
SUMMARY

| Groups | Count | Sum | Average | Variance |
|---|---|---|---|---|
| Low Risk | 62 | 1.21525255 | 0.0196008 | 0.000705036 |
| High Risk | 8 | 0.07956911 | 0.0099461 | 2.89373E-05 |
| T2DM | 10 | 0.1045982 | 0.0104598 | 8.98104E-05 |

ANOVA

| Source of Variation | SS | df | MS | F | P-value | F crit |
|---|---|---|---|---|---|---|
| Between Groups | 0.001225764 | 2 | 0.000612882 | 1.072104 | 0.347341 | 3.115366 |
| Within Groups | 0.044018039 | 77 | 0.000571663 | | | |
| Total | 0.045243803 | 79 | | | | |

ILGFBP3
SUMMARY

| Groups | Count | Sum | Average | Variance |
|---|---|---|---|---|
| Low Risk | 72 | 1358.60915 | 18.869571 | 22.88738922 |
| High Risk | 10 | 182.890594 | 18.289059 | 29.12214859 |
| T2DM | 11 | 172.725089 | 15.702281 | 6.586759577 |

ANOVA

| Source of Variation | SS | df | MS | F | P-value | F crit |
|---|---|---|---|---|---|---|
| Between Groups | 95.95508023 | 2 | 47.97754012 | 2.210979 | 0.115512 | 3.097698 |
| Within Groups | 1952.971568 | 90 | 21.69968408 | | | |
| Total | 2048.926648 | 92 | | | | |

Resistin
SUMMARY

| Groups | Count | Sum | Average | Variance |
|---|---|---|---|---|
| Low Risk | 72 | 1431.43619 | 19.881058 | 91.34763269 |
| High Risk | 10 | 194.100243 | 19.410024 | 102.8132046 |
| T2DM | 11 | 232.583922 | 21.143993 | 99.42808118 |

ANOVA

| Source of Variation | SS | df | MS | F | P-value | F crit |
|---|---|---|---|---|---|---|
| Between Groups | 18.85723729 | 2 | 9.428618644 | 0.100957 | 0.904074 | 3.097698 |
| Within Groups | 8405.281574 | 90 | 93.39201749 | | | |
| Total | 8424.138812 | 92 | | | | |

**IL1RA**
**SUMMARY**

| Groups | Count | Sum | Average | Variance |
|---|---|---|---|---|
| Low Risk | 63 | 33.843918 | 0.537205 | 3.715229918 |
| High Risk | 10 | 2.01804762 | 0.2018048 | 0.010442467 |
| T2DM | 11 | 2.22946169 | 0.2026783 | 0.01217606 |

**ANOVA**

| Source of Variation | SS | df | MS | F | P-value | F crit |
|---|---|---|---|---|---|---|
| Between Groups | 1.766943134 | 2 | 0.883471567 | 0.31038 | 0.734036 | 3.109311 |
| Within Groups | 230.5599977 | 81 | 2.846419725 | | | |
| Total | 232.3269408 | 83 | | | | |

**IL6**
**SUMMARY**

| Groups | Count | Sum | Average | Variance |
|---|---|---|---|---|
| Low Risk | 72 | 0.1085509 | 0.0015077 | 6.87579E-06 |
| High Risk | 10 | 0.02705613 | 0.0027056 | 8.4704E-06 |
| T2DM | 11 | 0.02321809 | 0.0021107 | 3.22869E-06 |

**ANOVA**

| Source of Variation | SS | df | MS | F | P-value | F crit |
|---|---|---|---|---|---|---|
| Between Groups | 1.46265E-05 | 2 | 7.31326E-06 | 1.103052 | 0.336301 | 3.097698 |
| Within Groups | 0.000596702 | 90 | 6.63002E-06 | | | |
| Total | 0.000611328 | 92 | | | | |

**IL1b**
**SUMMARY**

| Groups | Count | Sum | Average | Variance |
|---|---|---|---|---|
| Low Risk | 68 | 728.586007 | 10.7145 | 70.82159067 |
| High Risk | 10 | 129.897417 | 12.989742 | 95.87878545 |
| T2DM | 10 | 106.369486 | 10.636949 | 15.47093721 |

**ANOVA**

| Source of Variation | SS | df | MS | F | P-value | F crit |
|---|---|---|---|---|---|---|
| Between Groups | 46.33892841 | 2 | 23.16946421 | 0.342672 | 0.710847 | 3.103839 |
| Within Groups | 5747.194079 | 85 | 67.61404798 | | | |
| Total | 5793.533007 | 87 | | | | |

**CD40 Soluble**
**SUMMARY**

| Groups | Count | Sum | Average | Variance |
|---|---|---|---|---|
| Low Risk | 57 | 0.55702437 | 0.0097724 | 0.000721443 |

**ANOVA**

| Source of Variation | SS | df | MS | F | P-value | F crit |
|---|---|---|---|---|---|---|
| Between Groups | 0.000756618 | 2 | 0.000378309 | 0.671542 | 0.514091 | 3.123907 |

## FIG. 5C(contd.)

EP 2 177 908 A2

# FIG. 5D

| | | | | | |
|---|---|---|---|---|---|
| High Risk | 9 | 0.02809156 | 0.0031213 | 1.6634E-05 | |
| T2DM | 9 | 0.01481475 | 0.0016461 | 3.36272E-06 | |

**Within Groups**  0.040560788  72  0.000563344

**Total**  0.041317406  74

## AKT1
### SUMMARY

| Groups | Count | Sum | Average | Variance |
|---|---|---|---|---|
| Low Risk | 33 | 18.3035467 | 0.5546529 | 4.385196821 |
| High Risk | 5 | 0.96689016 | 0.193378 | 0.120495378 |
| T2DM | 3 | 0.05190269 | 0.0173009 | 7.1763E-05 |

### ANOVA

| Source of Variation | SS | df | MS | F | P-value | F crit |
|---|---|---|---|---|---|---|
| Between Groups | 1.233823097 | 2 | 0.616911548 | 0.166486 | 0.847249 | 3.244818 |
| Within Groups | 140.8084233 | 38 | 3.705484824 | | | |
| Total | 142.0422464 | 40 | | | | |

## IL-8
### SUMMARY

| Groups | Count | Sum | Average | Variance |
|---|---|---|---|---|
| Low Risk | 53 | 25.1476338 | 0.4744837 | 0.850037547 |
| High Risk | 9 | 7.20869006 | 0.8009656 | 5.197743533 |
| T2DM | 10 | 1.10225678 | 0.1102257 | 0.045298416 |

### ANOVA

| Source of Variation | SS | df | MS | F | P-value | F crit |
|---|---|---|---|---|---|---|
| Between Groups | 2.279264359 | 2 | 1.13963218 | 0.912324 | 0.406379 | 3.129644 |
| Within Groups | 86.19158645 | 69 | 1.249153427 | | | |
| Total | 88.47085081 | 71 | | | | |

## activin A
### SUMMARY

| Groups | Count | Sum | Average | Variance |
|---|---|---|---|---|
| Low Risk | 58 | 32.8673282 | 0.5666781 | 1.442015651 |
| High Risk | 7 | 0.85091747 | 0.1215596 | 0.004849452 |
| T2DM | 9 | 1.9729777 | 0.2192197 | 0.098888295 |

### ANOVA

| Source of Variation | SS | df | MS | F | P-value | F crit |
|---|---|---|---|---|---|---|
| Between Groups | 1.946776456 | 2 | 0.973388228 | 0.832506 | 0.439159 | 3.125764 |
| Within Groups | 83.01509521 | 71 | 1.169226693 | | | |
| Total | 84.96187166 | 73 | | | | |

# FIG. 5D(contd.)

ACE
SUMMARY

| Groups | Count | Sum | Average | Variance |
|---|---|---|---|---|
| Low Risk | 72 | 17876.869 | 248.28985 | 30281.09223 |
| High Risk | 10 | 2393.00582 | 239.30058 | 21894.09962 |
| T2DM | 11 | 3793.60834 | 344.87349 | 40837.6526 |

ANOVA

| Source of Variation | SS | df | MS | F | P-value | F crit |
|---|---|---|---|---|---|---|
| Between Groups | 93250.46471 | 2 | 46625.23236 | 1.522937 | 0.223636 | 3.097698 |
| Within Groups | 2755379.971 | 90 | 30615.33301 | | | |
| Total | 2848630.435 | 92 | | | | |

MMP-9
SUMMARY

| Groups | Count | Sum | Average | Variance |
|---|---|---|---|---|
| Low Risk | 72 | 24407821.8 | 338997.53 | 47692562604 |
| High Risk | 10 | 2657417.87 | 265741.79 | 48593207870 |
| T2DM | 11 | 4207584.34 | 382507.67 | 29134880725 |

ANOVA

| Source of Variation | SS | df | MS | F | P-value | F crit |
|---|---|---|---|---|---|---|
| Between Groups | 73795064391 | 2 | 36897532195 | 0.807021 | 0.449387 | 3.097698 |
| Within Groups | 4.11486E+12 | 90 | 45720662478 | | | |
| Total | 4.18865E+12 | 92 | | | | |

IL-10
SUMMARY

| Groups | Count | Sum | Average | Variance |
|---|---|---|---|---|
| Low Risk | 60 | 1.1944029 | 0.0199067 | 0.007257646 |
| High Risk | 9 | 0.04690808 | 0.005212 | 2.4724E-05 |
| T2DM | 11 | 0.03650612 | 0.0033187 | 3.68062E-06 |

ANOVA

| Source of Variation | SS | df | MS | F | P-value | F crit |
|---|---|---|---|---|---|---|
| Between Groups | 0.003732071 | 2 | 0.001866035 | 0.335371 | 0.716112 | 3.115366 |
| Within Groups | 0.428435705 | 77 | 0.0055641 | | | |
| Total | 0.432167776 | 79 | | | | |

VCAM
SUMMARY

| Groups | Count | Sum | Average | Variance |
|---|---|---|---|---|
| Low Risk | 72 | 57304.4461 | 795.89508 | 195153.4251 |
| High Risk | 10 | 7139.96228 | 713.99623 | 146428.2392 |
| T2DM | 11 | 9328.29215 | 848.02656 | 208209.0999 |

ANOVA

| Source of Variation | SS | df | MS | F | P-value | F crit |
|---|---|---|---|---|---|---|
| Between Groups | 96320.53327 | 2 | 48160.26664 | 0.251186 | 0.778421 | 3.097698 |
| Within Groups | 17255838.33 | 90 | 191731.537 | | | |
| Total | 17352158.87 | 92 | | | | |

**INF-gamma**
SUMMARY

| Groups | Count | Sum | Average | Variance |
|---|---|---|---|---|
| Low Risk | 72 | 1.98999756 | 0.0276389 | 5.18101E-05 |
| High Risk | 10 | 0.26501604 | 0.0265016 | 4.882E-05 |
| T2DM | 11 | 0.29715724 | 0.0270143 | 3.31149E-06 |

ANOVA

| Source of Variation | SS | df | MS | F | P-value | F crit |
|---|---|---|---|---|---|---|
| Between Groups | 1.36458E-05 | 2 | 6.82289E-06 | 0.14793 | 0.862701 | 3.097698 |
| Within Groups | 0.004151013 | 90 | 4.61224E-05 | | | |
| Total | 0.004164659 | 92 | | | | |

**HGF**
SUMMARY

| Groups | Count | Sum | Average | Variance |
|---|---|---|---|---|
| Low Risk | 72 | 21.4638351 | 0.2981088 | 0.387671716 |
| High Risk | 10 | 2.72221033 | 0.272221 | 0.020928655 |
| T2DM | 11 | 2.67441317 | 0.2431285 | 0.01191665 |

ANOVA

| Source of Variation | SS | df | MS | F | P-value | F crit |
|---|---|---|---|---|---|---|
| Between Groups | 0.03193245 | 2 | 0.015966225 | 0.051629 | 0.949709 | 3.097698 |
| Within Groups | 27.83221625 | 90 | 0.309246847 | | | |
| Total | 27.8641487 | 92 | | | | |

**FAS-Lg**
SUMMARY

| Groups | Count | Sum | Average | Variance |
|---|---|---|---|---|
| Low Risk | 72 | 12.7821734 | 0.1775302 | 0.003134941 |
| High Risk | 10 | 1.98261004 | 0.198261 | 0.001692512 |
| T2DM | 11 | 2.05300538 | 0.1866369 | 0.005045334 |

ANOVA

| Source of Variation | SS | df | MS | F | P-value | F crit |
|---|---|---|---|---|---|---|
| Between Groups | 0.004193302 | 2 | 0.002096651 | 0.654597 | 0.522108 | 3.097698 |
| Within Groups | 0.288266725 | 90 | 0.003202964 | | | |
| Total | 0.292460027 | 92 | | | | |

**NGF**
SUMMARY

| Groups | Count | Sum | Average | Variance |
|---|---|---|---|---|
| Low Risk | 72 | 259.590874 | 3.6054288 | 406.1253301 |
| High Risk | 10 | 43.9273288 | 4.3927329 | 59.03394385 |
| T2DM | 11 | 8.20710167 | 0.7461002 | 1.490273146 |

ANOVA

| Source of Variation | SS | df | MS | F | P-value | F crit |
|---|---|---|---|---|---|---|
| Between Groups | 90.1533857 | 2 | 45.07669285 | 0.138079 | 0.871214 | 3.097698 |
| Within Groups | 29381.10666 | 90 | 326.4567407 | | | |

# FIG. 5D(contd.)

EP 2 177 908 A2

# FIG. 5E

| Total | 29471.26005 | 92 | | | | |
|---|---|---|---|---|---|---|

IL18
SUMMARY

| Groups | Count | Sum | Average | Variance |
|---|---|---|---|---|
| Low Risk | 72 | 18.6022506 | 0.2583646 | 0.018316361 |
| High Risk | 10 | 4.02510087 | 0.4025101 | 0.018740797 |
| T2DM | 11 | 3.86827781 | 0.3516616 | 0.026416589 |

ANOVA

| Source of Variation | SS | df | MS | F | P-value | F crit |
|---|---|---|---|---|---|---|
| Between Groups | 0.238046784 | 2 | 0.119023392 | 6.180198 | 0.003055 | 3.097698 |
| Within Groups | 1.733294714 | 90 | 0.01925883 | | | |
| Total | 1.971341499 | 92 | | | | |

Fibrinogen
SUMMARY

| Groups | Count | Sum | Average | Variance |
|---|---|---|---|---|
| Low Risk | 72 | 1433743293 | 19913101 | 2.66421E+15 |
| High Risk | 9 | 139702020 | 15522447 | 1.57528E+14 |
| T2DM | 11 | 164372209 | 14942928 | 6.16979E+13 |

ANOVA

| Source of Variation | SS | df | MS | F | P-value | F crit |
|---|---|---|---|---|---|---|
| Between Groups | 3.48802E+14 | 2 | 1.74401E+14 | 0.08125 | 0.922031 | 3.09887 |
| Within Groups | 1.91036E+17 | 89 | 2.14647E+15 | | | |
| Total | 1.91385E+17 | 91 | | | | |

**Comp C4**

SUMMARY

| Groups | Count | Sum | Average | Variance |
|---|---|---|---|---|
| Low Risk | 72 | 60315521.1 | 837715.57 | 1.03588E+12 |
| High Risk | 10 | 10257949.7 | 1025795 | 7.4479E+11 |
| T2DM | 11 | 16149482 | 1468134.7 | 9.81511E+11 |

ANOVA

| Source of Variation | SS | df | MS | F | P-value | F crit |
|---|---|---|---|---|---|---|
| Between Groups | 3.88984E+12 | 2 | 1.94492E+12 | 1.943507 | 0.149164 | 3.097698 |
| Within Groups | 9.00655E+13 | 90 | 1.00073E+12 | | | |
| Total | 9.39554E+13 | 92 | | | | |

**PAI-1**

SUMMARY

| Groups | Count | Sum | Average | Variance |
|---|---|---|---|---|
| Low Risk | 72 | 10710.4567 | 148.75634 | 9838.151053 |
| High Risk | 10 | 1995.50152 | 199.55015 | 11810.75339 |
| T2DM | 11 | 2039.16962 | 185.37906 | 6962.53664 |

ANOVA

| Source of Variation | SS | df | MS | F | P-value | F crit |
|---|---|---|---|---|---|---|
| Between Groups | 31633.83714 | 2 | 15816.91857 | 1.627942 | 0.202061 | 3.097698 |
| Within Groups | 874430.8717 | 90 | 9715.898574 | | | |
| Total | 906064.7088 | 92 | | | | |

**Haptoglobin**

SUMMARY

| Groups | Count | Sum | Average | Variance |
|---|---|---|---|---|
| Low Risk | 72 | 361781883 | 5024748.4 | 3.4984E+13 |
| High Risk | 10 | 70463933.5 | 7046393.4 | 3.86077E+13 |
| T2DM | 11 | 48270115.5 | 4388192.3 | 1.10864E+13 |

ANOVA

| Source of Variation | SS | df | MS | F | P-value | F crit |
|---|---|---|---|---|---|---|
| Between Groups | 4.34501E+13 | 2 | 2.17251E+13 | 0.664556 | 0.517008 | 3.097698 |
| Within Groups | 2.9422E+15 | 90 | 3.26911E+13 | | | |
| Total | 2.98565E+15 | 92 | | | | |

FIG. 5E(contd.)

180

# FIG. 6

## INDIVIDUAL MARKER PERFORMANCE AT CLINICAL CUTOFFS

n | 85

| LEPTIN Diagnosis | Pre-Diabetes | Normal | Total |
|---|---|---|---|
| | Pre-Diabetes | | |
| Pre-Diabetes | 7 | 29 | 36 |
| Normal | 1 | 48 | 49 |
| Total | 8 | 77 | 85 |

| | Prevalence |
|---|---|
| Population | - |
| Sample | 0.094117647 |

| | | 95% CI |
|---|---|---|
| Sensitivity | 87.5% | 47.3% to 99.7% |
| Specificity | 62.3% | 50.6% to 73.1% |
| Positive predictive value | 19.4% | |
| Negative predictive value | 98.0% | |
| Efficiency | 64.7% | |

n | 85

| HAPTOGLOBIN Diagnosis | Pre-Diabetes | Normal | Total |
|---|---|---|---|
| | Pre-Diabetes | | |
| Pre-Diabetes | 5 | 45 | 50 |
| Normal | 3 | 32 | 35 |
| Total | 8 | 77 | 85 |

| | Prevalence |
|---|---|
| Population | - |
| Sample | 0.094117647 |

| | | 95% CI |
|---|---|---|
| Sensitivity | 62.5% | 24.5% to 91.5% |
| Specificity | 41.6% | 30.4% to 53.4% |
| Positive predictive value | 10.0% | |
| Negative predictive value | 91.4% | |
| Efficiency | 43.5% | |

EP 2 177 908 A2

COMBINED MARKER PERFORMANCE
USING EITHER MARKER CUTOFF AS A POSITIVE DIAGNOSIS

| n | 85 |
|---|---|

| | Pre-Diabetes | | |
|---|---|---|---|
| Either Marker Above Cutoff | Pre-Diabetes | Normal | Total |
| Pre-Diabetes | 7 | 58 | 65 |
| Normal | 1 | 19 | 20 |
| Total | 8 | 77 | 85 |

| | Prevalence |
|---|---|
| Population | - |
| Sample | 0.094117647 |

| | | 95% CI |
|---|---|---|
| Sensitivity | 87.5% | 47.3% to 99.7% |
| Specificity | 24.7% | 15.6% to 35.8% |

| | |
|---|---|
| Positive predictive value | 10.8% |
| Negative predictive value | 95.0% |

| | |
|---|---|
| Efficiency | 30.6% |

COMBINED MARRKER PERFORMANCE
USING MATHEMATICAL ALGORITHM (LOGISTIC REGRESSION)

| n | 85 |
|---|---|

| | Pre-Diabetes | | |
|---|---|---|---|
| Logistic Regression Model | Pre-Diabetes | Normal | Total |
| Pre-Diabetes | 7 | 25 | 32 |
| Normal | 1 | 52 | 53 |
| Total | 8 | 77 | 85 |

| | Prevalence |
|---|---|
| Population | - |
| Sample | 0.094117647 |

| | | 95% CI |
|---|---|---|
| Sensitivity | 87.5% | 47.3% to 99.7% |
| Specificity | 87.6% | 55.9% to 77.8% |

| | |
|---|---|
| Positive predictive value | 21.9% |
| Negative predictive value | 98.1% |

| | |
|---|---|
| Efficiency | 89.4% |

# FIG. 6(contd.)

EP 2 177 908 A2

## COMBINED MARRKER PERFORMANCE VERSUS INDIVIDUAL MARKERS - ROC CURVE ANALYSIS USING MATHEMATICAL ALGORITHM (LOGISTIC REGRESSION)

n  |  84  (cases excluded; 1 due to missing values)

| Pre-Diabetes | n |
|---|---|
| Normal | 76 |
| Pre-Diabetes | 8 |

| Curve | Area | SE | p | 95% CI of Area | Pre-Diabetes = Pre-Diabetes |
|---|---|---|---|---|---|
| HAPTOGLOBIN | 0.613 | 0.1173 | 0.1666 | 0.384 to 0.843 | have higher values |
| LEP | 0.786 | 0.0685 | <0.0001 | 0.652 to 0.921 | have higher values |
| 2Mod_1 | 0.794 | 0.0790 | <0.0001 | 0.640 to 0.949 | have higher values |

| Contrast | Difference | p |
|---|---|---|
| HAPTOGLOBIN v LEP | -0.173 | - |
| HAPTOGLOBIN v 2Mod_1 | -0.181 | 0.1093 |
| LEP v 2Mod_1 | -0.008 | - |

FIG. 6(contd.)

FIG. 6(contd.)

Legend:
- — — — No discrimination
- —◇— HEPTOGLOBIN
- ····□···· LEPTIN
- — △ — Logistic Regression

X-axis: 1 - Specificity (false positives)
Y-axis: Sensitivity (true positives)

FIG. 7

Changes in Individual DBRISKMARKER Influence
As Additional Markers Are Added to a DBRISKMARKER Panel

FIG. 8   Improving DBRISKMARKER Panel Performance As Additional Markers Are Added to the Panel

FIG. 9

**Histogram of OUT2$R2**

FIG. 10

FIG. 13

| | DBRISKMARKER1 | DBRISKMARKER2 | DBRISKMARKER3 |
|---|---|---|---|
| 3-Panel 1 | LEP | HAPTOGLOBIN | ILGFBP3 |
| 3-Panel 2 | LEP | TNFR1 | CD_26 |
| 3-Panel 3 | LEP | CD_26 | ILGFBP3 |
| 3-Panel 4 | LEP | HAPTOGLOBIN | C_PEPTIDE |
| 3-Panel 5 | LEP | HAPTOGLOBIN | ADIPONECTIN |
| 3-Panel 6 | LEP | HAPTOGLOBIN | MCSF |
| 3-Panel 7 | LEP | RAGE | ILGFBP3 |
| 3-Panel 8 | LEP | HAPTOGLOBIN | ADIPSIN |
| 3-Panel 9 | LEP | RESISTIN | ILGFBP3 |
| 3-Panel 10 | LEP | HAPTOGLOBIN | RESISTIN |
| 3-Panel 11 | LEP | HAPTOGLOBIN | CD_26 |
| 3-Panel 12 | LEP | HAPTOGLOBIN | COMPC4 |
| 3-Panel 13 | LEP | HAPTOGLOBIN | MMP2 |
| 3-Panel 14 | LEP | RAGE | CD_26 |
| 3-Panel 15 | LEP | HAPTOGLOBIN | ACE |
| 3-Panel 16 | LEP | HAPTOGLOBIN | RAGE |
| 3-Panel 17 | LEP | ADIPONECTIN | ILGFBP3 |
| 3-Panel 18 | LEP | HGF | ILGFBP3 |
| 3-Panel 19 | LEP | HAPTOGLOBIN | ICAM |
| 3-Panel 20 | LEP | HAPTOGLOBIN | E_SELECTIN |
| 3-Panel 21 | LEP | MMP2 | ILGFBP3 |
| 3-Panel 22 | LEP | CD_26 | ADIPONECTIN |
| 3-Panel 23 | LEP | RAGE | RESISTIN |
| 3-Panel 24 | LEP | HAPTOGLOBIN | VEGF |
| 3-Panel 25 | LEP | TNFR1 | ILGFBP3 |

FIG. 11

# FIG. 12

KEY INDIVIDUAL MARKERS

| Marker position<br>Core Marker | 1<br>LEPTIN | 2<br>HAPTOGLOBIN | 3<br>ILGFBP3 | 4<br>RESISTIN | 5<br>MMP2 | 6<br>ACE | 7<br>COMPC4 | 8<br>CD14 |
|---|---|---|---|---|---|---|---|---|
| Frequently Combined<br>With | HAPTOGLOBIN<br>ILGFBP3<br>CD-26<br>RAGE<br>ADIPONECTIN | LEP<br>ILGFBP3<br>TNFR1<br>ADIPONECTIN<br>CRP<br>ADIPSIN | LEP<br>HAPTOGLOBIN<br>RESISTIN<br>TNFR1<br>CRP | LEP<br>HAPTOGLOBIN<br>TNFR1-CD-26<br>CRP<br>RAGE | LEP<br>HAPTOGLOBIN<br>RESISTIN<br>TNFR1-CD-26<br>IL_18<br>ADIPSIN<br>PAI_1<br>CRP | LEP<br>HAPTOGLOBIN<br>RESISTIN<br>TNFR1-CD-26<br>PAI_1<br>IL-18<br>ADIPSIN<br>CRP | LEP<br>HAPTOGLOBIN<br>TNFR1-CD-26<br>RAGE<br>IL_18<br>ADIPONECTIN<br>CRP | LEP<br>CRP<br>HAPTOGLOBIN<br>TNFR1-CD-26<br>RAGE<br>IL_18<br>ADIPONECTIN |
| Key Marker<br>Substitution Strategies | CRP<br>TNFR1-CD-26<br>IL6SOLR<br>ADIPSIN<br>CD40_LIGAND | IL_18<br>TNFR1-CD-26<br>RAGE<br>FETUIN_A<br>ADIPONECTIN<br>HGF | ADIPSIN<br>TNFR1-CD-26<br>E_SELECTIN<br>ICAM<br>CD40_LIGAND<br>RAGE<br>C_PEPTIDE<br>ADIPONECTIN<br>VEGF | MCSF<br>TNFR1-CD-26<br>ADIPSIN<br>ACTIVIN_A<br>INFGAMMA | E_SELECTIN<br>MCSF<br>FIBRINOGEN | CD_26<br>E_SELECTIN<br>FIBRINOGEN | E_SELECTIN<br>VEGF<br>MCSF<br>ACTIVIN_A<br>C_PEPTIDE<br>ICAM | E_SELECTIN<br>MCSF<br>CD_26<br>ICAM<br>FAS<br>CD40_SOLUBLE |

KEY COMBINATION MARKERS

| Marker Position<br>Substitution Marker | 9<br>E_SELECTIN | 10<br>MCSF | 11<br>VEGF | 12<br>TNFR1 | 13<br>CD_26 | 14<br>FIBRINOGEN | 15<br>AGRP | 16<br>TNFALPHA |
|---|---|---|---|---|---|---|---|---|
| Alternative<br>Substitution Strategies | ADIPSIN<br>IL6SOLR<br>MMP2 | ICAM<br>ADIPSIN<br>ACTIVIN_A | CD40_LIGAND<br>ADIPSIN<br>INFGAMMA | ICAM<br>ACTIVIN_A<br>HGF | HGF<br>ACTIVIN_A<br>ICAM | ACTIVIN_A<br>CD40_LIGAND<br>ICAM | MMP2<br>INFGAMMA<br>ACTIVIN_A | CD40_LIGAND<br>ADIPSIN<br>IL_8 |

| Marker Position<br>Substitution Marker | 17<br>MCP_1 | 18<br>TGFB1 | 19<br>COMP_C3 | 20<br>AKT1 |
|---|---|---|---|---|
| Alternative<br>Substitution Strategies | ACTIVIN_A<br>MMP2<br>IL_6 | ICAM<br>C_PEPTIDE<br>ACTIVIN_A | ICAM<br>ADIPSIN<br>MMP2 | ACTIVIN_A<br>ADIPSIN<br>ICAM |

EP 2 177 908 A2

# FIG. 14

| | DBRISKMARKER1 | DBRISKMARKER2 | DBRISKMARKER3 | DBRISKMARKER4 | DBRISKMARKER5 | DBRISKMARKER6 | DBRISKMARKER7 | DBRISKMARKER8 |
|---|---|---|---|---|---|---|---|---|
| 8-Panel 1 | LEP | HAPTOGLOBIN | ILGFBP3 | RESISTIN | MMP2 | ACE | COMPC4 | CD14 |
| 8-Panel 2 | CRP | HAPTOGLOBIN | ILGFBP3 | RESISTIN | MMP2 | ACE | COMPC4 | CD14 |
| 8-Panel 3 | TNFR1 | HAPTOGLOBIN | ILGFBP3 | RESISTIN | MMP2 | ACE | COMPC4 | CD14 |
| 8-Panel 4 | IL6SOLR | HAPTOGLOBIN | ILGFBP3 | RESISTIN | MMP2 | ACE | COMPC4 | CD14 |
| 8-Panel 5 | LEP | IL 18 | ILGFBP3 | RESISTIN | MMP2 | ACE | COMPC4 | CD14 |
| 8-Panel 6 | LEP | CD 26 | ILGFBP3 | RESISTIN | MMP2 | ACE | COMPC4 | CD14 |
| 8-Panel 7 | LEP | RAGE | ILGFBP3 | RESISTIN | MMP2 | ACE | COMPC4 | CD14 |
| 8-Panel 8 | LEP | HAPTOGLOBIN | ADIPSIN | RESISTIN | MMP2 | ACE | COMPC4 | CD14 |
| 8-Panel 9 | LEP | HAPTOGLOBIN | CD 26 | RESISTIN | MMP2 | ACE | COMPC4 | CD14 |
| 8-Panel 10 | LEP | HAPTOGLOBIN | E SELECTIN | RESISTIN | MMP2 | ACE | COMPC4 | CD14 |
| 8-Panel 11 | LEP | HAPTOGLOBIN | ILGFBP3 | MCSF | MMP2 | ACE | COMPC4 | CD14 |
| 8-Panel 12 | LEP | HAPTOGLOBIN | ILGFBP3 | CD 26 | MMP2 | ACE | COMPC4 | CD14 |
| 8-Panel 13 | LEP | HAPTOGLOBIN | ILGFBP3 | ADIPSIN | MMP2 | ACE | COMPC4 | CD14 |
| 8-Panel 14 | LEP | HAPTOGLOBIN | ILGFBP3 | RESISTIN | E SELECTIN | ACE | COMPC4 | CD14 |
| 8-Panel 15 | LEP | HAPTOGLOBIN | ILGFBP3 | RESISTIN | MCSF | ACE | COMPC4 | CD14 |
| 8-Panel 16 | LEP | HAPTOGLOBIN | ILGFBP3 | RESISTIN | FIBRINOGEN | ACE | COMPC4 | CD14 |
| 8-Panel 17 | LEP | HAPTOGLOBIN | ILGFBP3 | RESISTIN | MMP2 | CD 26 | COMPC4 | CD14 |
| 8-Panel 18 | LEP | HAPTOGLOBIN | ILGFBP3 | RESISTIN | MMP2 | E SELECTIN | COMPC4 | CD14 |
| 8-Panel 19 | LEP | HAPTOGLOBIN | ILGFBP3 | RESISTIN | MMP2 | FIBRINOGEN | COMPC4 | CD14 |
| 8-Panel 20 | LEP | HAPTOGLOBIN | ILGFBP3 | RESISTIN | MMP2 | ACE | E SELECTIN | CD14 |
| 8-Panel 21 | LEP | HAPTOGLOBIN | ILGFBP3 | RESISTIN | MMP2 | ACE | VEGF | CD14 |
| 8-Panel 22 | LEP | HAPTOGLOBIN | ILGFBP3 | RESISTIN | MMP2 | ACE | MCSF | CD14 |
| 8-Panel 23 | LEP | HAPTOGLOBIN | ILGFBP3 | RESISTIN | MMP2 | ACE | COMPC4 | E SELECTIN |
| 8-Panel 24 | LEP | HAPTOGLOBIN | ILGFBP3 | RESISTIN | MMP2 | ACE | COMPC4 | MCSF |
| 8-Panel 25 | LEP | HAPTOGLOBIN | ILGFBP3 | RESISTIN | MMP2 | ACE | COMPC4 | CD 26 |

EP 2 177 908 A2

# FIG. 15

| n | | 80 |
|---|---|---|

| Pre-Diabetes | n |
|---|---|
| Normal | 61 |
| Pre-Diabetes | 19 |

| Curve | Area | SE | p | 95% CI of Area | CATEGORY = Pre-Diabetes |
|---|---|---|---|---|---|
| 3 DBRISKMARKER Panel | 0.704 | 0.0753 | 0.0033 | 0.557 to 0.852 | have higher values |
| 8 DBRISKMARKER Panel | 0.800 | 0.0535 | <0.0001 | 0.695 to 0.905 | have higher values |
| 18 DBRISKMARKER Panel | 0.841 | 0.0554 | <0.0001 | 0.733 to 0.950 | have higher values |

| Contrast | Difference | p |
|---|---|---|
| 3 Panel v 8 Panel | -0.096 | 0.1151 |
| 3 Panel v 18 Panel | -0.137 | 0.0461 |
| 8 Panel v 18 Panel | -0.041 | 0.3098 |

FIG. 15(contd.)

EP 2 177 908 A2

# FIG. 16

n |     84 (cases excluded: 1 due to missing values)

| CATEGORY | n |
|---|---|
| Normal | 64 |
| Pre-Diabetes | 20 |

| Curve | Area | SE | p | 95% CI of Area | CATEGORY = Pre-Diabetes |
|---|---|---|---|---|---|
| HAPTOGLOBIN | 0.612 | 0.0748 | 0.0675 | 0.465 to 0.758 | have higher values |
| ILGFBP3 | 0.566 | 0.0795 | 0.2017 | 0.411 to 0.722 | have higher values |
| LEP | 0.730 | 0.0671 | 0.0003 | 0.599 to 0.862 | have higher values |

| Contrast | Difference | p |
|---|---|---|
| HAPTOGLOBIN v ILGFBP3 | 0.045 | - |
| HAPTOGLOBIN v LEP | -0.119 | - |
| ILGFBP3 v LEP | -0.164 | - |

EP 2 177 908 A2

FIG. 16(contd.)

EP 2 177 908 A2

# FIG. 17

**IL -IRA Standard curve**

FIG. 1: standard curve for IL-1 Receptor Agonist

## FIG. 18

EP 2 177 908 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 72546205 P
- US 4727022 A, Skold
- US 4659678 A, Forrest
- US 4376110 A, David
- US 4275149 A, Litman
- US 4233402 A, Maggio
- US 4230767 A, Boguslaski
- WO 04056456 A
- WO 04088309 A
- US 5744305 A

### Non-patent literature cited in the description

- **O'Marcaigh AS ; Jacobson RM.** Estimating The Predictive Value Of A Diagnostic Test, How To Prevent Misleading Or Confusing Results. *Clin. Ped.,* 1993, vol. 32 (8), 485-491
- Clinical Interpretation Of Laboratory Procedures. **Shultz.** Fundamentals of Clinical Chemistry. W.B. Saunders Company, 1996, 192-199
- **Zweig et al.** ROC Curve Analysis: An Example Showing The Relationships Among Serum Lipid And Apolipoprotein Concentrations In Identifying Subjects With Coronory Artery Disease. *Clin. Chem.,* 1992, vol. 38 (8), 1425-1428
- **Kannel, W.B. et al.** *Am. J. Cardiol.,* 1976, vol. 38, 46-51
- **Stem, M.P. et al.** *Am. J. Epidemiol.,* 1984, vol. 120, 834-851
- **Stem, M.P. et al.** *Diabetes,* 1993, vol. 42, 706-714
- **Burke, J.P. et al.** *Arch. Intern. Med.,* 1999, vol. 159, 1450-1456
- **Eddy, D.M. ; Schlessinger, L.** *Diabetes Care,* 2003, vol. 26 (11), 3093-3101
- **Eddy, D.M. ; Schlessinger, L.** *Diabetes Care,* 2003, vol. 26 (11), 3102-3110
- **Lindström, J. ; Tuomilehto, J.** *Diabetes Care,* 2003, vol. 26 (3), 725-731
- **Griffin, S.J. et al.** *Diabetes Metab. Res. Rev.,* 2000, vol. 16, 164-171
- **DeLong E.R. et al.** *Biometrics,* 1988, vol. 44, 837-45
- **Hanson, R.L. et al.** *Diabetes,* 2002, vol. 51, 3120-3127
- **E. Maggio.** Enzyme-Immunoassay. CRC Press, Inc, 1980
- **Wirth, U. et al.** *Proteomics,* 2002, vol. 2 (10), 1445-51
- **Aalto, K. ; L. Korhonen et al.** Nerve growth factor in serum of children with systemic lupus erythematosus is correlated with disease activity. *Cytokine,* 2002, vol. 20 (3), 136-9
- **Abate, N. ; M. Chandalia et al.** ENPP1/PC-1 K121Q polymorphism and genetic susceptibility to type 2 Diabetes. *Diabetes,* 2005, vol. 54 (4), 1207-13
- **Adam, T. C. ; J. Jocken et al.** Decreased glucagon-like peptide 1 release after weight loss in overweight/obese subjects. *Obes Res,* 2005, vol. 13 (4), 710-6
- **Aguilar-Bryan, L. ; C. G. Nichols et al.** Cloning of the beta cell high-affinity sulfonylurea receptor: a regulator of insulin secretion. *Science,* 1995, vol. 268 (5209), 423-6
- **Al-Khalili, L. ; M. Forsgren et al.** Enhanced insulin-stimulated glycogen synthesis in response to insulin, metformin or rosiglitazone is associated with increased mRNA expression of GLUT4 and peroxisomal proliferator activator receptor gamma co-activator 1. *Diabetologia,* 2005, vol. 48 (6), 1173-9
- **Arosio, M. ; S. Porretti et al.** Elevated circulating somatostatin levels in acromegaly. *J Endocrinol Invest,* 2003, vol. 26 (6), 499-502
- **Astaneie, F. ; M. Afshari et al.** Total antioxidant capacity and levels of epidermal growth factor and nitric oxide in blood and saliva of insulin-dependent diabetic patients. *Arch Med Res,* 2005, vol. 36 (4), 376-81
- **Authier, F. ; M. Kouach et al.** Endosomal proteolysis of insulin-like growth factor-I at its C-terminal D-domain by cathepsin B. *FEBS Lett,* 2005, vol. 579 (20), 4309-16
- **Baranowska, B. ; E. Wolinska-Witort et al.** Plasma orexin A, orexin B, leptin, neuropeptide Y (NPY) and insulin in obese women. *Neuro Endocrinol Lett,* 2005, vol. 26 (4), 293-6
- **Baratta, R ; R. Di Paola et al.** Evidence for genetic epistasis in human insulin resistance: the combined effect of PC-1 (K121Q) and PPARgamma2 (P12A) polymorphisms. *J Mol Med,* 2003, vol. 81 (11), 718-23

- **Beale, E. G. ; R. E. Hammer et al.** Disregulated glyceroneogenesis: PCK1 as a candidate Diabetes and obesity gene. *Trends Endocrinol Metab,* 2004, vol. 15 (3), 129-35
- **Bell, C. G. ; D. Meyre et al.** Association of melanin-concentrating hormone receptor 1 5' polymorphism with early-onset extreme obesity. *Diabetes,* 2005, vol. 54 (10), 3049-55
- **Beranek, M. ; K. Kankova et al.** Polymorphisms in the von Willebrand factor gene are not associated with proliferative retinopathy in non-insulin-dependent Diabetes Mellitus. *Ophthalmic Res,* 2002, vol. 34 (5), 327-30
- **Bernassola, F. ; M. Federici et al.** Role of transglutaminase 2 in glucose tolerance: knockout mice studies and a putative mutation in a MODY patient. *Faseb J,* 2002, vol. 16 (11), 1371-8
- **Bertherat, J. ; F. Tenenbaum et al.** Somatostatin receptors 2 and 5 are the major somatostatin receptors in insulinomas: an in vivo and in vitro study. *J Clin Endocrinol Metab,* 2003, vol. 88 (11), 5353-60
- **Bluher, M. ; R. Unger et al.** Relation between glycaemic control, hyperinsulinaemia and plasma concentrations of soluble adhesion molecules in patients with impaired glucose tolerance or Type II Diabetes. *Diabetoloaia,* 2002, vol. 45 (2), 210-6
- **Boucher, J. ; B. Masri et al.** Apelin, a newly identified adipokine upregulated by insulin and obesity. *Endocrinology,* 2005, vol. 146 (4), 1764-71
- **Boutin, P. ; C. Dina et al.** GAD2 on chromosome 10p12 is a candidate gene for human obesity. *PLoS Biol,* 2003, vol. 1 (3), E68
- **Brackenridge, A. ; E. R. Pearson et al.** Contrasting insulin sensitivity of endogenous glucose production rate in subjects with hepatocyte nuclear factor-lbeta and -1 alpha mutations. *Diabetes,* 2006, vol. 55 (2), 405-11
- **Bruun, J. M. ; C. Verdich et al.** Association between measures of insulin sensitivity and circulating levels of interleukin-8, interleukin-6 and tumor necrosis factor-alpha. Effect of weight loss in obese men. *Eur J Endocrinol,* 2003, vol. 148 (5), 535-42
- **Bullmann, C. ; J. Kotzka et al.** Identification of a novel mutation in the arginine vasopressin-neurophysin II gene in familial central Diabetes insipidus. *Exp Clin Endocrinol Diabetes,* 2002, vol. 110 (3), 134-7
- **Carroll, J. S. ; E. W. Seely et al.** Digitalis-like factor response to hyperinsulinemia accompanying a euglycemic hyperinsulinemic clamp or oral glucose tolerance test. *Life Sci,* 2001, vol. 69 (7), 829-37
- **Chen, M. B. ; A. J. McAinch et al.** Impaired activation of AMP-kinase and fatty acid oxidation by globular adiponectin in cultured human skeletal muscle of obese type 2 diabetics. *J Clin Endocrinol Metab,* 2005, vol. 90 (6), 3665-72
- **Chen, M. P. ; F. M. Chung et al.** Elevated plasma level of visfatin/pre-B cell colony-enhancing factor in patients with type 2 Diabetes Mellitus. *J Clin Endocrinol Metab,* 2006, vol. 91 (1), 295-9
- **Cho, Y. M. ; M. D. Ritchie et al.** Multifactor-dimensionality reduction shows a two-locus interaction associated with Type 2 Diabetes Mellitus. *Diabetologia,* 2004, vol. 47 (3), 549-54
- **Chouchane, L. ; J. Danguir et al.** Genetic variation in the stress protein hsp70-2 gene is highly associated with obesity. *Int J Obes Relat Metab Disord,* 2001, vol. 25 (4), 462-6
- **Clemmons, D. R. ; M. Sleevi et al.** Recombinant, nonglycosylated human insulin-like growth factor-binding protein-3 (IGFBP-3) is degraded preferentially after administration to type II diabetics, resulting in increased endogenous glycosylated IGFBP-3. *J Clin Endocrinol Metab,* 2005, vol. 90 (12), 6561-8
- **Collin, G. B. ; J. D. Marshall et al.** Mutations in ALMS1 cause obesity, type 2 Diabetes and neurosensory degeneration in Alstrom syndrome. *Nat Genet,* 2002, vol. 31 (1), 74-8
- **Cosson, E. ; A. F. Bringuier et al.** Fas/Fas-Ligand pathway is impaired in patients with type 2 Diabetes. Influence of hypertension and insulin resistance. *Diabetes Metab,* 2005, vol. 31 (1), 47-54
- **Cryns, K. ; T. A. Sivakumaran et al.** Mutational spectrum of the WFS1 gene in Wolfram syndrome, nonsyndromic hearing impairment, Diabetes Mellitus, and psychiatric disease. *Hum Mutat,* 2003, vol. 22 (4), 275-87
- **Cummings, D. E. ; J. Q. Purnell et al.** A preprandial rise in plasma ghrelin levels suggests a role in meal initiation in humans. *Diabetes,* 2001, vol. 50 (8), 1714-9
- **Dantz, D. ; J. Bewersdorf et al.** Vascular endothelial growth factor: a novel endocrine defensive response to hypoglycemia. *J Clin Endocrinol Metab,* 2002, vol. 87 (2), 835-40
- **Delplanque, J. ; F. Vasseur et al.** Mutation screening of the urocortin gene: identification of new single nucleotide polymorphisms and association studies with obesity in French Caucasians. *J Clin Endocrinol Metab,* 2002, vol. 87 (2), 867-9
- **Dempfle, A. ; A.. Hinney et al.** Large quantitative effect of melanocortin-4 receptor gene mutations on body mass index. *J Med Genet,* 2004, vol. 41 (10), 795-800
- **Du, K. ; S. Herzig et al.** TRB3: a tribbles homolog that inhibits Akt/PKB activation by insulin in liver. *Science,* 2003, vol. 300 (5625), 1574-7
- **Dzienis-Straczkowska, S. ; M. Straczkowski et al.** Soluble tumor necrosis factor-alpha receptors in young obese subjects with normal and impaired glucose tolerance. *Diabetes Care,* 2003, vol. 26 (3), 875-80

- **Esposito, K. ; R. Marfella et al.** Plasma interleukin-18 concentrations are elevated in type 2 Diabetes. *Diabetes Care,* 2004, vol. 27 (1), 272
- **Faraj, M. ; P. J. Havel et al.** Plasma acylation-stimulating protein, adiponectin, leptin, and ghrelin before and after weight loss induced by gastric bypass surgery in morbidly obese subjects. *J Clin Endocrinol Metab,* 2003, vol. 88 (4), 1594-602
- **Feng, X. ; Z. Guo et al.** Identification of a negative response element in the human inducible nitric-oxide synthase (hiNOS) promoter: The role of NF-kappa B-repressing factor (NRF) in basal repression of the hiNOS gene. *Proc Natl Acad Sci U S A,* 2002, vol. 99 (22), 14212-7
- **Feng, Y. ; Q. Wang et al.** SGK1-mediated fibronectin formation in diabetic nephropathy. *Cell Physiol Biochem,* 2005, vol. 16 (4-6), 237-44
- **Fernandez-Real, J. M. ; M. Broch et al.** CD14 monocyte receptor, involved in the inflammatory cascade, and insulin sensitivity. *J Clin Endocrinol Metab,* 2003, vol. 88 (4), 1780-4
- **Fernandez-Real, J. M. ; M. Pugeat et al.** Serum corticosteroid-binding globulin concentration and insulin resistance syndrome: a population study. *J Clin Endocrinol Metab,* 2002, vol. 87 (10), 4686-90
- **Fontaine, C. ; G. Dubois et al.** The orphan nuclear receptor Rev-Erbalpha is a peroxisome proliferator-activated receptor (PPAR) gamma target gene and promotes PPARgamma-induced adipocyte differentiation. *J Biol Chem,* 2003, vol. 278 (39), 37672-80
- **Forbes, J. M. ; S. R. Thorpe et al.** Modulation of soluble receptor for advanced glycation end products by angiotensin-converting enzyme-1 inhibition in diabetic nephropathy. *J Am Soc Nephrol,* 2005, vol. 16 (8), 2363-72
- **Fukui, M. ; Y. Kitagawa et al.** Association between alcohol consumption and serum dehydroepiandrosterone sulphate concentration in men with Type 2 Diabetes: a link to decreased cardiovascular risk. *Diabet Med,* 2005, vol. 22 (10), 1446-50
- **Gavrila, A. ; J. L. Chan et al.** Circulating melanin-concentrating hormone, agouti-related protein, and alpha-melanocyte-stimulating hormone levels in relation to body composition: alterations in response to food deprivation and recombinant human leptin administration. *J Clin Endocrinol Metab,* 2005, vol. 90 (2), 1047-54
- **George, S. ; J. J. Rochford et al.** A family with severe insulin resistance and Diabetes due to a mutation in AKT2. *Science,* 2004, vol. 304 (5675), 1325-8
- **Ghosh, S. ; E. R. Hauser et al.** A large sample of finnish diabetic sib-pairs reveals no evidence for a non-insulin-dependent Diabetes Mellitus susceptibility locus at 2qter. *J Clin Invest,* 1998, vol. 102 (4), 704-9
- **Ghosh, S. ; R. M. Watanabe et al.** Type 2 Diabetes: evidence for linkage on chromosome 20 in 716 Finnish affected sib pairs. *Proc Natl Acad Sci U S A,* 1999, vol. 96 (5), 2198-203
- **Gonzalez, M. ; C. Flores et al.** Cell signalling-mediating insulin increase of mRNA expression for cationic amino acid transporters-1 and -2 and membrane hyperpolarization in human umbilical vein endothelial cells. *Pflugers Arch,* 2004, vol. 448 (4), 383-94
- **Gouni-Berthold, I. ; E. Giannakidou et al.** Association between the PPARalpha L162V polymorphism, plasma lipoprotein levels, and atherosclerotic disease in patients with Diabetes Mellitus type 2 and in nondiabetic controls. *Am Heart J,* 2004, vol. 147 (6), 1117-24
- **Grant, S. F. ; G. Thorleifsson et al.** Variant of transcription factor 7-like 2 (TCF7L2) gene confers risk of type 2 Diabetes. *Nat Genet,* 2006, vol. 38 (3), 320-3
- **Gunton, J. E. ; R. N. Kulkarni et al.** Loss of ARNT/HIF1beta mediates altered gene expression and pancreatic-islet dysfunction in human type 2 Diabetes. *Cell,* 2005, vol. 122 (3), 337-49
- **Gylvin, T. ; R. Nolsoe et al.** Mutation analysis of suppressor of cytokine signalling 3, a candidate gene in Type 1 Diabetes and insulin sensitivity. *Diabetologia,* 2004, vol. 47 (7), 1273-7
- **Haap, M. ; C. Thamer et al.** Metabolic characterization of a woman homozygous for the Ser113Leu missense mutation in carnitine palmitoyl transferase II. *J Clin Endocrinol Metab,* 2002, vol. 87 (5), 2139-43
- **Hamid, Y. H. ; S. A. Urhammer et al.** Variation in the interleukin-6 receptor gene associates with type 2 Diabetes in Danish whites. *Diabetes,* 2004, vol. 53 (12), 3342-5
- **Hansen, S. K. ; C. S. Rose et al.** Variation near the hepatocyte nuclear factor (HNF)-4alpha gene associates with type 2 Diabetes in the Danish population. *Diabetologia,* 2005, vol. 48 (3), 452-8
- **Hara, K. ; M. Horikoshi et al.** Absence of an association between the polymorphisms in the genes encoding adiponectin receptors and type 2 Diabetes. *Diabetologia,* 2005, vol. 48 (7), 1307-14
- **Hart, L. M. ; R. P. Stolk et al.** Prevalence of variants in candidate genes for type 2 Diabetes Mellitus in The Netherlands: the Rotterdam study and the Hoom study. *J Clin Endocrinol Metab,* 1999, vol. 84 (3), 1002-6
- **Hasegawa, G. ; M. Ohta et al.** Increased serum resistin levels in patients with type 2 Diabetes are not linked with markers of insulin resistance and adiposity. *Acta Diabetol,* 2005, vol. 42 (2), 104-9
- **Hauache, O. M. ; A. F. Reis et al.** Estimation of Diabetes risk in Brazilian population by typing for polymorphisms in HLA-DR-DQ, INS and CTLA-4 genes. *Dis Markers,* 2005, vol. 21 (3), 139-45

- **He, Z. H. ; L. H. Ma.** The aerobic fitness (VO2 peak) and alpha-fibrinogen genetic polymorphism in obese and non-obese Chinese boys. *Int J Sports Med,* 2005, vol. 26 (4), 253-7
- **Herlihy, O. M. ; B. A. Barrow et al.** Hyperglycaemic siblings of Type II (non-insulin-dependent) diabetic patients have increased PAI-1, central obesity and insulin resistance compared with their paired normoglycaemic sibling. *Diabetologia,* 2002, vol. 45 (5), 635-41
- **Hernandez, C. ; E. Carrasco et al.** Somatostatin molecular variants in the vitreous fluid: a comparative study between diabetic patients with proliferative diabetic retinopathy and nondiabetic control subjects. *Diabetes Care,* 2005, vol. 28 (8), 1941-7
- **Hirai, S. ; M. Yamanaka et al.** Activin A inhibits differentiation of 3T3-L1 preadipocyte. *Mol Cell Endocrinol,* 2005, vol. 232 (1-2), 21-6
- **Horike, N. ; H. Takemori et al.** Adipose-specific expression, phosphorylation of Ser794 in insulin receptor substrate-1, and activation in diabetic animals of salt-inducible kinase-2. *J Biol Chem,* 2003, vol. 278 (20), 18440-7
- **Hotamisligil, G. S. ; B. M. Spiegelman.** Tumor necrosis factor alpha: a key component of the obesity-Diabetes link. *Diabetes,* 1994, vol. 43 (11), 1271-8
- **Hristova, M. ; L. Aloe.** Metabolic syndrome--neurotrophic hypothesis. *Med Hypotheses,* 2006, vol. 66 (3), 545-9
- **Humpert, P. M. ; S. Kopf et al.** Plasma sRAGE is independently associated with urinary albumin excretion in type 2 Diabetes. *Diabetes Care,* 2006, vol. 29 (5), 1111-3
- **Ide, A. ; E. Kawasaki et al.** Genetic association between interleukin-10 gene promoter region polymorphisms and type 1 Diabetes age-at-onset. *Hum Immunol,* 2002, vol. 63 (8), 690-5
- **Illig, T. ; F. Bongardt et al.** Significant association of the interleukin-6 gene polymorphisms C-174G and A-598G with type 2 Diabetes. *J Clin Endocrinol Metab,* 2004, vol. 89 (10), 5053-8
- **Itoh, Y. ; Y. Kawamata et al.** Free fatty acids regulate insulin secretion from pancreatic beta cells through GPR40. *Nature,* 2003, vol. 422 (6928), 173-6
- **Jayagopal, V. ; E. S. Kilpatrick et al.** The biological variation of sex hormone-binding globulin in type 2 Diabetes: implications for sex hormone-binding globulin as a surrogate marker of insulin resistance. *Diabetes Care,* 2004, vol. 27 (1), 278-80
- **Jinchuan, Y. ; W. Zonggui et al.** Upregulation of CD40--CD40 ligand system in patients with Diabetes Mellitus. *Clin Chim Acta,* 2004, vol. 339 (1-2), 85-90
- **Johannesen, J. ; A. Pie et al.** Linkage of the human inducible nitric oxide synthase gene to type 1 Diabetes. *J Clin Endocrinol Metab,* 2001, vol. 86 (6), 2792-6
- **Johansen, T. ; C. Laurino et al.** Reduction of adiposity with prolonged growth hormone treatment in old obese rats: effects on glucose handling and early insulin signaling. *Growth Horm IGF Res,* 2005, vol. 15 (1), 55-63
- **Kalina, A. ; C. Szalai et al.** Association of plasma lipid levels with apolipoprotein E polymorphism in Type 2 Diabetes. *Diabetes Res Clin Pract,* 2002, vol. 56 (1), 63-8
- **Kalmar, T. ; I. Seres et al.** Correlation between the activities of lipoprotein lipase and paraoxonase in type 2 Diabetes Mellitus. *Diabetes Metab,* 2005, vol. 31 (6), 574-80
- **Kaneto, H. ; T. A. Matsuoka et al.** A crucial role of MafA as a novel therapeutic target for Diabetes. *J Biol Chem,* 2005, vol. 280 (15), 15047-52
- **Kim, H. S. ; S. Yumkham et al.** C-terminal part of AgRP stimulates insulin secretion through calcium release in pancreatic beta Rin5mf cells. *Neuropeptides,* 2005, vol. 39 (4), 385-93
- **Kim, J. H. ; H. D. Shin et al.** Peroxisome proliferator-activated receptor gamma coactivator 1 alpha promoter polymorphisms are associated with early-onset type 2 Diabetes Mellitus in the Korean population. *Diabetologia,* 2005, vol. 48 (7), 1323-30
- **Kim, M. J. ; M. Maachi et al.** Increased adiponectin receptor-1 expression in adipose tissue of impaired glucose-tolerant obese subjects during weight loss. *Eur J Endocrinol,* 2006, vol. 155 (1), 161-5
- **Koka, V. ; W. Wang et al.** Advanced glycation end products activate a chymase-dependent angiotensin II-generating pathway in diabetic complications. *Circulation,* 2006, vol. 113 (10), 1353-60
- **Konheim, Y. L. ; J. K. Wolford.** Association of a promoter variant in the inducible cyclooxygenase-2 gene (PTGS2) with type 2 Diabetes Mellitus in Pima Indians. *Hum Genet,* 2003, vol. 113 (5), 377-81
- **Koo, S. H. ; L. Flechner et al.** The CREB coactivator TORC2 is a key regulator of fasting glucose metabolism. *Nature,* 2005, vol. 437 (7062), 1109-11
- **Kotnik, P. ; J. Nielsen et al.** Altered expression of COX-1, COX-2, and mPGES in rats with nephrogenic and central Diabetes insipidus. *Am J Physiol Renal Physiol,* 2005, vol. 288 (5), F1053-68
- **Lakka, T. A. ; H. M. Lakka et al.** Effect of exercise training on plasma levels of C-reactive protein in healthy adults: the HERITAGE Family Study. *Eur Heart J,* 2005, vol. 26 (19), 2018-25
- **Laukkanen, O. ; J. Lindstrom et al.** Polymorphisms in the SLC2A2 (GLUT2) gene are associated with the conversion from impaired glucose tolerance to type 2 Diabetes: the Finnish Diabetes Prevention Study. *Diabetes,* 2005, vol. 54 (7), 2256-60

- **Laukkanen, O. ; J. Pihlajamaki et al.** Polymorphisms of the SUR (ABCC8) and Kir6.2 (KCNJ11) genes predict the conversion from impaired glucose tolerance to type 2 Diabetes. The Finnish Diabetes Prevention Study. *J Clin Endocrinol Metab,* 2004, vol. 89 (12), 6286-90
- **Lee, S. W. ; K. E. Song et al.** Alterations in peripheral blood levels of TIMP-1, MMP-2, and MMP-9 in patients with type-2 Diabetes. *Diabetes Res Clin Pract,* 2005, vol. 69 (2), 175-9
- **Legakis, I. ; T. Mantzouridis et al.** Positive correlation of galanin with glucose in type 2 Diabetes. *Diabetes Care,* 2005, vol. 28 (3), 759-60
- **Lewitt, M. S. ; K. Hall et al.** Altered response of insulin-like growth factor-binding protein 1 to nutritional deprivation in type 2 Diabetes Mellitus. *Metabolism,* 2005, vol. 54 (3), 275-80
- **Lim, H. S. ; A. D. Blann et al.** Soluble CD40 ligand, soluble P-selectin, interleukin-6, and tissue factor in Diabetes Mellitus: relationships to cardiovascular disease and risk factor intervention. *Circulation,* 2004, vol. 109 (21), 2524-8
- **Lindgren, C. M. ; M. M. Mahtani et al.** Genomewide search for type 2 Diabetes Mellitus susceptibility loci in Finnish families: the Botnia study. *Am J Hum Genet,* 2002, vol. 70 (2), 509-16
- **Lo, J. C. ; X. Zhao et al.** The association of genetic polymorphisms in sex hormone biosynthesis and action with insulin sensitivity and Diabetes Mellitus in women at midlife. *Am J Med,* 2006, vol. 119 (9), 69-78
- **Maedler, K. ; A. Fontana et al.** FLIP switches Fas-mediated glucose signaling in human pancreatic beta cells from apoptosis to cell replication. *Proc Natl Acad Sci U S A,* 2002, vol. 99 (12), 8236-41
- **Magnusson, M. ; O. Melander et al.** Elevated plasma levels ofNt-proBNP in patients with type 2 Diabetes without overt cardiovascular disease. *Diabetes Care,* 2004, vol. 27 (8), 1929-35
- **Mallone, R. ; P. C. Perin.** Anti-CD38 autoantibodies in type? Diabetes. *Diabetes Metab Res Rev,* 2006, vol. 22 (4), 284-94
- **Mannucci, E. ; L. Pala et al.** Hyperglycaemia increases dipeptidyl peptidase IV activity in Diabetes Mellitus. *Diabetologia,* 2005, vol. 48 (6), 1168-72
- **Marchal-Victorion, S. ; N. Vionnet et al.** Genetic, pharmacological and functional analysis of cholecystokinin-1 and cholecystokinin-2 receptor polymorphism in type 2 Diabetes and obese patients. *Pharmacogenetics,* 2002, vol. 12 (1), 23-30
- **Marculescu, R. ; G. Endler et al.** Interleukin-1 receptor antagonist genotype is associated with coronary atherosclerosis in patients with type 2 Diabetes. *Diabetes,* 2002, vol. 51 (12), 3582-5
- **Marques, R. G. ; M. J. Fontaine et al.** C-peptide: much more than a byproduct of insulin biosynthesis. *Pancreas,* 2004, vol. 29 (3), 231-8
- **Marselli, L. ; L. Trincavelli et al.** The role of peripheral benzodiazepine receptors on the function and survival of isolated human pancreatic islets. *Eur J Endocrinol,* 2004, vol. 151 (2), 207-14
- **Mathieu, C. ; C. Gysemans et al.** Vitamin D and Diabetes. *Diabetologia,* 2005, vol. 48 (7), 1247-57
- **Miele, C. ; A. Riboulet et al.** Human glycated albumin affects glucose metabolism in L6 skeletal muscle cells by impairing insulin-induced insulin receptor substrate (IRS) signaling through a protein kinase C alpha-mediated mechanism. *J Biol Chem,* 2003, vol. 278 (48), 47376-87
- **Migdalis, L. ; T. Thomaides et al.** Changes of gastric emptying rate and gastrin levels are early indicators of autonomic neuropathy in type II diabetic patients. *Clin Auton Res,* 2001, vol. 11 (4), 259-63
- **Milewicz, A. ; E. Mikulski et al.** Plasma insulin, cholecystokinin, galanin, neuropeptide Y and leptin levels in obese women with and without type 2 Diabetes Mellitus. *Int J Obes Relat Metab Disord,* 2000, vol. 24 (2), 152-3
- **Minn, A. H. ; C. Hafele et al.** Thioredoxin-interacting protein is stimulated by glucose through a carbohydrate response element and induces beta-cell apoptosis. *Endocrinology,* 2005, vol. 146 (5), 2397-405
- **Mironczuk, K. ; A. Okruszko et al.** Interleukin 18 and sICAM-1 serum levels in families with type 1 Diabetes Mellitus. *Rocz Akad Med Bialymst,* 2005, vol. 50, 151-4
- **Mitamura, Y. ; A. Tashimo et al.** Vitreous levels of placenta growth factor and vascular endothelial growth factor in patients with proliferative diabetic retinopathy. *Diabetes Care,* 2002, vol. 25 (12), 2352
- **Miyake, Y. ; H. Eguchi et al.** Glucose intolerance and serum aminotransferase activities in Japanese men. *J Hepatol,* 2003, vol. 38 (1), 18-23
- **Miyawaki, K. ; Y. Yamada et al.** Glucose intolerance caused by a defect in the entero-insular axis: a study in gastric inhibitory polypeptide receptor knockout mice. *Proc Natl Acad Sci U S A,* 1999, vol. 96 (26), 14843-7
- **Mohlke, K. L. ; A. D. Skol et al.** Evaluation of SLC2A10 (GLUT10) as a candidate gene for type 2 Diabetes and related traits in Finns. *Mol Genet Metab,* 2005, vol. 85 (4), 323-7
- **Mustafa, S. ; T. Vukovich et al.** Haptoglobin phenotype and gestational Diabetes. *Diabetes Care,* 2004, vol. 27 (9), 2103-7
- **Nair, S. ; Y. H. Lee et al.** 11beta-Hydroxysteroid dehydrogenase Type 1: genetic polymorphisms are associated with Type 2 Diabetes in Pima Indians independently of obesity and expression in adipocyte and muscle. *Diabetologia,* 2004, vol. 47 (6), 1088-95

- **Nakagawa, T. ; H. Furuta et al.** Molecular scanning of the betacellulin gene for mutations in type 2 diabetic patients. *Diabetes Res Clin Pract,* 2005, vol. 68 (3), 188-92
- **Nomura, S. ; A. Shouzu et al.** 5-HT2A receptor antagonist increases circulating adiponectin in patients with type 2 Diabetes. *Blood Coagul Fibrinolysis,* 2005, vol. 16 (6), 423-8
- **Noushmehr, H. ; E. D'Amico et al.** Fatty acid translocase (FAT/CD36) is localized on insulin-containing granules in human pancreatic betacells and mediates fatty acid effects on insulin secretion. *Diabetes,* 2005, vol. 54 (2), 472-81
- **O'Connell, T. ; D. R. Clemmons.** IGF-I/IGF-binding protein-3 combination improves insulin resistance by GH-dependent and independent mechanisms. *J Clin Endocrinol Metab,* 2002, vol. 87 (9), 4356-60
- **Ohki-Hamazaki, H. ; K. Watase et al.** Mice lacking bombesin receptor subtype-3 develop metabolic defects and obesity. *Nature,* 1997, vol. 390 (6656), 165-9
- **Okazawa, K. ; Y. Yoshimasa et al.** The haplotypes of the IRS-2 gene affect insulin sensitivity in Japanese patients with type 2 Diabetes. *Diabetes Res Clin Pract,* 2005, vol. 68 (1), 39-48
- **Pagliarini, D. J. ; S. E. Wiley et al.** Involvement of a mitochondrial phosphatase in the regulation of ATP production and insulin secretion in pancreatic beta cells. *Mol Cell,* 2005, vol. 19 (2), 197-207
- **Patti, M. E. ; A. J. Butte et al.** Coordinated reduction of genes of oxidative metabolism in humans with insulin resistance and Diabetes: Potential role of PGC1 and NRF1. *Proc Natl Acad Sci U S A,* 2003, vol. 100 (14), 8466-71
- **Repaske, D. R. ; J. A. Phillips, 3rd et al.** Molecular analysis of autosomal dominant neurohypophyseal Diabetes insipidus. *J Clin Endocrinol Metab,* 1990, vol. 70 (3), 752-7
- **Rodgers, J. T. ; C. Lerin et al.** Nutrient control of glucose homeostasis through a complex of PGC-1alpha and SIRT1. *Nature,* 2005, vol. 434 (7029), 113-8
- **Rose, C. S. ; J. Ek et al.** A -30G>A polymorphism of the beta-cell-specific glucokinase promoter associates with hyperglycemia in the general population of whites. *Diabetes,* 2005, vol. 54 (10), 3026-31
- **Rosmond, R. ; M. Chagnon et al.** Increased abdominal obesity, insulin and glucose levels in nondiabetic subjects with a T29C polymorphism of the transforming growth factor-beta1 gene. *Horm Res,* 2003, vol. 59 (4), 191-4
- **Roth, S. M. ; M. A. Schrager et al.** IGF2 genotype and obesity in men and women across the adult age span. *Int J Obes Relat Metab Disord,* 2002, vol. 26 (4), 585-7
- **Rudofsky, G., Jr. ; P. Reismann et al.** Reduction of postprandial hyperglycemia in patients with type 2 Diabetes reduces NF-kappaB activation in PBMCs. *Horm Metab Res,* 2004, vol. 36 (9), 630-8
- **Salopuro, T. ; J. Lindstrom et al.** Common variants in beta2- and beta3-adrenergic receptor genes and uncoupling protein 1 as predictors of the risk for type 2 Diabetes and body weight changes. The Finnish Diabetes Prevention Study. *Clin Genet,* 2004, vol. 66 (4), 365-7
- **Sano, H. ; S. C. Liu et al.** Insulin receptor substrate 4 associates with the protein IRAS. *J Biol Chem,* 2002, vol. 277 (22), 19439-47
- **Sauvaget, D. ; V. Chauffeton et al.** In vitro transcriptional induction of the human apolipoprotein A-II gene by glucose. *Diabetes,* 2004, vol. 53 (3), 672-8
- **Scassa, M. E. ; A. S. Guberman et al.** Hepatic nuclear factor 3 and nuclear factor 1 regulate 5-aminolevulinate synthase gene expression and are involved in insulin repression. *J Biol Chem,* 2004, vol. 279 (27), 28082-92
- **Schlosser, M. ; J. P. Banga et al.** Dynamic changes of GAD65 autoantibody epitope specificities in individuals at risk of developing type 1 Diabetes. *Diabetologia,* 2005, vol. 48 (5), 922-30
- **Several.** Nature Medicine Special Issue on Metabolic Syndrome. *Nature Medicine,* 2006, vol. 12 (1), 26-80
- **Several.** Third Annual World Congress on the Insulin Resistance Syndrome. *Diabetes Care,* 2006, vol. 29, 2165-2174
- **Shaat, N. ; M. Ekelund et al.** Association of the E23K polymorphism in the KCNJ11 gene with gestational Diabetes Mellitus. *Diabetologia,* 2005, vol. 48 (12), 2544-51
- **Shimomura, I. ; R. E. Hammer et al.** Insulin resistance and Diabetes Mellitus in transgenic mice expressing nuclear SREBP-1c in adipose tissue: model for congenital generalized lipodystrophy. *Genes Dev,* 1998, vol. 12 (20), 3182-94
- **Shumay, E. ; X. Song et al.** pp60Src mediates insulin-stimulated sequestration of the beta(2)-adrenergic receptor: insulin stimulates pp60Src phosphorylation and activation. *Mol Biol Cell,* 2002, vol. 13 (11), 3943-54
- **Siddiq, A. ; F. Lepretre et al.** A synonymous coding polymorphism in the alpha2-Heremans-schmid glycoprotein gene is associated with type 2 Diabetes in French Caucasians. *Diabetes,* 2005, vol. 54 (8), 2477-81
- **Sivitz, W. I. ; S. M. Wayson et al.** Leptin and body fat in type 2 Diabetes and monodrug therapy. *J Clin Endocrinol Metab,* 2003, vol. 88 (4), 1543-53
- **Song, K. H. ; S. H. Ko et al.** Prospective study of lipoprotein(a) as a risk factor for deteriorating renal function in type 2 diabetic patients with overt proteinuria. *Diabetes Care,* 2005, vol. 28 (7), 1718-23

- **Sosa-Pineda, B.** The gene Pax4 is an essential regulator of pancreatic beta-cell development. *Mol Cells,* 2004, vol. 18 (3), 289-94
- **Southgate, R. J. ; C. R. Bruce et al.** PGC-1 alpha gene expression is down-regulated by Akt- mediated phosphorylation and nuclear exclusion of FoxO1 in insulin-stimulated skeletal muscle. *Faseb J,* 2005, vol. 19 (14), 2072-4
- **Spranger, J. ; A. Kroke et al.** Inflammatory cytokines and the risk to develop type 2 Diabetes: results of the prospective population-based European Prospective Investigation into Cancer and Nutrition (EPIC)-Potsdam Study. *Diabetes,* 2003, vol. 52 (3), 812-7
- **Taniguchi, A. ; M. Fukushima et al.** Soluble E-selectin, leptin, triglycerides, and insulin resistance in nonobese Japanese type 2 diabetic patients. *Metabolism,* 2005, vol. 54 (3), 376-80
- **Tayebjee, M. H. ; H. S. Lim et al.** Matrix metalloproteinase-9 and tissue inhibitor of metalloproteinase-1 and -2 in type 2 Diabetes: effect of 1 year's cardiovascular risk reduction therapy. *Diabetes Care,* 2004, vol. 27 (8), 2049-51
- **Terazono, K. ; H. Yamamoto et al.** A novel gene activated in regenerating islets. *J Biol Chem,* 1988, vol. 263 (5), 2111-4
- **Theodorakis, M. J. ; O. Carlson et al.** Elevated plasma glucose-dependent insulinotropic polypeptide associates with hyperinsulinemia in impaired glucose tolerance. *Diabetes Care,* 2004, vol. 27 (7), 1692-8
- **Thomas, G. N. ; J. A. Critchley et al.** Renin-angiotensin system gene polymorphisms and retinopathy in chinese patients with type 2 Diabetes. *Diabetes Care,* 2003, vol. 26 (5), 1643-4
- **Thomas, M. K. ; K. M. Yao et al.** Bridge-1, a novel PDZ-domain coactivator of E2A-mediated regulation of insulin gene transcription. *Mol Cell Biol,* 1999, vol. 19 (12), 8492-504
- **Tohjima, T. ; N. Honda et al.** Decreased activity of arachidonate 12-lipoxygenase in platelets of Japanese patients with non-insulin-dependent Diabetes Mellitus. *Metabolism,* 1998, vol. 47 (3), 257-63
- **Tokuyama, Y. ; K. Matsui et al.** Five missense mutations in glucagon-like peptide 1 receptor gene in Japanese population. *Diabetes Res Clin Pract,* 2004, vol. 66 (1), 63-9
- **Torres, S. H. ; J. B. De Sanctis et al.** Inflammation and nitric oxide production in skeletal muscle of type 2 diabetic patients. *J Endocrinol,* 2004, vol. 181 (3), 419-27
- **Tsiavou, A. ; E. Hatziagelaki et al.** Correlation between intracellular interferon-gamma (IFN-gamma) production by CD4+ and CD8+ lymphocytes and IFN-gamma gene polymorphism in patients with type 2 Diabetes Mellitus and latent autoimmune Diabetes of adults (LADA). *Cytokine,* 2005, vol. 31 (2), 135-41
- **Tsuchida, H. ; M. Bjornholm et al.** Gene expression of the p85alpha regulatory subunit of phosphatidylinositol 3-kinase in skeletal muscle from type 2 diabetic subjects. *Pflugers Arch,* 2002, vol. 445 (1), 25-31
- **Ukkola, O. ; M. Chagnon et al.** Genetic variation at the adipsin locus and response to long-term overfeeding. *Eur J Clin Nutr,* 2003, vol. 57 (9), 1073-8
- **Ukkola, O. ; J. Salonen et al.** Role of candidate genes in the lipid responses to intensified treatment in Type 2 Diabetes. *J Endocrinol Invest,* 2005, vol. 28 (10), 871-5
- **Um, S. H. ; F. Frigerio et al.** Absence of S6KI protects against age-and diet-induced obesity while enhancing insulin sensitivity. *Nature,* 2004, vol. 431 (7005), 200-5
- **Vanttinen, M. ; P. Nuutila et al.** Single nucleotide polymorphisms in the peroxisome proliferator-activated receptor delta gene are associated with skeletal muscle glucose uptake. *Diabetes,* 2005, vol. 54 (12), 3587-91
- **Vettor, R. ; G. Milan et al.** Review article: adipocytokines and insulin resistance. *Aliment Pharmacol Ther,* 2005, vol. 22 (2), 3-10
- **Wahab, N. A. ; L. Schaefer et al.** Glomerular expression of thrombospondin-1, transforming growth factor beta and connective tissue growth factor at different stages of diabetic nephropathy and their interdependent roles in mesangial response to diabetic stimuli. *Diabetologia,* 2005, vol. 48 (12), 2650-60
- **Wakabayashi, M. ; H. Ohi et al.** Acquired Loss of Erythrocyte Complement Receptor Type 1 in Patients with Diabetic Nephropathy Undergoing Hemodialysis. *Nephron Exp Nephrol,* 2006, vol. 104 (3), e89-e95
- **Wautier, M. P. ; E. Boulanger et al.** AGEs, macrophage colony stimulating factor and vascular adhesion molecule blood levels are increased in patients with diabetic microangiopathy. *Thromb Haemost,* 2004, vol. 91 (5), 879-85
- **Wei, F. Y. ; K. Nagashima et al.** Cdk5-dependent regulation of glucose-stimulated insulin secretion. *Nat Med,* 2005, vol. 11 (10), 1104-8
- **Wolford, J. K. ; K. A. Yeatts et al.** Sequence variation in PPARG may underlie differential response to troglitazone. *Diabetes,* 2005, vol. 54 (11), 3319-25
- **Wu, B. ; J. Takahashi et al.** Variants of calpain-10 gene and its association with type 2 Diabetes Mellitus in a Chinese population. *Diabetes Res Clin Pract,* 2005, vol. 68 (2), 155-61
- **Xue, Y. M. ; L. Zhou et al.** Correlation between angiotensin II type 1 receptor gene polymorphism and type 2 Diabetes Mellitus complicated by hypertension. *Di Yi Jun Yi Da Xue Xue Bao,* 2002, vol. 22 (5), 444-6

- **Yagui, K. ; F. Shimada et al.** A missense mutation in the CD38 gene, a novel factor for insulin secretion: association with Type II Diabetes Mellitus in Japanese subjects and evidence of abnormal function when expressed in vitro. *Diabetologia,* 1998, vol. 41 (9), 1024-8
- **Yang, Q. ; T. E. Graham et al.** Serum retinol binding protein 4 contributes to insulin resistance in obesity and type 2 Diabetes. *Nature,* 2005, vol. 436 (7049), 356-62
- **Yang, Y. ; E. K. Chung et al.** Diversity in intrinsic strengths of the human complement system: serum C4 protein concentrations correlate with C4 gene size and polygenic variations, hemolytic activities, and body mass index. *J Immunol,* 2003, vol. 171 (5), 2734-45
- **Ye, L. ; Y. Xu et al.** Association of polymorphism in neurogenic differentiation factor 1 gene with type 2 Diabetes. *Zhonghua Yi Xue Yi Chuan Xue Za Zhi,* 2002, vol. 19 (6), 484-7
- **Zacharova, J. ; J. L. Chiasson et al.** Leptin receptor gene variation predicts weight change in subjects with impaired glucose tolerance. *Obes Res,* 2005, vol. 13 (3), 501-6
- **Zeggini, E. ; J. Parkinson et al.** Association studies of insulin receptor substrate 1 gene (IRS1) variants in type 2 Diabetes samples enriched for family history and early age of onset. *Diabetes,* 2004, vol. 53 (12), 3319-22
- **Zhang, J. ; R. I. Holt et al.** Plasma adiponectin concentrations are independently predicted by fat insulin sensitivity in women and by muscle insulin sensitivity in men. *Diabetes Care,* 2005, vol. 28 (3), 755-6
- **Zhu, F. ; L. Ji et al.** The role of urotensin II gene in the genetic susceptibility to type 2 Diabetes in Chinese population. *Zhonghua Yi Xue Za Zhi,* 2002, vol. 82 (21), 1473-5
- **Zimmermann, R. ; J. G. Strauss et al.** Fat mobilization in adipose tissue is promoted by adipose triglyceride lipase. *Science,* 2004, vol. 306 (5700), 1383-6